# EUROPEAN PATENT SPECIFICATION

(11) **EP 4 171 549 B1**
(45) Date of publication and mention of the grant of the patent: **25.03.2026**
(21) Application number: 21762534.2
(22) Date of filing: 25.06.2021
(51) Int. Cl.: A61K 31/4166, A61K 31/513, A61K 39/395, A61K 31/4164, A61K 31/4168, A61K 31/506, A61K 31/4178, A61K 31/497, A61K 31/4439, A61K 31/501, A61K 31/4245, A61K 31/427, A61K 31/4196, A61K 31/454, A61K 31/496, A61P 35/00, A61P 35/02, A61P 35/04, A61K 31/7072, A61K 31/541

(54) **COMBINATION THERAPY WITH DEOXYURIDINE TRIPHOSPHATASE INHIBITORS**
KOMBINATIONSTHERAPIE MIT DEOXYURIDIN-TRIPHOSPHATASE-HEMMERN
POLYTHÉRAPIE AVEC DES INHIBITEURS DE DÉSOXYURIDINE TRIPHOSPHATASE

(30) Priority: 26.06.2020 US 202063044926 P
(43) Date of publication of application: 03.05.2023
(73) Proprietor: CV6 Therapeutics (NI) Limited, Belfast BT1 6DN (GB)
(72) Inventor: MULLIGAN, Karl Andrew, Belfast BT4 2WG (GB); WILSON, Peter Michael, Antrim BT39 0DL (GB); WILSON, Melissa J LaBonte, Antrim BT39 0DL (GB); LADNER, Robert D., Holywood Down BT18 9BX (GB)
(74) Representative: Carpmaels & Ransford LLP
(86) International application number: PCT/US2021/039248
(87) International publication number: WO 2021/263205

(56) References cited:
- EP-A1- 3 400 963
- WO-A1-2017/006271
- WO-A1-2017/006282
- US-A1- 2011 212 467
- WILSON PETER M ET AL: "Standing the test of time : targeting thymidylate biosynthesis in cancer therapy", NATURE REVIEWS CLINICAL ONCOLOGY, NATURE, NY, US, vol. 11, no. 5, 1 January 2014 (2014-01-01), pages 282 - 298, XP009509081, ISSN: 1759-4774, DOI: 10.1038/NRCLINONC.2014.51
- DOI TOSHIHIKO ET AL: "First-in-human phase 1 study of novel dUTPase inhibitor TAS-114 in combination with S-1 in Japanese patients with advanced solid tumors", INVESTIGATIONAL NEW DRUGS, SPRINGER US, NEW YORK, vol. 37, no. 3, 4 December 2018 (2018-12-04), pages 507 - 518, XP036810294, ISSN: 0167-6997, [retrieved on 20181204], DOI: 10.1007/S10637-018-0697-3

## Description

### CROSS-REFERENCE TO RELATED APPLICATIONS

The present application claims priority to U.S. Provisional Patent Application No. 63/044,926 under 35 U.S.C. 119(e), filed on June 26, 2020.

### FIELD OF DISCLOSURE

The present disclosure is, in some aspects, in the field of combination therapy of deoxyuridine triphosphatase inhibitors with other active agents for the treatment of cancer.

### SUMMARY

In one aspect, presented herein are a deoxyuridine triphosphatase (dUTPase) inhibitor, an inhibitor of thymidylate biosynthesis and an immunotherapy agent, for use in a method of enhancing a therapeutic efficacy of an immunotherapy agent in a subject in need thereof; wherein the method comprises administering to the subject an effective amount of the deoxyuridine triphosphatase (dUTPase) inhibitor, an effective amount of the inhibitor of thymidylate biosynthesis, and the immunotherapy agent, wherein the dUTPase inhibitor is 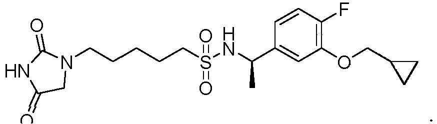 the inhibitor of thymidylate biosynthesis is 5-fluorouracil (5-FU);
and the immunotherapy agent is an anti-PD-1 (CD279) antibody.

Also presented herein is a deoxyuridine triphosphatase (dUTPase) inhibitor for use in a method of enhancing a therapeutic efficacy of an immunotherapy agent in a subject in need thereof; wherein the method comprises administering to the subject an effective amount of the deoxyuridine triphosphatase (dUTPase) inhibitor, an effective amount of an inhibitor of thymidylate biosynthesis, and an immunotherapy agent, wherein the dUTPase inhibitor is 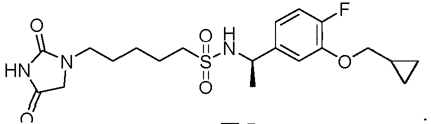 the inhibitor of thymidylate biosynthesis is 5-fluorouracil (5-FU);
and the immunotherapy agent is an anti-PD-1 (CD279) antibody.
Also presented herein is an inhibitor of thymidylate biosynthesis for use in a method of enhancing a therapeutic efficacy of an immunotherapy agent in a subject in need thereof; wherein the method comprises administering to the subject an effective amount of a deoxyuridine triphosphatase (dUTPase) inhibitor, an effective amount of the inhibitor of thymidylate biosynthesis, and an immunotherapy agent, wherein the dUTPase inhibitor is 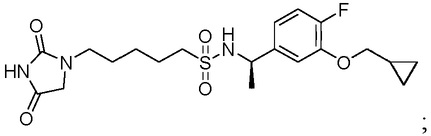 the inhibitor of thymidylate biosynthesis is 5-fluorouracil (5-FU);
and the immunotherapy agent is an anti-PD-1 (CD279) antibody.
Also presented herein is an immunotherapy agent for use in a method of enhancing a therapeutic efficacy of the immunotherapy agent in a subject in need thereof; wherein the method comprises administering to the subject an effective amount of a deoxyuridine triphosphatase (dUTPase) inhibitor, an effective amount of an inhibitor of thymidylate biosynthesis, and the immunotherapy agent, wherein the dUTPase inhibitor is 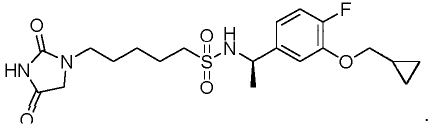 the inhibitor of thymidylate biosynthesis is 5-fluorouracil (5-FU);
and the immunotherapy agent is an anti-PD-1 (CD279) antibody.
In one aspect, the amount is an effective amount, e.g., a therapeutically effective amount.

In another aspect, presented herein are a deoxyuridine triphosphatase (dUTPase) inhibitor, an inhibitor of thymidylate biosynthesis and an immunotherapy agent, for use in a method of treating cancer in a subject in need thereof; wherein the method comprises administering to the subject an effective amount of the deoxyuridine triphosphatase (dUTPase) inhibitor, an effective amount of the inhibitor of thymidylate biosynthesis, and the immunotherapy agent, wherein the dUTPase inhibitor is 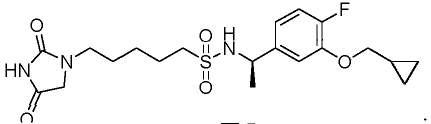 the inhibitor of thymidylate biosynthesis is 5-fluorouracil (5-FU);
and the immunotherapy agent is an anti-PD-1 (CD279) antibody.
Also presented is a deoxyuridine triphosphatase (dUTPase) inhibitor for use in a method of treating cancer in a subject in need thereof; wherein the method comprises administering to the subject an effective amount of the deoxyuridine triphosphatase (dUTPase) inhibitor, an effective amount of an inhibitor of thymidylate biosynthesis, and an immunotherapy agent, wherein the dUTPase inhibitor is 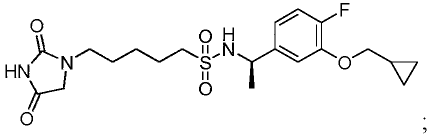 the inhibitor of thymidylate biosynthesis is 5-fluorouracil (5-FU);
and the immunotherapy agent is an anti-PD-1 (CD279) antibody.
Also presented is an inhibitor of thymidylate biosynthesis for use in a method of treating cancer in a subject in need thereof; wherein the method comprises administering to the subject an effective amount of a deoxyuridine triphosphatase (dUTPase) inhibitor, an effective amount of the inhibitor of thymidylate biosynthesis, and an immunotherapy agent, wherein the dUTPase inhibitor is 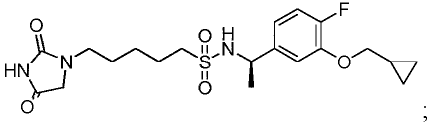 the inhibitor of thymidylate biosynthesis is 5-fluorouracil (5-FU);
and the immunotherapy agent is an anti-PD-1 (CD279) antibody.
Also presented is an immunotherapy agent for use in a method of treating cancer in a subject in need thereof; wherein the method comprises administering to the subject an effective amount of a deoxyuridine triphosphatase (dUTPase) inhibitor, an effective amount of an inhibitor of thymidylate biosynthesis, and the immunotherapy agent, wherein the dUTPase inhibitor is 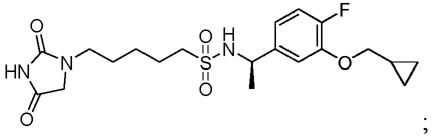 the inhibitor of thymidylate
biosynthesis is 5-fluorouracil (5-FU);
and the immunotherapy agent is an anti-PD-1 (CD279) antibody.
In one aspect, the amount is an effective amount, e.g., a therapeutically effective amount.

### BRIEF DESCRIPTION OF DRAWINGS

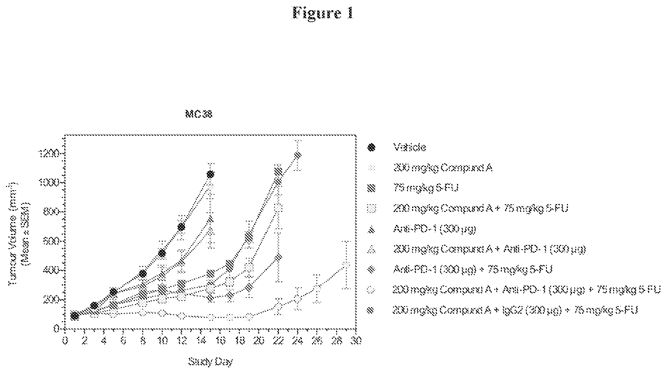

**Figure 1** depicts antitumor efficacy of Compound A in combination with 5-FU and an anti-PD-1 antibody in a murine MC38 syngeneic colon cancer model over the course of a 29 day study. Data presented is the mean tumor volume ± SEM. The data points for each group stop on the day the first animal was removed from study for that treatment when max permissible tumor volume of 1000 mm³ ± 50 mm³ was reached. One-way ANOVA at day 19 and day 22 = *p*<0.0001 with Tukey's Multiple Comparisons test: *p*<0.05 and 0.001 when Compound A + 5-FU + Anti-PD-1 is compared to 5-FU + anti-PD-1 on day 19.

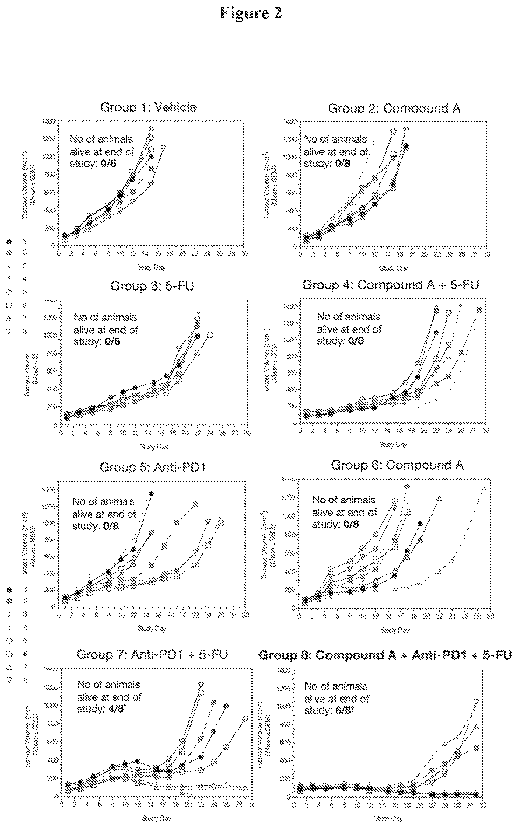

**Figure 2** depicts individual tumor volume for each animal (n=8) within each treatment group for a MC38 syngeneic colon cancer model over the course of a 29 day study. The data points for each animal stop on the day the animal was removed from study when max permissible tumor volume of 1000 mm³ ± 50 mm³ was reached. *Includes one complete regression; †Includes four histopathologically confirmed complete responses

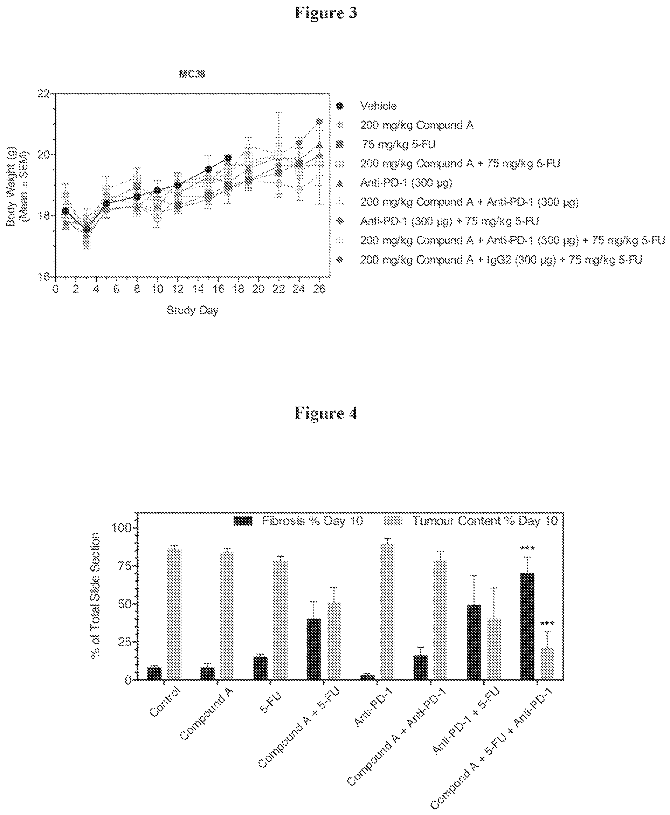

**Figure 3** depicts mouse body weight in grams (mean ± SEM) across all treatment groups in a murine MC38 syngeneic colon cancer model over the study. Days 16 to 26 have varying numbers of animals per group due to animals reaching maximum permissible tumor volume and being removed from study. One-way ANOVA *p* = ns. No statistically significant difference between means of each treatment group up to day 16 when all animals were on study.
**Figure 4** depicts histopathological analysis of tumor specimens for fibrosis vs tumor content following treatment in a murine MC38 syngeneic colon cancer model. Graph shows the mean ± SEM percentage of tumor content and fibrosis for the MC38 syngeneic colon tumors removed on day 10. One-way ANOVA at day 10 = *p*<0.0001; multiple comparisons test for fibrosis: vehicle vs Compound A + 5-FU + Anti-PD-1 ****p*=<0.001; multiple comparisons test for tumor content: vehicle vs Compound A + 5-FU + Anti-PD-1 ****p*=<0.001.

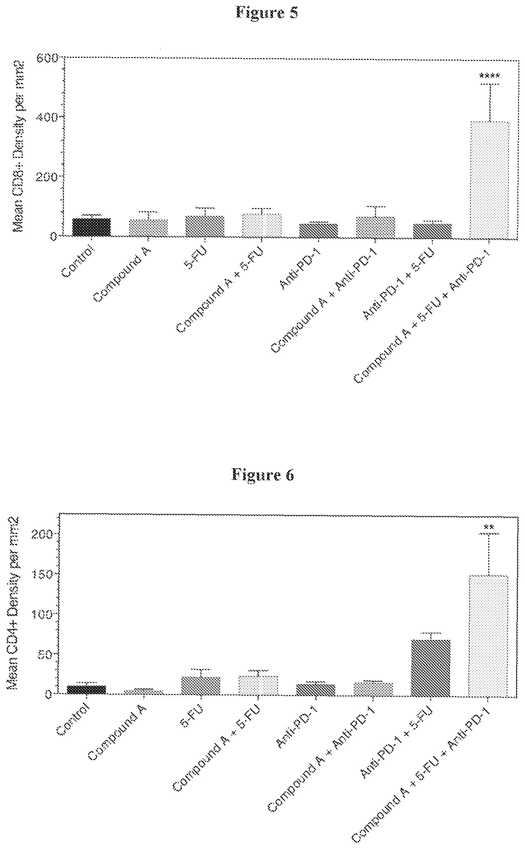

**Figure 5** depicts immunohistochemistry analysis of tumor specimens showing enhanced CD8+ T-cell infiltration following treatment with Compound A + 5-FU and an Anti-PD-1 antibody at Day 10 in a murine MC38 syngeneic colon cancer model. Graph shows the mean ± SEM intratumoral density for CD8+ positive T-cells per mm² for the MC38 syngeneic colon tumors removed on day 10 from each treatment group. One-way ANOVA at day 10 = ****p*<0.001; multiple comparisons test for CD8⁺: 5-FU + Anti-PD-1 vs Compound A + 5-FU + Anti-PD-1 *****p*=<0.0001.
**Figure 6** depicts immunohistochemistry analysis of tumor specimens showing enhanced CD4+ T-cell infiltration following treatment with Compound A + 5-FU and an Anti-PD-1 antibody at Day 10 in a murine MC38 syngeneic colon cancer model. Graph shows the mean ± SEM intratumoral density for CD4+ positive T-cells per mm² for the MC38 syngeneic colon tumors removed on day 10 from each treatment group. One-way ANOVA at day 10 = *****p*<0.0001; multiple comparisons test for CD4⁺: 5-FU + Anti-PD-1 vs Compound A + 5-FU + Anti-PD-1 ***p*=<0.01.

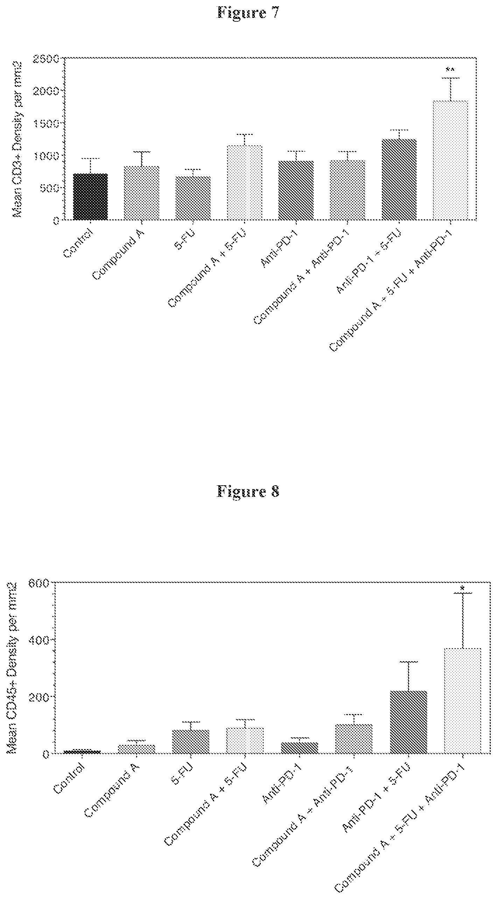

**Figure 7** depicts immunohistochemistry analysis of tumor specimens showing enhanced CD3+ immune cell infiltration following treatment with Compound A + 5-FU and an Anti-PD-1 antibody at Day 10 in a murine MC38 syngeneic colon cancer model. Graph shows the mean ± SEM intratumoral density for CD3+ positive immune cells per mm² for the MC38 syngeneic colon tumors removed on day 10 from each treatment group. One-way ANOVA at day 10 = *p*<0.05; multiple comparisons test for CD3+: vehicle vs Compound A + 5-FU + Anti-PD-1 ***p*=<0.01.
**Figure 8** depicts immunohistochemistry analysis of tumor specimens showing enhanced CD45+ immune cell infiltration following treatment with Compound A + 5-FU and an Anti-PD-1 antibody at Day 10 in a murine MC38 syngeneic colon cancer model. Graph shows the mean ± SEM intratumoral density for CD45+ positive immune cells per mm² for the MC38 syngeneic colon tumors (n = 4) removed on day 10 from each treatment group. One-way ANOVA at day 10: *p*=0.06; multiple comparisons test for CD45+ on day 10: vehicle vs Compound A + 5-FU + Anti-PD-1 *p=<0.05.

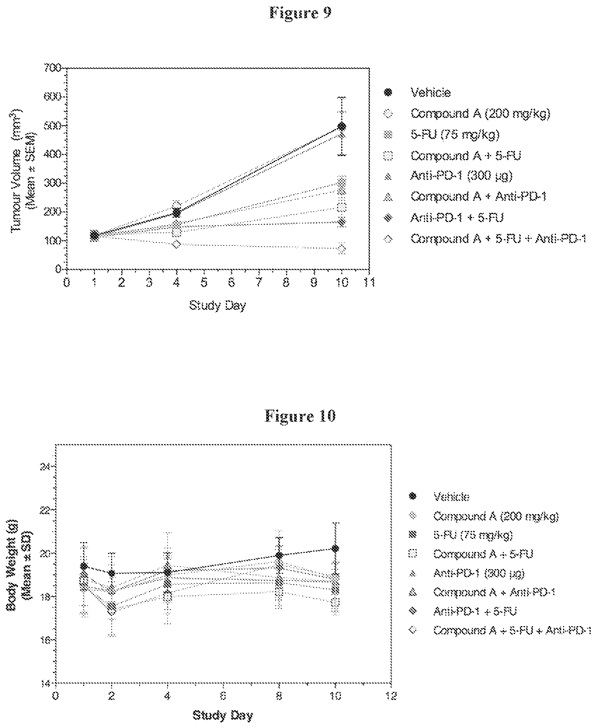

**Figure 9** depicts tumor volume for the duration of a 10-day study in a murine MC38 syngeneic colon cancer model. Data presented is the mean ± SEM with n = 8 animals on day 4 and n = 4 animals on day 10 (the first cohort for biomarker analysis were euthanized on day 4). One-way ANOVA at day 4: *p*<0.001 and day 10: *p*<0.0001. Tukey's Multiple Comparisons test for vehicle vs Compound A + 5-FU + Anti-PD-1 at day 4: *p*<0.001 and day 10: *p*<0.001.
**Figure 10** depicts mouse body weight across all treatment groups in a murine MC38 syngeneic colon cancer model. Graph shows the mean ± SEM bodyweight in grams for each treatment group for the MC38 syngeneic colon cancer model over the duration of the study. Data presented is the mean ± SEM with n = 8 animals on day 4 and n = 4 animals on day 10 (the first cohort for biomarker analysis were euthanized on day 4). One-way ANOVA *p*=ns on days 4 and days 10.

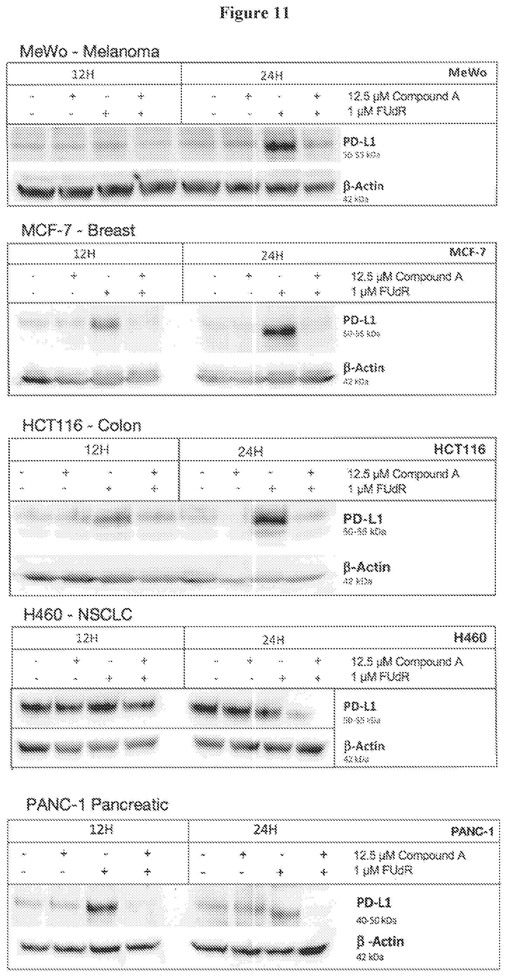

**Figure 11** depicts Western blotting measuring total PD-L1 expression, demonstrating that Compound A blocks the FUdR-mediated induction of PD-L1 and leads to a decrease in expression of PD-L1 in melanoma, breast, colon, non-small cell lung (NSCLC) and pancreatic cancer cell lines. Cancer cell lines were treated with vehicle control, 12.5 µM Compound A, 1 µM FUdR and a combination of 12.5 µM Compound A and 1 µM FUdR. The expression of PD-L1 was measured at both 12 and 24 hours post-treatment. Melanoma, MeWo; breast, MCF-7; colon, HCT116; NSCLC, H460 and pancreatic, PANC-1. Beta-actin is an additional protein used to control for total protein loading

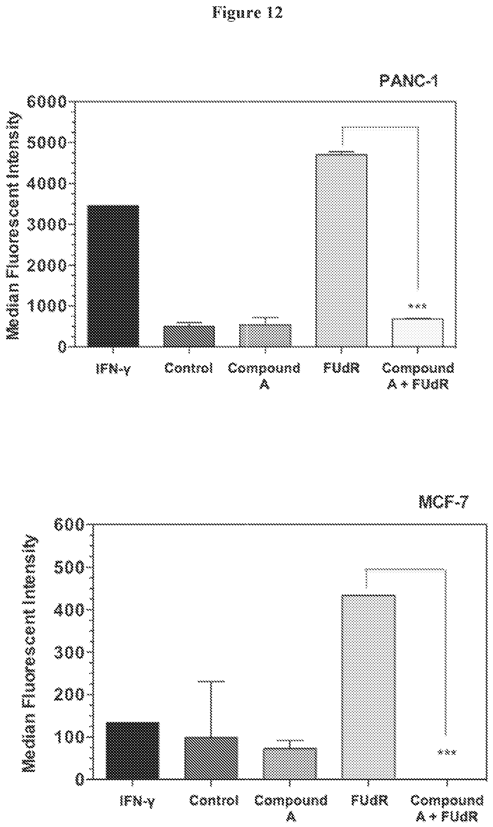

**Figure 12** depicts cell-surface PD-L1 expression detected by flow cytometry in PANC-1 pancreatic cancer and MCF-7 breast cancer cells treated with Compound A, FUdR and a combination of Compound A and FUdR. Interferon gamma (IFN-γ) was used as a positive control known to stimulate cell-surface PD-L1 expression in PANC-1 cells. Percentage positive population and median fluorescent intensity values were measured and analyzed in Microsoft Excel and GraphPad Prism 6. Statistical analysis consisted of one-way ANOVA with Tukey's multiple comparisons testing.

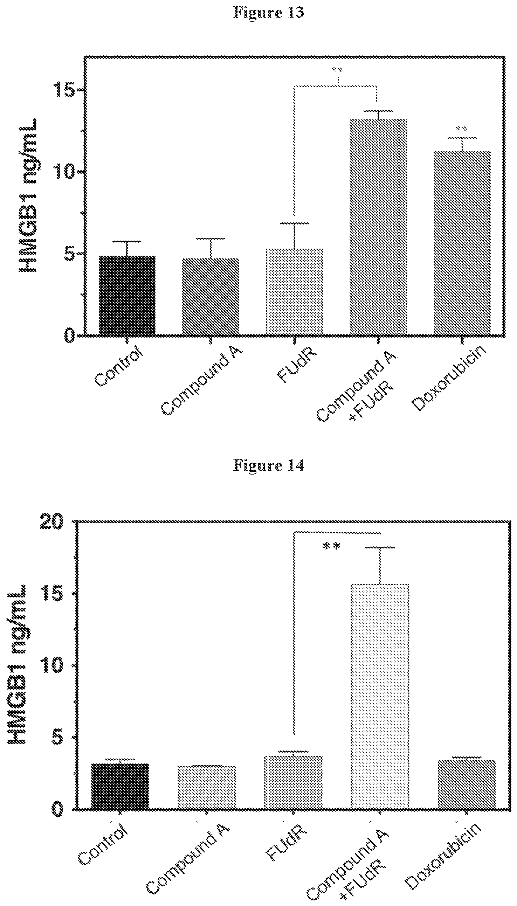

**Figure 13** depicts extracellular release of HMGB1 into cell culture media detected by ELISA in HCT116 colon cancer cells treated with vehicle control, 12.5 µM Compound A, 1 µM FUdR and a combination of 12.5 µM Compound A and 1 µM FUdR. Doxorubicin was used as a positive control for HMGB 1 and known stimulator of immunogenic cell death in some cell lines. Statistical analysis consisted of one-way ANOVA with Tukey's multiple comparisons test between vehicle control and doxorubicin (p<0.01) and between FUdR and Compound A + FUdR (p<0.01).
**Figure 14** depicts extracellular release of HMGB1 into cell culture media detected by ELISA in JU77 mesothelioma cells treated with vehicle control, 12.5 µM Compound A, 1 µM FUdR and a combination of 12.5 µM Compound A and 1 µM FUdR. Doxorubicin was used as a positive control for HMGB 1 and known stimulator of immunogenic cell death in some cell lines. Statistical analysis consisted of one-way ANOVA with Tukey's multiple comparisons test between FUdR and the combination of Compound A + FUdR (p<0.01).

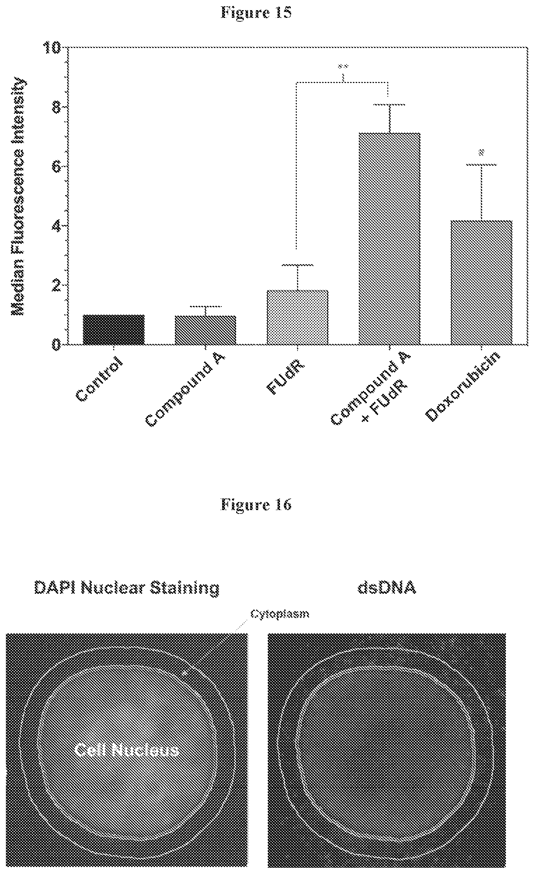

**Figure 15** depicts cell-surface calreticulin expression detected by flow cytometry in PANC-1 pancreatic cancer cells treated with 6.25 µM Compound A, 1 µM FUdR and a combination of 6.25 µM Compound A and 1 µM FUdR. Doxorubicin was used as a positive control known to stimulate cell-surface calreticulin expression in some cell lines. Median fluorescent intensity values of cells that stained positive for calreticulin was measured and analyzed in Microsoft Excel and GraphPad Prism 6. Statistical analysis consisted of one-way ANOVA with Tukey's multiple comparisons testing between FUdR and the combination of Compound A + FUdR (p<0.01).
**Figure 16** depicts non-limiting example showing software settings for the quantification of cytoplasmic dsDNA. Cancer cell nucleus stained with DAPI to identify the cell nucleus (left) and an anti-dsDNA antibody (right) with a detection ring beyond the nuclear envelope to detect nuclear dsDNA in the cytoplasm. The nucleus was encircled and detection parameters are: Gaps to ring = 3 and Ring size = 25. The outer ring indicated the region for detection of dsDNA.

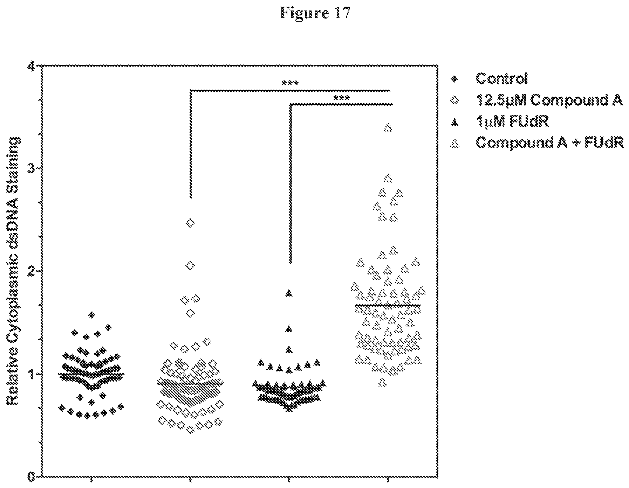

**Figure 17** depicts relative cytoplasmic dsDNA density as detected by fluorescence microscopy and quantified by ImageJ in HCT116 colon cancer cells treated with vehicle control (DMSO), 12.5 µM Compound A, 1 µM FUdR and a combination of 12.5 µM Compound A and 1 µM FUdR for 24 hours. Data presented is from at least 69 individual cells each quantified for cytoplasmic dsDNA, the horizontal line indicating the mean relative cytoplasmic dsDNA density. Statistical analysis consisted of One-way ANOVA (p<0.0001) with Tukey's Multiple Comparisons Test: Compound A treatment and FUdR treatment were not significantly different (ns) when compared to control. Compound A vs Compound A + FUdR = p<0.001 and the comparison of FUdR vs Compound A + FUdR = p<0.001.

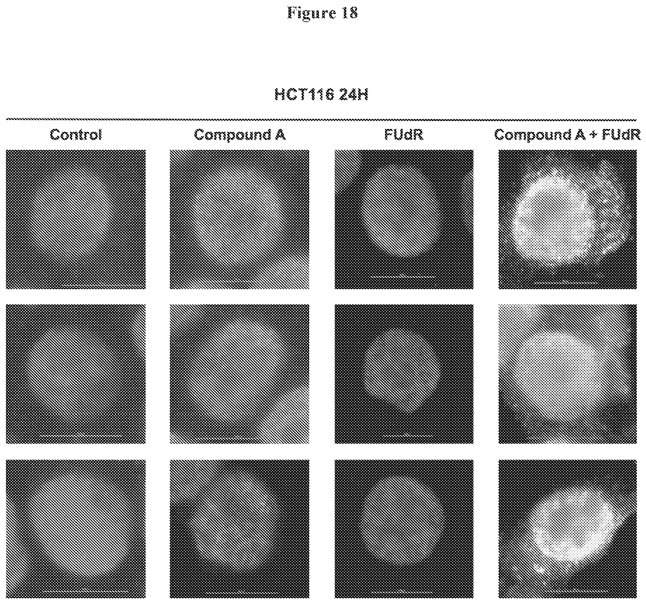

**Figure 18** depicts representative images showing cytoplasmic dsDNA in HCT116 colon cancer cells treated with vehicle control (DMSO), 12.5 µM Compound A, 1 µM FUdR and a combination of 12.5 µM Compound A and 1 µM FUdR for 24 hours. Treatment with the combination of 12.5 µM Compound A and 1 µM FUdR clearly demonstrated a marked increase in fluorescent signal outside the nucleus indicative of the release of nuclear DNA into the cytoplasm with clear evidence of distinct micronuclei. All images were captured under identical experimental conditions.

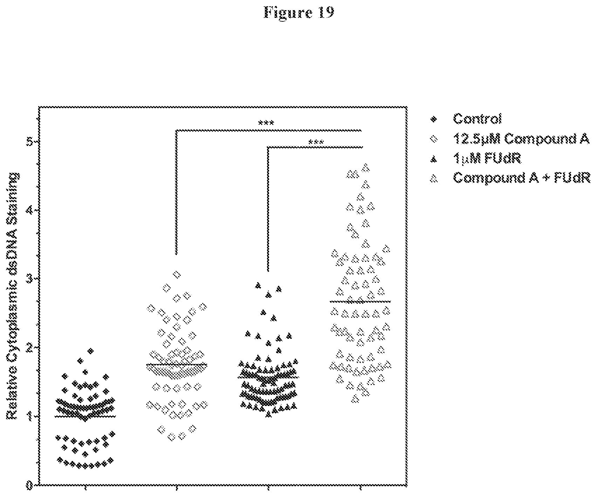

**Figure 19** depicts relative cytoplasmic dsDNA density as detected by fluorescence microscopy and quantified by ImageJ in PANC-1 pancreatic cancer cells treated with vehicle control (DMSO), 12.5 µM Compound A, 1 µM FUdR and a combination of 12.5 µM Compound A and 1 µM FUdR for 24 hours. Data presented is at least 64 individual cells each quantified for cytoplasmic dsDNA, the horizontal line indicates the mean relative cytoplasmic dsDNA density. Statistical analysis consisted of One-way ANOVA (p<0.0001) with Tukey's Multiple Comparisons Test: The comparison of FUdR vs Compound A + FUdR = p<0.001 and the comparison of Compound A vs Compound A + FUdR = p<0.001.

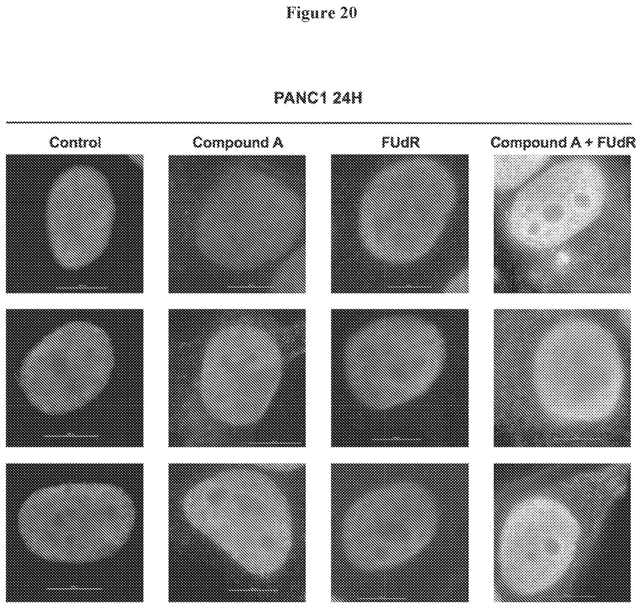

**Figure 20** depicts representative images showing cytoplasmic dsDNA in PANC-1 pancreatic cancer cells treated with vehicle control (DMSO), 12.5 µM Compound A, 1 µM FUdR and a combination of 12.5 µM Compound A and 1 µM FUdR for 24 hours. Treatment with the combination of 12.5 µM Compound A and 1 µM FUdR clearly demonstrated a marked increase in fluorescent signal outside the nucleus indicative of the release of nuclear DNA into the cytoplasm with clear evidence of distinct micronuclei. All images were captured under identical experimental conditions.

### DETAILED DESCRIPTION

### Definitions

Throughout this disclosure, various publications, patents and published patent specifications are referenced by an identifying citation.

The practice of the present technology will employ, unless otherwise indicated, conventional techniques of organic chemistry, pharmacology, immunology, molecular biology, microbiology, cell biology and recombinant DNA, which are within the skill of the art. See, *e.g*., Sambrook, Fritsch and Maniatis, Molecular Cloning: A Laboratory Manual, 2nd edition (1989); Current Protocols In Molecular Biology (F. M. Ausubel, et al. eds., (1987)); the series Methods in Enzymology (Academic Press); PCR 2: A Practical Approach (M.J. MacPherson, B.D. Hames and G.R. Taylor eds., (1995)); Antibodies, a Laboratory Manual, and Animal Cell Culture (R.I. Freshney, ed. (1987)).

As used in the specification and claims, the singular form "a," "an" and "the" include plural references unless the context clearly dictates otherwise. For example, the term "a cell" includes a plurality of cells, including mixtures thereof.

As used herein, the term "comprising" is intended to mean that the compounds, compositions and methods include the recited elements, but not exclude others. "Consisting essentially of" when used to define compounds, compositions and methods, shall mean excluding other elements of any essential significance to the combination. Thus, a composition consisting essentially of the elements as defined herein would not exclude trace contaminants, e.g., from the isolation and purification method and pharmaceutically acceptable carriers, preservatives, and the like. "Consisting of" shall mean excluding more than trace elements of other ingredients. Embodiments defined by each of these transition terms are within the scope of this technology.

All numerical designations, e.g., pH, temperature, time, concentration, and molecular weight, including ranges, are approximations which are varied (+) or (-) by increments of 1, 5, or 10%. It is to be understood, although not always explicitly stated that all numerical designations are preceded by the term "about." It also is to be understood, although not always explicitly stated, that the reagents described herein are merely exemplary and that equivalents of such are known in the art.

"Alkyl" refers to monovalent saturated aliphatic hydrocarbyl groups having from 1 to 10 carbon atoms and preferably 1 to 6 carbon atoms. This term includes, by way of example, linear and branched hydrocarbyl groups such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃)(CH₃CH₂)CH-), t-butyl ((CH₃)₃C-), n-pentyl (CH₃CH₂CH₂CH₂CH₂-), and neopentyl ((CH₃)₃CCH₂-).

"Alkenyl" refers to monovalent straight or branched hydrocarbyl groups having from 2 to 10 carbon atoms and preferably 2 to 6 carbon atoms or preferably 2 to 4 carbon atoms and having at least 1 and preferably from 1 to 2 sites of vinyl (>C=C<) unsaturation. Such groups are exemplified, for example, by vinyl, allyl, and but-3-en-1-yl. Included within this term are the *cis* and *trans* isomers or mixtures of these isomers.

"Alkynyl" refers to straight or branched monovalent hydrocarbyl groups having from 2 to 10 carbon atoms and preferably 2 to 6 carbon atoms or preferably 2 to 3 carbon atoms and having at least 1 and preferably from 1 to 2 sites of acetylenic (-C≡C-) unsaturation. Examples of such alkynyl groups include acetylenyl (-C≡CH), and propargyl (-CH₂C≡CH).

"Substituted alkyl" refers to an alkyl group having from 1 to 5, preferably 1 to 3, or more preferably 1 to 2 substituents selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein.

"Heteroalkyl" refers to an alkyl group one or more carbons is replaced with -O-, -S-, SO₂, a P containing moiety as provided herein, -NR^{Q}-, 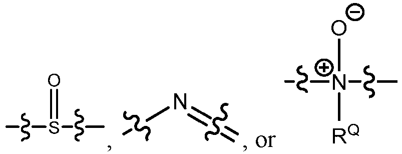 moieties where R^{Q} is H or C₁-C₆ alkyl. Substituted heteroalkyl refers to a heteroalkyl group having from 1 to 5, preferably 1 to 3, or more preferably 1 to 2 substituents selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein.

"Substituted alkenyl" refers to alkenyl groups having from 1 to 3 substituents, and preferably 1 to 2 substituents, selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxyl, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein and with the proviso that any hydroxyl or thiol substitution is not attached to a vinyl (unsaturated) carbon atom.

"Heteroalkenyl" refers to an alkenyl group one or more carbons is replaced with -O-, -S-, SO₂, a P containing moiety as provided herein, -NR^{Q}-, 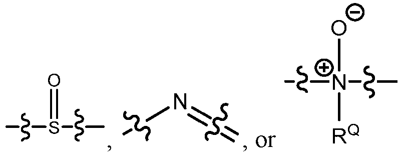 moieties where R^{Q} is H or C₁-C₆ alkyl. Substituted heteroalkenyl refers to a heteroalkenyl group having from 1 to 5, preferably 1 to 3, or more preferably 1 to 2 substituents selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein.

"Substituted alkynyl" refers to alkynyl groups having from 1 to 3 substituents, and preferably 1 to 2 substituents, selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein and with the proviso that any hydroxyl or thiol substitution is not attached to an acetylenic carbon atom.

"Heteroalkynyl" refers to an alkynyl group one or more carbons is replaced with -O-, -S-, SO₂, a P containing moiety as provided herein, -NR^{Q}-, 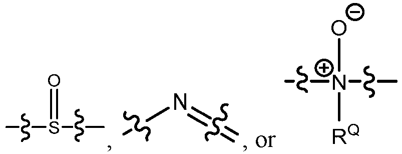 moieties where R^{Q} is H or C₁-C₆ alkyl. Substituted heteroalkynyl refers to a heteroalkynyl group having from 1 to 5, preferably 1 to 3, or more preferably 1 to 2 substituents selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein.

"Alkylene" refers to divalent saturated aliphatic hydrocarbyl groups having from 1 to 10 carbon atoms, preferably having from 1 to 6 and more preferably 1 to 3 carbon atoms that are either straight-chained or branched. This term is exemplified by groups such as methylene (-CH₂-), ethylene (-CH₂CH₂-), n-propylene (-CH₂CH₂CH₂-), iso-propylene (-CH₂CH(CH₃)- or -CH(CH₃)CH₂-), butylene (-CH₂CH₂CH₂CH₂-), isobutylene (-CH₂CH(CH₃)CH₂-), sec-butylene (-CH₂CH₂(CH₃)CH-), and the like. Similarly, "alkenylene" and "alkynylene" refer to an alkylene moiety containing respective 1 or 2 carbon carbon double bonds or a carbon carbon triple bond.

"Substituted alkylene" refers to an alkylene group having from 1 to 3 hydrogens replaced with substituents selected from the group consisting of alkyl, substituted alkyl, alkoxy, substituted alkoxy, acyl, acylamino, acyloxy, amino, substituted amino, aminoacyl, aryl, substituted aryl, aryloxy, substituted aryloxy, cyano, halogen, hydroxyl, nitro, carboxyl, carboxyl ester, cycloalkyl, substituted cycloalkyl, heteroaryl, substituted heteroaryl, heterocyclic, substituted heterocyclic, and oxo wherein said substituents are defined herein. In some embodiments, the alkylene has 1 to 2 of the aforementioned groups, or having from 1-3 carbon atoms replaced with -O-, -S-, or -NR^{Q}- moieties where R^{Q} is H or C₁-C₆ alkyl. It is to be noted that when the alkylene is substituted by an oxo group, 2 hydrogens attached to the same carbon of the alkylene group are replaced by "=O". "Substituted alkenylene" and " substituted alkynylene" refer to alkenylene and substituted alkynylene moieties substituted with substituents as described for substituted alkylene.

"Alkynylene" refers to straight or branched divalent hydrocarbyl groups having from 2 to 10 carbon atoms and preferably 2 to 6 carbon atoms or preferably 2 to 3 carbon atoms and having at least 1 and preferably from 1 to 2 sites of acetylenic (-C≡C-) unsaturation. Examples of such alkynylene groups include -C≡C- and -CH₂C≡C-.

"Substituted alkynylene" refers to alkynylene groups having from 1 to 3 substituents, and preferably 1 to 2 substituents, selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein and with the proviso that any hydroxyl or thiol substitution is not attached to an acetylenic carbon atom.

"Heteroalkylene" refers to an alkylene group wherein one or more carbons is replaced with -O-, -S-, SO₂, a P containing moiety as provided herein, -NR^{Q}-, 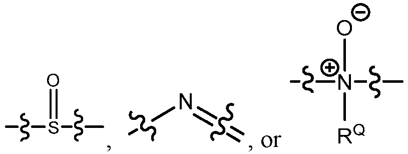 moieties where R^{Q} is H or C₁-C₆ alkyl. "Substituted heteroalkylene" refers to heteroalkynylene groups having from 1 to 3 substituents, and preferably 1 to 2 substituents, selected from the substituents disclosed for substituted alkylene.

"Heteroalkenylene" refers to an alkenylene group wherein one or more carbons is replaced with -O-, -S-, SO₂, a P containing moiety as provided herein, -NR^{Q}-, 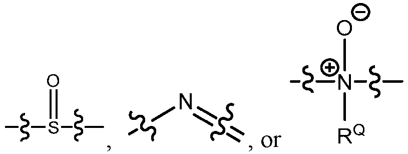 moieties where R^{Q} is H or C₁-C₆ alkyl. "Substituted heteroalkenylene" refers to heteroalkynylene groups having from 1 to 3 substituents, and preferably 1 to 2 substituents, selected from the substituents disclosed for substituted alkenylene.

"Heteroalkynylene" refers to an alkynylene group wherein one or more carbons is replaced with -O-, -S-, SO₂, a P containing moiety as provided herein, -NR^{Q}-, 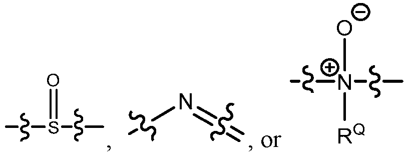 moieties where R^{Q} is H or C₁-C₆ alkyl. "Substituted heteroalkynylene" refers to heteroalkynylene groups having from 1 to 3 substituents, and preferably 1 to 2 substituents, selected from the substituents disclosed for substituted alkynylene.

"Alkoxy" refers to the group -O-alkyl wherein alkyl is defined herein. Alkoxy includes, by way of example, methoxy, ethoxy, n-propoxy, isopropoxy, n-butoxy, t-butoxy, sec-butoxy, and n-pentoxy.

"Substituted alkoxy" refers to the group -O-(substituted alkyl) wherein substituted alkyl is defined herein.

"Acyl" refers to the groups H-C(O)-, alkyl-C(O)-, substituted alkyl-C(O)-, alkenyl-C(O)-, substituted alkenyl-C(O)-, alkynyl-C(O)-, substituted alkynyl-C(O)-, cycloalkyl-C(O)-, substituted cycloalkyl-C(O)-, cycloalkenyl-C(O)-, substituted cycloalkenyl-C(O)-, aryl-C(O)-, substituted aryl-C(O)-, heteroaryl-C(O)-, substituted heteroaryl-C(O)-, heterocyclic-C(O)-, and substituted heterocyclic-C(O)-, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein. Acyl includes the "acetyl" group CH₃C(O)-.

"Acylamino" refers to the groups -NR⁴⁷C(O)alkyl, -NR⁴⁷C(O)substituted alkyl, -NR⁴⁷C(O)cycloalkyl, -NR⁴⁷C(O)substituted cycloalkyl, -NR⁴⁷C(O)cycloalkenyl, -NR⁴⁷C(O)substituted cycloalkenyl, -NR⁴⁷C(O)alkenyl, -NR⁴⁷C(O)substituted alkenyl, -NR⁴⁷C(O)alkynyl, -NR⁴⁷C(O)substituted alkynyl, -NR⁴⁷C(O)aryl, -NR⁴⁷C(O)substituted aryl, -NR⁴⁷C(O)heteroaryl, -NR⁴⁷C(O)substituted heteroaryl, -NR⁴⁷C(O)heterocyclic, and -NR⁴⁷C(O)substituted heterocyclic wherein R⁴⁷ is hydrogen or alkyl and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Acyloxy" refers to the groups alkyl-C(O)O-, substituted alkyl-C(O)O-, alkenyl-C(O)O-, substituted alkenyl-C(O)O-, alkynyl-C(O)O-, substituted alkynyl-C(O)O-, aryl-C(O)O-, substituted aryl-C(O)O-, cycloalkyl-C(O)O-, substituted cycloalkyl-C(O)O-, cycloalkenyl-C(O)O-, substituted cycloalkenyl-C(O)O-, heteroaryl-C(O)O-, substituted heteroaryl-C(O)O-, heterocyclic-C(O)O-, and substituted heterocyclic-C(O)O- wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

An animal, subject or patient for diagnosis or treatment refers to an animal such as a mammal, or a human, ovine, bovine, feline, canine, equine, simian, etc. Non-human animals subject to diagnosis or treatment include, for example, simians, murine, such as, rat, mice, canine, leporid, livestock, sport animals, and pets.

"Amino" refers to the group -NH₂.

"Substituted amino" refers to the group -NR⁴⁸R⁴⁹ where R⁴⁸ and R⁴⁹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, substituted heterocyclic, -SO₂-alkyl, -SO₂-substituted alkyl, -SO₂-alkenyl, -SO₂-substituted alkenyl, -SO_{Z}-cycloalkyl, -SO₂-substituted cylcoalkyl, -SO₂-cycloalkenyl, -SO₂-substituted cylcoalkenyl, -SO₂-aryl, -SO₂-substituted aryl, -SO₂-heteroaryl, -SO₂-substituted heteroaryl, -SO₂-heterocyclic, and -SO₂-substituted heterocyclic and wherein R⁴⁸ and R⁴⁹ are optionally joined, together with the nitrogen bound thereto to form a heterocyclic or substituted heterocyclic group, provided that R⁴⁸ and R⁴⁹ are both not hydrogen, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein. When R⁴⁸ is hydrogen and R⁴⁹ is alkyl, the substituted amino group is sometimes referred to herein as alkylamino. When R⁴⁸ and R⁴⁹ are alkyl, the substituted amino group is sometimes referred to herein as dialkylamino. When referring to a monosubstituted amino, it is meant that either R⁴⁸ or R⁴⁹ is hydrogen but not both. When referring to a disubstituted amino, it is meant that neither R⁴⁸ nor R⁴⁹ are hydrogen.

"Aminocarbonyl" refers to the group -C(O)NR⁵⁰R⁵¹ where R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminothiocarbonyl" refers to the group -C(S)NR⁵⁰R⁵¹ where R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminocarbonylamino" refers to the group -NR⁴⁷C(O)NR⁵⁰R⁵¹ where R⁴⁷ is hydrogen or alkyl and R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminothiocarbonylamino" refers to the group -NR⁴⁷C(S)NR⁵⁰R⁵¹ where R⁴⁷ is hydrogen or alkyl and R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminocarbonyloxy" refers to the group -O-C(O)NR⁵⁰R⁵¹ where R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminosulfonyl" refers to the group -SO₂NR⁵⁰R⁵¹ where R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminosulfonyloxy" refers to the group -O-SO₂NR⁵⁰R⁵¹ where R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminosulfonylamino" refers to the group -NR⁴⁷SO₂NR⁵⁰R⁵¹ where R⁴⁷ is hydrogen or alkyl and R⁵⁰ and R⁵¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Amidino" refers to the group -C(=NR⁵²)NR⁵⁰R⁵1 where R⁵⁰, R⁵¹, and R⁵² are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R⁵⁰ and R⁵¹ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aryl" or "Ar" refers to a monovalent aromatic carbocyclic group of from 6 to 14 carbon atoms having a single ring *(e.g.,* phenyl) or multiple condensed rings *(e.g.,* naphthyl or anthryl) which condensed rings may or may not be aromatic (e.g., 2-benzoxazolinone, 2H-1,4-benzoxazin-3(4H)-one-7-yl, and the like) provided that the point of attachment is at an aromatic carbon atom. Preferred aryl groups include phenyl and naphthyl.

"Substituted aryl" refers to aryl groups which are substituted with 1 to 5, preferably 1 to 3, or more preferably 1 to 2 substituents selected from the group consisting of alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein.

"Arylene" refers to a divalent aromatic carbocyclic group of from 6 to 14 carbon atoms having a single ring or multiple condensed rings. "Substituted arylene" refers to an arylene having from 1 to 5, preferably 1 to 3, or more preferably 1 to 2 substituents as defined for aryl groups.

"Heteroarylene" refers to a divalent aromatic group of from 1 to 10 carbon atoms and 1 to 4 heteroatoms selected from the group consisting of oxygen, nitrogen and sulfur within the ring. "Substituted heteroarylene" refers to heteroarylene groups that are substituted with from 1 to 5, preferably 1 to 3, or more preferably 1 to 2 substituents selected from the group consisting of the same group of substituents defined for substituted aryl.

"Aryloxy" refers to the group -O-aryl, where aryl is as defined herein, that includes, by way of example, phenoxy and naphthoxy.

"Substituted aryloxy" refers to the group -O-(substituted aryl) where substituted aryl is as defined herein.

"Arylthio" refers to the group -S-aryl, where aryl is as defined herein.

"Substituted arylthio" refers to the group -S-(substituted aryl), where substituted aryl is as defined herein.

"Carbonyl" refers to the divalent group -C(O)- which is equivalent to -C(=O)-.

"Carboxyl" or "carboxy" refers to -COOH or salts thereof.

"Carboxyl ester" or "carboxy ester" refers to the group -C(O)(O)-alkyl, -C(O)(O)-substituted alkyl, -C(O)O-alkenyl, -C(O)(O)-substituted alkenyl, -C(O)(O)-alkynyl, - C(O)(O)-substituted alkynyl, -C(O)(O)-aryl, -C(O)(O)-substituted-aryl, -C(O)(O)-cycloalkyl, -C(O)(O)-substituted cycloalkyl, -C(O)(O)-cycloalkenyl, -C(O)(O)-substituted cycloalkenyl, -C(O)(O)-heteroaryl, -C(O)(O)-substituted heteroaryl, -C(O)(O)-heterocyclic, and -C(O)(O)-substituted heterocyclic wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"(Carboxyl ester)amino refers to the group -NR⁴⁷C(O)(O)-alkyl, -NR¹⁷C(O)(O)-substituted alkyl, -NR⁴⁷C(O)O-alkenyl, -NR⁴⁷C(O)(o)-substituted alkenyl, -NR⁴⁷C(O)(O)-alkynyl, -NR⁴⁷C(O)(o)-substituted alkynyl, -NR⁴⁷C(O)(O)-aryl, -NR⁴⁷C(O)(O)-substitutedaryl, -NR⁴⁷C(O)(O)-cycloalkyl, -NR⁴⁷C(O)(O)-substituted cycloalkyl, -NR⁴⁷C(O)(O)-cycloalkenyl, -NR⁴⁷C(O)(o)-substituted cycloalkenyl, -NR⁴⁷C(O)(O)-heteroaryl, - NR⁴⁷C(O)(O)-substituted heteroaryl, -NR⁴⁷C(O)(O)-heterocyclic, and -NR⁴⁷C(O)(O)-substituted heterocyclic wherein R⁴⁷ is alkyl or hydrogen, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"(Carboxyl ester)oxy refers to the group -O-C(O)O-alkyl, -O-C(O)O-substituted alkyl, -O-C(O)O-alkenyl, -O-C(O)O-substituted alkenyl, -O-C(O)O-alkynyl, -O-C(O)(O)-substituted alkynyl, -O-C(O)O-aryl, -O-C(O)O-substituted-aryl, -O-C(O)O-cycloalkyl, - O-C(O)O-substituted cycloalkyl, -O-C(O)O-cycloalkenyl, -O-C(O)O-substituted cycloalkenyl, -O-C(O)O-heteroaryl, -O-C(O)O-substituted heteroaryl, -O-C(O)O-heterocyclic, and -O-C(O)O-substituted heterocyclic wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

A "composition" as used herein, refers to an active agent, such as a compound as disclosed herein and a carrier, inert or active. The carrier can be, without limitation, solid such as a bead or resin, or liquid, such as phosphate buffered saline.

Administration or treatment in "combination" refers to administering two agents such that their pharmacological effects are manifest at the same time. Combination does not require administration at the same time or substantially the same time, although combination can include such administrations.

"Cyano" refers to the group -CN.

"Cycloalkyl" refers to cyclic alkyl groups of from 3 to 10 carbon atoms having single or multiple cyclic rings including fused, bridged, and spiro ring systems. The fused ring can be an aryl ring provided that the non aryl part is joined to the rest of the molecule. Examples of suitable cycloalkyl groups include, for instance, adamantyl, cyclopropyl, cyclobutyl, cyclopentyl, and cyclooctyl.

"Cycloalkenyl" refers to non-aromatic cyclic alkyl groups of from 3 to 10 carbon atoms having single or multiple cyclic rings and having at least one >C=C< ring unsaturation and preferably from 1 to 2 sites of >C=C< ring unsaturation.

"Substituted cycloalkyl" and "substituted cycloalkenyl" refers to a cycloalkyl or cycloalkenyl group having from 1 to 5 or preferably 1 to 3 substituents selected from the group consisting of oxo, thioxo, alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein.

"Cyclopropano" refers to:

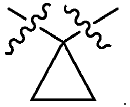

"Cyclobutano" refers to:

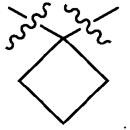

"Cycloalkyloxy" refers to -O-cycloalkyl.

"Substituted cycloalkyloxy refers to -O-(substituted cycloalkyl).

"Cycloalkylthio" refers to -S-cycloalkyl.

"Substituted cycloalkylthio" refers to -S-(substituted cycloalkyl).

"Cycloalkenyloxy" refers to -O-cycloalkenyl.

"Substituted cycloalkenyloxy" refers to -O-(substituted cycloalkenyl).

"Cycloalkenylthio" refers to -S-cycloalkenyl.

"Substituted cycloalkenylthio" refers to -S-(substituted cycloalkenyl).

"Guanidino" refers to the group -NHC(=NH)NH₂.

"Substituted guanidino" refers to -NR⁵³C(=NR⁵³)N(R⁵³)₂ where each R⁵³ is independently selected from the group consisting of hydrogen, alkyl, substituted alkyl, aryl, substituted aryl, heteroaryl, substituted heteroaryl, cycloalkyl, substituted cycloalkyl, heterocyclic, and substituted heterocyclic and two R⁵³ groups attached to a common guanidino nitrogen atom are optionally joined together with the nitrogen bound thereto to form a heterocyclic or substituted heterocyclic group, provided that at least one R⁵³ is not hydrogen, and wherein said substituents are as defined herein.

"Halo" or "halogen" refers to fluoro, chloro, bromo and iodo.

"Hydroxy" or "hydroxyl" refers to the group -OH.

"Heteroaryl" refers to an aromatic group of from 1 to 10 carbon atoms and 1 to 4 heteroatoms selected from the group consisting of oxygen, nitrogen and sulfur within the ring. Such heteroaryl groups can have a single ring (*e.g*., pyridinyl or furyl) or multiple condensed rings (*e.g*., indolizinyl or benzothienyl) wherein the condensed rings may or may not be aromatic and/or contain a heteroatom provided that the point of attachment is through an atom of the aromatic heteroaryl group. In one embodiment, the nitrogen and/or the sulfur ring atom(s) of the heteroaryl group are optionally oxidized to provide for the N-oxide (N→O), sulfinyl, or sulfonyl moieties. Certain non-limiting examples include pyridinyl, pyrrolyl, indolyl, thiophenyl, oxazolyl, thiazolyl, and furanyl.

"Substituted heteroaryl" refers to heteroaryl groups that are substituted with from 1 to 5, preferably 1 to 3, or more preferably 1 to 2 substituents selected from the group consisting of the same group of substituents defined for substituted aryl.

"Heteroaryloxy" refers to -O-heteroaryl.

"Substituted heteroaryloxy" refers to the group -O-(substituted heteroaryl).

"Heteroarylthio" refers to the group -S-heteroaryl.

"Substituted heteroarylthio" refers to the group -S-(substituted heteroaryl).

"Heterocycle" or "heterocyclic" or "heterocycloalkyl" or "heterocyclyl" refers to a saturated or partially saturated, but not aromatic, group having from 1 to 10 ring carbon atoms and from 1 to 4 ring heteroatoms selected from the group consisting of nitrogen, sulfur, or oxygen. Heterocycle encompasses single ring or multiple condensed rings, including fused bridged and spiro ring systems. In fused ring systems, one or more the rings can be cycloalkyl, aryl, or heteroaryl provided that the point of attachment is through a non-aromatic ring. In one embodiment, the nitrogen and/or sulfur atom(s) of the heterocyclic group are optionally oxidized to provide for the N-oxide, sulfinyl, or sulfonyl moieties.

"Substituted heterocyclic" or "substituted heterocycloalkyl" or "substituted heterocyclyl" refers to heterocyclyl groups that are substituted with from 1 to 5 or preferably 1 to 3 of the same substituents as defined for substituted cycloalkyl.

"Heterocyclyloxy" refers to the group -O-heterocycyl.

"Substituted heterocyclyloxy" refers to the group -O-(substituted heterocycyl).

"Heterocyclylthio" refers to the group -S-heterocycyl.

"Substituted heterocyclylthio" refers to the group -S-(substituted heterocycyl).

Examples of heterocycle and heteroaryls include, but are not limited to, azetidine, pyrrole, furan, thiophene, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthyl pyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, and tetrahydrofuranyl.

"Nitro" refers to the group -NO₂.

"Oxo" refers to the atom (=O).

Phenylene refers to a divalent aryl ring, where the ring contains 6 carbon atoms.

Substituted phenylene refers to phenylenes which are substituted with 1 to 4, preferably 1 to 3, or more preferably 1 to 2 substituents selected from the group consisting of alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, substituted sulfonyloxy, thioacyl, thiol, alkylthio, and substituted alkylthio, wherein said substituents are as defined herein.

"Spirocycloalkyl" and "spiro ring systems" refers to divalent cyclic groups from 3 to 10 carbon atoms having a cycloalkyl or heterocycloalkyl ring with a spiro union (the union formed by a single atom which is the only common member of the rings) as exemplified by the following structure:

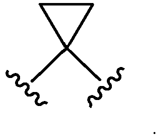

"Sulfonyl" refers to the divalent group -S(O)₂-.

"Substituted sulfonyl" refers to the group -SO₂-alkyl, -SO₂-substituted alkyl, -SO₂-alkenyl, -SO₂-substituted alkenyl, -SO_{Z}-cycloalkyl, -SO₂-substituted cylcoalkyl, -SO₂-cycloalkenyl, -SO₂-substituted cylcoalkenyl, -SO₂-aryl, -SO₂-substituted aryl, -SO₂-heteroaryl, -SO₂-substituted heteroaryl, -SO₂-heterocyclic, -SO₂-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein. Substituted sulfonyl includes groups such as methyl-SO₂-, phenyl-SO₂-, and 4-methylphenyl-SO₂-.

"Substituted sulfonyloxy" refers to the group -OSO₂-alkyl, -OSO₂-substituted alkyl, -OSO₂-alkenyl, -OSO₂-substituted alkenyl, -OSO₂-cycloalkyl, -OSO₂-substituted cylcoalkyl, -OSO₂-cycloalkenyl, -OSO₂-substituted cylcoalkenyl, -OSO₂-aryl, -OSO₂-substituted aryl, -OSO₂-heteroaryl, -OSO₂-substituted heteroaryl, -OSO₂-heterocyclic, -OSO₂-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Thioacyl" refers to the groups H-C(S)-, alkyl-C(S)-, substituted alkyl-C(S)-, alkenyl-C(S)-, substituted alkenyl-C(S)-, alkynyl-C(S)-, substituted alkynyl-C(S)-, cycloalkyl-C(S)-, substituted cycloalkyl-C(S)-, cycloalkenyl-C(S)-, substituted cycloalkenyl-C(S)-, aryl-C(S)-, substituted aryl-C(S)-, heteroaryl-C(S)-, substituted heteroaryl-C(S)-, heterocyclic-C(S)-, and substituted heterocyclic-C(S)-, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Thiol" refers to the group -SH.

"Thiocarbonyl" refers to the divalent group -C(S)- which is equivalent to -C(=S)-.

"Thioxo" refers to the atom (=S).

"Alkylthio" refers to the group -S-alkyl wherein alkyl is as defined herein.

"Substituted alkylthio" refers to the group -S-(substituted alkyl) wherein substituted alkyl is as defined herein.

A substituted ring can be substituted with one or more fused and/or spiro cycles. Such fused cycles include a fused cycloalkyl, a fused heterocyclyl, a fused aryl, a fused heteroaryl ring, each of which rings can be unsubstituted or substituted. Such spiro cycles include a fused cycloalkyl and a fused heterocyclyl, each of which rings can be unsubstituted or substituted.

"Optionally substituted" refers to a group selected from that group and a substituted form of that group. Substituents are such as those defined hereinabove. In one embodiment, substituents are selected from C₁-C₁₀ or C₁-C₆ alkyl, substituted C₁-C₁₀ or C₁-C₆ alkyl, C₂-C₆ alkenyl, C₂-C₆ alkynyl, C₆-C₁₀ aryl, C₃-C₈ cycloalkyl, C₂-C₁₀ heterocyclyl, C₁-C₁₀ heteroaryl, substituted C₂-C₆ alkenyl, substituted C₂-C₆ alkynyl, substituted C₆-C₁₀ aryl, substituted C₃-C₈ cycloalkyl, substituted C₂-C₁₀ heterocyclyl, substituted C₁-C₁₀ heteroaryl, halo, nitro, cyano, -CO₂H or a C₁-C₆ alkyl ester thereof.

Unless indicated otherwise, the nomenclature of substituents that are not explicitly defined herein are arrived at by naming the terminal portion of the functionality followed by the adjacent functionality toward the point of attachment. For example, the substituent "alkoxycarbonylalkyl" refers to the group (alkoxy)-C(O)-(alkyl)-.

It is understood that in all substituted groups defined above, polymers arrived at by defining substituents with further substituents to themselves (e.g., substituted aryl having a substituted aryl group as a substituent which is itself substituted with a substituted aryl group, etc.) are not intended for inclusion herein. In such cases, the maximum number of such substituents is three. That is to say that each of the above definitions is constrained by a limitation that, for example, substituted aryl groups are limited to -substituted aryl-(substituted aryl)-substituted aryl.

It is understood that the above definitions are not intended to include impermissible substitution patterns (e.g., methyl substituted with 5 fluoro groups). Such impermissible substitution patterns are well known to the skilled artisan.

"Tautomer" refer to alternate forms of a compound that differ in the position of a proton, such as enol-keto and imine-enamine tautomers, or the tautomeric forms of heteroaryl groups containing a ring atom attached to both a ring -NH- moiety and a ring =N- moiety such as pyrazoles, imidazoles, benzimidazoles, triazoles, and tetrazoles.

As used herein, the term stereochemically pure denotes a compound which has 80% or greater by weight of the indicated stereoisomer and 20% or less by weight of other stereoisomers. In a further embodiment, the compound of Formula (I) has 90% or greater by weight of the stated stereoisomer and 10% or less by weight of other stereoisomers. In a yet further embodiment, the compound of Formula (I) has 95% or greater by weight of the stated stereoisomer and 5% or less by weight of other stereoisomers. In a still further embodiment, the compound of Formula (I) has 97% or greater by weight of the stated stereoisomer and 3% or less by weight of other stereoisomers.

"Pharmaceutically acceptable salt" refers to salts of a compound, which salts are suitable for pharmaceutical use and are derived from a variety of organic and inorganic counter ions well known in the art and include, when the compound contains an acidic functionality, by way of example only, sodium, potassium, calcium, magnesium, ammonium, and tetraalkylammonium; and when the molecule contains a basic functionality, salts of organic or inorganic acids, such as hydrochloride, hydrobromide, tartrate, mesylate, acetate, maleate, and oxalate (see Stahl and Wermuth, eds., "Handbook of Pharmaceutically Acceptable Salts," (2002), Verlag Helvetica Chimica Acta, Zürich, Switzerland), for a discussion of pharmaceutical salts, their selection, preparation, and use.

Generally, pharmaceutically acceptable salts are those salts that retain substantially one or more of the desired pharmacological activities of the parent compound and which are suitable for *in vivo* administration. Pharmaceutically acceptable salts include acid addition salts formed with inorganic acids or organic acids. Inorganic acids suitable for forming pharmaceutically acceptable acid addition salts include, by way of example and not limitation, hydrohalide acids (*e.g*., hydrochloric acid, hydrobromic acid, hydroiodic acid, etc.), sulfuric acid, nitric acid, phosphoric acid, and the like.

Organic acids suitable for forming pharmaceutically acceptable acid addition salts include, by way of example and not limitation, acetic acid, trifluoroacetic acid, propionic acid, hexanoic acid, cyclopentanepropionic acid, glycolic acid, oxalic acid, pyruvic acid, lactic acid, malonic acid, succinic acid, malic acid, maleic acid, fumaric acid, tartaric acid, citric acid, palmitic acid, benzoic acid, 3-(4-hydroxybenzoyl) benzoic acid, cinnamic acid, mandelic acid, alkylsulfonic acids (*e.g*., methanesulfonic acid, ethanesulfonic acid, 1,2-ethane-disulfonic acid, 2-hydroxyethanesulfonic acid, etc.), arylsulfonic acids (*e.g.,* benzenesulfonic acid, 4-chlorobenzenesulfonic acid, 2-naphthalenesulfonic acid, 4-toluenesulfonic acid, camphorsulfonic acid, etc.), glutamic acid, hydroxynaphthoic acid, salicylic acid, stearic acid, muconic acid, and the like.

Pharmaceutically acceptable salts also include salts formed when an acidic proton present in the parent compound is either replaced by a metal ion *(e.g.,* an alkali metal ion, an alkaline earth metal ion, or an aluminum ion) or by an ammonium ion *(e.g.,* an ammonium ion derived from an organic base, such as, ethanolamine, diethanolamine, triethanolamine, morpholine, piperidine, dimethylamine, diethylamine, triethylamine, and ammonia).

An "effective amount" is an amount sufficient to effect beneficial or desired results. An effective amount can be administered in one or more administrations, applications or dosages. Such delivery is dependent on a number of variables including the time period for which the individual dosage unit is to be used, the bioavailability of the therapeutic agent, the route of administration, etc. It is understood, however, that specific dose levels of the therapeutic agents disclosed herein for any particular subject depends upon a variety of factors including the activity of the specific compound employed, bioavailability of the compound, the route of administration, the age of the animal and its body weight, general health, sex, the diet of the animal, the time of administration, the rate of excretion, the drug combination, and the severity of the particular disorder being treated and form of administration. In general, one will desire to administer an amount of the compound that is effective to achieve a serum level commensurate with the concentrations found to be effective *in vivo.* These considerations, as well as effective formulations and administration procedures are well known in the art and are described in standard textbooks.

"Therapeutically effective amount" of a drug or an agent refers to an amount of the drug or the agent that is an amount sufficient to obtain a pharmacological response such as inhibiting a biological target (e.g., dUTPase); or alternatively, is an amount of the drug or agent that, when administered to a patient with a specified disorder or disease, is sufficient to have the intended effect, e.g., treatment, alleviation, amelioration, palliation or elimination of one or more manifestations of the specified disorder or disease in the patient. A therapeutic effect does not necessarily occur by administration of one dose, and may occur only after administration of a series of doses. Thus, a therapeutically effective amount may be administered in one or more administrations.

As used herein, "treating" or "treatment" of a disease in a subject refers to (1) preventing the symptoms or disease from occurring in a subject that is predisposed or does not yet display symptoms of the disease; (2) inhibiting the disease or arresting its development; or (3) ameliorating or causing regression of the disease or the symptoms of the disease. As understood in the art, "treatment" is an approach for obtaining beneficial or desired results, including clinical results. For the purposes of this technology, beneficial or desired results can include one or more, but are not limited to, alleviation or amelioration of one or more symptoms, diminishment of extent of a condition (including a disease), stabilized (i.e., not worsening) state of a condition (including disease), delay or slowing of condition (including disease), progression, amelioration or palliation of the condition (including disease), states and remission (whether partial or total), whether detectable or undetectable. In one aspect, treatment excludes prophylaxis.

When the disease is cancer, the following clinical endpoints are non-limiting examples of treatment: (1) elimination of a cancer in a subject or in a tissue/organ of the subject or in a cancer loci; (2) reduction in tumor burden (such as number of cancer cells, number of cancer foci, number of cancer cells in a foci, size of a solid cancer, concentrate of a liquid cancer in the body fluid, and/or amount of cancer in the body); (3) stabilizing or delay or slowing or inhibition of cancer growth and/or development, including but not limited to, cancer cell growth and/or division, size growth of a solid tumor or a cancer loci, cancer progression, and/or metastasis (such as time to form a new metastasis, number of total metastases, size of a metastasis, as well as variety of the tissues/organs to house metastatic cells); (4) less risk of having a cancer growth and/or development; (5) inducing an immune response of the patient to the cancer, such as higher number of tumor-infiltrating immune cell, higher number of activated immune cells, or higher number cancer cell expressing an immunotherapy target, or higher level of expression of an immunotherapy target in a cancer cell; (6) higher probability of survival and/or increased duration of survival, such as increased overall survival (OS, which may be shown as 1-year, 2-year, 5-year, 10-year, or 20-year survival rate), increased progression free survival (PFS), increased disease free survival (DFS), increased time to tumor recurrence (TTR) and increased time to tumor progression (TTP). In some embodiments, the subject after treatment experiences one or more endpoints selected from tumor response, reduction in tumor size, reduction in tumor burden, increase in overall survival, increase in progression free survival, inhibiting metastasis, improvement of quality of life, minimization of drug-related toxicity, and avoidance of side-effects (e.g., decreased treatment emergent adverse events). In some embodiments, improvement of quality of life includes resolution or improvement of cancer-specific symptoms, such as but not limited to fatigue, pain, nausea/vomiting, lack of appetite, and constipation; improvement or maintenance of psychological well-being (e.g., degree of irritability, depression, memory loss, tension, and anxiety); improvement or maintenance of social well-being (e.g., decreased requirement for assistance with eating, dressing, or using the restroom; improvement or maintenance of ability to perform normal leisure activities, hobbies, or social activities; improvement or maintenance of relationships with family). In some embodiments, improved patient quality of life that is measured qualitatively through patient narratives or quantitatively using validated quality of life tools known to those skilled in the art, or a combination thereof. Additional non-limiting examples of endpoints include reduced hospital admissions, reduced drug use to treat side effects, longer periods off-treatment, and earlier return to work or caring responsibilities. In one aspect, prevention or prophylaxis is excluded from treatment.

As used herein, immune cells are cells of the immune system, including but not limited to lymphocytes (such as, T-cells, B-cells, natural killer (NK) cells, and natural killer T (NKT) cells), myeloid-derived cells (such as granulocytes (basophils, eosinophils, neutrophils, mast cells), monocytes, macrophages, and dendritic cells (DC)). T cells are divided into two broad categories: CD8+ T cells or CD4+ T cells, based on which protein is present on the cell's surface. CD8+ T cells also are called cytotoxic T cells or cytotoxic lymphocytes (CTLs). The four major CD4+ T-cell subsets are TH1, TH2, TH17, and Treg, with "TH" referring to "T helper cell." T cells may also refer to gamma delta T cell. Dendritic cells (DC) are an important antigen-presenting cell (APC), and they also can develop from monocytes. In some embodiments, the immune cells refer to a killer cell, including but not limited to: a cytotoxic T cell, a gamma delta T cell, a NK cell and a NK-T cell. In one embodiment, the immune cell is a CD45+ cell.

The term "subject," "host," "individual," and "patient" are as used interchangeably herein to refer to animals, typically mammalian animals. Any suitable mammal can be treated by a method described herein. Non-limiting examples of mammals include humans, non-human primates (e.g., apes, gibbons, chimpanzees, orangutans, monkeys, macaques, and the like), domestic animals (e.g., dogs and cats), farm animals (e.g., horses, cows, goats, sheep, pigs) and experimental animals (e.g., mouse, rat, rabbit, guinea pig). In some embodiments, a mammal is a human. A mammal can be any age or at any stage of development (e.g., an adult, teen, child, infant, or a mammal in utero). A mammal can be male or female. In some embodiments, a subject is a human. In some embodiments, a subject has or is diagnosed of having or is suspected of having a cancer.

In certain embodiments, the terms "disease" "disorder" and "condition" are used interchangeably herein, referring to a cancer, a status of being diagnosed with a cancer, or a status of being suspect of having a cancer. "Cancer", which is also referred to herein as "tumor", is a known medically as an uncontrolled division of abnormal cells in a part of the body, benign or malignant. In one embodiment, cancer refers to a malignant neoplasm, a broad group of diseases involving unregulated cell division and growth, and invasion to nearby parts of the body. Non-limiting examples of cancers include carcinomas, sarcomas, leukemia and lymphoma, e.g., colon cancer, colorectal cancer, rectal cancer, gastric cancer, esophageal cancer, head and neck cancer, breast cancer, brain cancer, lung cancer, stomach cancer, liver cancer, gall bladder cancer, or pancreatic cancer. In one embodiment, the term "cancer" refers to a solid tumor, which is an abnormal mass of tissue that usually does not contain cysts or liquid areas, including but not limited to, sarcomas, carcinomas, and certain lymphomas (such as Non-Hodgkin's lymphoma). In another embodiment, the term "cancer" refers to a liquid cancer, which is a cancer presenting in body fluids (such as, the blood and bone marrow), for example, leukemias (cancers of the blood) and certain lymphomas.

Additionally or alternatively, a cancer may refer to a local cancer (which is an invasive malignant cancer confined entirely to the organ or tissue where the cancer began), a metastatic cancer (referring to a cancer that spreads from its site of origin to another part of the body), a non-metastatic cancer, a primary cancer (a term used describing an initial cancer a subject experiences), a secondary cancer (referring to a metastasis from primary cancer or second cancer unrelated to the original cancer), an advanced cancer, an unresectable cancer, or a recurrent cancer. As used herein, an advanced cancer refers to a cancer that had progressed after receiving one or more of: the first line therapy, the second line therapy, or the third line therapy.

The term "contacting" means direct or indirect binding or interaction between two or more. A particular example of direct interaction is binding. A particular example of an indirect interaction is where one entity acts upon an intermediary molecule, which in turn acts upon the second referenced entity. Contacting as used herein includes in solution, in solid phase, *in vitro, ex vivo,* in a cell and *in vivo.* Contacting *in vivo* can be referred to as administering, or administration.

As used herein, the term "administration" and "administering" are used to mean introducing an agent into a subject. Routes of administration include, but are not limited to, oral (such as a tablet, capsule or suspension), topical, transdermal, intranasal, vaginal, rectal, subcutaneous intravenous, intravenous, intraarterial, intramuscular, intraosseous, intraperitoneal, intraocular, subconjunctival, sub-Tenon's, intravitreal, retrobulbar, intracameral, intratumoral, epidural and intrathecal.

An "immunotherapy agent" means a type of cancer treatment which uses a patient's own immune system to fight cancer, including but not limited to a physical intervene, a chemical substance, a biological molecule or particle, a cell, a tissue or organ, or any combinations thereof, enhancing or activating or initiating a patient's immune response against cancer. In the present invention, the immunotherapy agent is an anti-PD-1 antibody CD279. Examples of immunotherapy agents include antibodies, immune regulators, checkpoint inhibitors, an antisense oligonucleotide (ASO), a RNA interference (RNAi), a Clustered Regularly Interspaced Short Palindromic Repeat (CRISPR) system, a viral vector, an anti-cancer cell therapy (e.g., transplanting an anti-cancer immune cell optionally amplified and/or activated in vivo, or administering an immune cell expressing a chimeric antigen receptor (CAR)), a CAR therapy, and cancer vaccines. As used herein, unless otherwise specified, an immunotherapy agent is not an inhibitor of thymidylate biosynthesis, or an anthracycline or other topoisomerase II inhibitor. As used herein, immune checkpoint refers to a regulator and/or modulator of the immune system (such as an immune response, an anti-tumor immune response, a nascent anti-tumor immune response, an anti-tumor immune cell response, an anti-tumor T cell response, and/or an antigen recognition of T cell receptor in the process of immune response). Their interaction activates either inhibitory or activating immune signaling pathways. Thus a checkpoint may contain one of the two signals: an stimulatory immune checkpoint that stimulates an immune response, and an inhibitory immune checkpoint inhibiting an immune response. In some embodiments, the immune checkpoint is crucial for self-tolerance, which prevents the immune system from attacking cells indiscriminately. However, some cancers can protect themselves from attack by stimulating immune checkpoint targets. In some embodiments, the immune checkpoints are present on T cells, antigen-presenting cells (APCs) and/or tumor cells.

One target of an immunotherapy agent is a tumor-specific antigen while the immunotherapy directs or enhances the immune system to recognize and attack tumor cells. Non-limiting examples of such agent includes a cancer vaccine presenting a tumor-specific antigen to the patient's immune system, a monoclonal antibody or an antibody-drug conjugate specifically binding to a tumor-specific antigen, a bispecific antibody specifically binding to a tumor-specific antigen and an immune cell (such as a T-cell engager or a NK-cell engager), an immune cell (such as a killer cell) specifically binding to a tumor-specific antigen (such as a CAR-T cell, a CAR-NK cell, and a CAR-NKT cell), a polynucleotide (or a vector comprising the same) transfecting/transducing an immune cell to express an tumor-specific antibody of an antigen binding fragment thereof (such as a CAR), or a polynucleotide (or a vector comprising the same) transfecting/transducing a cancer cell to express an antigen or a marker which can be recognized by an immune cell.

In accordance with the invention, the target of the immunotherapy is an inhibitory immune checkpoint which suppresses the nascent anti-tumor immune response, which is PD-1 as defined in the claims. Another exemplified target is an inhibitory immune checkpoint which suppresses the nascent anti-tumor immune response, such as A2AR, B7-H3, B7-H4, BTLA, CTLA-4, CTLA-4/B7-1/B7-2, IDO, KIR, LAG3, NOX2, PD-1, PD-L1 and TIM-3, VISTA, SIGLEC7 (Sialic acid-binding immunoglobulin-type lectin 7, also designated as CD328) and SIGLEC9 (Sialic acid-binding immunoglobulin-type lectin 9, also designated as CD329). Non-limiting examples of such agent includes an antagonist or inhibitor of an inhibitory immune checkpoint, an agent reducing the expression and/or activity of an inhibitory immune checkpoint (such as via an antisense oligonucleotide (ASO), a RNA interference (RNAi), or a Clustered Regularly Interspaced Short Palindromic Repeat (CRISPR) system), an antibody or an antibody-drug conjugate or a ligand specifically binding to and reducing (or inhibiting) the activity of an inhibitory immune checkpoint, an immune cell with reduced (or inhibited) an inhibitory immune checkpoint (and optionally specifically binding to a tumor-specific antigen, such as a CAR-T cell, a CAR-NK cell, and a CAR-NKT cell), and a polynucleotide (or a vector comprising the same) transfecting/transducing an immune cell or a cancer cell to reduce or inhibit an inhibitory immune checkpoint thereof. Reducing expression or activity of such inhibitory immune checkpoint enhances immune response of a patient to a cancer.

A further possible immunotherapy target is a stimulatory checkpoint molecule (including but not limited to 4-1BB, CD27, CD28, CD40, CD122, CD137, OX40, GITR and ICOS), wherein the immunotherapy agent actives or enhances the anti-tumor immune response. Non-limiting examples of such agent includes an agonist of a stimulatory checkpoint, an agent increasing the expression and/or activity of a stimulating immune checkpoint, an antibody or an antibody-drug conjugate or a ligand specifically binding to and activating or enhancing the activity of a stimulating immune checkpoint, an immune cell with increased expression and/or activity of a stimulating immune checkpoint (and optionally specifically binding to a tumor-specific antigen, such as a CAR-T cell, a CAR-NK cell, and a CAR-NKT cell), and a polynucleotide (or a vector comprising the same) transfecting/transducing an immune cell or a cancer cell to express a stimulating immune checkpoint thereof.

Additional or alternative targets may be utilized by an immunotherapy agent, such as an immune regulating agent, including but not limited to, an agent activating an immune cell, an agent recruiting an immune cell to a cancer or a cancer cell, or an agent increasing immune cell infiltrated into a solid tumor and/or a cancer loci. Non-limiting examples of such agent is an immune regulator or a variant, a mutant, a fragment, an equivalent thereof.

In some embodiments, an immunotherapy agent utilizes one or more targets, such as a bispecific T cell engager, a bispecific NK cell engager, or a CAR cell therapy. In some embodiments, the immunotherapy agent targets one or more immune regulatory or effector cells.

As used herein, the term "antibody" collectively refers to immunoglobulins or immunoglobulin-like molecules including by way of example and without limitation, IgA, IgD, IgE, IgG and IgM, combinations thereof, and similar molecules produced during an immune response in any vertebrate, for example, in mammals such as humans, goats, rabbits, rat, canine, donkey, mice, camelids (such as dromedaries, llamas, and alpacas), as well as non-mammalian species, such as shark immunoglobulins. Unless specifically noted otherwise, the term "antibody" includes intact immunoglobulins and "antibody fragments" or "antigen binding fragments" that specifically bind to a molecule of interest (or a group of highly similar molecules of interest) to the substantial exclusion of binding to other molecules (for example, antibodies and antibody fragments that have a binding constant for the molecule of interest that is at least 10³ M⁻¹ greater, at least 10⁴ M⁻¹ greater or at least 10⁵ M⁻¹ greater than a binding constant for other molecules in a biological sample). The term "antibody" also includes genetically engineered forms such as chimeric antibodies (for example, murine or humanized non-primate antibodies), heteroconjugate antibodies (such as, bispecific antibodies). See also, Pierce Catalog and Handbook, 1994-1995 (Pierce Chemical Co., Rockford, Ill.); Owen et al., Kuby Immunology, 7th Ed., W.H. Freeman & Co., 2013; Murphy, Janeway's Immunobiology, 8th Ed., Garland Science, 2014; Male et al., Immunology (Roitt), 8th Ed., Saunders, 2012; Parham, The Immune System, 4th Ed., Garland Science, 2014. The term "antibody" includes any protein or peptide containing molecule that comprises at least a portion of an immunoglobulin molecule, such as the whole antibody and any antigen binding fragment or a single chain thereof. The terms "antibody," "antibodies" and "immunoglobulin" also include immunoglobulins of any isotype, fragments of antibodies which retain specific binding to antigen, including, but not limited to, Fab, Fab', F(ab)₂, Fv, scFv, dsFv, Fd fragments, dAb, VH, VL, VhH, and V-NAR domains; minibodies, diabodies, triabodies, tetrabodies and kappa bodies; multispecific antibody fragments formed from antibody fragments and one or more isolated. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, at least one portion of a binding protein, chimeric antibodies, humanized antibodies, single-chain antibodies, and fusion proteins comprising an antigen-binding portion of an antibody and a non-antibody protein. The variable regions of the heavy and light chains of the immunoglobulin molecule contain a binding domain that interacts with an antigen. The constant regions of the antibodies (Abs) may mediate the binding of the immunoglobulin to host tissues. The antibodies can be polyclonal, monoclonal, multispecific (e.g., bispecific antibodies), and antibody fragments, so long as they exhibit the desired biological activity.

As used herein, the term "monoclonal antibody" refers to an antibody produced by a single clone of B-lymphocytes or by a cell into which the light and heavy chain genes of a single antibody have been transfected. Monoclonal antibodies are produced by methods known to those of skill in the art, for instance by making hybrid antibody-forming cells from a fusion of myeloma cells with immune spleen cells. Monoclonal antibodies include humanized monoclonal antibodies.

In some embodiments, the antibody is a bispecific immune cell engager, referring to a bispecific monoclonal antibody that is capable of recognizing and specifically binding to a tumor antigen (such as CD19, EpCAM, MCSP, HER2, EGFR or CS-1) and an immune cell, and directing an immune cell to cancer cells, thereby treating a cancer. Non-limiting examples of such antibody include bispecific T cell engager, bispecific cytotoxic T lymphocytes (CTL) engager, and bispecific NK cell engager. In one embodiment, the engager is a fusion protein consisting of two single-chain variable fragments (scFvs) of different antibodies. Additionally or alternatively, the immune cell is a killer cell, including but not limited to: a cytotoxic T cell, a gamma delta T cell, a NK cell and a NK-T cell.

As used herein, the term "antigen binding domain" refers to any protein or polypeptide domain that can specifically bind to an antigen target.

The term "chimeric antigen receptor" (CAR), as used herein, refers to a fused protein comprising an extracellular domain capable of binding to an antigen, a transmembrane domain derived from a polypeptide different from a polypeptide from which the extracellular domain is derived, and at least one intracellular domain. The "chimeric antigen receptor (CAR)" is sometimes called a "chimeric receptor", a "T-body", or a "chimeric immune receptor (CIR)." The "extracellular domain capable of binding to an antigen" means any oligopeptide or polypeptide that can bind to a certain antigen. The "intracellular domain" or "intracellular signaling domain" means any oligopeptide or polypeptide known to function as a domain that transmits a signal to cause activation or inhibition of a biological process in a cell. In certain embodiments, the intracellular domain may comprise, alternatively consist essentially of, or yet further comprise one or more costimulatory signaling domains in addition to the primary signaling domain. The "transmembrane domain" means any oligopeptide or polypeptide known to span the cell membrane and that can function to link the extracellular and signaling domains. A chimeric antigen receptor may optionally comprise a "hinge domain" which serves as a linker between the extracellular and transmembrane domains.

As used herein, a CAR therapy may refer to administrating an immune cell expressing a CAR into a subject as well as contacting a vector expressing a CAR in an immune cell (such as *in vivo*).

As used herein, the term "NK cell," also known as natural killer cell, refers to a type of lymphocyte that originates in the bone marrow and play a critical role in the innate immune system. NK cells provide rapid immune responses against viral-infected cells, tumor cells or other stressed cell, even in the absence of antibodies and major histocompatibility complex on the cell surfaces. NK cells for using in a cell therapy and/or a CAR therapy may either be isolated or obtained from a commercially available source. Non-limiting examples of commercial NK cell lines include lines NK-92 (ATCC^{®} CRL-2407^{™}) NK-92MI (ATCC^{®} CRL-2408^{™}). Further examples include but are not limited to NK lines HANK1, KHYG-1, NKL, NK-YS, NOI-90, and YT. Non-limiting exemplary sources for such commercially available cell lines include the American Type Culture Collection, or ATCC, (http://www.atcc.org/) and the German Collection of Microorganisms and Cell Cultures (https://www.dsmz.de/).

As used herein, the term "T cell," refers to a type of lymphocyte that matures in the thymus. T cells play an important role in cell-mediated immunity and are distinguished from other lymphocytes, such as B cells, by the presence of a T-cell receptor on the cell surface. T-cells for using in a cell therapy and/or a CAR therapy may either be isolated or obtained from a commercially available source. "T cell" includes all types of immune cells expressing CD3 including T-helper cells (CD4+ cells), cytotoxic T-cells (CD8+ cells), natural killer T-cells, T-regulatory cells (Treg) and gamma-delta T cells. A "cytotoxic cell" includes CD8+ T cells, natural-killer (NK) cells, and neutrophils, which cells are capable of mediating cytotoxicity responses. Non-limiting examples of commercially available T-cell lines include lines BCL2 (AAA) Jurkat (ATCC^{®} CRL-2902^{™}), BCL2 (S70A) Jurkat (ATCC^{®} CRL-2900^{™}), BCL2 (S87A) Jurkat (ATCC^{®} CRL-2901^{™}), BCL2 Jurkat (ATCC^{®} CRL-2899^{™}) Neo Jurkat (ATCC^{®} CRL-2898^{™}) TALL-104 cytotoxic human T cell line (ATCC # CRL-11386). Further examples include but are not limited to mature T-cell lines, *e.g.,* such as Deglis, EBT-8, HPB-MLp-W, HUT 78, HUT 102, Karpas 384, Ki 225, My-La, Se-Ax, SKW-3, SMZ-1 and T34; and immature T- cell lines, e.g., ALL-SIL, Be13, CCRF-CEM, CML-T1, DND-41, DU.528, EU-9, HD-Mar, HPB-ALL, H-SB2, HT-1, JK-T1, Jurkat, Karpas 45, KE-37, KOPT-K1, K-T1, L-KAW, Loucy, MAT, MOLT-1, MOLT 3, MOLT-4, MOLT 13, MOLT-16, MT-1, MT-ALL, P12/Ichikawa, Peer, PER0117, PER-255, PF-382, PFI-285, RPMI-8402, ST-4, SUP-T1 to T14, TALL-1, TALL-101, TALL-103/2, TALL-104, TALL-105, TALL-106, TALL-107, TALL-197, TK-6, TLBR-1, -2, -3, and -4, CCRF-HSB-2 (CCL-120.1), J.RT3-T3.5 (ATCC TIB-153), J45.01 (ATCC CRL-1990), J.CaM1.6 (ATCC CRL-2063), RS4;11 (ATCC CRL-1873), CCRF-CEM (ATCC CRM-CCL-119); and cutaneous T-cell lymphoma lines, *e.g*., HuT78 (ATCC CRM-TIB-161), MJ[G11] (ATCC CRL-8294), HuT102 (ATCC TIB-162). Null leukemia cell lines, including but not limited to REH, NALL-1, KM-3, L92-221, are another commercially available source of immune cells for using in a CAR therapy, as are cell lines derived from other leukemias and lymphomas, such as K562 erythroleukemia, THP-1 monocytic leukemia, U937 lymphoma, HEL erythroleukemia, HL60 leukemia, HMC-1 leukemia, KG-1 leukemia, U266 myeloma. Non-limiting exemplary sources for such commercially available cell lines include the American Type Culture Collection, or ATCC, (http://www.atcc.org/) and the German Collection of Microorganisms and Cell Cultures (https://www.dsmz.de/).

As used herein, a "tumor-specific antigen" refers to an antigenic substance produced in tumor cells, capable of triggering an immune response in a subject. In some embodiments, such tumor-specific antigen is not expressed on or in a cell in the subject, which is not a cancer cell. In some embodiment, such tumor-specific antigen may still be expressed in or on some non-cancer cells. For example, a tumor-specific antigen may not be expressed on the cell surface of a non-cancer cell in the subject. In one embodiment, the tumor-specific antigen may be expressed in or on a non-cancer cell of the subject, but in a much lower level compared to a cancer cell. In another embodiment, the tumor-specific antigen may be expressed in or on a non-cancer cell of the subject which is not adjacent to a cancer or a cancer cell. Non-limiting examples of a tumor-specific antigen includes: Alphafetoprotein (AFP), Beta-2-microglobulin (B2M), Beta-human chorionic gonadotropin (Beta-hCG), Bladder Tumor Antigen (BTA), C-kit/CD117, CA15-3/CA27.29, CA19-9, CA-125, CA 27.29, Calcitonin, Carcinoembryonic antigen (CEA), Chromogranin A (CgA), Cytokeratin fragment 21-1, Des-gamma-carboxy prothrombin (DCP), Estrogen receptor (ER)/progesterone receptor (PR), Epithelial tumor antigen (ETA), Fibrin/fibrinogen, Gastrin, HE4, overexpressed HER2/neu, 5-HIAA, Lactate dehydrogenase, Melanoma-associated antigen (MAGE), MUC-1, Neuron-specific enolase (NSE), Nuclear matrix protein 22, Programmed death ligand 1 (PD-L1), Prostate-specific antigen (PSA), Prostatic Acid Phosphatase (PAP), Soluble mesothelin-related peptides (SMRP), Somatostatin receptor, Tyrosinase, Thyroglobulin, abnormal products of ras, p53, alpha folate receptor, 5T4, αvβ6 integrin, BCMA, B7-H3, B7-H6, CAIX, CD16, CD19, CD20, CD22, CD25, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvni, EGP2, EGP40, EPCAM, EphA2, EpCAM, FAP, fetal AchR, FRoc, GD2, GD3, Glypican-3 (GPC3), HL A- A 1 +M AGE 1 , HLA-A2+MAGE1, HL A- A3 +M AGE 1, HLA-Al+NY-ESO-1, HL A-A2+NY-ESO- 1 , HLA-A3+NY-ESO- 1 , IL- 1 lRoc, IL-13Ra2, Lambda, Lewis- Y, Kappa, Mesothelin, Mucl, Mucl6, NCAM, NKG2D Ligands, NY-ESO-1, PRAME, PSCA, PSMA, ROR1, SSX, Survivin, TAG72, TEMs, VEGFR2, and WT-1.

As used herein, a "vector" refers to a construct which is capable of delivering, and, in some embodiments expressing, a polynucleotide in to a cell. Non-limiting examples of delivery vectors include viral vectors, nucleic acid expression vectors (such as a plasmid), naked DNA, and certain eukaryotic cells (e.g., producer cells). In some embodiments, nucleic acids described by the disclosure are delivered via a viral vector. Examples of viral vectors include retroviral vectors (e.g., Maloney murine leukemia virus, MML-V), adenoviral vectors (e.g., AD 100), lentiviral vectors (e.g., HIV and FIV-based vectors), and herpesvirus vectors (e.g., HSV, HSV-1, HSV-2), as described by Chira et al. (Oncotarget, 2015, 6(31); 30673-30703). In some embodiments, nucleic acids described by the disclosure are delivered by an adeno-associated virus (AAV) vector (e.g., a recombinant AAV (rAAV) vector).

As used herein, the term "CRISPR" refers to a technique of sequence specific genetic manipulation relying on the clustered regularly interspaced short palindromic repeats pathway. CRISPR can be used to perform nucleic acid editing and/or regulation, as well as to simply target proteins to a specific genomic or mRNA location. Nucleic acid editing refers to a type of genetic engineering in which the nucleotide sequence of a target polynucleotide is changed through introduction of deletions, insertions, cleavages, or base substitutions to the polynucleotide sequence. In some aspects, CRISPR-mediated editing utilizes the pathways of nonhomologous end-joining (NHEJ) or homologous recombination to perform the edits. Nucleic acid regulation refers to increasing or decreasing the production of specific gene products such as protein or RNA.

The term "gRNA" or "guide RNA" as used herein refers to the guide RNA sequences used to target specific genes for correction employing the CRISPR technique. Techniques of designing gRNAs and donor therapeutic polynucleotides for target specificity are well known in the art. For example, Doench, J., et al. Nature biotechnology 2014; 32(12):1262-7, Mohr, S. et al. (2016) FEBS Journal 283: 3232-38, and Graham, D., et al. Genome Biol. 2015; 16: 260. In some embodiments, a gRNA comprises or alternatively consists essentially of, or yet further consists of a fusion polynucleotide comprising, or consisting essentially of, or yet further consisting of, CRISPR RNA (crRNA) and trans-activating CRISPR RNA (tracrRNA); or a polynucleotide comprising, or consisting essentially of, or yet further consisting of, CRISPR RNA (crRNA) and trans-activating CRISPR RNA (tracrRNA). In some embodiments, a gRNA is synthetic (Kelley, M. et al. (2016) J of Biotechnology 233 (2016) 74-83). In some embodiments, a gRNA comprises, or consists essentially of, or consists of, at least a first region that hybridizes to target polynucleotide; and a second region comprising, or consisting essentially of, or yet further consisting of, a gRNA scaffold (*e.g*., scaffold sequence).

The term "Cas9" refers to a CRISPR associated endonucleoase referred to by this name (UniProtKB G3ECR1 (CAS9_STRTR)) as well as orthologs and biological equivalents thereof. In some embodiments, Cas9 lacks endonuclease activity. For example, in some embodiments, Cas9 is dead Cas-9 or dCas9, which lacks endonuclease activity but retains the ability to target a target polynucleotide in the presence of a gRNA. Orthologs of Cas9 include but are not limited to *Streptococcus pyogenes* Cas9 ("spCas9"); Cas9 from *Streptococcus thermophiles, Legionella pneumophilia, Neisseria lactamica, Neisseria meningitides, Francisella novicida*; and Cpf1 (which performs cutting functions analogous to Cas9) from various bacterial species including *Acidaminococcus* spp. and *Francisella novicida U112.* In some embodiments, the Cas9 is "split-Cas9" in which Cas9 is split into two halves - C-Cas9 and N-Cas9 - and fused with a two intein moieties. See e.g. Volz et al. (2015) Nat Biotechnol. 33(2):139-42; Wright et al. (2015) PNAS 112(10) 2984-89. A non-limiting exemplary split-Cas9 has a C-Cas9 comprising residues 574-1398 and N-Cas9 comprising residues 1-573. An exemplary split-Cas9 for dCas9 involves two domains comprising these same residues of dCas9, denoted dC-Cas9 and dN-Cas9.

The terms "gRNA scaffold" and "scaffold sequence" are used interchangeably and refer to a region with the guide RNA that is involved in the binding of a CRISPR associated endonuclease (e.g., Cas protein).

The term "RNA interference" (RNAi) refers to sequence-specific or gene specific suppression of gene expression (protein synthesis) that is mediated by short interfering RNA (siRNA) or microRNA (miRNA).

The term "short interfering RNA" (siRNA) refers to double-stranded RNA molecules (dsRNA), generally, from about 10 to about 30 nucleotides in length that are capable of mediating RNA interference (RNAi), or 11 nucleotides in length, 12 nucleotides in length, 13 nucleotides in length, 14 nucleotides in length, 15 nucleotides in length, 16 nucleotides in length, 17 nucleotides in length, 18 nucleotides in length, 19 nucleotides in length, 20 nucleotides in length, 21 nucleotides in length, 22 nucleotides in length, 23 nucleotides in length, 24 nucleotides in length, 25 nucleotides in length, 26 nucleotides in length, 27 nucleotides in length, 28 nucleotides in length, or 29 nucleotides in length. As used herein, the term siRNA includes short hairpin RNAs (shRNAs).

shRNAs comprise a single strand of RNA that forms a stem-loop structure, where the stem consists of the complementary sense and antisense strands that comprise a double-stranded siRNA, and the loop is a linker of varying size. The stem structure of shRNAs generally is from about 10 to about 30 nucleotides in length. For example, the stem can be 10-30 nucleotides in length, or alternatively, 12-28 nucleotides in length, or alternatively, 15-25 nucleotides in length, or alternatively, 19-23 nucleotides in length, or alternatively, 21-23 nucleotides in length.

The term "double stranded RNA" (dsRNA) refers to double stranded RNA molecules that may be of any length and may be cleaved intracellularly into smaller RNA molecules, such as siRNA. In cells that have a competent interferon response, longer dsRNA, such as those longer than about 30 base pair in length, may trigger the interferon response. In other cells that do not have a competent interferon response, dsRNA may be used to trigger specific RNAi.

Tools to assist siRNA design are readily available to the public. For example, a computer-based siRNA design tool is available on the internet at www.dharmacon.com, Ambion-www.ambion.com/jp/techlib/misc/siRNA_finder.html;ThermoScientific-Dharmacon-www.dharmacon.com/DesignCenter/DesignCenterPage.aspx; Bioinformatics Research Center-sysbio.kribb.re.kr:8080/AsiDesigner/menuDesigner.jsf;andInvitrogenrnaidesigner.invitrogen. com/rnaiexpress/.

The term "antisense oligonucleotide" (ASO) refers to a synthetic single strand of nucleic acids that bind to RNA, thereby altering or reducing the expression of the RNA. The ASO generally is from about 5 to about 70 nucleotides in length. For example, the ASO can be 5-50 nucleotides in length, or alternatively, 8-50 nucleotides in length, or alternatively, 15-40 nucleotides in length, or alternatively, 10-30 nucleotides in length, or alternatively, 8-40 nucleotides in length.

The term "cell" as used herein may refer to either a prokaryotic or an eukaryotic cell, optionally obtained from a subject or a commercially available source.

"An inhibitor of thymidylate biosynthesis" means an inhibitor which directly or indirectly impacts the thymidylate biosynthesis pathway. In the present invention, the inhibitor of thymidylate biosynthesis is 5-fluorouracil (5-FU). Examples of an inhibitor of thymidylate biosynthesis include thymidylate synthase inhibitors and inhibitors of folatemediated one-carbon metabolism. Other examples include the fluoropyrimidines (e.g., 5-fluorouracil (5-FU) or 5-FU based adjuvant therapy, S-1, and capecitabine (Xeloda^{®})); and antifolates (e.g., pemetrexed (Alimta^{®}) and methotrexate). Additional non-limiting examples include prodrug derivatives of inhibitors of thymidylate biosynthesis as well as formulations of inhibitors of thymidylate biosynthesis with modulatory co-factors.

"dUTPase" means any of the following, which are considered to be synonymous, "deoxyuridine triphosphate nucleotidohydrolase", "deoxyuridine triphosphate pyrophosphatase", "dUTP nucleotidohydrolase", "dUTP pyrophosphatase", and other equivalent nomenclature for the dUTPase enzyme. In one aspect, dUTPase intends DUT-N and DUT-M. In other aspects, it is DUT-N only, or alternatively, DUT-M only. The amino acid and coding sequences for dUTPase are known in the art and disclosed in U.S. Patent No. 5,962,246. Methods for expressing and screening for expression level of the enzyme are disclosed in U.S. Patent No. 5,962,246 and Ladner et al. (US Patent Publ. No. 2011/0212467A1).

"DUT-N" means the nuclear form of dUTPase.

"DUT-M" means the mitochondrial or cytoplasmic form of dUTPase.

5-Fluorouracil (5-FU) belongs to the family of therapy drugs called pyrimidine based anti-metabolites. It is a pyrimidine analog, which is transformed into different cytotoxic metabolites that are then incorporated into DNA and RNA thereby inducing cell cycle arrest and apoptosis. Chemical equivalents are pyrimidine analogs which result in disruption of DNA replication. Chemical equivalents inhibit cell cycle progression at S phase resulting in the disruption of cell cycle and consequently apoptosis. Equivalents to 5-FU include prodrugs, analogs and derivative thereof such as 5'-deoxy-5-fluorouridine (doxifluoroidine), 1-tetrahydrofuranyl-5-fluorouracil (ftorafur), capecitabine (Xeloda^{®}), S-1 (MBMS-247616, consisting of tegafur and two modulators, a 5-chloro-2,4-dihydroxypyridine and potassium oxonate), ralititrexed (tomudex), nolatrexed (Thymitaq, AG337), LY231514 and ZD9331, as described for example in Papamichael (1999) The Oncologist 4:478-487.

"5-FU based adjuvant therapy" refers to 5-FU alone or alternatively the combination of 5-FU with one or more other treatments, that include, but are not limited to radiation, methyl-CCNU, leucovorin, oxaliplatin (such as cisplatin), irinotecan, mitomycin, cytarabine, doxorubicin, cyclophosphamide, and levamisole, as well as an immunotherapy. Specific treatment adjuvant regimens are known in the art such as weekly Fluorouracil/Leucovorin, weekly Fluorouracil/Leucovorin + Bevacizumab, FOLFOX, FOLFOX-4, FOLFOX6, modified FOLFOX6 (mFOLFOX6), FOLFOX6 with bevacizumab, mFOLFOX6 + Cetuximab, mFOLFOX6 + Panitumumab, modified FOLFOX7 (mFOLFOX7), FOLFIRI, FOLFIRI with Bevacizumab, FOLFIRI + Ziv-aflibercept, FOLFIRI with Cetuximab, FOLFIRI + Panitumumab, FOLFIRI + Ramucirumab, FOLFOXIRI, FOLFIRI with FOLFOX6, FOLFOXIRI + Bevacizumab, FOLFOXIRI + Cetuximab, FOLFOXIRI + Panitumumab, Roswell Park Fluorouracil/Leucovorin, Roswell Park Fluorouracil/Leucovorin + Bevacizumab, Simplified Biweekly Infusional Fluorouracil/Leucovorin, Simplified Biweekly Infusional Fluorouracil/Leucovorin + Bevacizumab, and MOF (semustine (methyl-CCNU), vincrisine (Oncovin^{®}) and 5-FU). For a review of these therapies see Beaven and Goldberg (2006) Oncology 20(5):461-470 as well as www.cancertherapyadvisor.com/home/cancer-topics/gastrointestinal-cancers/gastrointestinal-cancers-treatment-regimens/colon-cancer-treatment-regimens/. Other chemotherapeutics can be added, e.g., oxaliplatin or irinotecan.

Capecitabine is a prodrug of (5-FU) that is converted to its active form by the tumor-specific enzyme PynPase following a pathway of three enzymatic steps and two intermediary metabolites, 5'-deoxy-5-fluorocytidine (5'-DFCR) and 5'-deoxy-5-fluorouridine (5'-DFUR). Capecitabine is marketed by Roche under the trade name Xeloda^{®}.

Leucovorin (Folinic acid) is an adjuvant used in cancer therapy. It is used in synergistic combination with 5-FU to improve efficacy of the chemotherapeutic agent. Without being bound by theory, addition of Leucovorin is believed to enhance efficacy of 5-FU by inhibiting thymidylate synthase. It has been used as an antidote to protect normal cells from high doses of the anticancer drug methotrexate and to increase the antitumor effects of fluorouracil (5-FU) and tegafur-uracil. It is also known as citrovorum factor and Wellcovorin. This compound has the chemical designation of L-Glutamic acid N-[4-[[(2-amino-5-formyl-1,4,5,6,7,8-hexahydro-4-oxo-6-pteridinyl)methyl]amino]benzoyl], calcium salt (1:1).

"Oxaliplatin" (Eloxatin) is a platinum-based chemotherapy drug in the same family as cisplatin and carboplatin. It is typically administered in combination with fluorouracil and leucovorin in a combination known as FOLFOX for the treatment of colorectal cancer. Compared to cisplatin, the two amine groups are replaced by cyclohexyldiamine for improved antitumor activity. The chlorine ligands are replaced by the oxalato bidentate derived from oxalic acid in order to improve water solubility. Equivalents to Oxaliplatin are known in the art and include, but are not limited to cisplatin, carboplatin, aroplatin, lobaplatin, nedaplatin, and JM-216 (see McKeage et al. (1997) J. Clin. Oncol. 201:1232-1237 and in general, Chemotherapy for Gynecological Neoplasm, Curr. Therapy and Novel Approaches, in the Series Basic and Clinical Oncology, Angioli et al. Eds., 2004).

"FOLFOX" is an abbreviation for a type of combination therapy that is used to treat cancer. This therapy includes leucovorin ("FOL"), 5-FU ("F"), and oxaliplatin ("OX") and encompasses various regimens, such as FOLFOX-4, FOLFOX-6, modified FOLOX-6, and FOLFOX-7, which vary in doses and ways in which each of the three drugs are administered. "FOLFIRI" is an abbreviation for a type of combination therapy that is used treat cancer and comprises, or alternatively consists essentially of, or yet further consists of 5-FU, leucovorin, and irinotecan. Information regarding these treatments are available on the National Cancer Institute's web site, cancer.gov, last accessed on May 30, 2020 as well as www.cancertherapyadvisor.com/home/cancer-topics/gastrointestinal-cancers/gastrointestinal-cancers-treatment-regimens/colon-cancer-treatment-regimens/, last accessed on May 30, 2020.

Irinotecan (CPT-11) is sold under the trade name of Camptosar. It is a semisynthetic analogue of the alkaloid camptothecin, which is activated by hydrolysis to SN-38 and targets topoisomerase I. Chemical equivalents are those that inhibit the interaction of topoisomerase I and DNA to form a catalytically active topoisomerase I-DNA complex. Chemical equivalents inhibit cell cycle progression at G2-M phase resulting in the disruption of cell proliferation.

S-1 consists of three agents (at a molar ratio of 1:0.4:1): tegafur, 5-chloro-2-4-dihydroxypyridine, and potassium oxonate.

The term "adjuvant" therapy refers to administration of a therapy or chemotherapeutic regimen to a patient in addition to the primary or initial treatment, such as after removal of a tumor by surgery. Adjuvant therapy is typically given to minimize or prevent a possible cancer reoccurrence. Alternatively, "neoadjuvant" therapy refers to administration of therapy or chemotherapeutic regimen before surgery, typically in an attempt to shrink the tumor prior to a surgical procedure to minimize the extent of tissue removed during the procedure. Additionally or alternatively, such adjuvant therapy potentials (i.e., sensitizes the subject to the original therapy) the subject may help reach one or more of clinical end points of the cancer treatment.

The phrase "first line" or "second line" or "third line" etc., refers to the order of treatment received by a patient. First line therapy regimens are treatments given first, whereas second or third line therapy are given after the first line therapy or after the second line therapy, respectively. The National Cancer Institute defines first line therapy as "the first treatment for a disease or condition. In patients with cancer, primary treatment can be surgery, chemotherapy, radiation therapy, or a combination of these therapies. First line therapy is also referred to those skilled in the art as primary therapy and primary treatment." See National Cancer Institute website as www.cancer.gov, last visited on May 1, 2008. Typically, a patient is given a subsequent chemotherapy regimen because the patient did not shown a positive clinical or sub-clinical response to the first line therapy or the first line therapy has stopped.

As used herein, the term "antifolate" intends a drug or biologic that impairs the function of folic acids, e.g., an antimetabolite agent that inhibits the use of a metabolite, i.e. another chemical that is part of normal metabolism. In cancer treatment, antimetabolites interfere with DNA production, thus cell division and growth of the tumor. Non-limiting examples of these agents are dihydrofolate reductase inhibitors, such as methotrexate, Aminopterin, and Pemetrexed; thymidylate synthase inhibitors, such as Raltitrexed or Pemetrexed; purine based, i.e. an adenosine deaminase inhibitor, such as Pentostatin, a thiopurine, such as Thioguanine and Mercaptopurine, a halogenated/ribonucleotide reductase inhibitor, such as Cladribine, Clofarabine, Fludarabine, or a guanine/guanosine: thiopurine, such as Thioguanine; or Pyrimidine based, i.e. cytosine/cytidine: hypomethylating agent, such as Azacitidine and Decitabine, a DNA polymerase inhibitor, such as Cytarabine, a ribonucleotide reductase inhibitor, such as Gemcitabine, or a thymine/thymidine: thymidylate synthase inhibitor, such as a Fluorouracil (5-FU).

In one aspect, the term "chemical equivalent" means the ability of the chemical to selectively interact with its target protein, DNA, RNA or fragment thereof as measured by the inactivation of the target protein, incorporation of the chemical into the DNA or RNA or other suitable methods. Chemical equivalents include, but are not limited to, those agents with the same or similar biological activity and include, without limitation a pharmaceutically acceptable salt or mixtures thereof that interact with and/or inactivate the same target protein, DNA, or RNA as the reference chemical.

The terms "oligonucleotide" or "polynucleotide" or "portion," or "segment" thereof refer to a stretch of polynucleotide residues which is long enough to use in PCR or various hybridization procedures to identify or amplify identical or related parts of mRNA or DNA molecules. The polynucleotide compositions of this invention include RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g., methyl phosphonates, phosphotriesters, phosphoamidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.). Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

When a genetic marker, e.g., over expression of dUTPase, is used as a basis for selecting a patient for a treatment described herein, the genetic marker is measured before and/or during treatment, and the values obtained are used by a clinician in assessing any of the following: (a) probable or likely suitability of an individual to initially receive treatment(s); (b) probable or likely unsuitability of an individual to initially receive treatment(s); (c) responsiveness to treatment; (d) probable or likely suitability of an individual to continue to receive treatment(s); (e) probable or likely unsuitability of an individual to continue to receive treatment(s); (f) adjusting dosage; (g) predicting likelihood of clinical benefits; or (h) toxicity. As would be well understood by one in the art, measurement of the genetic marker in a clinical setting is a clear indication that this parameter was used as a basis for initiating, continuing, adjusting and/or ceasing administration of the treatments described herein.

A damage-associated molecular pattern (DAMP), is also known as dangerassociated molecular patterns, danger signals, and alarmin, are host biomolecules that can initiate and perpetuate a noninfectious inflammatory response. As used herein, the term DAMP is able to initiate and perpetuate an inflammatory response to a cancer cell or to facilitating or promoting or accelerating treatment of a cancer cell to achieve one or more of clinical endpoints. One non-limiting example is "HMG domain," "high mobility group (HMG) box domain," or "HMGB" referring to an amino acid sequence that is involved in binding DNA (Stros et al., Cell Mol Life Sci. 64(19-20):2590-606 (2007)). In one embodiment, the structure of the HMG-box domain consists of three helices in an irregular array. In another embodiment, an HMG-box domain enables a protein to bind non-B-type DNA conformations (kinked or unwound) with high affinity. HMG-box domains can be found in high mobility group proteins, which are involved in the regulation of DNA-dependent processes such as transcription, replication and DNA repair, all of which require changing the conformation of chromatin (Thomas (2001) Biochem. Soc. Trans. 29(Pt 4):395-401). HMGB1 is a high mobility group box (HMGB) 1 protein that is reported to bind to and distort the minor groove of DNA. Recombinant or isolated protein and polypeptide are commercially available from Atgenglobal, ProSpecBio, Protein1 and Abnova.

HMG-box proteins are found in a variety of eukaryotic organisms and can be broadly divided into two groups, based on sequence-dependent and sequence-independent DNA recognition; the former usually contain one HMG-box motif, while the latter can contain multiple HMG-box motifs. Non-limiting examples of polypeptides comprising an HMG-box domain include HMG1(HMGB1), HMG2(HMGB2), HMGB3 and HMGB4 nonhistone components of chromatin; SRY (sex determining region Y protein) involved in differential gonadogenesis; the SOX family of transcription factors (Harley et al. (2003) Endocr. Rev. 24(4):466-87); sequence-specific LEF1 (lymphoid enhancer binding factor 1) and TCF-1 (T-cell factor 1) involved in regulation of organogenesis and thymocyte differentiation (Labbé et al. (2000) Proc. Natl. Acad. Sci. USA 97(15):8358-63); structurespecific recognition protein SSRP involved in transcription and replication; MTF1 mitochondrial transcription factor; nucleolar transcription factors UBF 1/2 (upstream binding factor) involved in transcription by RNA polymerase I; Abf2 yeast ARS-binding factor (Cho et al. (2001) Biochim. Biophys. Acta. 1522(3):175-86); yeast transcription factors lxr1, Rox1, Nhp6b and Spp41; mating type proteins (MAT) involved in the sexual reproduction of fungi (Barve et al. (2003) Fungal Genet. Biol. 39(2):151-67); and the YABBY plant-specific transcription factors. Exemplary sequences of polypeptides comprising an HMG-box domain include NP_002119 (human HMGB1), UniProtKB - P09429 (human HMGB1), NP_001124160 (human HMGB2), UniProtKB - P26583 (human HMGB2), NP_005333 (human HMGB3), UniProtKB - 015347 (human HMGB3), NP_660206 (human HMGB4), and UniProtKB - Q8WW32 (human HMGB4). In addition, polynucleotides encoding a HMG-box domain/ protein includes, but not limited to: a human HMGB1 transcript, such as NM_001313892.1, NM_001313893.1, NM_001363661.1, NM_001370339.1, NM_001370340.1, NM_001370341.1, and NM_002128.7; a human HMGB2 transcript, such as NM_001130688.1, NM_001130689.1, and NM_002129.4; a human HMGB3 transcript, such as NM_001301228.1, NM_001301229.2, NM_001301231.2, and NM_005342.4; and a human HMGB4 transcript, such as NM_001352984.2 and NM_145205.5.

### Methods

In one aspect, provided herein is a deoxyuridine triphosphatase (dUTPase) inhibitor, an inhibitor of thymidylate biosynthesis and an immunotherapy agent, for use in a method of enhancing a therapeutic efficacy of an immunotherapy agent in a subject in need thereof, wherein the method comprises administering to the subject an effective amount of the deoxyuridine triphosphatase (dUTPase) inhibitor, an effective amount of the inhibitor of thymidylate biosynthesis, and the immunotherapy agent, wherein the dUTPase inhibitor is 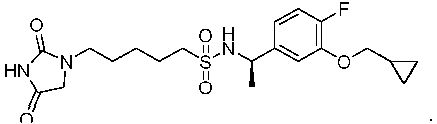 the inhibitor of thymidylate biosynthesis is 5-fluorouracil (5-FU);
and the immunotherapy agent is an anti-PD-1 (CD279) antibody, wherein the subject was administered or is concomitantly administered or will be administered the immunotherapy agent. In other embodiments, the method further comprises administering to the subject an effective amount of an inhibitor of folate-mediated one-carbon metabolism. In other embodiments, the method further comprises administering to the subject an effective amount of an anthracycline or other topoisomerase II inhibitor.

Also provided herein is a deoxyuridine triphosphatase (dUTPase) inhibitor for use in a method of enhancing a therapeutic efficacy of an immunotherapy agent in a subject in need thereof; wherein the method comprises administering to the subject an effective amount of the deoxyuridine triphosphatase (dUTPase) inhibitor, an effective amount of an inhibitor of thymidylate biosynthesis, and an immunotherapy agent, wherein the dUTPase inhibitor is 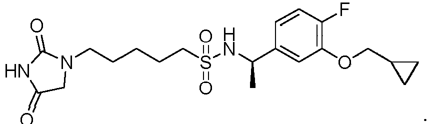 the inhibitor of thymidylate biosynthesis is 5-fluorouracil (5-FU);
and the immunotherapy agent is an anti-PD-1 (CD279) antibody, wherein the subject was administered or is concomitantly administered or will be administered the immunotherapy agent. In other embodiments, the method further comprises administering to the subject an effective amount of an inhibitor of folate-mediated one-carbon metabolism. In other embodiments, the method further comprises administering to the subject an effective amount of an anthracycline or other topoisomerase II inhibitor.

Also provided herein is an inhibitor of thymidylate biosynthesis for use in a method of enhancing a therapeutic efficacy of an immunotherapy agent in a subject in need thereof; wherein the method comprises administering to the subject an effective amount of a deoxyuridine triphosphatase (dUTPase) inhibitor, an effective amount of the inhibitor of thymidylate biosynthesis, and an immunotherapy agent, wherein the dUTPase inhibitor is 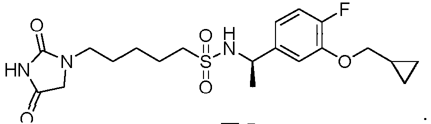 the inhibitor of thymidylate biosynthesis is 5-fluorouracil (5-FU);
and the immunotherapy agent is an anti-PD-1 (CD279) antibody, wherein the subject was administered or is concomitantly administered or will be administered the immunotherapy agent. In other embodiments, the method further comprises administering to the subject an effective amount of an inhibitor of folate-mediated one-carbon metabolism. In other embodiments, the method further comprises administering to the subject an effective amount of an anthracycline or other topoisomerase II inhibitor.

Also provided herein is an immunotherapy agent for use in a method of enhancing a therapeutic efficacy of the immunotherapy agent in a subject in need thereof; wherein the method comprises administering to the subject an effective amount of a deoxyuridine triphosphatase (dUTPase) inhibitor, an effective amount of an inhibitor of thymidylate biosynthesis, and the immunotherapy agent, wherein the dUTPase inhibitor is 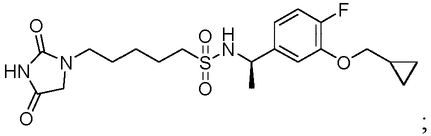 the inhibitor of thymidylate biosynthesis is 5-fluorouracil (5-FU); and the immunotherapy agent is an anti-PD-1 (CD279) antibody, wherein the subject was administered or is concomitantly administered or will be administered the immunotherapy agent. In other embodiments, the method further comprises administering to the subject an effective amount of an inhibitor of folate-mediated one-carbon metabolism. In other embodiments, the method further comprises administering to the subject an effective amount of an anthracycline or other topoisomerase II inhibitor.

The invention is defined in the claims. Embodiments which are outside the scope of the claims are described for context only.

In another aspect, provided herein is a method of enhancing a therapeutic efficacy of an immunotherapy agent in a subject in need thereof, comprising, consisting essentially of, or consisting of administering to the subject an effective amount of a dUTPase inhibitor and the immunotherapy agent, and an effective amount of an inhibitor of thymidylate biosynthesis.

In another aspect, provided herein is a method of enhancing a therapeutic efficacy of an immunotherapy agent in a subject in need thereof, comprising, consisting essentially of, or consisting of administering to the subject an effective amount of a dUTPase inhibitor and the immunotherapy agent, and an effective amount of an inhibitor of folate-mediated one-carbon metabolism.

In another aspect, provided herein is a method of enhancing a therapeutic efficacy of an immunotherapy agent in a subject in need thereof, comprising, consisting essentially of, or consisting of administering to the subject an effective amount of a dUTPase inhibitor and the immunotherapy agent, and an effective amount of an anthracycline or other topoisomerase II inhibitor.

Additionally or alternatively, provided herein is a method of enhancing a therapeutic efficacy of an inhibitor of thymidylate biosynthesis in a subject in need thereof, comprising, consisting essentially of, or consisting of administering to the subject an effective amount of a deoxyuridine triphosphatase (dUTPase) inhibitor, wherein the subject was administered or is concomitantly administered or will be administered with the inhibitor of thymidylate biosynthesis. In some embodiments, the method further comprises administering to the subject one or more selected from an effective amount of an immunotherapy agent, and an effective amount of an anthracycline or other topoisomerase II inhibitor. In other embodiments, the method further comprises administering to the subject an effective amount of an immunotherapy agent. In other embodiments, the method further comprises administering to the subject an effective amount of an inhibitor of folate-mediated one-carbon metabolism. In other embodiments, the method further comprises administering to the subject an effective amount of an anthracycline or other topoisomerase II inhibitor.

Additionally or alternatively, provided herein is a method of enhancing a therapeutic efficacy of an immunotherapy agent combined with an inhibitor of thymidylate biosynthesis in a subject in need thereof, comprising, consisting essentially of, or consisting of administering to the subject an effective amount of a deoxyuridine triphosphatase (dUTPase) inhibitor, wherein the subject was administered or is concomitantly administered or will be administered with the immunotherapy agent and the inhibitor of thymidylate biosynthesis. In some embodiments, the method further comprises administering to the subject one or more selected from an effective amount of an inhibitor of folate-mediated one-carbon metabolism, and an effective amount of an anthracycline or other topoisomerase II inhibitor. In other embodiments, the method further comprises administering to the subject an effective amount of an inhibitor of folate-mediated one-carbon metabolism. In other embodiments, the method further comprises administering to the subject an effective amount of an anthracycline or other topoisomerase II inhibitor.

In some embodiments, the therapeutic efficacy of the immunotherapy agent and/or an inhibitor of thymidylate biosynthesis is enhanced by at least about 10%, or alternatively at least about 20%, or alternatively at least about 30%, or alternatively at least about 40%, or alternatively at least about 50%, or alternatively at least about 60%, or alternatively at least about 70%, or alternatively at least about 80%, or alternatively at least about 90%, or alternatively at least about 1-fold, or alternatively at least about 1.1-fold, or alternatively at least about 1.2-fold, or alternatively at least about 1.3-fold, or alternatively at least about 1.4-fold, or alternatively at least about 1.5-fold, or alternatively at least about 1.6-fold, or alternatively at least about 1.7-fold, or alternatively at least about 1.8-fold, or alternatively at least about 1.9-fold, or alternatively at least about 2-fold versus administration of the immunotherapy agent and/or an inhibitor of thymidylate biosynthesis without the enhancement. This also includes 2-, 3-, 4-, 5-, 6-, 7-, 8-, 9-, 10-, 11-, 12-, 13-, 14-, 15-, 16-, 17-, 18-, 19-, 20-, 25-, 30-, 35-, 40-, 45-, 50-, 55-, 60-, 65-, 70-, 75-, 80-, 85-, 90-, 95-, or 100-fold or more, including increments therein, of enhancement of therapeutic efficacy of the immunotherapy agent versus administration of the immunotherapy agent as monotherapy.

In certain embodiments, a therapeutic efficacy of a treatment refers to achieving one or more of clinical endpoints, optionally selected from the following:
(1) elimination of a cancer in a subject or in a tissue/organ of the subject or in a cancer loci;
(2) reduction in tumor burden (such as number of cancer cells, number of cancer foci, number of cancer cells in a foci, size of a solid cancer, concentrate of a liquid cancer in the body fluid, and/or amount of cancer in the body);
(3) stabilizing or delay or slowing or inhibition of cancer growth and/or development, including but not limited to, cancer cell growth and/or division, size growth of a solid tumor or a cancer loci, cancer progression, and/or metastasis (such as time to form a new metastasis, number of total metastases, size of a metastasis, as well as variety of the tissues/organs to house metastatic cells);
(4) less risk of having a cancer growth and/or development;
(5) inducing an immune response of the patient to the cancer, such as higher number of tumor-infiltrating immune cell, higher number of activated immune cells, or higher number cancer cell expressing an immunotherapy target, or higher level of expression of an immunotherapy target in a cancer cell; and
(6) higher probability of survival and/or increased duration of survival, such as increased overall survival (OS, which may be shown as 1-year, 2-year, 5-year, 10-year, or 20-year survival rate), increased progression free survival (PFS), increased disease free survival (DFS), increased time to tumor recurrence (TTR) and increased time to tumor progression (TTP).

In certain embodiments, enhancing a therapeutic efficacy refers to achieving one or more of clinical endpoints of treatment to a greater extend and/or in a faster speed and/or using less time, optionally compared to the treatment without the enhancement method/step. Additionally or alternatively, enhancing a therapeutic efficacy also refers to achieving more clinical endpoints, optionally compared to the treatment without the enhancement method/step.

Methods and tools for measuring such therapeutic efficacy is known to one of skill in the art, including measuring a clinical endpoint in a human patient and/or in an animal/tissue/cell model mimicking a patient having a cancer. For example, therapeutic efficacy may be monitored by CT scan or blood work analysis. In addition, tumor markers may be assessed. Non-limiting experimental settings can be found in the Examples.

In another aspect, provided herein is a method of treating cancer in a subject in need thereof, the method comprising, consisting essentially of, or consisting of administering to the subject an effective amount of a dUTPase inhibitor and an effective amount of an immunotherapy agent. In some embodiments, the method further comprises administering to the subject one or more selected from an effective amount of an inhibitor of thymidylate biosynthesis, and an effective amount of an anthracycline or other topoisomerase II inhibitor.

In another aspect, provided herein is a method of treating cancer in a subject in need thereof, the method comprising, consisting essentially of, or consisting of administering to the subject an effective amount of a dUTPase inhibitor, an effective amount of an immunotherapy agent, and one or more selected from an effective amount of an inhibitor of thymidylate biosynthesis, and an effective amount of an anthracycline or other topoisomerase II inhibitor.

In another aspect, provided herein is a method of treating cancer in a subject in need thereof, the method comprising, consisting essentially of, or consisting of administering to the subject an effective amount of a dUTPase inhibitor, an effective amount of an immunotherapy agent, and an effective amount of an inhibitor of thymidylate biosynthesis.

In another aspect, provided herein is a method of treating cancer in a subject in need thereof, the method comprising, consisting essentially of, or consisting of administering to the subject an effective amount of a dUTPase inhibitor, an effective amount of an immunotherapy agent, and an effective amount of an inhibitor of folate-mediated one-carbon metabolism.

In another aspect, provided herein is a method of treating cancer in a subject in need thereof, the method comprising, consisting essentially of, or consisting of administering to the subject an effective amount of a dUTPase inhibitor, an effective amount of an immunotherapy agent, and an effective amount of an anthracycline or other topoisomerase II inhibitor.

In yet another aspect, provided herein is a method of treating cancer in a subject in need thereof, the method comprising, consisting essentially of, or consisting of administering to the subject an effective amount of a dUTPase inhibitor and an effective amount of an inhibitor of thymidylate biosynthesis. In some embodiments, the method further comprises administering to the subject one or more selected from an effective amount of an immunotherapy agent, and an effective amount of an anthracycline or other topoisomerase II inhibitor.

In some embodiments, the subject after treatment experiences one or more clinical endpoints as disclosed herein. In one embodiment, the endpoints are selected from tumor response, reduction in tumor size, reduction in tumor burden, increase in overall survival, increase in progression free survival, and inhibiting metastasis.

In some embodiments, the cancer is selected from cancers of the: circulatory system, for example, heart (sarcoma [angiosarcoma, fibrosarcoma, rhabdomyosarcoma, liposarcoma], myxoma, rhabdomyoma, fibroma, lipoma and teratoma), mediastinum and pleura, and other intrathoracic organs, vascular tumors and tumor-associated vascular tissue; respiratory tract, for example, nasal cavity and middle ear, accessory sinuses, larynx, trachea, bronchus and lung such as small cell lung cancer (SCLC), non-small cell lung cancer (NSCLC), bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; gastrointestinal system, for example, esophagus (squamous cell carcinoma, adenocarcinoma, leiomyosarcoma, lymphoma), stomach (carcinoma, lymphoma, leiomyosarcoma), gastric, pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); gastrointestinal stromal tumors and neuroendocrine tumors arising at any site; genitourinary tract, for example, kidney (adenocarcinoma, Wilm's tumor [nephroblastoma], lymphoma, leukemia), bladder and/or urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); liver, for example, hepatoma (hepatocellular carcinoma), cholangiocarcinoma, hepatoblastoma, angiosarcoma, hepatocellular adenoma, hemangioma, pancreatic endocrine tumors (such as pheochromocytoma, insulinoma, vasoactive intestinal peptide tumor, islet cell tumor and glucagonoma); bone, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; nervous system, for example, neoplasms of the central nervous system (CNS), primary CNS lymphoma, skull cancer (osteoma, hemangioma, granuloma, xanthoma, osteitis deformans), meninges (meningioma, meningiosarcoma, gliomatosis), brain cancer (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); reproductive system, for example, gynecological, uterus (endometrial carcinoma), cervix (cervical carcinoma, pre- tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma) and other sites associated with female genital organs; placenta, penis, prostate, testis, and other sites associated with male genital organs; hematologic system, for example, blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; oral cavity, for example, lip, tongue, gum, floor of mouth, palate, and other parts of mouth, parotid gland, and other parts of the salivary glands, tonsil, oropharynx, nasopharynx, pyriform sinus, hypopharynx, and other sites in the lip, oral cavity and pharynx; skin, for example, malignant melanoma, cutaneous melanoma, basal cell carcinoma, squamous cell carcinoma, Karposi's sarcoma, moles dysplastic nevi, lipoma, angioma, dermatofibroma, and keloids; and other tissues comprising connective and soft tissue, retroperitoneum and peritoneum, eye, intraocular melanoma, and adnexa, breast, head or/and neck, anal region, thyroid, parathyroid, adrenal gland and other endocrine glands, secondary and unspecified malignant neoplasm of lymph nodes, secondary malignant neoplasm of respiratory and digestive systems and secondary malignant neoplasm of other sites. Additionally or alternatively, the cancer is a solid tumor or a liquid cancer. In some embodiments, the cancer is a primary cancer. In another embodiment, the cancer is metastatic.

In some embodiments, the cancer comprises, consists essentially of, or consists of a carcinoma, a sarcoma, a myeloma, a leukemia, or a lymphoma. In some embodiments, the cancer comprises, consists essentially of, or consists of a carcinoma. In some embodiments, the cancer comprises, consists essentially of, or consists of a sarcoma. In some embodiments, the cancer comprises, consists essentially of, or consists of a myeloma. In some embodiments, the cancer comprises, consists essentially of, or consists of a leukemia. In some embodiments, the cancer comprises, consists essentially of, or consists of a lymphoma.

In another aspect, provided herein is a method of inhibiting growth of a cancer cell comprising, consisting essentially of, or consisting of contacting the cell with an effective amount of a dUTPase inhibitor and an effective amount of an immunotherapy agent. In some embodiments, the method further comprises contacting the cell with one or more selected from an effective amount of an inhibitor of thymidylate biosynthesis, and an effective amount of an anthracycline or other topoisomerase II inhibitor. In some embodiments, the contacting occurs *in vitro.* In some embodiments, the contacting occurs *in vivo.*

In another aspect, provided herein is a method of inhibiting growth of a cancer cell comprising, consisting essentially of, or consisting of contacting the cell with an effective amount of a dUTPase inhibitor, an effective amount of an immunotherapy agent, and one or more selected from an effective amount of an inhibitor of thymidylate biosynthesis, and an effective amount of an anthracycline or other topoisomerase II inhibitor. In some embodiments, the contacting occurs *in vitro.* In some embodiments, the contacting occurs *in vivo.*

In another aspect, provided herein is a method of inhibiting growth of a cancer cell comprising, consisting essentially of, or consisting of contacting the cell with an effective amount of a dUTPase inhibitor, an effective amount of an immunotherapy agent, and an effective amount of an inhibitor of thymidylate biosynthesis. In some embodiments, the contacting occurs *in vitro.* In some embodiments, the contacting occurs *in vivo.*

In another aspect, provided herein is a method of inhibiting growth of a cancer cell comprising, consisting essentially of, or consisting of contacting the cell with an effective amount of a dUTPase inhibitor, an effective amount of an immunotherapy agent, and an effective amount of an inhibitor of folate-mediated one-carbon metabolism. In some embodiments, the contacting occurs *in vitro.* In some embodiments, the contacting occurs *in vivo.*

In another aspect, provided herein is a method of inhibiting growth of a cancer cell comprising, consisting essentially of, or consisting of contacting the cell with an effective amount of a dUTPase inhibitor, an effective amount of an immunotherapy agent, and an effective amount of an anthracycline or other topoisomerase II inhibitor. In some embodiments, the contacting occurs *in vitro.* In some embodiments, the contacting occurs *in vivo.*

In another aspect, provided herein is a method of inhibiting growth of a cancer cell comprising, consisting essentially of, or consisting of contacting the cell with an effective amount of a dUTPase inhibitor and an effective amount of an inhibitor of thymidylate biosynthesis. In some embodiments, the method further comprises contacting the cell with one or more selected from an effective amount of an immunotherapy agent, and an effective amount of an anthracycline or other topoisomerase II inhibitor. In some embodiments, the contacting occurs *in vitro.* In some embodiments, the contacting occurs *in vivo.*

In some embodiments, the cancer cell is a primary cell isolated from a biopsy or cultured cancer cell that is cultured in the lab or obtained from a commercial vendor such as the American Type Culture Collection (ATCC), or a cancer cell in an animal model for evaluating therapeutic efficacy of potential therapies.

In some embodiments, inhibition of growth of the cancer cell is measured by comparing growth of a cancer cell after contacting the cell with the dUTPase inhibitor, the immunotherapy agent, and one or more selected from the inhibitor of thymidylate biosynthesis, the anthracycline or other topoisomerase II inhibitor with growth of a cancer cell without any such contact (i.e., growth of a control sample). Methods and assays for detecting and/or quantifying the growth are known to one skilled in the art.

In another aspect, provided herein is a method for one or more of:
a. stimulating cytoplasmic DNA release in a cancer cell;
b. decreasing expression or activity of an inhibitory immune checkpoint molecule (such as PD-L1) in a cancer cell that expresses the inhibitory immune checkpoint molecule;
c. inducing release or expression of a damage-associated molecule pattern (DAMP) (such as any one or more of ATP, calreticulin, HMGB1, HMGB2, HMGB3, and HMGB4) from a cancer cell,
the method comprising, consisting essentially of, or consisting of contacting the cancer cell with an effective amount of a dUTPase inhibitor; and one or more selected from an effective amount of an inhibitor of thymidylate biosynthesis, and an effective amount of an anthracycline or other topoisomerase II inhibitor.

In another aspect, provided herein is a method for one or more of:
a. stimulating cytoplasmic DNA release in a cancer cell;
b. decreasing expression or activity of an inhibitory immune checkpoint molecule (such as PD-L1) in a cancer cell that expresses the inhibitory immune checkpoint molecule;
c. inducing release or expression of a DAMP (such as any one or more of ATP, calreticulin, HMGB 1, HMGB2, HMGB3, and HMGB4) from a cancer cell,
the method comprising, consisting essentially of, or consisting of contacting the cancer cell with an effective amount of a dUTPase inhibitor; and an effective amount of an inhibitor of thymidylate biosynthesis.

In another aspect, provided herein is a method for one or more of:
a. stimulating cytoplasmic DNA release in a cancer cell;
b. decreasing expression or activity of an inhibitory immune checkpoint molecule (such as PD-L1) in a cancer cell that expresses the inhibitory immune checkpoint molecule;
c. inducing release or expression of a DAMP (such as any one or more of ATP, calreticulin, HMGB 1, HMGB2, HMGB3, and HMGB4) from a cancer cell,
the method comprising, consisting essentially of, or consisting of contacting the cancer cell with an effective amount of a dUTPase inhibitor; and an effective amount of an inhibitor of folate-mediated one-carbon metabolism.

In another aspect, provided herein is a method for one or more of:
a. stimulating cytoplasmic DNA release in a cancer cell;
b. decreasing expression or activity of an inhibitory immune checkpoint molecule (such as PD-L1) in a cancer cell that expresses the inhibitory immune checkpoint molecule;
c. inducing release or expression of a DAMP (such as any one or more of ATP, calreticulin, HMGB 1, HMGB2, HMGB3, and HMGB4) from a cancer cell,
the method comprising, consisting essentially of, or consisting of contacting the cancer cell with an effective amount of a dUTPase inhibitor; and an effective amount of an anthracycline or other topoisomerase II inhibitor.

In another aspect, provided herein is a method for one or more of:
a. stimulating cytoplasmic DNA release in a cancer cell;
b. increasing expression or activity of a stimulatory immune checkpoint molecule in a cancer cell that expresses the stimulatory immune checkpoint molecule;
c. inducing release or expression of a damage-associated molecule pattern (DAMP) (such as any one or more of ATP, calreticulin, HMGB1, HMGB2, HMGB3, and HMGB4) from a cancer cell,
the method comprising, consisting essentially of, or consisting of contacting the cancer cell with an effective amount of a dUTPase inhibitor; and one or more selected from an effective amount of an inhibitor of thymidylate biosynthesis, and an effective amount of an anthracycline or other topoisomerase II inhibitor.

In another aspect, provided herein is a method for one or more of:
a. stimulating cytoplasmic DNA release in a cancer cell;
b. increasing expression or activity of a stimulatory immune checkpoint molecule in a cancer cell that expresses the stimulatory immune checkpoint molecule;
c. inducing release or expression of a DAMP (such as any one or more of ATP, calreticulin, HMGB 1, HMGB2, HMGB3, and HMGB4) from a cancer cell,
the method comprising, consisting essentially of, or consisting of contacting the cancer cell with an effective amount of a dUTPase inhibitor; and an effective amount of an inhibitor of thymidylate biosynthesis.

In another aspect, provided herein is a method for one or more of:
a. stimulating cytoplasmic DNA release in a cancer cell;
b. increasing expression or activity of a stimulatory immune checkpoint molecule in a cancer cell that expresses the stimulatory immune checkpoint molecule;
c. inducing release or expression of a DAMP (such as any one or more of ATP, calreticulin, HMGB 1, HMGB2, HMGB3, and HMGB4) from a cancer cell,
the method comprising, consisting essentially of, or consisting of contacting the cancer cell with an effective amount of a dUTPase inhibitor; and an effective amount of an inhibitor of folate-mediated one-carbon metabolism.

In another aspect, provided herein is a method for one or more of:
a. stimulating cytoplasmic DNA release in a cancer cell;
b. increasing expression or activity of a stimulatory immune checkpoint molecule in a cancer cell that expresses the stimulatory immune checkpoint molecule;
c. inducing release or expression of a DAMP (such as any one or more of ATP, calreticulin, HMGB 1, HMGB2, HMGB3, and HMGB4) from a cancer cell,
the method comprising, consisting essentially of, or consisting of contacting the cancer cell with an effective amount of a dUTPase inhibitor; and an effective amount of an anthracycline or other topoisomerase II inhibitor.

Methods and assays for detecting and/or quantifying the released DNA, the expression of an inhibitory or stimulatory immune checkpoint molecule, or the expression of a DAMP can be performed by one of skill in the art, for example using a nucleotide molecule capable of hybridizing to a released DNA and/or an antibody specifically binding to a checkpoint or DAMP. Such methods include but are not limited to, an immunoassay, a southern blot, a polymerase chain reaction (PCR), a quantitative PCR, a DNA sequence, a western blot, an enzyme-linked immunosorbent assay (ELISA), or lateral flow strips (also known as lateral flow devices).

In some embodiments, the DAMP comprises ATP, calreticulin, HMGB 1, HMGB2, HMGB3, and HMGB4. In some embodiments, the DAMP comprises HMGB1, HMGB2, HMGB3, HMGB4, or functional equivalents thereof. In some embodiments, the DAMP comprises HMGB1 or functional equivalents thereof. In some embodiments, the DAMP comprises HMGB2 or functional equivalents thereof. In some embodiments, the DAMP comprises HMGB3 or functional equivalents thereof. In some embodiments, the DAMP comprises HMGB4 or functional equivalents thereof. In some embodiments, the DAMP comprises ATP. In some embodiments, the DAMP comprises calreticulin.

In some embodiments, the cancer cell is a cell of a cancer selected from cancers of the: circulatory system, for example, heart (sarcoma [angiosarcoma, fibrosarcoma, rhabdomyosarcoma, liposarcoma], myxoma, rhabdomyoma, fibroma, lipoma and teratoma), mediastinum and pleura, and other intrathoracic organs, vascular tumors and tumor-associated vascular tissue; respiratory tract, for example, nasal cavity and middle ear, accessory sinuses, larynx, trachea, bronchus and lung such as small cell lung cancer (SCLC), non-small cell lung cancer (NSCLC), bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; gastrointestinal system, for example, esophagus (squamous cell carcinoma, adenocarcinoma, leiomyosarcoma, lymphoma), stomach (carcinoma, lymphoma, leiomyosarcoma), gastric, pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); gastrointestinal stromal tumors and neuroendocrine tumors arising at any site; genitourinary tract, for example, kidney (adenocarcinoma, Wilm's tumor [nephroblastoma], lymphoma, leukemia), bladder and/or urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); liver, for example, hepatoma (hepatocellular carcinoma), cholangiocarcinoma, hepatoblastoma, angiosarcoma, hepatocellular adenoma, hemangioma, pancreatic endocrine tumors (such as pheochromocytoma, insulinoma, vasoactive intestinal peptide tumor, islet cell tumor and glucagonoma); bone, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; nervous system, for example, neoplasms of the central nervous system (CNS), primary CNS lymphoma, skull cancer (osteoma, hemangioma, granuloma, xanthoma, osteitis deformans), meninges (meningioma, meningiosarcoma, gliomatosis), brain cancer (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); reproductive system, for example, gynecological, uterus (endometrial carcinoma), cervix (cervical carcinoma, pre- tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma) and other sites associated with female genital organs; placenta, penis, prostate, testis, and other sites associated with male genital organs; hematologic system, for example, blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; oral cavity, for example, lip, tongue, gum, floor of mouth, palate, and other parts of mouth, parotid gland, and other parts of the salivary glands, tonsil, oropharynx, nasopharynx, pyriform sinus, hypopharynx, and other sites in the lip, oral cavity and pharynx; skin, for example, malignant melanoma, cutaneous melanoma, basal cell carcinoma, squamous cell carcinoma, Karposi's sarcoma, moles dysplastic nevi, lipoma, angioma, dermatofibroma, and keloids; and other tissues comprising connective and soft tissue, retroperitoneum and peritoneum, eye, intraocular melanoma, and adnexa, breast, head or/and neck, anal region, thyroid, parathyroid, adrenal gland and other endocrine glands, secondary and unspecified malignant neoplasm of lymph nodes, secondary malignant neoplasm of respiratory and digestive systems and secondary malignant neoplasm of other sites. Additionally or alternatively, the cancer is a solid tumor or a liquid cancer. In some embodiments, the cancer is a primary cancer. In another embodiment, the cancer is a metastasis.

In some embodiments, the cancer cell is from a carcinoma, a sarcoma, a myeloma, a leukemia, or a lymphoma. In some embodiments, the cancer cell is from a carcinoma. In some embodiments, the cancer cell is from a sarcoma. In some embodiments, the cancer cell is from a myeloma. In some embodiments, the cancer cell is from a leukemia. In some embodiments, the cancer cell is from a lymphoma.

In some embodiments, any method or steps/embodiments of a method as disclosed herein may be further combined with another anti-cancer therapy, such as chemotherapy other than those specified herein, radiation therapy, surgery and others. Other combined therapy may include but not limited to: oncolytic virus infecting and killing a cancer cell (such as an oncolytic HSV), an antisense oligonucleotide (ASO) killing or damaging a cancer cell, a RNA interference (RNAi) killing or damaging a cancer cell, a Clustered Regularly Interspaced Short Palindromic Repeat (CRISPR) system killing or damaging a cancer cell, an exosome killing or damaging a cancer cell, and a vector delivering each thereof.

### Immunotherapy agent

In accordance with the invention, the immunotherapy agent for use, as defined in the claims, is an anti-PD-1 (CD279) antibody. Other embodiments are described for context only. In some embodiments, the immunotherapy agent comprises, consists essentially of, or consists of one or more selected from monoclonal antibodies (such as a monospecific, bispecific or multispecific antibody recognizing a tumor-specific antigen and/or an immune checkpoint), antibody-drug conjugates (e.g., recognizing a tumor-specific antigen and/or an immune checkpoint wherein the conjugated drug kill or damage a cancer cell expressing the tumor-specific antigen and/or inhibit an inhibitory immune checkpoint and/or active a stimulating immune checkpoint), a CAR therapy, a cell therapy(e.g., transplanting an anti-cancer immune cell optionally amplified and/or activated in vivo, or administering an immune cell expressing a chimeric antigen receptor (CAR)), immune regulators, cancer vaccines, an inhibitor or antagonist of an inhibitory immune checkpoint (referred to herein as a "checkpoint inhibitor", such as a chemical substance, an antisense oligonucleotide (ASO), a RNA interference (RNAi), a Clustered Regularly Interspaced Short Palindromic Repeat (CRISPR) system, a vector delivering each thereof), an activator or agonist of a stimulatory immune checkpoint (such as an activating ligand). In some embodiments, the immunotherapy agent comprises, consists essentially of, or consists of one or more monoclonal antibodies, bispecific antibodies and antibody fragments. In one embodiment, the immunotherapy agent comprises, consists essentially of, or consists of one or more of bispecific antibodies specifically binding to a tumor-specific antigen and engages an immune cell, such as a bispecific T-cell engager, a bispecific NK-cell engager, a bispecific NKT-cell engager, a bispecific gamma-delta T-cell engager, and a bispecific cytotoxic T-cell engager. In some embodiments, the immunotherapy agent comprises, consists essentially of, or consists of one or more antibody-drug conjugates. In some embodiments, the immunotherapy agent comprises, consists essentially of, or consists of one or more CAR cell therapy, such as administration of an immune cell expressing a CAR, including but not limited to CAR T cells, CAR NK cells, CAR NKT cells, CAR CD8+ T cells, CAR cytotoxic T cells, CAR gamma-delta T cells. In some embodiments, the immunotherapy agent comprises, consists essentially of, or consists of one or more cancer vaccines, such as a polypeptide or a polynucleotide mimicking a tumor-specific antigen and capable of inducing an immune response to the antigen in a subject. In some embodiments, the immunotherapy agent comprises, consists essentially of, or consists of one or more oncolytic virus therapy, such as a viral vector specifically infecting and optionally duplicating in a cancer cell and delivering an immunotherapy agent to the cancer cell. In one embodiment, the oncolytic virus is an HSV, optionally selected from HSV-1 and HSV-2. In a further embodiment, the oncolytic virus increases the expression optionally on the cell surface of a tumor-specific antigen in a cancer cell; and/or reduces the expression and/or activity of an inhibitory immune checkpoint in a cancer cell; and/or increases the expression and/or activity of a stimulatory immune checkpoint in a cancer cell.

Non-limiting examples of monoclonal antibodies include rituximab, blinatumomab, alemtuzumab, ibritumomab tiuxetan, bevacizumab, bevacizumab-awwb, cetuximab, panitumumab, ofatumumab, denosumab, pertuzumab, obinutuzumab, elotuzumab, ramucirumab, dinutuximab, daratumumab, trastuzumab, trastuzumab-dkst, nivolumab, pembrolizumab, cemiplimab, spartalizumab, camrelizumab, sintilimab, tislelizumab, toripalimab, AMF 514 (MEDI0680), balstilimab, avelumab, durvalumab, atezolizumab, ipilimumab, tremelimumab, zalifrelimab, and AGEN1181. In some embodiments, the monoclonal antibody is combined with another agent. For example, rituximab may be formulated with hyaluronidase human.

Non-limiting examples of antibody-drug conjugates include moxetumomab pasudotox-tdfk, brentuximab vedotin, trastuzumab emtansine, inotuzumab ozogamicin, gemtuzumab ozogamicin, tagraxofusp-erzs, polatuzumab vedotin-piiq, enfortumab vedotin-ejfv, trastuzumab deruxtecan, and sacituzumab govitecan-hziy.

Non-limiting examples of CAR T-cell therapy include tisagenlecleucel and axicabtagene ciloleucel.

Non-limiting examples of immune regulators include interleukins, aldesleukin, interferon alfa-2a/2b, pexidartinib, erythropoietin, granulocyte-macrophage colony-stimulating factor (GM-CSF), granulocyte colony-stimulating factor (G-CSF), thalidomide, lenalidomide, pomalidomide, and imiquimod.

Non-limiting examples of cancer vaccines include BCG live (THERACYS^{®}) or sipuleucel-T (PROVENGE^{®}).

Non-limiting examples of oncolytic virus therapy include oncorine (H101) and talimogene laherparepvec (IMLYGIC^{®}).

In some embodiments, the immunotherapy agent comprises, consists essentially of, or consists of a checkpoint inhibitor.

In some embodiments, the checkpoint inhibitor comprises, consists essentially of, or consists of a non-antibody agent. In some embodiments, the checkpoint inhibitor comprises, consists essentially of, or consists of GS4224, AMP-224, CA-327, CA-170, BMS-1001, BMS-1166, peptide-57, M7824, MGD013, CX-072, UNP-12, NP-12, or a combination of two or more thereof.

In some embodiments, the checkpoint inhibitor comprises, consists essentially of, or consists of one or more selected from an anti-PD-1 agent, an anti-PD-L1 agent, an anti-CTLA-4 agent, an anti-LAG-3 agent, an anti-TIM-3 agent, an anti-TIGIT agent, an anti-VISTA agent, an anti-B7-H3 agent, an anti-BTLA agent, an anti-ICOS agent, an anti-GITR agent, an anti-4-1BB agent, an anti-OX40 agent, an anti-CD27 agent, an anti-CD28 agent, an anti-CD40 agent, and an anti-Siglec-15 agent. In some embodiments, the anti-PD-1 agent, the anti-PD-L1 agent, the anti-CTLA-4 agent, the anti-LAG-3 agent, the anti-TIM-3 agent, the anti-TIGIT agent, the anti-VISTA agent, the anti-B7-H3 agent, the anti-BTLA agent, the anti-ICOS agent, the anti-GITR agent, the anti-4-1BB agent, the anti-OX40 agent, the anti-CD27 agent, the anti-CD28 agent, the anti-CD40 agent, or the anti-Siglec-15 agent is an antagonist. In some embodiments, the anti-PD-1 agent, the anti-PD-L1 agent, the anti-CTLA-4 agent, the anti-LAG-3 agent, the anti-TIM-3 agent, the anti-TIGIT agent, the anti-VISTA agent, the anti-B7-H3 agent, the anti-BTLA agent, the anti-ICOS agent, the anti-GITR agent, the anti-4-1BB agent, the anti-OX40 agent, the anti-CD27 agent, the anti-CD28 agent, the anti-CD40 agent, or the anti-Siglec-15 agent is an agonist. In some embodiments, the anti-PD-1 agent, the anti-PD-L1 agent, the anti-CTLA-4 agent, the anti-LAG-3 agent, the anti-TIM-3 agent, the anti-TIGIT agent, the anti-VISTA agent, the anti-B7-H3 agent, the anti-BTLA agent, the anti-ICOS agent, the anti-GITR agent, the anti-4-1BB agent, the anti-OX40 agent, the anti-CD27 agent, the anti-CD28 agent, the anti-CD40 agent, or the anti-Siglec-15 agent is an inhibitor. In some embodiments, the anti-LAG-3 agent comprises, consists essentially of, or consists of AK104, KN046, eftilagimod alpha, relatlimab, LAG525, MK-4280, REGN3767, TSR-033, BI754111, Sym022, FS118, or MGD013. In some embodiments, the anti-TIM-3 agent comprises, consists essentially of, or consists of CA-327, TSR-022, MBG453, Sym023, INCAGN2390, LY3321367, BMS-986258, SHR-1702, or RO7121661. In some embodiments, the anti-TIGIT agent comprises, consists essentially of, or consists of MK-7684, etigilimab, tiragolumab, BMS-986207, AB-154, or ASP-8374. In some embodiments, the anti-VISTA agent comprises, consists essentially of, or consists of JNJ-61610588 or CA-170. In some embodiments, the anti-B7-H3 agent comprises, consists essentially of, or consists of enoblituzumab, MGD009, or omburtamab. In some embodiments, the anti-BTLA agent comprises, consists essentially of, or consists of TAB004/JS004. In some embodiments, the anti-Siglec-15 agent comprises, consists essentially of, or consists of NC318. In some embodiments, the checkpoint inhibitor comprises, consists essentially of, or consists of AK104 or KN046.

In some embodiments, the checkpoint inhibitor comprises, consists essentially of, or consists of an anti-PD1 agent or an anti-PD-L1 agent.

In some embodiments, the anti-PD1 agent comprises, consists essentially of, or consists of an anti-PD1 antibody or an antigen binding fragment thereof. In some embodiments, the anti-PD1 antibody comprises, consists essentially of, or consists of nivolumab, pembrolizumab, cemiplimab, spartalizumab, camrelizumab, sintilimab, tislelizumab, toripalimab, AMF 514 (MEDI0680), balstilimab, or a combination of two or more thereof.

In some embodiments, the anti-PD-L1 agent comprises, consists essentially of, or consists of an anti-PD-L1 antibody or an antigen binding fragment thereof. In some embodiments, the anti-PD-L1 antibody comprises, consists essentially of, or consists of avelumab, durvalumab, atezolizumab, envafolimab, or a combination of two or more thereof.

In some embodiments, the checkpoint inhibitor comprises, consists essentially of, or consists of an anti-CTLA-4 agent. In some embodiments, the anti-CTLA-4 agent comprises, consists essentially of, or consists of an anti-CTLA-4 antibody or an antigen binding fragment thereof. In some embodiments, the anti-CTLA-4 antibody comprises, consists essentially of, or consists of ipilimumab, tremelimumab, zalifrelimab, or AGEN1181, or a combination thereof.

In some embodiments, the immunotherapy agent comprises, consists essentially of, or consists of pembrolizumab, optionally in treating a non-small cell lung cancer. In a further embodiment, the pembrolizumab therapy comprises, consists essentially of, or consists of administration of pembrolizumab to a subject at a dose of 200 mg every 3 weeks. In some embodiments, the immunotherapy agent comprises, consists essentially of, or consists of nivolumab. In a further embodiment, the nivolumab therapy comprises, consists essentially of, or consists of nivolumab administration to a subject 240 mg once every 2 weeks and 480 mg once every 4 weeks. In some embodiments, the immunotherapy agent comprises, consists essentially of, or consists of ipilimumab. In a further embodiment, the ipilimumab therapy comprises, consists essentially of, or consists of administration of ipilimumab to a subject at a dose of 1, 3 or 10 mg/kg every 3 weeks for a total of 4 doses. In some embodiments, the immunotherapy agent comprises, consists essentially of, or consists of avelumab. In a further embodiment, the avelumab therapy comprises, consists essentially of, or consists of administration of avelumab at a dose of 800 mg every 2 weeks. In some embodiment, the immunotherapy agent comprises, consists essentially of, or consists of durvalumab. In a further embodiment, the durvalumab therapy comprises, consists essentially of, or consists of administration of durvalumab to a subject at a dose of 10 mg/kg every 2 weeks. In some embodiments, the immunotherapy agent comprises, consists essentially of, or consists of atezolizumab. In a further embodiment, the atezolizumab therapy comprises, consists essentially of, or consists of administration of atezolizumab to a subject at a dose of 1200 mg intravenously (i.v.) over 60 minutes every 3 weeks.

### Inhibitors of thymidylate biosynthesis

In accordance with the invention, the inhibitor of thymidylate biosynthesis for use, as defined in the claims, is 5-fluorouracil (5-FU). Other embodiments are described for context only. In some embodiments, the inhibitor of thymidylate biosynthesis comprises, consists essentially of, or consists of a thymidylate synthase inhibitor. In some embodiments, the inhibitor of thymidylate biosynthesis comprises, consists essentially of, or consists of an inhibitor of folate-mediated one-carbon metabolism.

In some embodiments, the inhibitor of thymidylate biosynthesis comprises, consists essentially of, or consists of 5-fluorouracil (5-FU), pemetrexed, raltitrexed, nolatrexed, plevitrexed, GS7904L, capecitabine, methotrexate, pralatrexate, CT-900, NUC-3373, or a combination of two or more thereof. In some embodiments, the inhibitor of thymidylate biosynthesis comprises, consists essentially of, or consists of 5-FU. In some embodiments, the inhibitor of thymidylate biosynthesis comprises, consists essentially of, or consists of pemetrexed. In some embodiments, the inhibitor of thymidylate biosynthesis comprises, consists essentially of, or consists of raltitrexed. In some embodiments, the inhibitor of thymidylate biosynthesis comprises, consists essentially of, or consists of nolatrexed. In some embodiments, the inhibitor of thymidylate biosynthesis comprises, consists essentially of, or consists of plevitrexed. In some embodiments, the inhibitor of thymidylate biosynthesis comprises, consists essentially of, or consists of GS7904L. In some embodiments, the inhibitor of thymidylate biosynthesis comprises, consists essentially of, or consists of capecitabine. In some embodiments, the inhibitor of thymidylate biosynthesis comprises, consists essentially of, or consists of methotrexate. In some embodiments, the inhibitor of thymidylate biosynthesis comprises, consists essentially of, or consists of pralatrexate. In some embodiments, the inhibitor of thymidylate biosynthesis comprises, consists essentially of, or consists of CT-900. In some embodiments, the inhibitor of thymidylate biosynthesis comprises, consists essentially of, or consists of NUC-3373.

In some embodiments, the inhibitor of thymidylate biosynthesis comprises, consists essentially of, or consists of 5-FU based adjuvant therapy. In some embodiments, the 5-FU based adjuvant therapy comprises, consists essentially of, or consists of S-1, a combination of S-1 and folinic acid, FOLFOX, FOLFOX-4, FOLFIRI, MOF, deflexifol, or a combination of 5-FU with one or more selected from radiation, methyl-CCNU, leucovorin, arfolitixorin, oxaliplatin (such as cisplatin), irinotecan, mitomycin, cytarabine, and levamisole. In some embodiments, the 5-FU based adjuvant therapy comprises, consists essentially of, or consists of FOLFOX, FOLFOX-4, FOLFIRI, MOF, deflexifol, or a combination of 5-FU with one or more selected from radiation, methyl-CCNU, leucovorin, oxaliplatin (such as cisplatin), irinotecan, mitomycin, cytarabine, and levamisole. In some embodiments, the inhibitor of thymidylate biosynthesis comprises, consists essentially of, or consists of S-1. In some embodiments, the inhibitor of thymidylate biosynthesis comprises, consists essentially of, or consists of a combination of S-1 and folinic acid. In some embodiments, the inhibitor of thymidylate biosynthesis comprises, consists essentially of, or consists of FOLFOX. In some embodiments, the inhibitor of thymidylate biosynthesis comprises, consists essentially of, or consists of FOLFOX-4. In some embodiments, the inhibitor of thymidylate biosynthesis comprises, consists essentially of, or consists of FOLFIRI. In some embodiments, the inhibitor of thymidylate biosynthesis comprises, consists essentially of, or consists of MOF. In some embodiments, the inhibitor of thymidylate biosynthesis comprises, consists essentially of, or consists of deflexifol. In some embodiments, the inhibitor of thymidylate biosynthesis comprises, consists essentially of, or consists of a combination of 5-FU with one or more selected from radiation, methyl-CCNU, leucovorin, arfolitixorin, oxaliplatin (such as cisplatin), irinotecan, mitomycin, cytarabine, and levamisole. In some embodiments, the inhibitor of thymidylate biosynthesis comprises, consists essentially of, or consists of a combination of 5-FU with one or more selected from radiation, methyl-CCNU, leucovorin, oxaliplatin (such as cisplatin), irinotecan, mitomycin, cytarabine, and levamisole.

In some embodiments, the inhibitor of thymidylate biosynthesis is formulated for nanoparticle-based delivery.

In some embodiments, the inhibitor of thymidylate biosynthesis is a 5-FU. Various regimens may be utilized by one of skill in the art (e.g., an oncologist), such as those listed below:
(1) Colorectal cancer: 500 mg/m², i.v. bolus on day 1; 1 hour prior to administering 5-FU bolus, give leucovorin 500 mg/ m², i.v. over 2 hours. Repeat weekly on days 1, 8, 15, 22, 29, and 36 every 8 weeks for 4 to 6 cycles.
(2) Adjuvant treatment of high-risk stage II or stage III rectal cancer in combination with radiation therapy: 500 mg/m², i.v. bolus daily for 5 days on days 1 and 36 beginning 22 to 70 days after surgery; radiation therapy for 6 weeks is begun on day 64 after initiation of 5-FU therapy. 5-FU 225 mg/m²/day, i.v. continuous infusion is given throughout radiation therapy. Then, 5-FU 450 mg/m², i.v. bolus daily for 5 days beginning 1 month after radiation (i.e., days 134 to 138) and repeated in 4 weeks.
(3) For the treatment of metastatic colorectal cancer in combination with irinotecan and leucovorin, with or without bevacizumab (FOLFIRI with or without bevacizumab): 400 mg/m², i.v. bolus on day 1, followed by 5-FU 1,200 mg/m²/day on days 1 and 2 by continuous i.v. infusion (CIV) (total infusional dose, 2,400 mg/m² over 46 hours) for cycles 1 and 2. If there is no toxicity greater than grade 1, the 5-FU infusion dose may be increased to 3,000 mg/m² for all subsequent cycles.
(4) For the treatment of advanced colorectal cancer in combination with leucovorin (LV) and oxaliplatin with or without bevacizumab (FOLFOX4 with or without bevacizumab): 400 mg/m², i.v. bolus over 2 to 4 minutes, followed by 5-FU 600 mg/m² continuous i.v. infusion (CIV) over 22 hours on day 1. Prior to 5-FU bolus on day 1, administer oxaliplatin 85 mg/m², i.v. and leucovorin 200 mg/m², i.v. (both over 120 minutes via Y-site). If giving FOLFOX4 plus bevacizumab, administer bevacizumab 10 mg/kg i.v. over 30 to 90 minutes prior to chemotherapy on day 1. On day 2, repeat leucovorin 200 mg/m², i.v. over 2 hours followed by 5-FU 400 mg/m², i.v. bolus, then 5-FU 600 mg/m² CIV over 22 hours. The order of administration is (bevacizumab) followed by oxaliplatin and leucovorin, followed by 5-FU. This 2-day regimen is repeated every 2 weeks until disease progression or unacceptable toxicity.

In some embodiments, the inhibitor of thymidylate biosynthesis is capecitabine. Various regimens may be utilized by one of skill in the art (e.g., an oncologist), such as those listed below:
(1) Stage III colon cancer, adjuvant following surgery (monotherapy): 1.25 g/m² twice daily for 14 days, subsequent courses repeated after a 7-day interval, recommended duration of treatment is 6 months, adjust dose according to tolerability.
(2) Stage III colon cancer, adjuvant following surgery (combination therapy): 0.8-1 g/m² twice daily for 14 days, subsequent courses repeated after a 7-day interval, recommended duration of treatment is 6 months, adjust dose according to tolerability.
(3) Metastatic colorectal cancer (monotherapy):1.25 g/m² twice daily for 14 days, subsequent courses repeated after a 7-day interval, adjust dose according to tolerability.
(4) Metastatic colorectal cancer (combination therapy): 0.8-1 g/m² twice daily for 14 days, subsequent courses repeated after a 7-day interval, adjust dose according to tolerability.
(5) Advanced gastric cancer (first-line treatment in combination with a platinum-based regimen): 0.8-1 g/m² twice daily for 14 days, subsequent courses repeated after a 7-day interval, alternatively 625 mg/m² twice daily given continuously, adjust dose according to tolerability.

In some embodiments, the inhibitor of thymidylate biosynthesis is methotrexate. Various regimens may be utilized by one of skill in the art (e.g., an oncologist), such as those listed below:
(1) Choriocarcinoma and similar trophoblastic diseases: Methotrexate is administered orally or intramuscularly in doses of 15 to 30 mg daily for a five-day course. Such courses are usually repeated for 3 to 5 times as required.
(2) Lymphomas: In Burkitt's tumor, Stages I-II, Recommended dosage is 10 to 25 mg/day orally for 4 to 8 days.
(3) Mycosis fungoides (cutaneous T cell lymphoma): Dosage in early stages is usually 5 to 50 mg once weekly. Dose reduction or cessation is guided by patient response and hematologic monitoring.
(4) Osteosarcoma: Methotrexate is used in combination with other agents. In addition to high-dose methotrexate with leucovorin rescue, these agents may include doxorubicin, cisplatin, and the combination of bleomycin, cyclophosphamide and dactinomycin (BCD). The starting dose for high-dose methotrexate treatment is 12 grams/m².

### Anthracyclines or other topoisomerase II inhibitors

The following embodiments are described for context only.

In some embodiments, the anthracycline or other topoisomerase II inhibitor comprises, consists essentially of, or consists of an anthracycline. In some embodiments, the anthracycline comprises, consists essentially of, or consists of one or more selected from daunorubicin, doxorubicin (including liposomal doxorubicin), epirubicin, idarubicin, mitoxantrone, and valrubicin. In some embodiments, the anthracycline comprises, consists essentially of, or consists of daunorubicin. In some embodiments, the anthracycline comprises, consists essentially of, or consists of doxorubicin (including liposomal doxorubicin). In some embodiments, the anthracycline comprises, consists essentially of, or consists of epirubicin. In some embodiments, the anthracycline comprises, consists essentially of, or consists of idarubicin. In some embodiments, the anthracycline comprises, consists essentially of, or consists of valrubicin.

In some embodiments, the anthracycline or other topoisomerase II inhibitor comprises, consists essentially of, or consists of a compound selected from mitoxantrone, etoposide and teniposide. In some embodiments, the other topoisomerase II inhibitor comprises, consists essentially of, or consists of mitoxantrone. In some embodiments, the other topoisomerase II inhibitor comprises, consists essentially of, or consists of etoposide. In some embodiments, the other topoisomerase II inhibitor comprises, consists essentially of, or consists of teniposide.

### dUTPase inhibitors

In accordance with the invention, the deoxyuridine triphosphatase (dUTPase) inhibitor for use, as defined in the claims, is 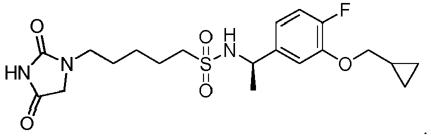 Other embodiments are described for context only. In some embodiments, the dUTPase inhibitor is a compound of Formula (I): 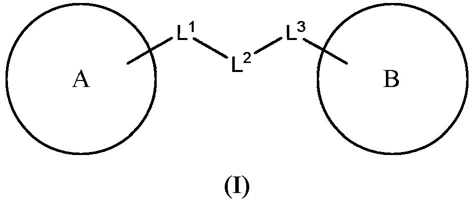
or a tautomer thereof, or a prodrug of each thereof; or a deuterium isotope of each of the above wherein up to 10, preferably up to 6, more preferably up to 3 hydrogen atoms that are attached to one or more carbon atoms are replaced with deuterium(s); or a pharmaceutically acceptable salt of each of the foregoing; or a pharmaceutically acceptable solvate of each of the above mentioned,
wherein
A is an optionally substituted 5-membered heterocyclyl containing a -C(O)NZC(O)-moiety, a -C(O)OC(O) moiety, a -C(O)CR¹⁰C(O) moiety, or a -C(O)NR¹⁰C(O) moiety; or
A is a 5-membered heteroaryl or a 5-membered substantially planar heterocyclyl (i.e., a heterocyclyl wherein at least 3 or at least 4 atoms can stably be in a same plane) substituted at 1,3 positions with substituents selected from halo, optionally substituted hydroxy, and optionally substituted -SH groups, preferably two fluoros, wherein the 5-membered heteroaryl or substantially planar heterocyclyl is further optionally substituted; or
A is 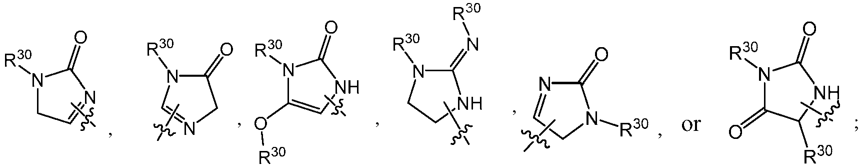
each R¹⁰ independently is hydrogen, an optionally substituted C₁-C₁₀ alkoxy, or an optionally substituted C₁-C₁₀ alkyl, preferably R¹⁰ is hydrogen;
each R³⁰ independently is hydrogen; an optionally substituted C₁-C₁₀ alkoxy; optionally substituted amino, such as -NH₂ or a mono or di-substituted form thereof; an optionally substituted C₁-C₁₀ alkyl; optionally substituted hydroxy; or Z; or A and L¹, preferably, R³⁰, wherein R³⁰ is attached to an atom that is adjacent to the atom attached to L¹, and L¹ together with the atoms they are attached to form a 5-7 membered ring;
L¹ is a linker having 2-13 chain atoms selected from C, N, O, S, and/or P, wherein the linker is optionally substituted; or
L¹ is -L¹¹-L¹²-L¹³-, wherein L¹¹ is attached to A and L¹¹ is O, S, NR, C₁-C₂ alkylene, C₂ alkenylene, C₂ heteroalkylene, C₃ heteroalkenylene, L¹² is arylene or heteroarylene, L¹³ is a bond or an optionally substituted C₁-C₅ alkylene, and R is H or C₁-C₃ alkyl;
L² is -SO₂NR⁵⁰-, wherein the sulfur is attached to L¹; -NR⁵⁰SO₂-, wherein the nitrogen is attached to L¹; -C(O)NR⁵⁰-, wherein the carbon is attached to L¹;-NR⁵⁰C(O)-, wherein the nitrogen is attached to L¹; -NR⁵⁰SO₂NR⁵⁰-; or -NR⁵⁰CONR⁵⁰-;
each R⁵⁰ independently is hydrogen, an optionally substituted C₁-C₆ alkyl, an optionally substituted C₂-C₆ heteroalkyl, an optionally substituted C₂-C₆ alkenyl, an optionally substituted C₃-C₆ heteroalkenyl, an optionally substituted C₂-C₆ alkynyl, an optionally substituted C₃-C₆ heteroalkynyl, or Z;
Z is 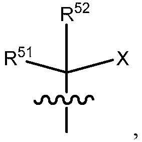
each R⁵¹ and R⁵² independently is hydrogen or an optionally substituted C₁-C₁₀ alkyl;
X is an optionally substituted hydroxy group, an optionally substituted NH₂ group, or an optionally substituted SH group;
L³ is a bond, an optionally substituted C₁-C₆ alkylene, an optionally substituted C₂-C₆ heteroalkylene, an optionally substituted C₂-C₆ alkenylene, an optionally substituted C₃-C₆ heteroalkenylene, an optionally substituted C₂-C₆ alkynylene, or an optionally substituted C₃-C₆ heteroalkynylene; and
B is an optionally substituted 6-10 membered aryl; an optionally substituted 5-15 membered heteroaryl; an optionally substituted 4-15 membered heterocyclyl; or an optionally substituted 3-15 membered cycloalkyl, if cycloalkyl, then preferably at least a 4 membered, or more preferably a 5-10 membered cycloalkyl.

In some embodiments, A is

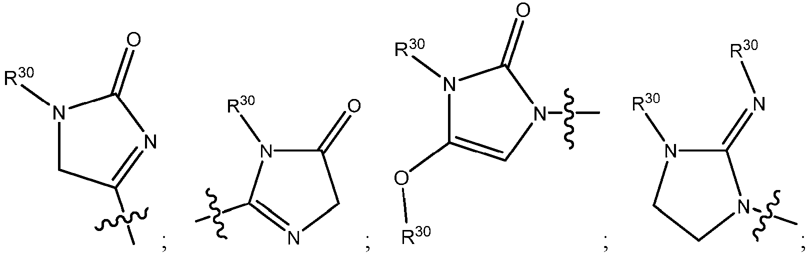

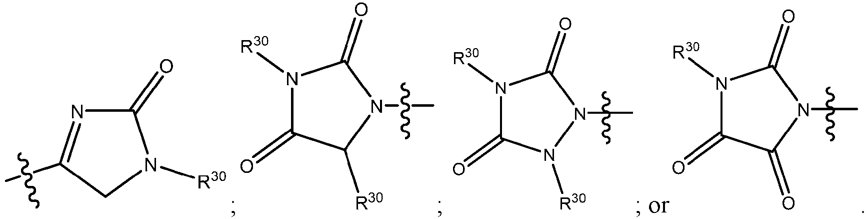

In some embodiments, the compound provided herein is a prodrug. As used herein, "prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor (for example, see the reference Nogrady, 1985, Medicinal Chemistry A Biochemical Approach, Oxford University Press, New York, pages 388-392). A prodrug may be synthesized using reactants other than the corresponding drug. Examples of prodrugs and methods of making them are also provided in US Patent Application Publication No. 20160024127.

In some embodiments, the compound provided herein contains one or more deuterium. Examples of a deuterium containing compound provided herein, wherein up to 10, preferably up to 6, more preferably up to 3 hydrogen atoms that are attached to carbon atoms are replaced with a deuterium, include, without limitation: a compound where a methyl group is converted to -CH₂D, -CHD₂, or -CD₃; a compound where a methylene group is converted to a -CHD- or -CD₂-, a phenyl ring where one or more hydrogen atoms are replaced with deuterium atoms, etc.

In some embodiments, A is an optionally substituted 5-membered heterocyclyl containing a -C(O)NZC(O)- moiety. In some embodiments, A is an optionally substituted 5-membered heterocyclyl containing a -C(O)OC(O) moiety. In some embodiments, A is an optionally substituted 5-membered heterocyclyl containing a -C(O)CR¹⁰C(O) moiety. In some embodiments, A is an optionally substituted 5-membered heterocyclyl containing a - C(O)NR¹⁰C(O) moiety.

In some embodiments, R¹⁰ is hydrogen. In some embodiments, R¹⁰ is an optionally substituted C₁-C₁₀ alkoxy. In some embodiments, R¹⁰ is an optionally substituted C₁-C₁₀ alkyl.

In some embodiments, A is a 5-membered heteroaryl substituted at 1,3 positions with substituents selected from halo, optionally substituted hydroxy, and optionally substituted -SH groups, preferably two fluoros, wherein the 5-membered heteroaryl is further optionally substituted. In some embodiments, A is a 5-membered heteroaryl substituted at 1,3 positions with halo, wherein the 5-membered heteroaryl is further optionally substituted. In some embodiments, the 5-membered heteroaryl is substituted at 1,3 positions with two fluoros, wherein the 5-membered heteroaryl is further optionally substituted. In some embodiments, A is a 5-membered heteroaryl substituted at 1,3 positions with optionally substituted hydroxy, wherein the 5-membered heteroaryl is further optionally substituted. In some embodiments, A is a 5-membered heteroaryl substituted at 1,3 positions with optionally substituted -SH groups, wherein the 5-membered heteroaryl is further optionally substituted.

Non-limiting and illustrative examples of a 5-membered heteroaryl substituted at 1,3 positions with substituents selected from halo, optionally substituted hydroxy, optionally substituted -SH groups include, without limitation: 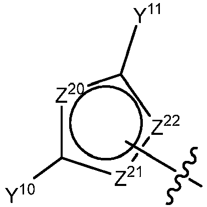 such as 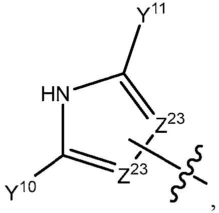 where Y¹⁰ and Y¹¹ independently are selected from a halo, preferably chloro or fluoro, hydroxy, -SH, substituted hydroxy, and substituted -SH; Z²⁰-Z²² are independently selected from optionally substituted CH, optionally substituted NH, N, S, SO₂, SO, and O, provided that the combination of Z²⁰-Z²² provides a planar valence matched heteroaryl or a tautomer thereof; and each Z²³ independently is CH or N.

In some embodiments, Y¹⁰ is a halo. In some embodiments, Y¹⁰ is a chloro. In some embodiments, Y¹⁰ is a fluoro. In some embodiments, Y¹⁰ is hydroxy. In some embodiments, Y¹⁰ is -SH. In some embodiments, Y¹⁰ is a substituted hydroxy. In some embodiments, Y¹⁰ is a substituted -SH.

In some embodiments, Y¹¹ is a halo. In some embodiments, Y¹¹ is a chloro. In some embodiments, Y¹¹ is a fluoro. In some embodiments, Y¹¹ is hydroxy. In some embodiments, Y¹¹ is -SH. In some embodiments, Y¹¹ is a substituted hydroxy. In some embodiments, Y¹¹ is a substituted -SH.

In some embodiments, Z²⁰ is an optionally substituted CH. In some embodiments, Z²⁰ is an optionally substituted NH. In some embodiments, Z²⁰ is N. In some embodiments, Z²⁰ is S. In some embodiments, Z²⁰ is SO₂. In some embodiments, Z²⁰ is SO. In some embodiments, Z²⁰ is O.

In some embodiments, Z²¹ is an optionally substituted CH. In some embodiments, Z²¹ is an optionally substituted NH. In some embodiments, Z²¹ is N. In some embodiments, Z²¹ is S. In some embodiments, Z²¹ is SO₂. In some embodiments, Z²¹ is SO. In some embodiments, Z²¹ is O.

In some embodiments, Z²² is an optionally substituted CH. In some embodiments, Z²² is an optionally substituted NH. In some embodiments, Z²² is N. In some embodiments, Z²² is S. In some embodiments, Z²² is SO₂. In some embodiments, Z²² is SO. In some embodiments, Z²² is O.

In some embodiments, Z²³ is an optionally substituted CH. In some embodiments, Z²³ is N.

In some embodiments, A is a 5-membered substantially planar heterocyclyl (i.e., a heterocyclyl wherein at least 3 or at least 4 atoms can stably be in a same plane) substituted at 1,3 positions with substituents selected from halo, optionally substituted hydroxy, and optionally substituted -SH groups, preferably two fluoros, wherein the 5-membered substantially planar heterocyclyl is further optionally substituted. In some embodiments, A is a 5-membered substantially planar heterocyclyl substituted at 1,3 positions with halo, wherein the 5-membered substantially planar heterocyclyl is further optionally substituted. In some embodiments, the 5-membered substantially planar heterocyclyl is substituted at 1,3 positions with two fluoros, wherein the 5-membered substantially planar heterocyclyl is further optionally substituted. In some embodiments, A is a 5-membered substantially planar heterocyclyl substituted at 1,3 positions with optionally substituted hydroxy, wherein the 5-membered substantially planar heterocyclyl is further optionally substituted. In some embodiments, A is a 5-membered substantially planar heterocyclyl substituted at 1,3 positions with optionally substituted -SH groups, wherein the 5-membered substantially planar heterocyclyl is further optionally substituted.

Examples of a 5-membered substantially planar heterocyclyl substituted at 1,3 positions with halo, optionally substituted hydroxy, and optionally substituted -SH groups, have similar structures as the corresponding 5-membered heteroaryl except that the 5-membered ring is not an aromatic ring.

In some embodiments, A is:

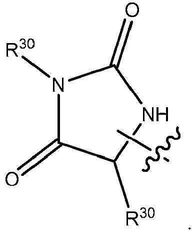

In some embodiments, A is:

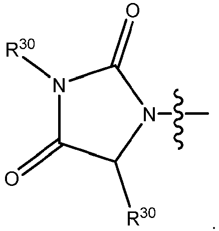

In some embodiments, A is:

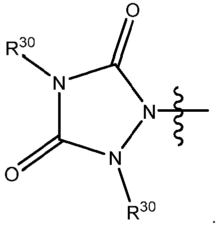

In some embodiments, A is:

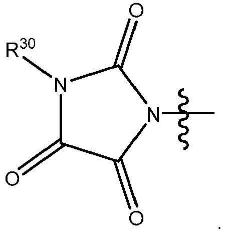

In some embodiments, A is:

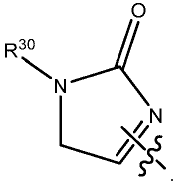

In some embodiments, A is:

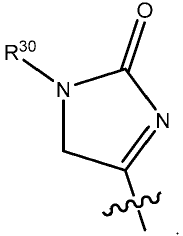

In some embodiments, A is:

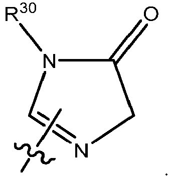

In some embodiments, A is:

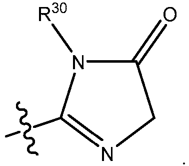

In some embodiments, A is:

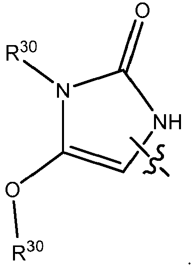

In some embodiments, A is:

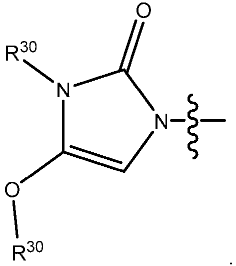

In some embodiments, A is:

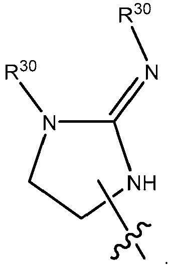

In some embodiments, A is:

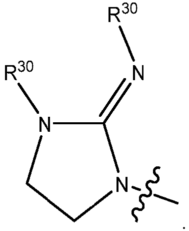

In some embodiments, A is:

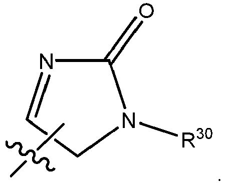

In some embodiments, A is:

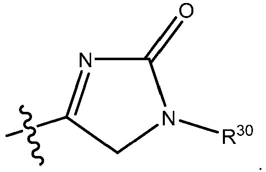

In some embodiments, R³⁰ is hydrogen. In some embodiments, R³⁰ is an optionally substituted C₁-C₁₀ alkoxy. In some embodiments, R³⁰ is optionally substituted amino, such as -NH₂ or a mono or di-substituted form thereof. In some embodiments, R³⁰ is an optionally substituted C₁-C₁₀ alkyl. In some embodiments, R³⁰ is an optionally substituted hydroxy. In some embodiments, R³⁰ is a prodrug moiety. Non-limiting and illustrative prodrug moieties include formyl ethers, and formyl esters as disclosed herein. In some embodiments, R³⁰ is Z.

Illustrative and non-limiting examples of R³⁰ include a substituted hydroxy or - CH₂OC(O)R⁸⁰, wherein R⁸⁰ is H or an optionally substituted C₁-C₁₀ alkyl. In some embodiments, R⁸⁰ is hydrogen. In some embodiments, R⁸⁰ is an optionally substituted C₁-C₁₀ alkyl.

In some embodiments, A and L¹, preferably, R³⁰ and L¹ together with the atoms they are attached to form a 5-7 membered ring.

In some embodiments, A is selected from the group consisting of:

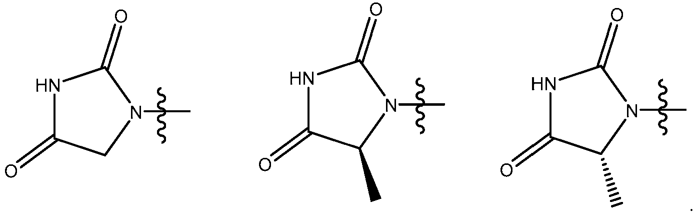

In some embodiments, A is

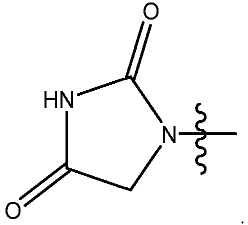

In some embodiments, A is

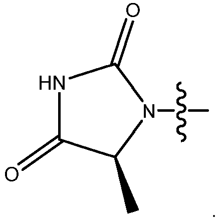

In some embodiments, A is

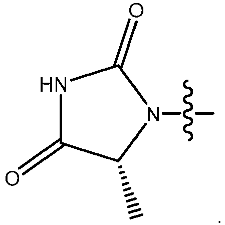

The A moieties disclosed herein including herein above, can, in some embodiments, be further substituted with 1-3, preferably 1-2, more preferably, 1 R³⁰ substituent as provided herein. In some embodiments, where R³⁰ and L¹ are joined to adjacent atoms (i.e., atoms having a 1,2 positional relation), R³⁰ and a portion of L¹, together with the intervening atoms can form a 5-6 membered, optionally substituted cycloalkyl or heterocyclyl ring.

In some embodiments, A is not:

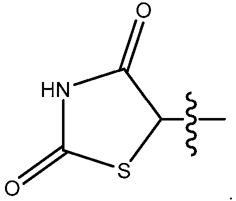

In some embodiments, A is not:

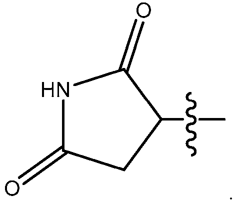

In some embodiments, L¹ is a linker having 2-13 chain atoms selected from C, N, O, S, and/or P, wherein the linker is optionally substituted. In various embodiments, L¹ having 2-13 chain atoms selected from C, N, O, S, and/or P can be: alkylene, alkenylene, alkynylene, wherein one or more carbon atoms are replaced with O, S, SO, SO₂, optionally substituted NH, 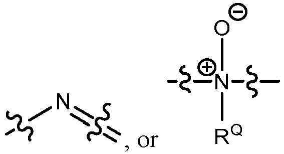 moieties where R^{Q} is H or C₁-C₆ alkyl optionally substituted -CO-NH-, optionally substituted -SO₂-NH-, optionally substituted -P(O)(OH)-, optionally substituted phosphoramide and optionally substituted phosporamidate, (such as -P(O)NH₂-, -P(O)(OH)NH-, etc.), optionally substituted oligoethylene glycol, optionally substituted oligo ethanolamine, and the likes, as will be apparent to the skilled artisan based on the disclosure provided herein.

In some embodiments, L¹ is -(CH₂)_{q}-. In some embodiments, one or more hydrogens are optionally substituted with C₁-C₃ alkyl. In some embodiments, at least two or more geminal hydrogens together with the carbon(s) to which they are attached are optionally replaced with an optionally substituted 3-5 membered heterocyclyl. In some embodiments, at least two or more geminal hydrogens together with the carbon(s) to which they are attached are optionally replaced with an optionally substituted 3-5 membered cycloalkyl. In some embodiments, the optionally substituted 3-5 membered cycloalkyl is an optionally substituted cyclopropano. In some embodiments, the optionally substituted 3-5 membered cycloalkyl is an optionally substituted cyclobutano. In some embodiments, the optionally substituted 3-5 membered cycloalkyl is an optionally substituted cyclopentano. In some embodiments, the optionally substituted 3-5 membered heterocyclyl is an optionally substituted tetrahydrofurano.

In some embodiments, q is 3. In some embodiments, q is 4. In some embodiments, q is 5. In some embodiments, q is 6. In some embodiments, q is 7. In some embodiments, q is 8.

In some embodiments, L¹ is: 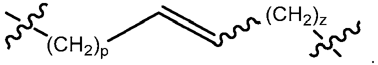 In some related embodiments, one or more hydrogens are optionally substituted with C₁-C₃ alkyl. In some embodiments, at least two or more geminal hydrogens together with the carbon(s) to which they are attached are optionally replaced with an optionally substituted 3-5 membered heterocyclyl. In some embodiments, at least two or more geminal hydrogens together with the carbon(s) to which they are attached are optionally replaced with an optionally substituted 3-5 membered cycloalkyl. In some embodiments, the optionally substituted 3-5 membered cycloalkyl is an optionally substituted cyclopropano. In some embodiments, the optionally substituted 3-5 membered cycloalkyl is an optionally substituted cyclobutano. In some embodiments, the optionally substituted 3-5 membered cycloalkyl is an optionally substituted cyclopentano. In some embodiments, the optionally substituted 3-5 membered cycloalkyl is an optionally substituted tetrahydrofurano.

In some embodiments, p is 0. In some embodiments, p is 1. In some embodiments, p is 2. In some embodiments, p is 3. In some embodiments, p is 4. In some embodiments, p is 5.

In some embodiments, z is 0. In some embodiments, z is 1. In some embodiments, z is 2. In some embodiments, z is 3. In some embodiments, z is 4. In some embodiments, z is 5.

In some embodiments, L¹ is -(CH₂)ₘ-X¹⁵-(CH₂)ₙ-. In some embodiments, one or more hydrogens are optionally substituted with C₁-C₃ alkyl. In some embodiments, at least two or more geminal hydrogens together with the carbon(s) to which they are attached are optionally replaced with an optionally substituted 3-5 membered heterocyclyl. In some embodiments, at least two or more geminal hydrogens together with the carbon(s) to which they are attached are optionally replaced with an optionally substituted 3-5 membered cycloalkyl. In some embodiments, the optionally substituted 3-5 membered cycloalkyl is an optionally substituted cyclopropano. In some embodiments, the optionally substituted 3-5 membered cycloalkyl is an optionally substituted cyclobutano. In some embodiments, the optionally substituted 3-5 membered cycloalkyl is an optionally substituted cyclopentano. In some embodiments, the optionally substituted 3-5 membered heterocyclyl is an optionally substituted tetrahydrofurano.

In some embodiments, m is 0. In some embodiments, m is 1. In some embodiments, m is 2. In some embodiments, m is 3.

In some embodiments, n is 0. In some embodiments, n is 1. In some embodiments, n is 2. In some embodiments, n is 3. In some embodiments, n is 4. In some embodiments, n is 5. In some embodiments, n is 6. In some embodiments, n is 7.

In some embodiments, X¹⁵ is NR⁴⁰. In some embodiments, X¹⁵ is NR⁴⁰(+)-O(-). In some embodiments, R⁴⁰ is H. In some embodiments, R⁴⁰ is C₁-C₁₀ alkyl. In some embodiments, R⁴⁰ is C₁-C₃ alkyl. In some embodiments, X¹⁵ is O. In some embodiments, X¹⁵ is S. In some embodiments, X¹⁵ is SO. In some embodiments, X¹⁵ is SO₂.

In some embodiments, L¹ is: 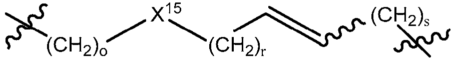 where X¹⁵ is defined as above.
In some related embodiments, one or more hydrogens are optionally substituted with C₁-C₃ alkyl. In some embodiments, at least two or more geminal hydrogens together with the carbon(s) to which they are attached are optionally replaced with an optionally substituted 3-5 membered heterocyclyl. In some embodiments, at least two or more geminal hydrogens together with the carbon(s) to which they are attached are optionally replaced with an optionally substituted 3-5 membered cycloalkyl. In some embodiments, the optionally substituted 3-5 membered cycloalkyl is an optionally substituted cyclopropano. In some embodiments, the optionally substituted 3-5 membered cycloalkyl is an optionally substituted cyclobutano. In some embodiments, the optionally substituted 3-5 membered cycloalkyl is an optionally substituted cyclopentano. In some embodiments, the optionally substituted 3-5 membered heterocyclyl is an optionally substituted tetrahydrofurano.

In some embodiments, o is 0. In some embodiments, o is 1. In some embodiments, o is 2. In some embodiments, o is 3.

In some embodiments, r is 1. In some embodiments, r is 2. In some embodiments, r is 3.

In some embodiments, s is 0. In some embodiments, s is 1. In some embodiments, s is 2. In some embodiments, s is 3. In some embodiments, s is 4.

In some embodiments, L¹ is selected from the group consisting of: 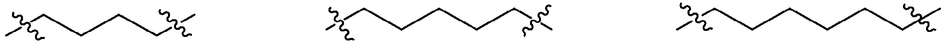 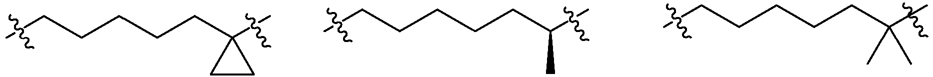 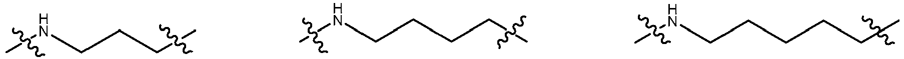 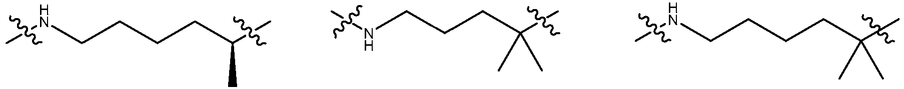 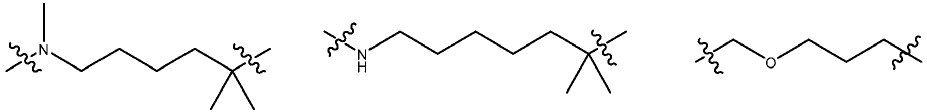 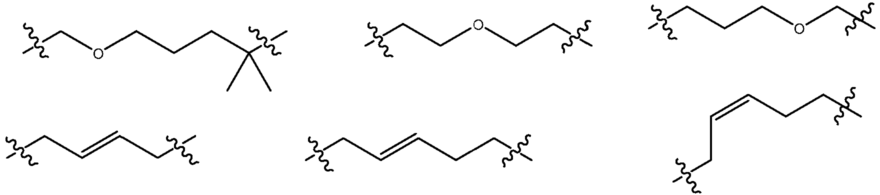 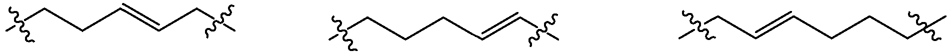 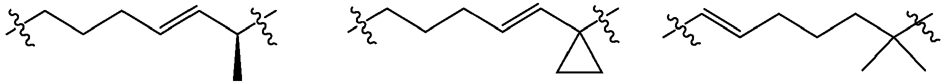 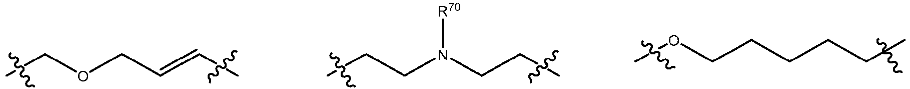 wherein the left side of the moieties are attached to A.

In some related embodiments, 1-5, preferably, 1-3 hydrogen atoms of the L¹ are optionally substituted, preferred substituents including without limitation, C₁-C₆ alkyl optionally substituted with 1-3 halo, such as fluoro, and/or C₁-C₆ alkoxy; optionally substituted C₁-C₆ alkoxy; and halo, preferably fluoro, wherein the left side of the moieties are attached to A and wherein R⁷⁰ is an optionally substituted C₁-C₁₀ alkyl. In some embodiments, L¹ is optionally substituted wherein 1-5 hydrogen atoms are optionally substituted. In some embodiments, L¹ is optionally substituted wherein 1-3 hydrogen atoms are optionally substituted. In some embodiments, substituents include without limitation C₁-C₆ alkyl optionally substituted with 1-3 halo, such as fluoro. In some embodiments, substituents include without limitation C₁-C₆ alkyl optionally substituted with C₁-C₆ alkoxy. In some embodiments, substituents include without limitation an optionally substituted C₁-C₆ alkoxy. In some embodiments, substituents include without limitation a halo. In some embodiments, substituents include a fluoro.

In some embodiments, L¹ is: 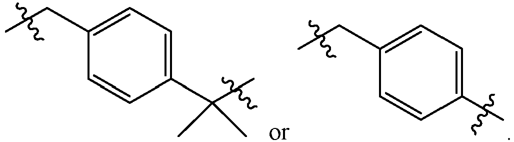 or an optionally substituted version of each thereof wherein 1-5, preferably, 1-3 hydrogen atoms are optionally substituted, preferred substituents including without limitation, C₁-C₆ alkyl optionally substituted with 1-3 halo, such as fluoro, and/or C₁-C₆ alkoxy; optionally substituted C₁-C₆ alkoxy; and halo, preferably fluoro, wherein the left side of the moieties are attached to A.

In some embodiments, L¹ is:

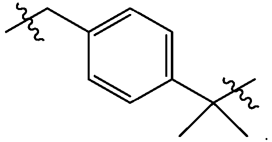

In some embodiments, L¹ is:

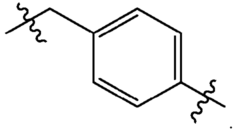

In some embodiments, L¹ is optionally substituted wherein 1-5 hydrogen atoms are optionally substituted. In some embodiments, L¹ is optionally substituted wherein 1-3 hydrogen atoms are optionally substituted. In some embodiments, substituents include without limitation C₁-C₆ alkyl optionally substituted with 1-3 halo, such as fluoro. In some embodiments, substituents include without limitation C₁-C₆ alkyl optionally substituted with C₁-C₆ alkoxy. In some embodiments, substituents include without limitation an optionally substituted C₁-C₆ alkoxy. In some embodiments, substituents include without limitation a halo. In some embodiments, substituents include a fluoro.

In some embodiments, L² is -SO₂NR⁵⁰-, wherein the sulfur is attached to L¹. In some embodiments, L² is -NR⁵⁰SO₂-, wherein the nitrogen is attached to L¹. In some embodiments, L² is -C(O)NR⁵⁰-, wherein the carbon is attached to L¹. In some embodiments, L² is -NR⁵⁰C(O)-, wherein the nitrogen is attached to L¹. In some embodiments, L² is - NR⁵⁰SO₂NR⁵⁰-. In some embodiments, L² is -NR⁵⁰CONR⁵⁰-.

In some embodiments, R⁵⁰ is hydrogen. In some embodiments, R⁵⁰ is an optionally substituted C₁-C₆ alkyl. In some embodiments, R⁵⁰ is an optionally substituted C₂-C₆ heteroalkyl. In some embodiments, R⁵⁰ is an optionally substituted C₂-C₆ alkenyl. In some embodiments, R⁵⁰ is an optionally substituted C₃-C₆ heteroalkenyl. In some embodiments, R⁵⁰ is an optionally substituted C₂-C₆ alkynyl. In some embodiments, R⁵⁰ is an optionally substituted C₃-C₆ heteroalkynyl. In some embodiments, R⁵⁰ is Z.

In some embodiments, Z is 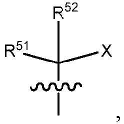 wherein each R⁵¹ and R⁵² independently is hydrogen or an optionally substituted C₁-C₁₀ alkyl and X is an optionally substituted hydroxy group, an optionally substituted NH₂ group, or an optionally substituted SH group.

In some embodiments, R⁵¹ is hydrogen. In some embodiments, R⁵¹ is an optionally substituted C₁-C₁₀ alkyl. In some embodiments, R⁵² is hydrogen. In some embodiments, R⁵² is an optionally substituted C₁-C₁₀ alkyl.

In some embodiments, X is an optionally substituted hydroxy group. In some embodiments, X is an optionally substituted NH₂ group. In some embodiments, X is an optionally substituted SH group.

As used herein, an optionally substituted hydroxy group refers to without limitation alkylated, arylated, cycloalkylated, heterocyclylated, acylated, carboxylated (i.e., generating a carbonate, carbamate, a thiocarbonate, a thiacarbamate containing alkyl, aryl, heteroaryl, and/or heterocyclyl, and such other moieties), phosphorylated, phosphonylated, sulfonylated, forms of a hydroxy group, as would be apparent to the skilled artisan in view of this disclosure.

As used herein, an optionally substituted NH₂ group refers to without limitation alkylated, arylated, cycloalkylated, heterocyclylated, acylated, carboxylated (i.e., generating a carbonate, carbamate, a thiocarbonate, a thiacarbamate containing alkyl, aryl, heteroaryl, and/or heterocyclyl, and such other moieties), phosphorylated, phosphonylated, sulfonylated, forms of a NH₂ group, as would be apparent to the skilled artisan in view of this disclosure.

As used herein, an optionally substituted SH group refers to without limitation alkylated, arylated, cycloalkylated, heterocyclylated, acylated, carboxylated (i.e., generating a carbonate, carbamate, a thiocarbonate, a thiacarbamate containing alkyl, aryl, heteroaryl, and/or heterocyclyl, and such other moieties), phosphorylated, phosphonylated, sulfonylated, forms of a -SH group, as would be apparent to the skilled artisan in view of this disclosure.

In some embodiments, L³ is a bond. In some embodiments, L³ is an optionally substituted C₁-C₆ alkylene. In some embodiments, L³ is -CH₂-. In some embodiments, L³ is an optionally substituted C₂-C₆ heteroalkylene. In some embodiments, L³ is an optionally substituted C₂-C₆ alkenylene. In some embodiments, L³ is an optionally substituted C₃-C₆ heteroalkenylene. In some embodiments, L³ is an optionally substituted C₂-C₆ alkynylene. In some embodiments, L³ is an optionally substituted C₃-C₆ heteroalkynylene. In some embodiments, L¹ is a linker optionally substituted with a C₃-C₆ cycloalkyl, preferably a cyclopropyl or a cyclobutyl. In some embodiments, the C₁-C₆ alkylene is optionally substituted with a C₃-C₆ cycloalkyl.

In some embodiments, L³ is selected from the group consisting of: 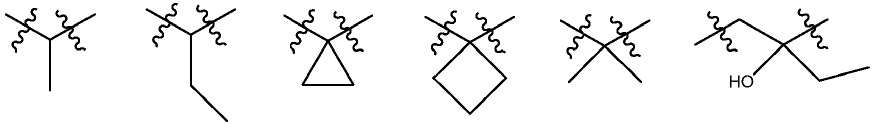 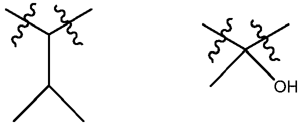 and optionally substituted versions thereof wherein 1-5, preferably, 1-3 hydrogen atoms are optionally substituted, preferred substituents including without limitation, C₁-C₆ alkyl optionally substituted with 1-3 halo, such as fluoro, and/or C₁-C₆ alkoxy; optionally substituted C₁-C₆ alkoxy; and halo, preferably fluoro, wherein the left side of the moieties are attached to L².

In some embodiments, L³ is:

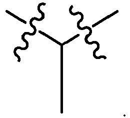

In some embodiments, L³ is:

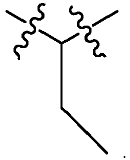

In some embodiments, L³ is:

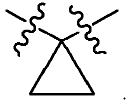

In some embodiments, L³ is:

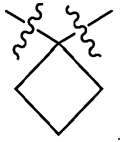

In some embodiments, L³ is:

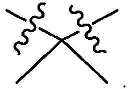

In some embodiments, L³ is:

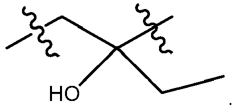

In some embodiments, the left side is attached to A.

In some embodiments, L³ is:

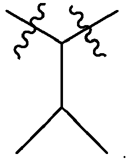

In some embodiments, L³ is:

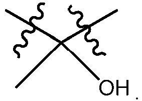

In some embodiments, the left side is attached to A.

In some embodiments, the L³ is optionally substituted wherein 1-5 hydrogen atoms are optionally substituted. In some embodiments, L³ is an optionally substituted version thereof wherein 1-3 hydrogen atoms are optionally substituted. In some embodiments, substituents include without limitation C₁-C₆ alkyl optionally substituted with 1-3 halo, such as fluoro. In some embodiments, substituents include without limitation C₁-C₆ alkyl optionally substituted with C₁-C₆ alkoxy. In some embodiments, substituents include without limitation an optionally substituted C₁-C₆ alkoxy. In some embodiments, substituents include without limitation a halo. In some embodiments, substituents include a fluoro.

In some embodiments, when L³ is 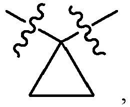 then A is a hydantoin moiety as disclosed herein.

In some embodiments, when L³ is a bond, then A is a hydantoin moiety as disclosed herein.

As used herein, a hydantoin moiety refers to: 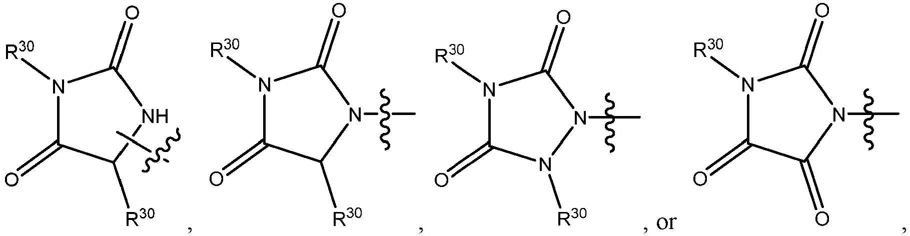 wherein R³⁰ is as defined above.

In some embodiments, a hydantoin moiety is:

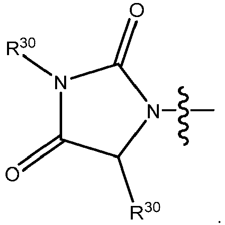

In some embodiments, L³ is not:

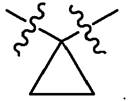

In some embodiments, L³ is selected from the group consisting of: 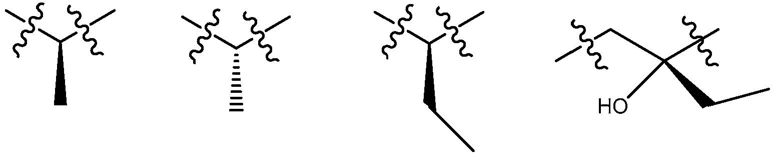 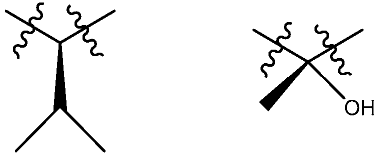 and optionally substituted versions thereof wherein 1-5, preferably, 1-3 hydrogen atoms are optionally substituted, preferred substituents including without limitation, C₁-C₆ alkyl optionally substituted with 1-3 halo, such as fluoro, and/or C₁-C₆ alkoxy; optionally substituted C₁-C₆ alkoxy; and halo, preferably fluoro, wherein the left side of the moieties are attached to L².

In some embodiments, L³ is: 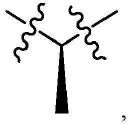 wherein the left side is attached to A.

In some less preferred embodiments, L³ is: 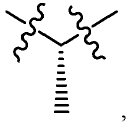 wherein the left side is attached to A.

In some embodiments, L³ is: 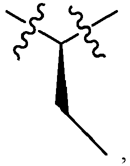 wherein the left side is attached to A.

In some embodiments, L³ is: 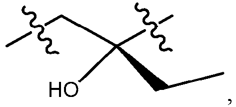 wherein the left side is attached to A.

In some embodiments, L³ is: 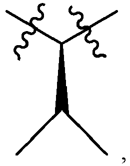 wherein the left side is attached to A.

In some embodiments, L³ is: 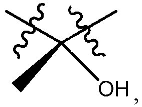 wherein the left side is attached to A.

In some embodiments, the L³ is optionally substituted, wherein 1-5 hydrogen atoms are optionally substituted. In some embodiments, L¹ is an optionally substituted version thereof wherein 1-3 hydrogen atoms are optionally substituted. In some embodiments, substituents include without limitation C₁-C₆ alkyl optionally substituted with 1-3 halo, such as fluoro. In some embodiments, substituents include without limitation C₁-C₆ alkyl optionally substituted with C₁-C₆ alkoxy. In some embodiments, substituents include without limitation an optionally substituted C₁-C₆ alkoxy. In some embodiments, substituents include without limitation a halo. In some embodiments, substituents include a fluoro.

In some embodiments, B is an optionally substituted 6-10 membered aryl. In some embodiments, B is an optionally substituted 5-15 membered heteroaryl. In some embodiments, B is an optionally substituted 4-15 membered heterocyclyl. In some embodiments, B is an optionally substituted 3-15 membered cycloalkyl. In some embodiments, if B is a 3-15 membered cycloalkyl, then B is at least a 4 membered cycloalkyl. In some embodiments, if B is a 3-15 membered cycloalkyl, then B is a 5-10 membered cycloalkyl.

In some embodiments, B is selected from the group consisting of: 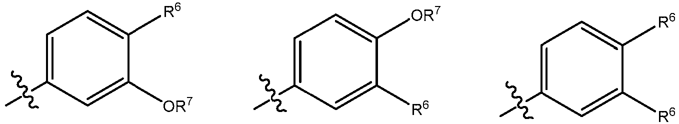 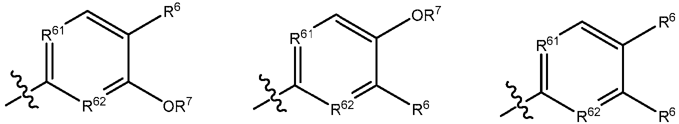 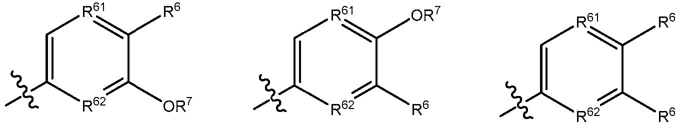 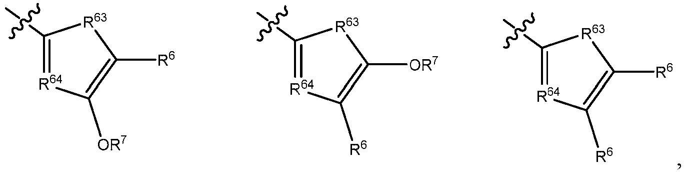 wherein
each R⁶ independently is hydrogen, an optionally substituted C₁-C₆ alkoxy, or halo;
each R⁷ independently is an optionally substituted C₁-C₆ alkyl, an optionally substituted C₂-C₆ alkenyl, an optionally substituted C₂-C₆ alkynyl, an optionally substituted C₃-C₈ cycloalkyl, an optionally substituted C₃-C₁₀ heteroaryl, an optionally substituted C₃-C₁₀ heterocyclyl, or an optionally substituted C₆-C₁₀ aryl such as optionally substituted phenyl; or
R⁶ and R⁷ together with the atoms they are attached to form an optionally substituted 5-7 membered ring; or 2 R⁶ groups together with the atoms they are attached to form an optionally substituted 5-7 membered ring;
each R⁶¹ and R⁶² is independently N or CH, provided that at least one of R⁶¹ and R⁶² is N,
each R⁶³ is independently NR⁹⁰, S, or O;
each R⁶⁴ is independently N or CH; and
each R⁹⁰ is independently hydrogen or R⁷,
and wherein one or more hydrogen atoms on the 5 and 6 membered aryl or heteroaryl rings shown above can be further optionally substituted.

In some embodiments, B is:

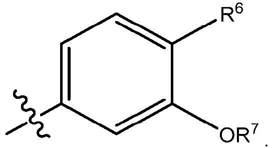

In some embodiments, B is:

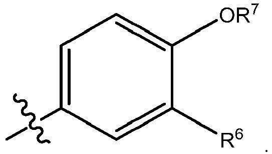

In some embodiments, B is:

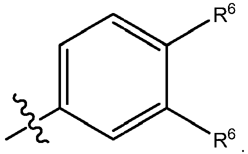

In some embodiments, B is:

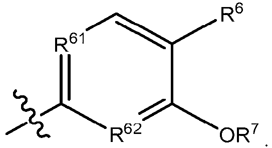

In some embodiments, B is:

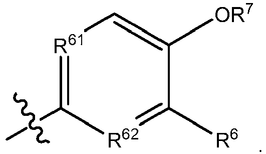

In some embodiments, B is:

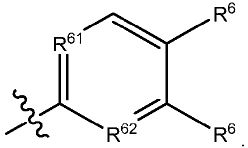

In some embodiments, B is:

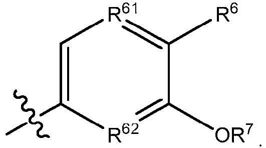

In some embodiments, B is:

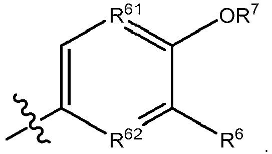

In some embodiments, B is:

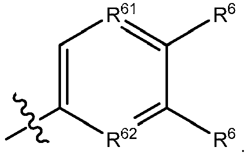

In some embodiments, B is:

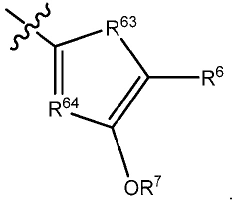

In some embodiments, B is:

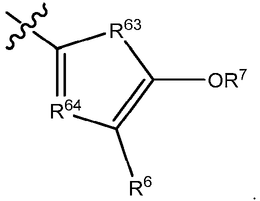

In some embodiments, B is:

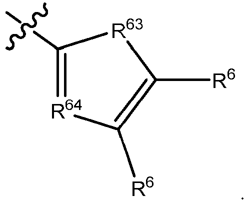

In some embodiments, R⁶ is hydrogen. In some embodiments, R⁶ is an optionally substituted C₁-C₆ alkoxy. In some embodiments, R⁶ is halo.

In some embodiments, R⁷ is an optionally substituted C₁-C₆ alkyl. In some embodiments, R⁷ is an optionally substituted C₂-C₆ alkenyl. In some embodiments, R⁷ is an optionally substituted C₂-C₆ alkynyl. In some embodiments, R⁷ is an optionally substituted C₃-C₈ cycloalkyl. In some embodiments, R⁷ is an optionally substituted C₃-C₁₀ heteroaryl. In some embodiments, R⁷ is an optionally substituted C₃-C₁₀ heterocyclyl. In some embodiments, R⁷ is an optionally substituted C₆-C₁₀ aryl. In some embodiments, the optionally substituted C₆-C₁₀ aryl is an optionally substituted phenyl.

In some embodiments, R⁶ and R⁷ together with the atoms they are attached to form an optionally substituted 5-7 membered ring. In some embodiments, 2 R⁶ groups together with the atoms they are attached to form an optionally substituted 5-7 membered ring.

In some embodiments, one of R⁶¹ and R⁶² is N. In some embodiments, both the R⁶¹ and R⁶² are N.

In some embodiments, R⁶³ is NR⁹⁰. In some embodiments, R⁶³ is S. In some embodiments, R⁶³ is O.

In some embodiments, R⁶⁴ is N. In some embodiments, R⁶⁴ is CH.

In some embodiments, R⁹⁰ is hydrogen. In some embodiments, R⁹⁰ is R⁷.

In some embodiments, B is 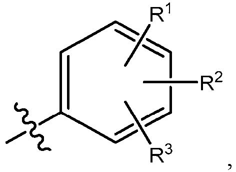 wherein
each R¹-R³ independently is H, halo, an optionally substituted C₁-C₆ alkyl, an optionally substituted 4-15 membered heterocyclyl, or -OR²⁰ or, if two of R¹ -R³ are on adjacent carbon atoms, then two such substituents together with the atoms they are attached to form an optionally substituted 5-7 membered ring;
R²⁰ is (CH₂)_{w}-R²¹, an optionally substituted C₃-C₆ cycloalkyl, or an optionally substituted C₁-C₆ alkyl;
R²¹ is an optionally substituted C₁-C₁₀ alkyl, an optionally substituted C₂-C₁₀ alkenyl, an optionally substituted C₂-C₁₀ alkynyl, an optionally substituted C₃-C₆ cycloalkyl, optionally substituted phenyl, optionally substituted 5-15 membered heteroaryl, an optionally substituted 4-15 membered heterocyclyl, or 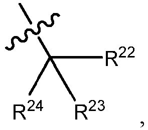 wherein each R²²-R²⁴ independently is an optionally substituted C₁-C₃ alkyl or hydroxy or two of R²²-R²⁴ together with the carbon atoms they are attached to form a 3-7 membered, preferably a 3-5 membered, or a 5-7 membered ring; and
w is 1, 2, 3, 4, or 5.

In some embodiments, R¹ is H. In some embodiments, R¹ is halo. In some embodiments, R¹ is an optionally substituted C₁-C₆ alkyl. In some embodiments, R¹ is H. In some embodiments, R¹ is an optionally substituted 4-15 membered heterocyclyl. In some embodiments, R¹ is -OR²⁰.

In some embodiments, R² is H. In some embodiments, R² is halo. In some embodiments, R² is an optionally substituted C₁-C₆ alkyl. In some embodiments, R² is H. In some embodiments, R² is an optionally substituted 4-15 membered heterocyclyl. In some embodiments, R² is -OR²⁰.

In some embodiments, R³ is H. In some embodiments, R³ is halo. In some embodiments, R³ is an optionally substituted C₁-C₆ alkyl. In some embodiments, R³ is H. In some embodiments, R³ is an optionally substituted 4-15 membered heterocyclyl. In some embodiments, R³ is -OR²⁰.

In some embodiments, if two of R¹ -R³ are on adjacent carbon atoms, then two such substituents together with the atoms they are attached to form an optionally substituted 5-7 membered ring.

In some embodiments, each R¹-R³ independently is H. In some embodiments, each R¹-R³ independently is F. In some embodiments, each R¹-R³ independently is Cl. In some embodiments, each R¹-R³ independently is C₁-C₃ alkyl. In some embodiments, each R¹-R³ independently is OR²⁰.

In some embodiments, R²⁰ is (CH₂)_{w}-R²¹. In some embodiments, R²⁰ is an optionally substituted C₃-C₆ cycloalkyl. In some embodiments, R²⁰ is an optionally substituted C₁-C₆ alkyl. In some embodiments, R²⁰ is a C₁-C₆ alkyl. In some embodiments, R²⁰ is a C₁-C₆ alkyl substituted with 1-3 fluoro. In some embodiments, R²⁰ is a C₁-C₆ alkyl substituted with 1-2, preferably, a single hydroxy.

In some embodiments, R²⁰ is CH₂-R²¹. In some embodiments, R²⁰ is methyl optionally substituted with 2 or 3 fluorine atoms. In some embodiments, R²⁰ is C₃-C₆ cycloalkyl.

In some embodiments, w is 1. In some embodiments, w is 2. In some embodiments, w is 3. In some embodiments, w is 4. In some embodiments, w is 5.

In some embodiments, R²¹ is C₁-C₁₀ alkyl. In some embodiments, R²¹ is a branched C₃-C₁₀ alkyl optionally substituted with one or more hydroxy or fluoro. In some embodiments, R²¹ is isopropyl or t-butyl optionally substituted with one or more hydroxy or fluoro.

In some embodiments, R²¹ is

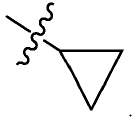

In some embodiments, R²¹ is

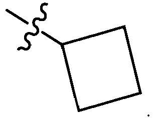

In some embodiments, R²¹ is

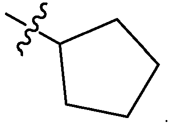

In some embodiments, R²¹ is

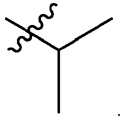

In some embodiments, R²¹ is

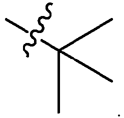

In some embodiments, R²¹ is

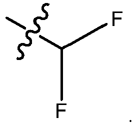

In some embodiments, R²¹ is

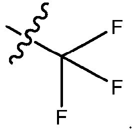

In some embodiments, R²¹ is

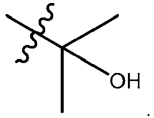

In some embodiments, R²¹ is a C₃-C₆ cycloalkyl. In some embodiments, R²¹ is a C₃-C₆ cycloalkyl substituted with 1-3, preferably 1-2 substituents. In some embodiments, R²¹ is a cyclopropyl. In some embodiments, R²¹ is a cyclopropyl substituted with 1-3, preferably 1-2 substituents. In some embodiments, R²¹ is a cyclobutyl. In some embodiments, R²¹ is a cyclobutyl substituted with 1-3, preferably 1-2 substituents. In some embodiments, R²¹ is a cyclopentyl. In some embodiments, R²¹ is a cyclopentyl substituted with 1-3, preferably 1-2 substituents. In some embodiments, R²¹ is an optionally substituted C₁-C₁₀ alkyl. In some embodiments, R²¹ is an optionally substituted C₂-C₁₀ alkenyl. In some embodiments, R²¹ is an optionally substituted C₂-C₁₀ alkynyl. In some embodiments, R²¹ is an optionally substituted 4-15 membered heterocyclyl.

In some embodiments, R²¹ is

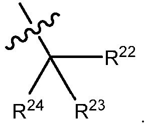

In some embodiments, R²² is an optionally substituted C₁-C₃ alkyl. In some embodiments, R²² is hydroxy. In some embodiments, R²² is H.

In some embodiments, R²³ is an optionally substituted C₁-C₃ alkyl. In some embodiments, R²³ is hydroxy.

In some embodiments, R²⁴ is an optionally substituted C₁-C₃ alkyl. In some embodiments, R²⁴ is hydroxy.

In some embodiments, each R²²-R²⁴ independently is an optionally substituted C₁-C₃ alkyl. In some embodiments, each R²²-R²⁴ independently is a hydroxy.

In some embodiments, two of R²²-R²⁴ together with the carbon atoms they are attached to form a 3-7 membered ring. In some embodiments, two of R²²-R²⁴ together with the carbon atoms they are attached to form a 5-7 membered ring. In some embodiments, the ring is optionally substituted cycloalkyl. In some embodiments, the ring is optionally substituted heterocyclyl.

In some embodiments, B is 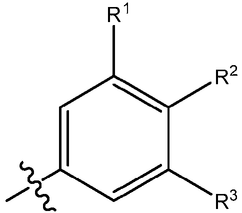 wherein
R¹, R², and R³ are as defined above; or
R¹ and R² together with the atoms they are attached to form an optionally substituted 5-7 membered ring; or
R² and R³ together with the atoms they are attached to form an optionally substituted 5-7 membered ring.

In some embodiments, R¹ and R² together with the atoms they are attached to form an optionally substituted 5-7 membered ring. In some embodiments, R² and R³ together with the atoms they are attached to form an optionally substituted 5-7 membered ring.

In some embodiments, wherein R¹ is H.

In some embodiments, R² is F. In some embodiments, R² is H.

In some embodiments, R² is H or -OR²⁰.

In some embodiments, R³ is F or H.

In some embodiments, R³ is H. In some embodiments, R³ is -OR²⁰, wherein R²⁰ is as defined above.

In some embodiments, B is: 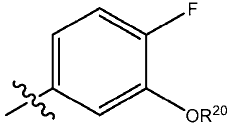 and wherein R²⁰ is as defined above.

In some embodiments, provided herein is a compound wherein A is: 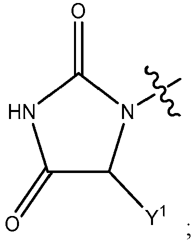
Y¹ is H or C₁-C₃ alkyl;
L¹ is an optionally substituted C₃-C₁₀ alkylene, further wherein at least two geminal hydrogens together with the carbon(s) to which they are attached are optionally replaced with cyclopropano or cyclobutano; optionally substituted C₃-C₁₀ alkenylene, optionally substituted C₃-C₁₀ heteroalkylene, optionally substituted C₃-C₁₀ heteroalkenylene, or -L¹¹-L¹²-L¹³-; wherein L¹¹ is attached to A and L¹¹ is O, S, NR, C₁-C₂ alkylene, C₂ alkenylene, C₂ heteroalkylene, C₃ heteroalkenylene; L¹² is arylene or heteroarylene; L¹³ is a bond or an optionally substituted C₁-C₅ alkylene; and R is H or C₁-C₃ alkyl;
L² is -S(O)₂NH-, wherein the sulfur is attached to L¹ or -NHS(O)₂-, wherein the nitrogen is attached to L¹;
L³ is a bond or an optionally substituted C₁-C₆ alkylene, preferably 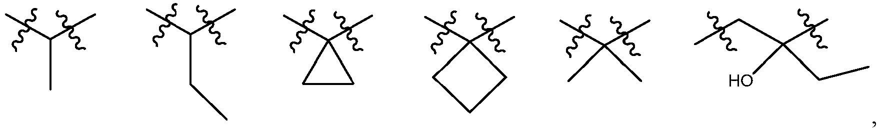 more preferably: 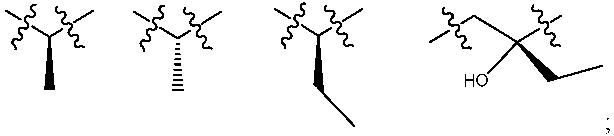
B is: 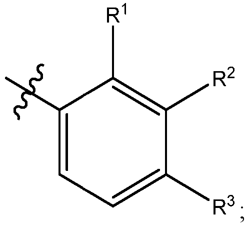
each R¹-R³ independently is H, F, Cl, C₁-C₃ alkyl, or OR²⁰;
R²⁰ is CH₂-R²¹; methyl optionally substituted with 2 or 3 fluorine atoms; C₃-C₆ cycloalkyl; or C₁-C₆ alkyl;
R²¹ is an optionally substituted C₃-C₆ cycloalkyl; an optionally substituted C₆-C₁₀ aryl; an optionally substituted 5-15 membered heteroaryl; an optionally substituted 4-15 membered heterocyclyl; C₁-C₁₀ alkyl, preferably branched C₃-C₁₀ alkyl, more preferably isopropyl or t-butyl, optionally substituted with one or more hydroxy or fluoro; C₃-C₆ cycloalkyl, preferably cyclopropyl, cyclobutyl, cyclopentyl; or 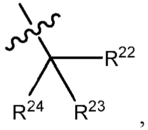 wherein each R²²-R²⁴ independently is an optionally substituted C₁-C₃ alkyl or hydroxyl, or two of R²² -R²⁴ together with the atoms they are attached to form an optionally substituted 3-7 membered ring.

In some embodiments, provided herein is a compound wherein A is 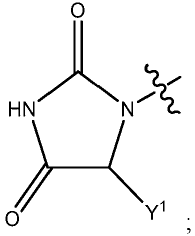
Y¹ is H or C₁-C₃ alkyl;
L¹ is an optionally substituted C₃-C₁₀ alkylene, further wherein at least two geminal hydrogens together with the carbon(s) to which they are attached are optionally replaced with cyclopropano or cyclobutano; optionally substituted C₃-C₁₀ alkenylene, optionally substituted C₃-C₁₀ heteroalkylene, optionally substituted C₃-C₁₀ heteroalkenylene, or -L¹¹-L¹²-L¹³-, wherein L¹¹ is attached to A and L¹¹ is O, S, NR, C₁-C₂ alkylene, C₂ alkenylene, C₂ heteroalkylene, C₃ heteroalkenylene, L¹² is arylene or heteroarylene, L¹³ is a bond or an optionally substituted C₁-C₅ alkylene, and R is H or C₁-C₃ alkyl;
L² is -S(O)₂NH-, wherein the sulfur is attached to L¹ or -NHS(O)₂-, wherein the nitrogen is attached to L¹;
L³ is a bond or an optionally substituted C₁-C₆ alkylene;
B is 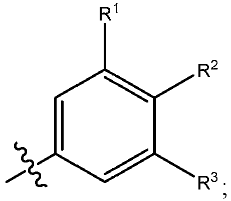
each R¹-R³ independently is H, F, Cl, C₁-C₃ alkyl, or -OR²⁰; or
R¹ and R² together with the atoms they are attached to form an optionally substituted 5-7 membered ring; or
R² and R³ together with the atoms they are attached to form an optionally substituted 5-7 membered ring;
R²⁰ is CH₂-R²¹; methyl optionally substituted with 2 or 3 fluorine atoms; C₃-C₆ cycloalkyl; or C₁-C₆ alkyl;
R²¹ is an optionally substituted C₃-C₆ cycloalkyl; an optionally substituted C₆-C₁₀ aryl; an optionally substituted 5-15 membered heteroaryl; an optionally substituted 4-15 membered heterocyclyl; C₁-C₁₀ alkyl, preferably branched C₃-C₁₀ alkyl optionally substituted with one or more hydroxy or fluoro; C₃-C₆ cycloalkyl; or 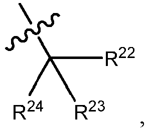 wherein each R²²-R²⁴ independently is an optionally substituted C₁-C₃ alkyl or hydroxy; or
two of R²² -R²⁴ together with the atoms they are attached to form an optionally substituted 3-7 membered ring.

In some embodiments, Y¹ is H. In some embodiments, Y¹ is C₁-C₃ alkyl.

In some embodiments, L¹ is an optionally substituted C₃-C₁₀ alkylene, further wherein at least two geminal hydrogens together with the carbon(s) to which they are attached are optionally replaced with cyclopropano or cyclobutano. In some embodiments, L¹ is an optionally substituted C₃-C₁₀ alkenylene. In some embodiments, L¹ is optionally substituted C₃-C₁₀ heteroalkylene. In some embodiments, L¹ is optionally substituted C₃-C₁₀ heteroalkenylene.

In some embodiments, L¹ is -L¹¹-L¹²-L¹³-, wherein L¹¹ is attached to A. In some embodiments, L¹¹ is O. In some embodiments, L¹¹ is S. In some embodiments, L¹¹ is C₁-C₂ alkylene. In some embodiments, L¹¹ is C₂ alkenylene. In some embodiments, L¹¹ is C₂ heteroalkylene. In some embodiments, L¹¹ is C₃ heteroalkenylene.

In some embodiments, L¹¹ is NR. In some embodiments, R is H. In some embodiments, R is C₁-C₃ alkyl.

In some embodiments, L¹² is arylene. In some embodiments, L¹² is heteroarylene.

In some embodiments, L¹³ is a bond. In some embodiments, L¹³ is an optionally substituted C₁-C₅ alkylene.

In some embodiments, L² is -S(O)₂NH-, wherein the sulfur is attached to L¹ or - NHS(O)₂-, wherein the nitrogen is attached to L¹.

In some embodiments, L³ is a bond. In some embodiments, L³ is an optionally substituted C₁-C₆ alkylene.

In some embodiments, R¹ is H. In some embodiments, R¹ is F. In some embodiments, R¹ is Cl. In some embodiments, R¹ is C₁-C₃ alkyl. In some embodiments, R¹ is -OR²⁰.

In some embodiments, R² is H. In some embodiments, R² is F. In some embodiments, R² is Cl. In some embodiments, R² is C₁-C₃ alkyl. In some embodiments, R² is -OR²⁰.

In some embodiments, R³ is H. In some embodiments, R³ is F. In some embodiments, R³ is Cl. In some embodiments, R³ is C₁-C₃ alkyl. In some embodiments, R³ is -OR²⁰.

In some embodiments, R¹ and R² together with the atoms they are attached to form an optionally substituted 5-7 membered ring. In some embodiments, R² and R³ together with the atoms they are attached to form an optionally substituted 5-7 membered ring.

In some embodiments, R²⁰ is CH₂-R²¹. In some embodiments, R²⁰ is a methyl optionally substituted with 2 or 3 fluorine atoms. In some embodiments, R²⁰ is C₃-C₆ cycloalkyl. In some embodiments, R²⁰ is C₁-C₆ alkyl.

In some embodiments, R²¹ is C₁-C₁₀ alkyl. In some embodiments, R²¹ is a branched C₃-C₁₀ alkyl optionally substituted with one or more hydroxy or fluoro. In some embodiments, R²¹ is C₃-C₆ cycloalkyl.

In some embodiments, R²¹ is

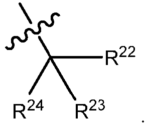

In some embodiments, R²² is an optionally substituted C₁-C₃ alkyl. In some embodiments, R²² is hydroxy.

In some embodiments, R²³ is an optionally substituted C₁-C₃ alkyl. In some embodiments, R²³ is hydroxy.

In some embodiments, R²⁴ is an optionally substituted C₁-C₃ alkyl. In some embodiments, R²⁴ is hydroxy.

In some embodiments, two of R²² -R²⁴ together with the atoms they are attached to form an optionally substituted 5-7 membered ring.

In some embodiments, B is selected from the group consisting of:

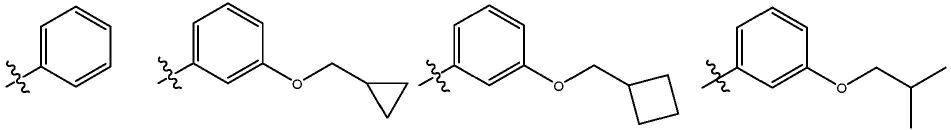

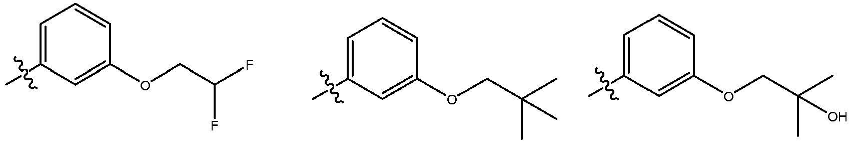

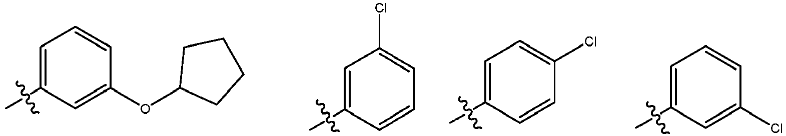

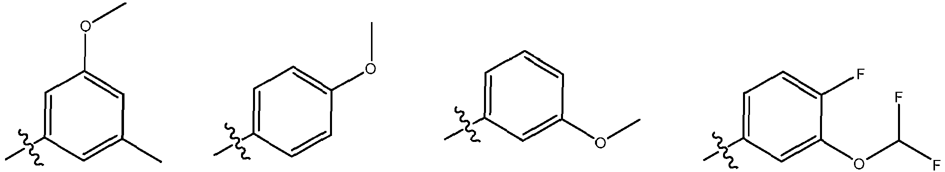

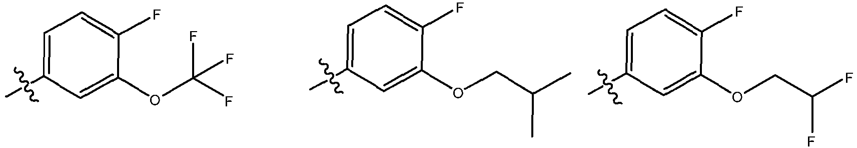

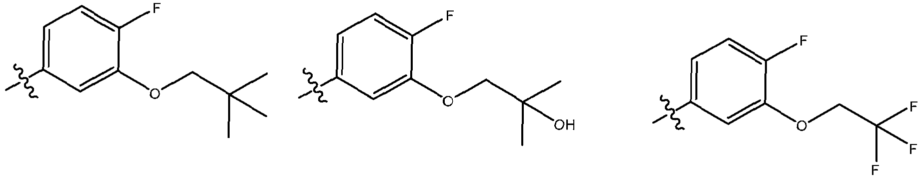

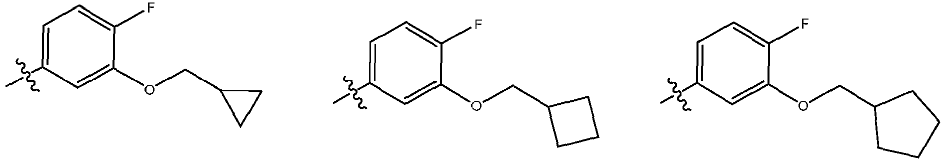

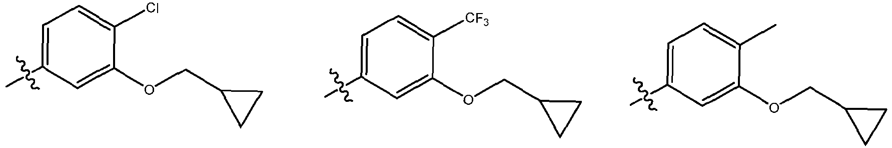

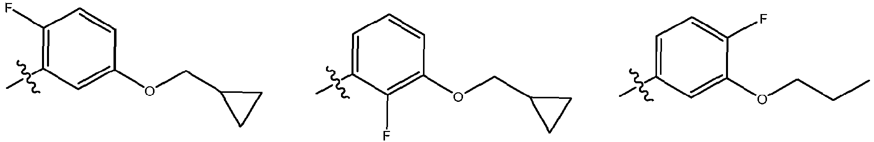

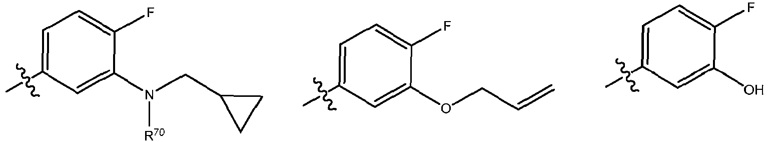

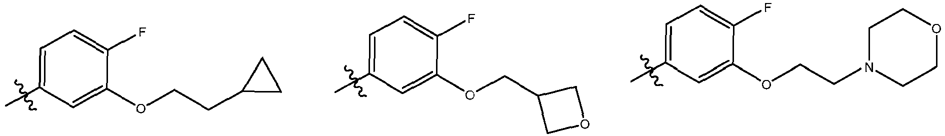

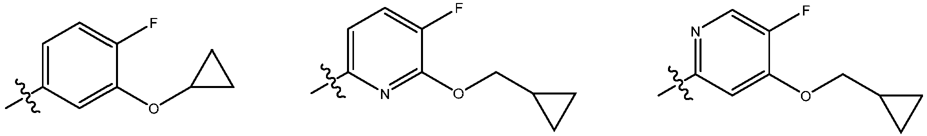

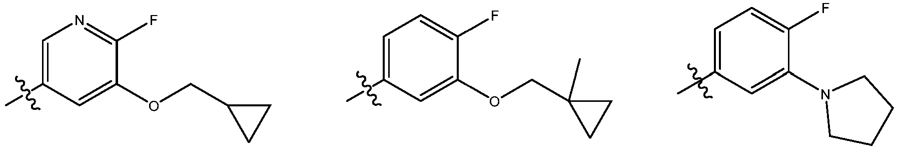

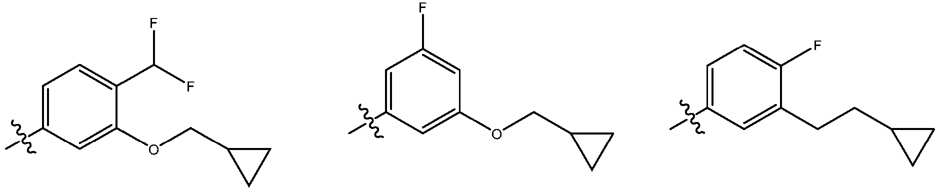

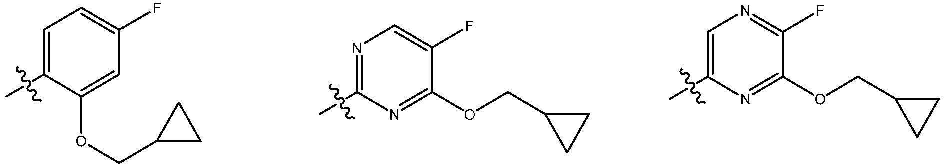

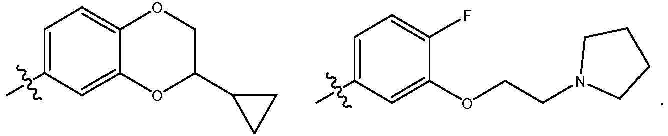

In some embodiments, the alkoxy group is further substituted wherein 1-5, preferably, 1-3 hydrogen atoms are substituted, preferred substituents including without limitation, C₁-C₆ alkyl optionally substituted with 1-3 halo, such as fluoro, and/or C₁-C₆ alkoxy; optionally substituted C₁-C₆ alkoxy; and halo, preferably fluoro. In some embodiments, substituents include without limitation C₁-C₆ alkyl substituted with 1-3 halo, such as fluoro. In some embodiments, substituents include without limitation C₁-C₆ alkyl optionally substituted with C₁-C₆ alkoxy. In some embodiments, substituents include without limitation a substituted C₁-C₆ alkoxy. In some embodiments, substituents include without limitation one or more halo. In some embodiments, substituents include one or more fluoro. In some embodiments, the ring moiety such as the cyclopropyl group is further substituted with 1-3 halo, preferably 1-2 halo. In some embodiments, the ring moiety, such as the cyclopropyl group, is further substituted with 1-2 halo. In some embodiments, the methylene group between the oxygen atom and the ring moiety, such as the cyclopropyl group, is substituted with 1-2 C₁-C₆ alkyl, preferably methyl, ethyl, or propyl groups. In some embodiments, the methylene group is substituted with methyl groups. In some embodiments, the methylene group is substituted with ethyl groups. In some embodiments, the methylene group is substituted with propyl groups. In some embodiments, R⁷⁰ is an optionally substituted C₁-C₁₀ alkyl.

In some embodiments, the alkoxy group is further optionally substituted wherein 1-5 hydrogen atoms are optionally substituted. In some embodiments, substituents include without limitation C₁-C₆ alkyl optionally substituted with 1-3 halo, such as fluoro. In some embodiments, substituents include without limitation C₁-C₆ alkyl optionally substituted with C₁-C₆ alkoxy. In some embodiments, substituents include without limitation an optionally substituted C₁-C₆ alkoxy. In some embodiments, substituents include without limitation a halo. In some embodiments, substituents include a fluoro.

In some embodiments, the ring moiety such as the cyclopropyl group is further optionally substituted with 1-3 halo. In some embodiments, the ring moiety, such as the cyclopropyl group, is further optionally substituted with 1-2 halo.

In some embodiments, the methylene group between the oxygen atom and the ring moiety, such as the cyclopropyl group, is optionally substituted with 1-2 C₁-C₆ alkyl. In some embodiments, the methylene group is optionally substituted with methyl groups. In some embodiments, the methylene group is optionally substituted with ethyl groups. In some embodiments, the methylene group is optionally substituted with propyl groups.

In some embodiments, B is:

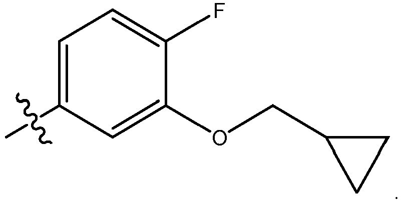

In some embodiments, the compound of Formula (I) is not

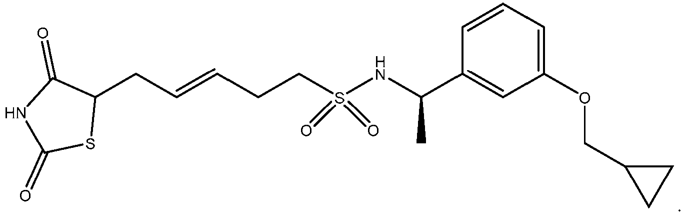

This disclosure also provides a stereochemically pure enantiomer of a compound as described herein, its tautomer, diastereoisomer or its pharmaceutically acceptable salt. Methods to purify and identify the pure enantiomer are known in the art and described herein.

In some embodiments, the dUTPase inhibitor is a compound selected from Table 1 below.

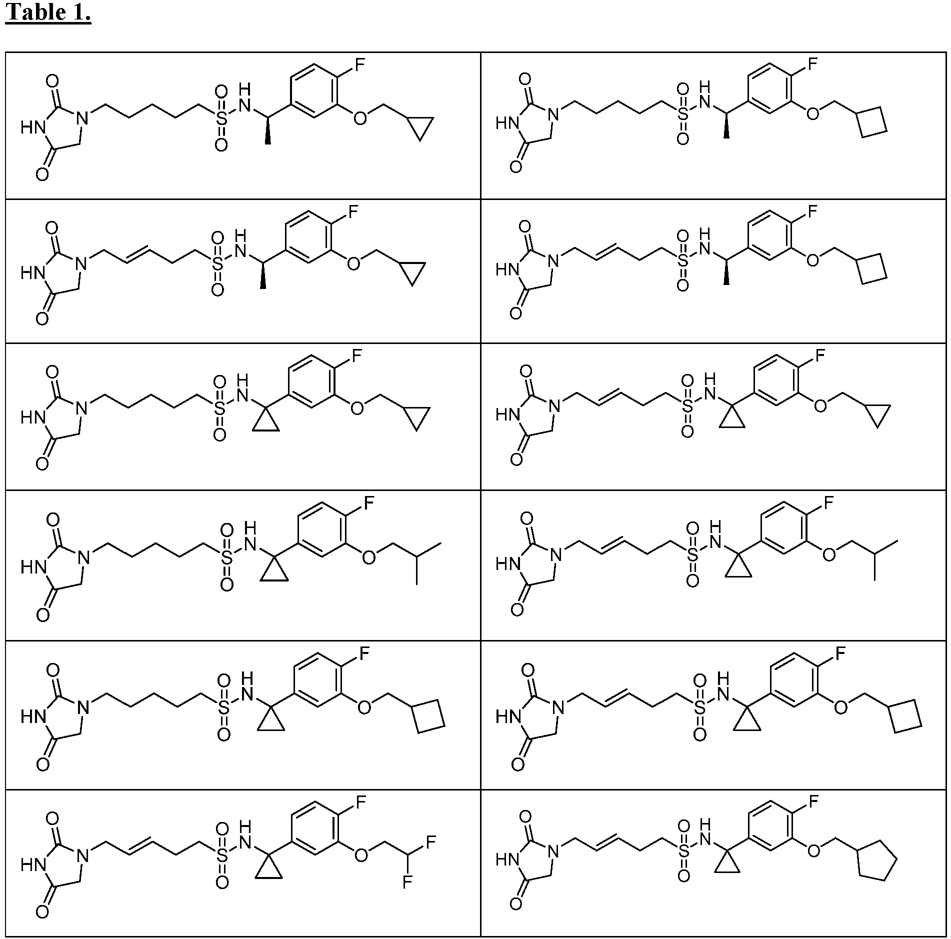

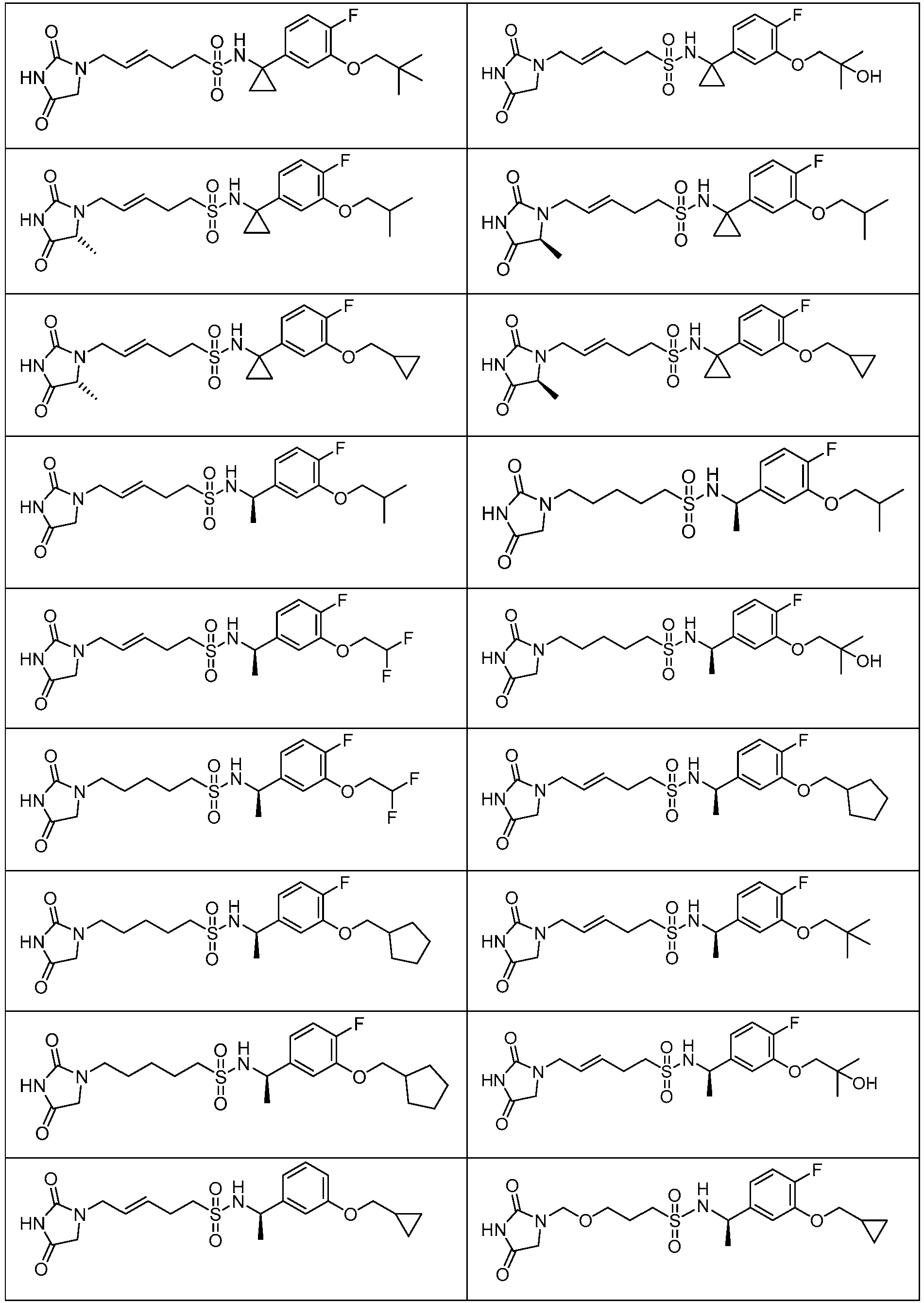

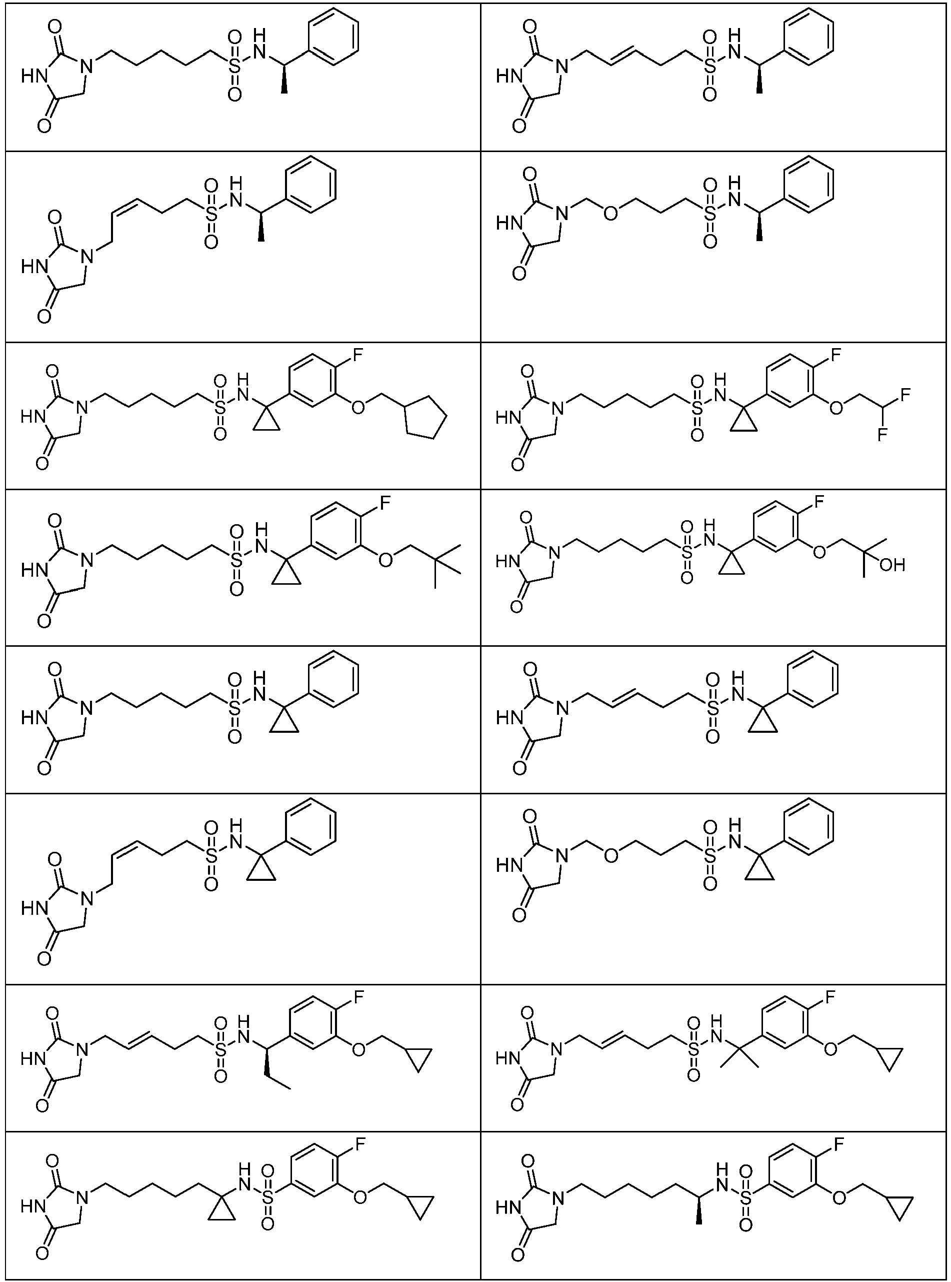

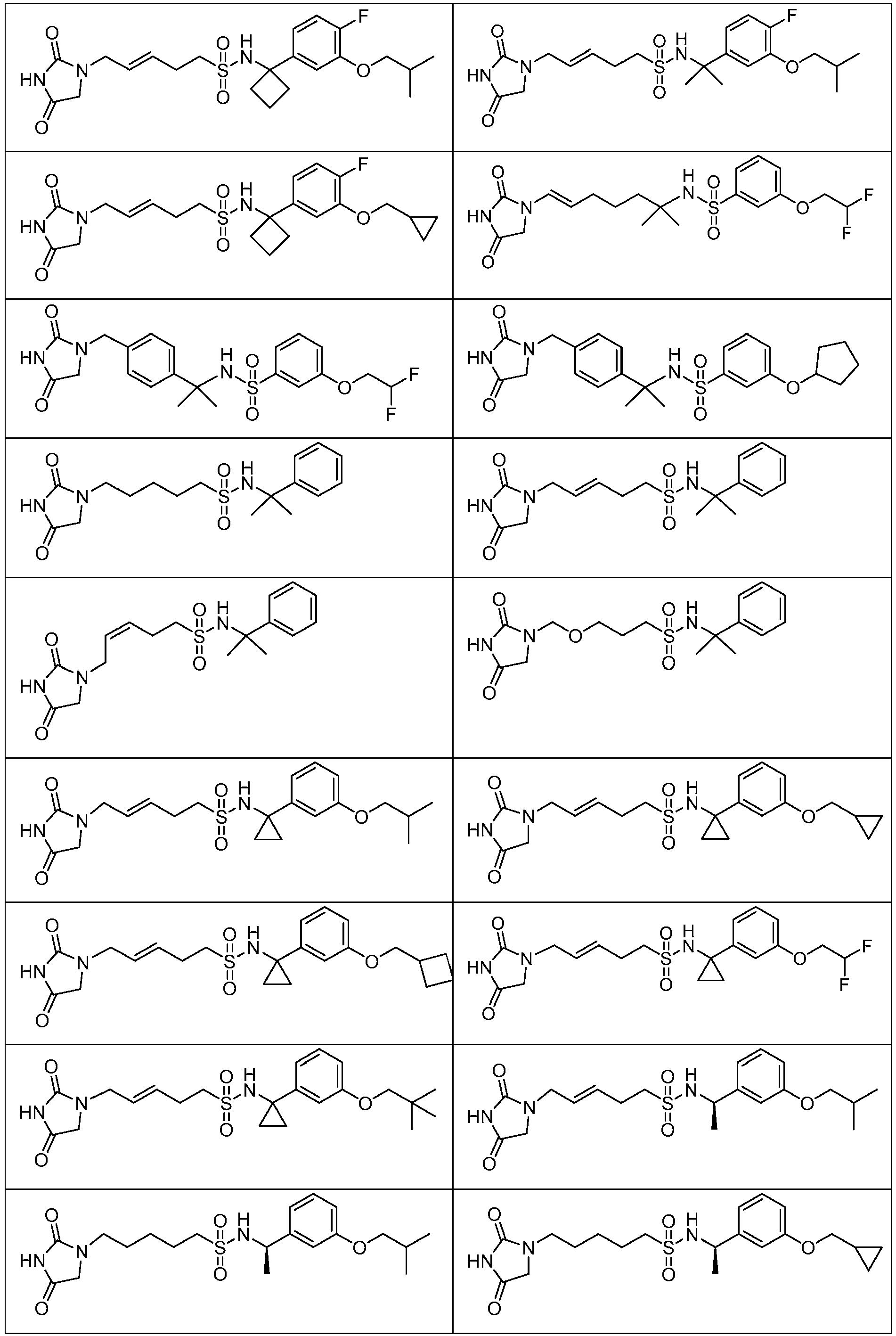

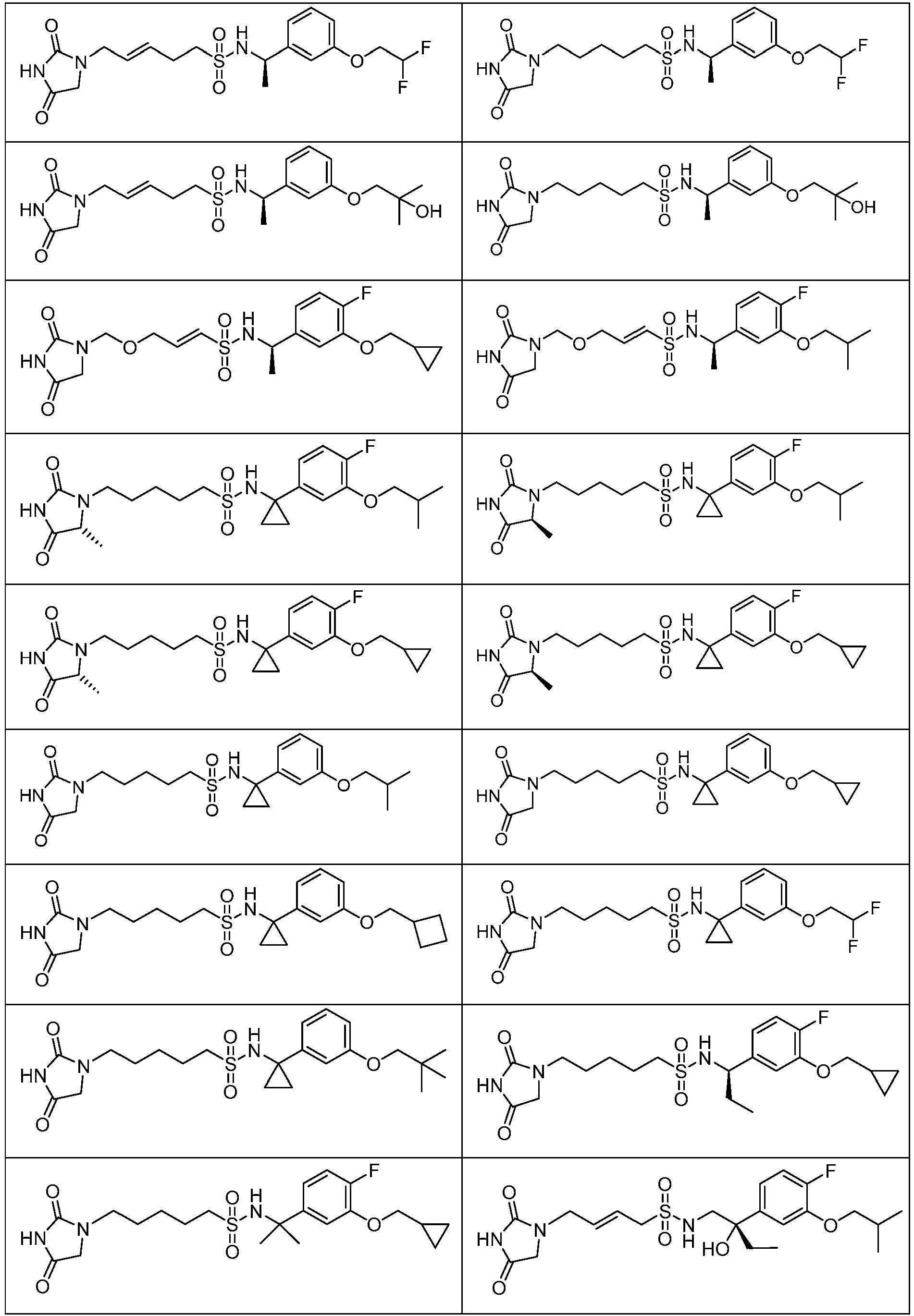

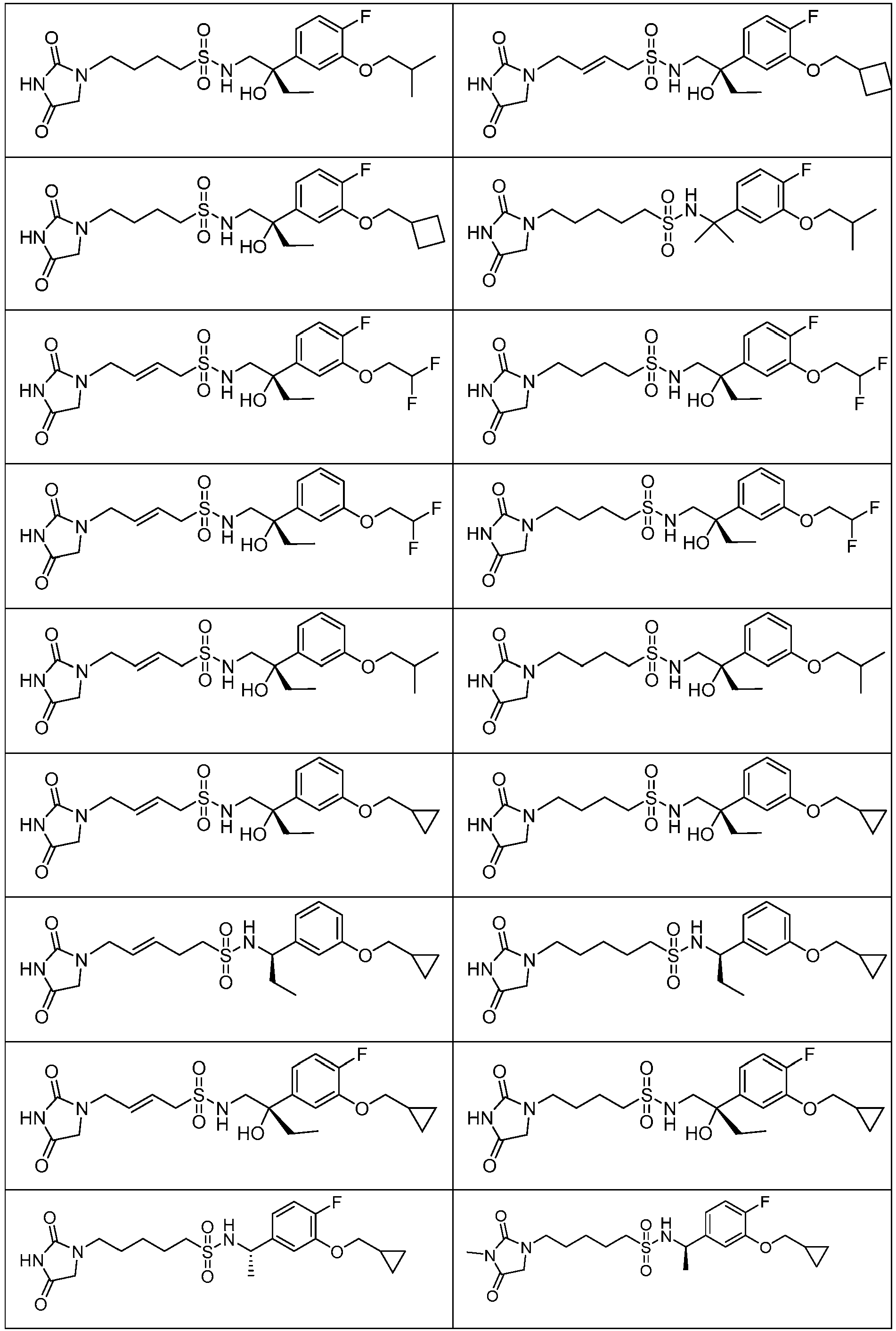

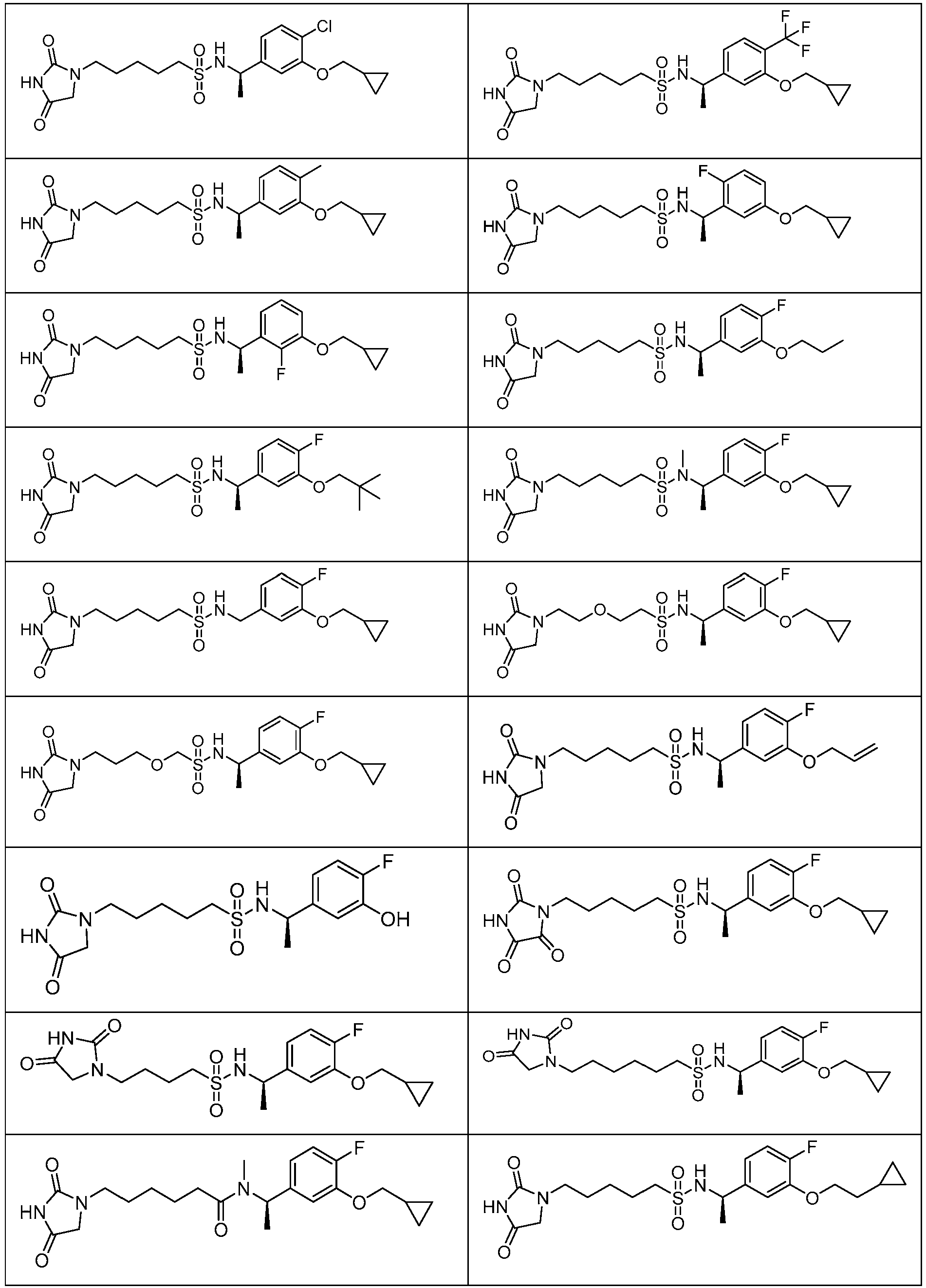

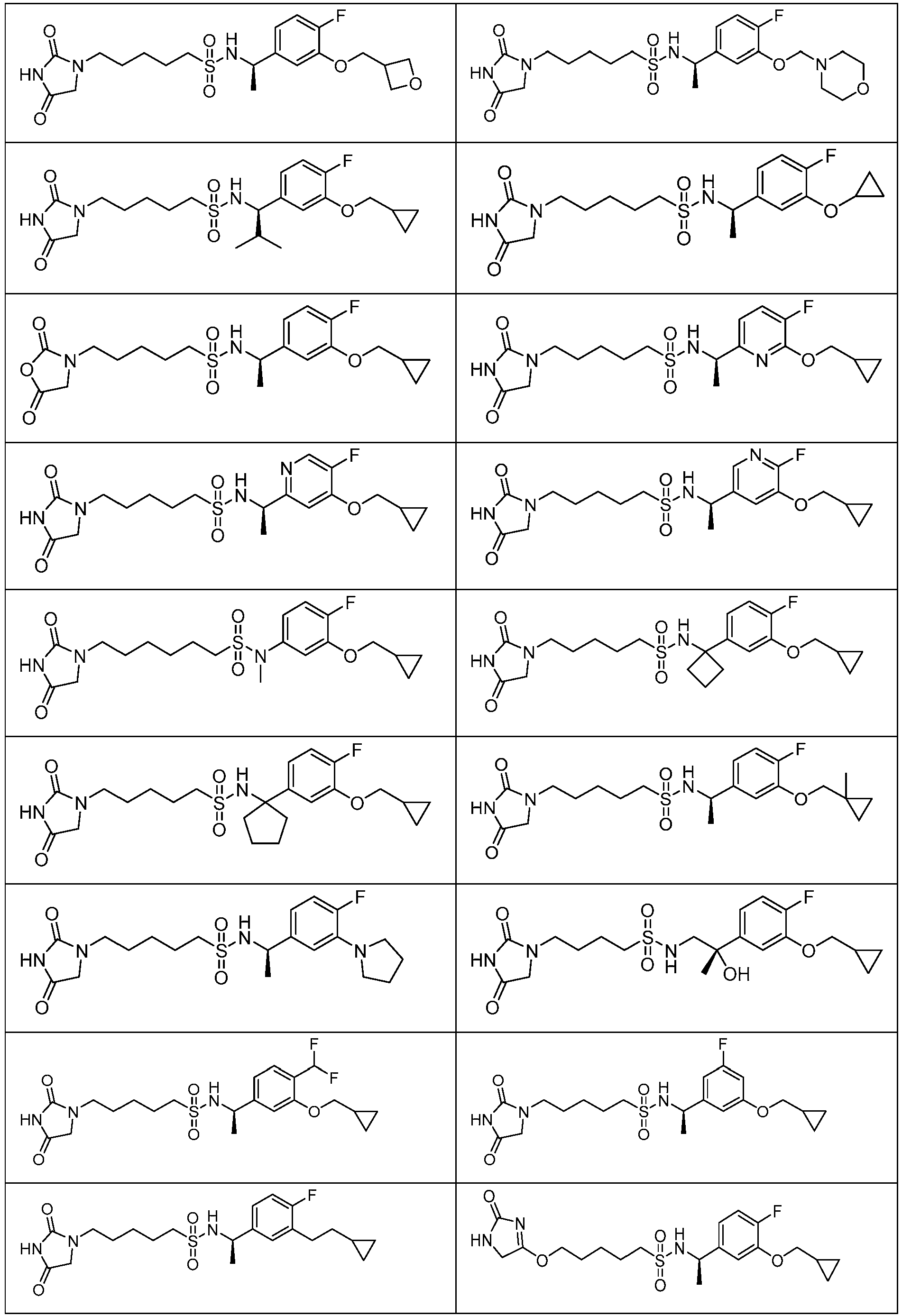

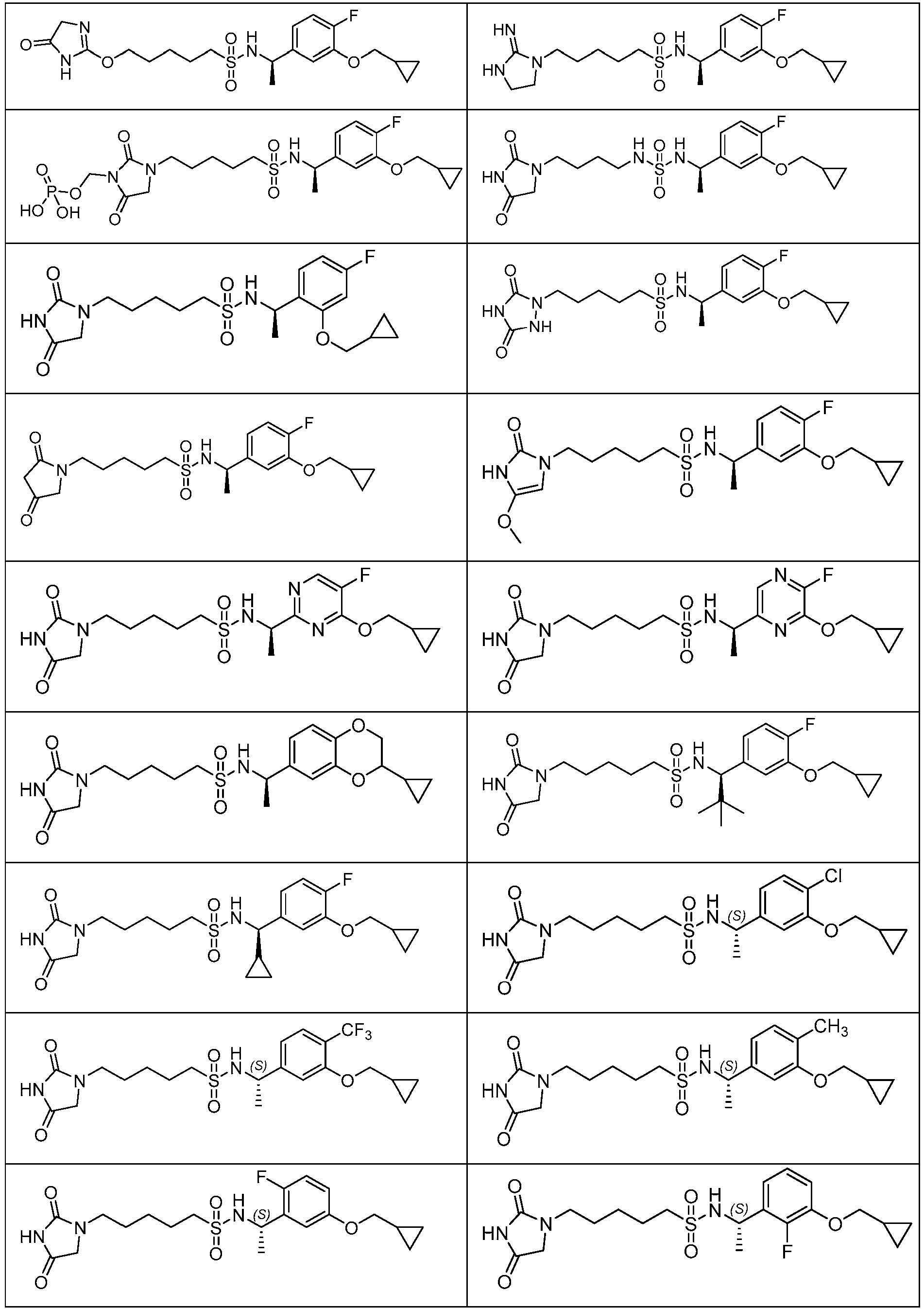

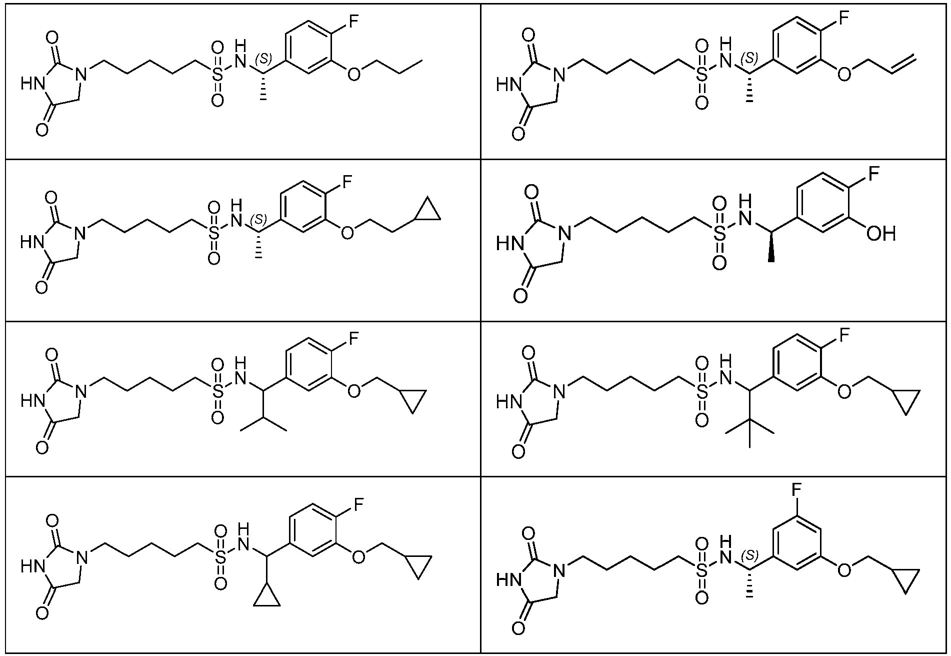

In some embodiments, the dUTPase inhibitor is a compound selected from Table 2 below.

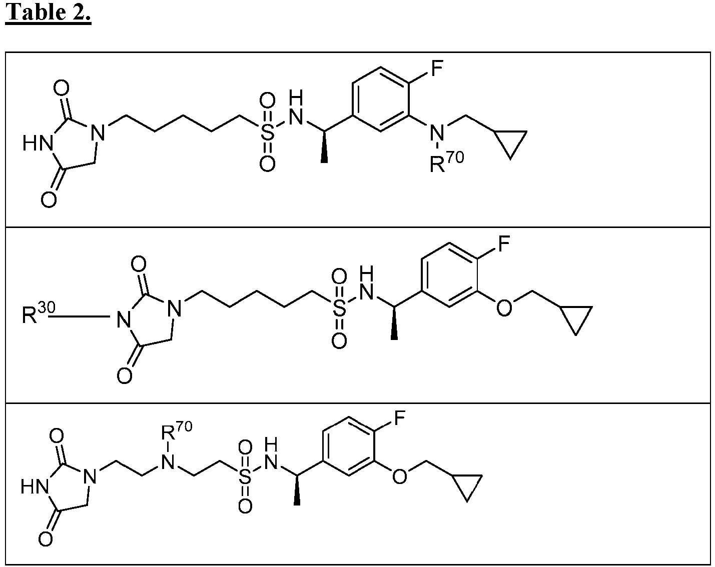

wherein R⁷⁰ is as defined above and R³⁰ is as defined above.

In some embodiments, the dUTPase inhibitor is a compound selected from Table 3 below.

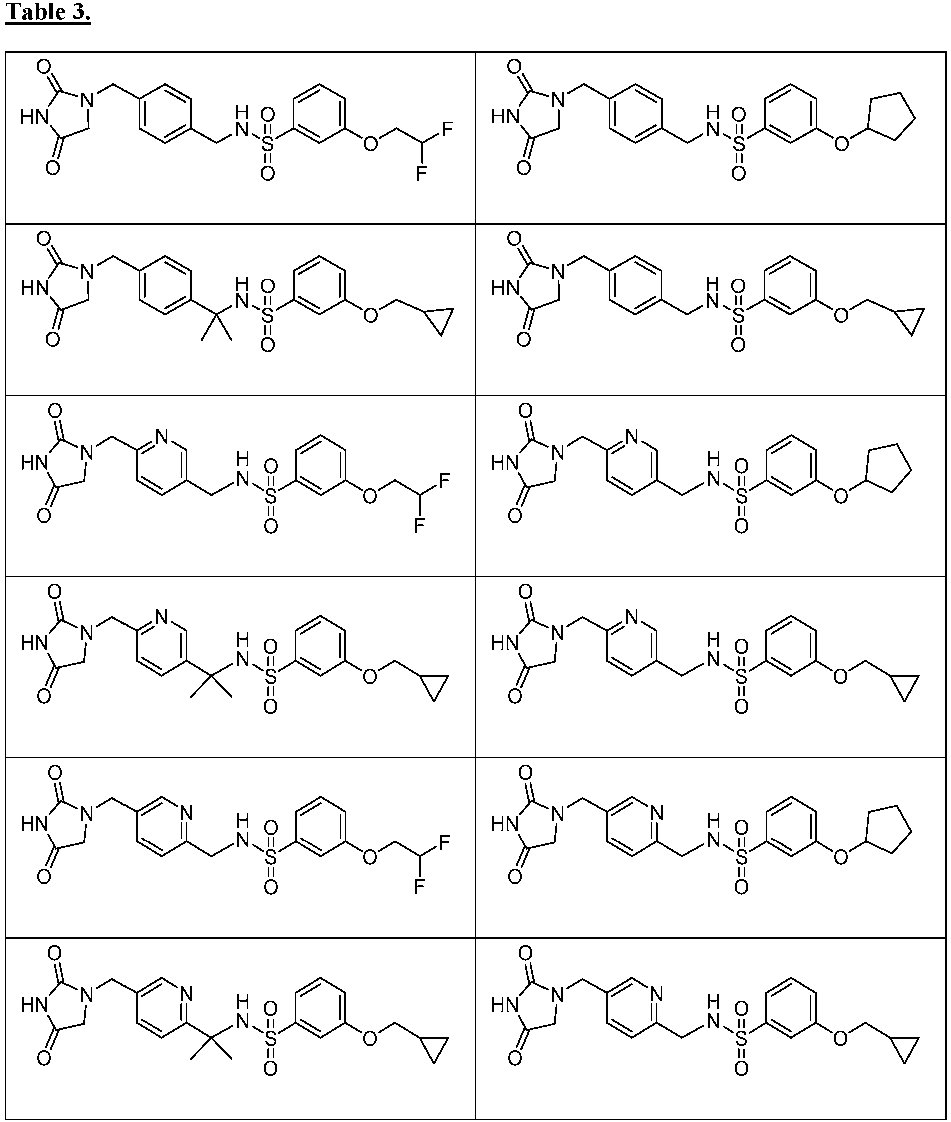

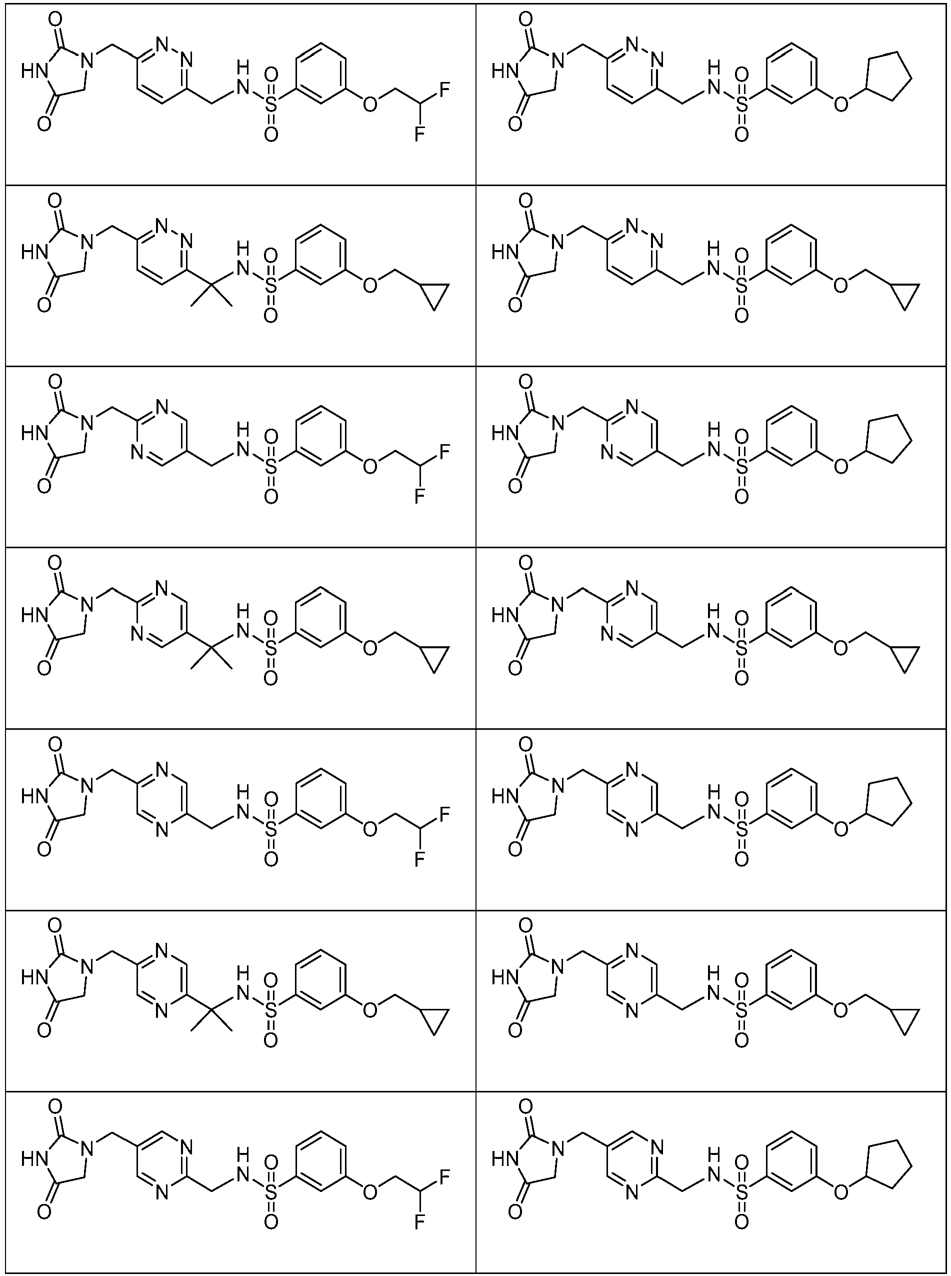

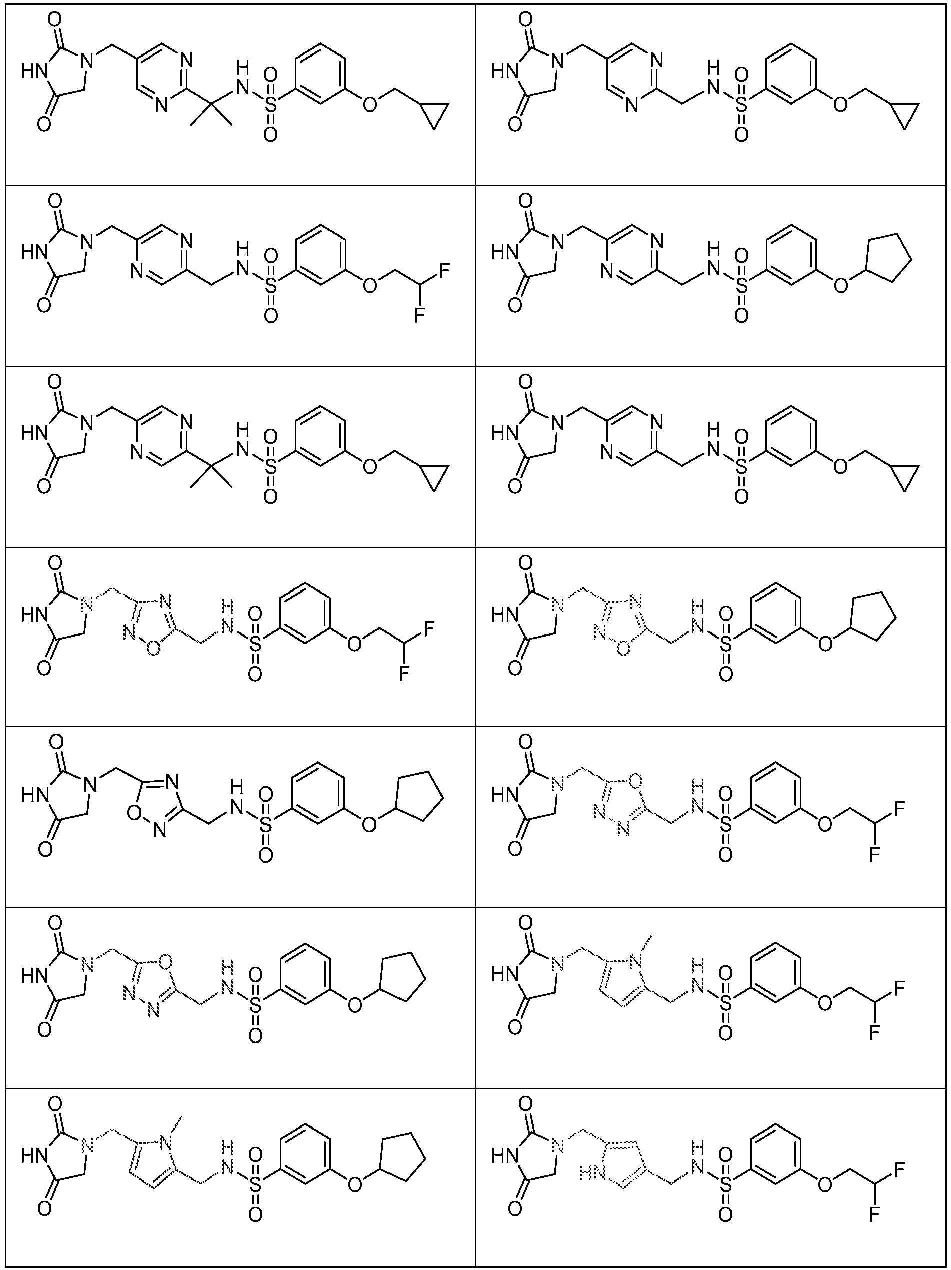

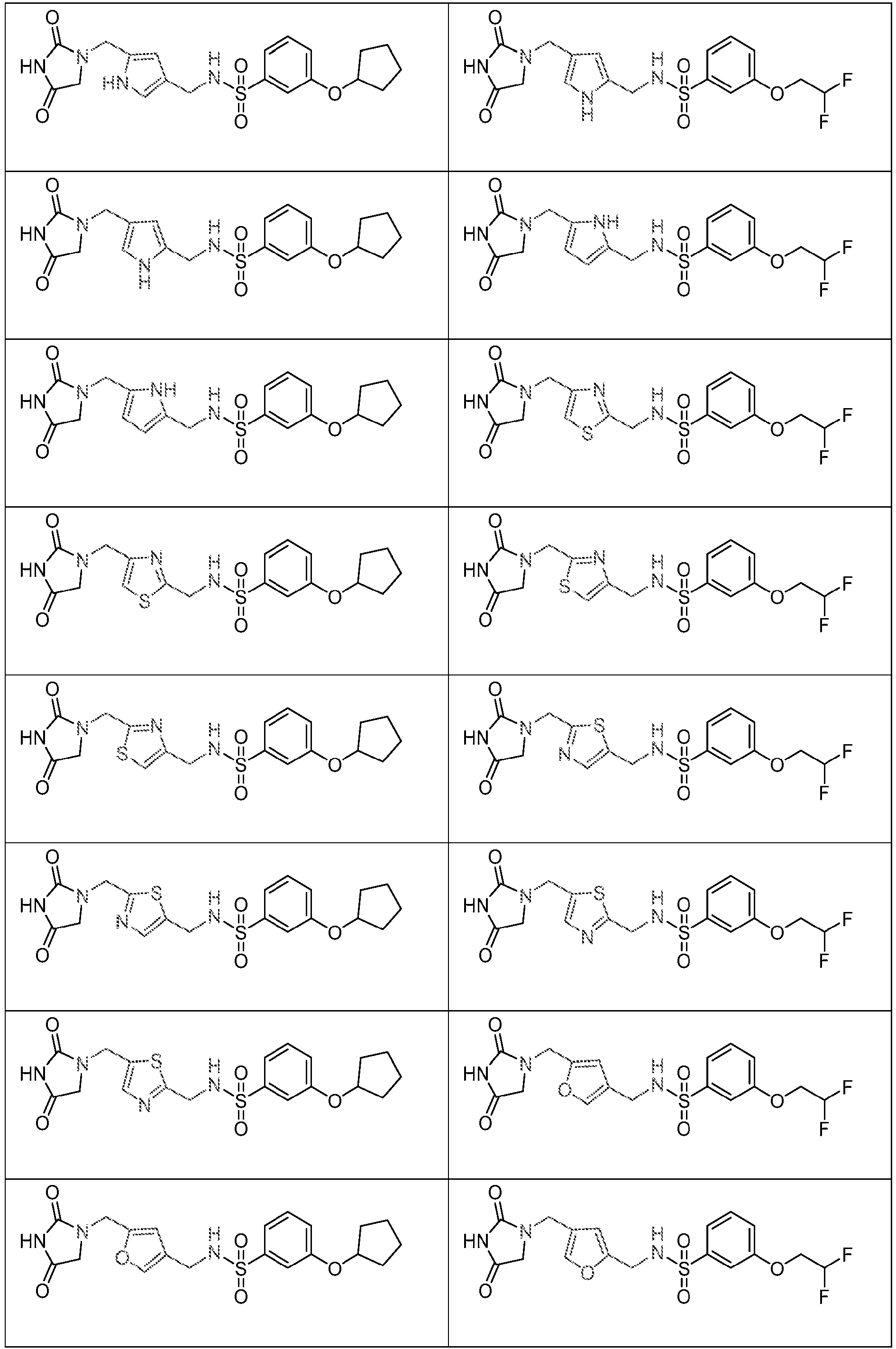

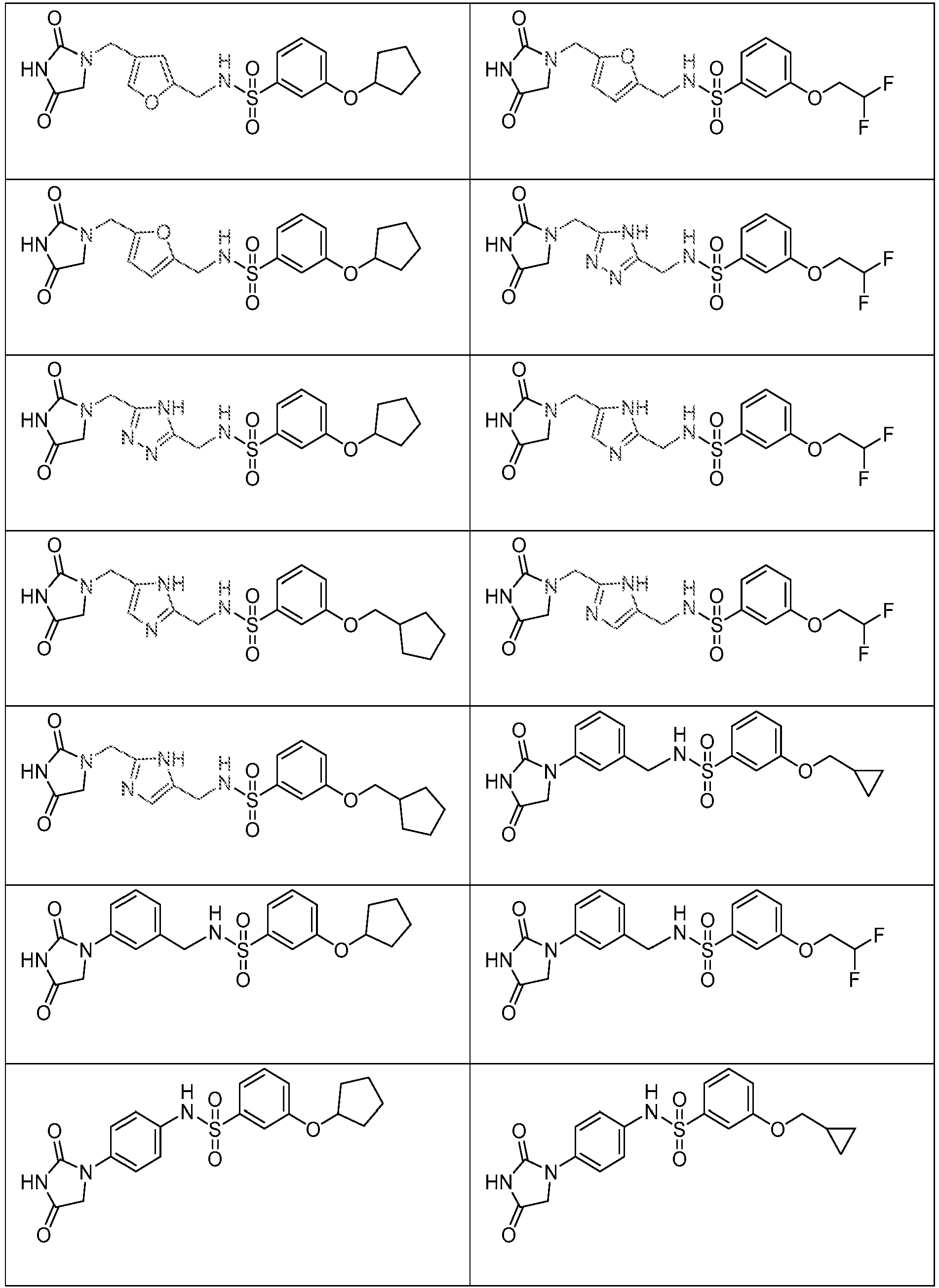

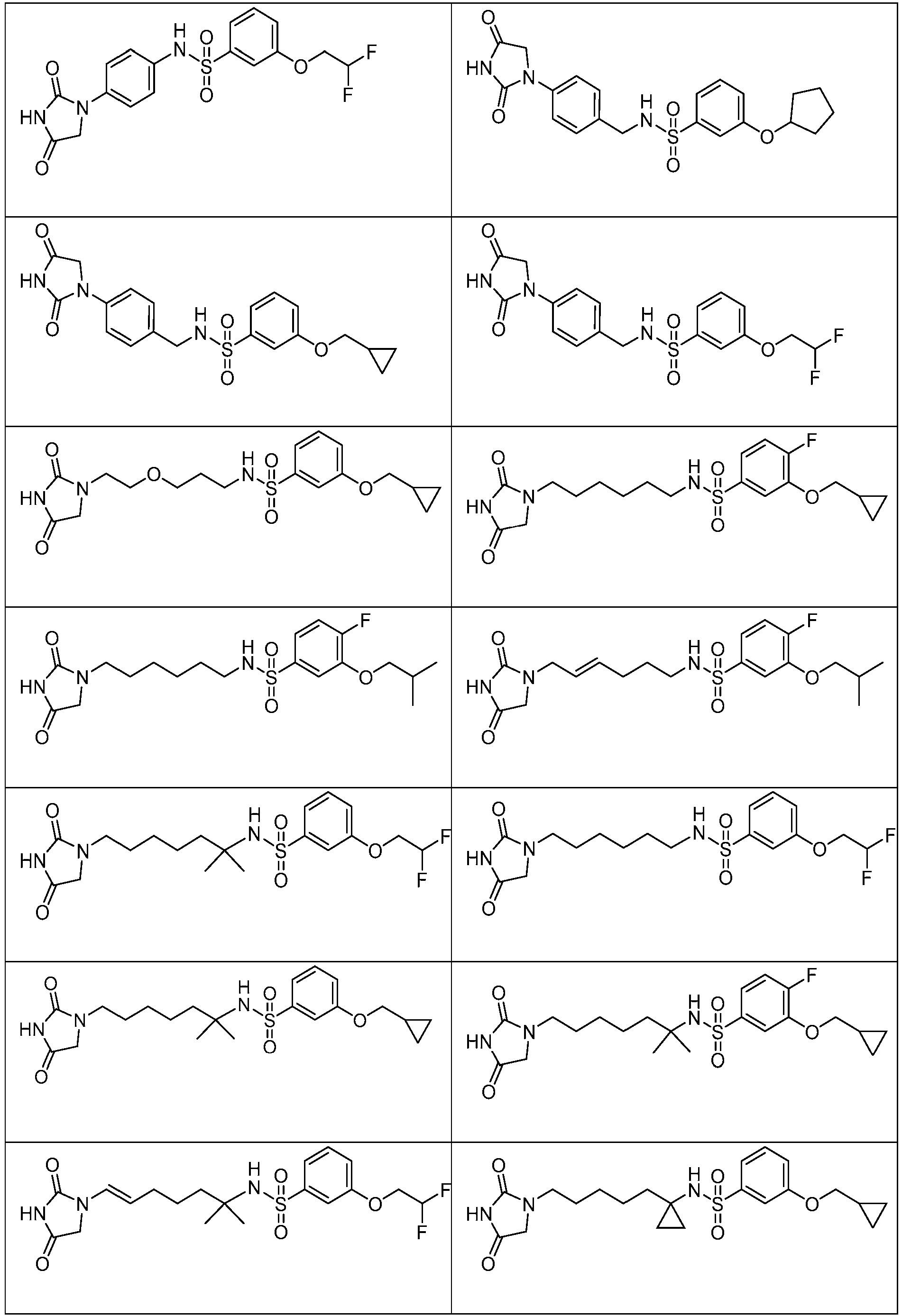

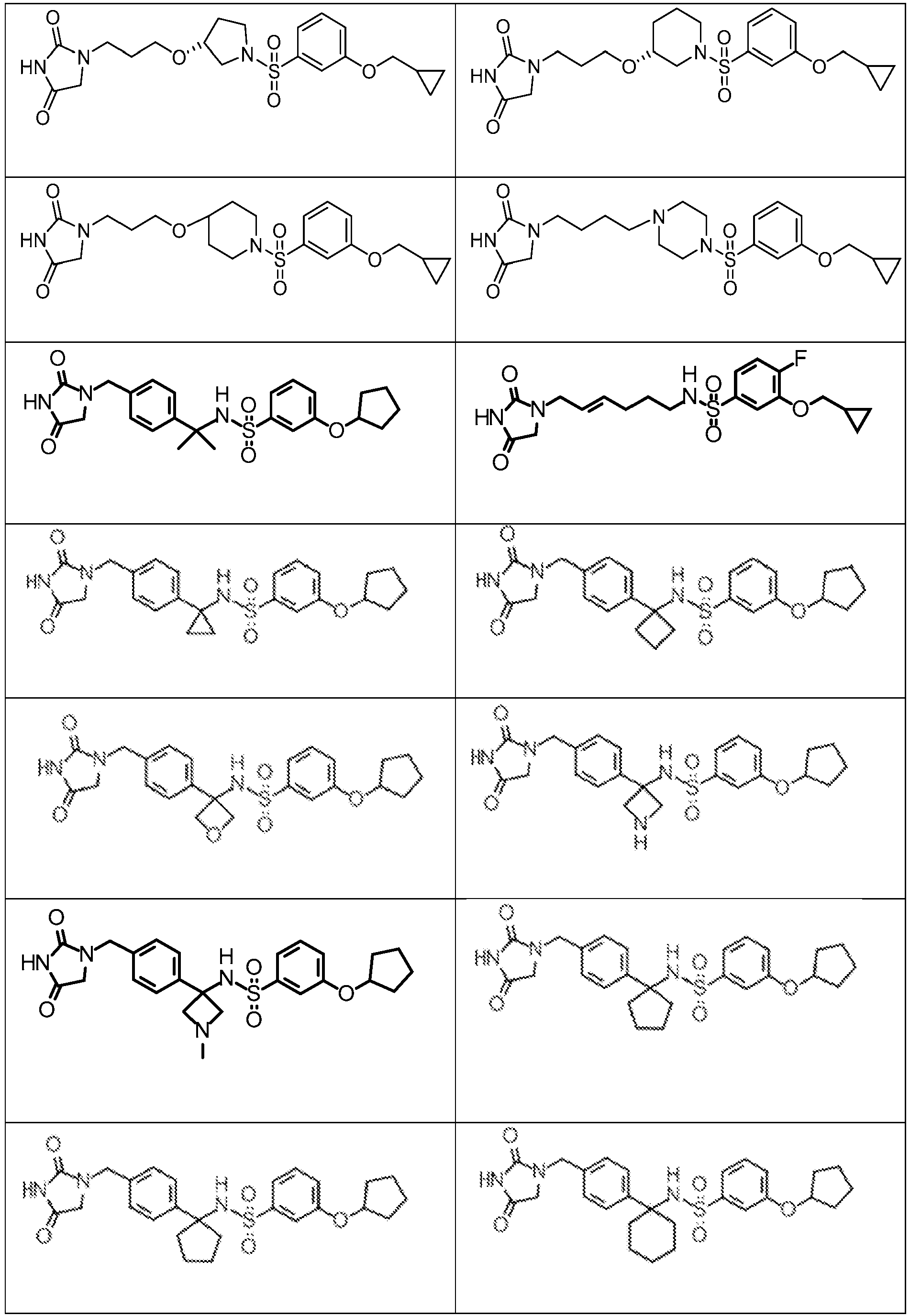

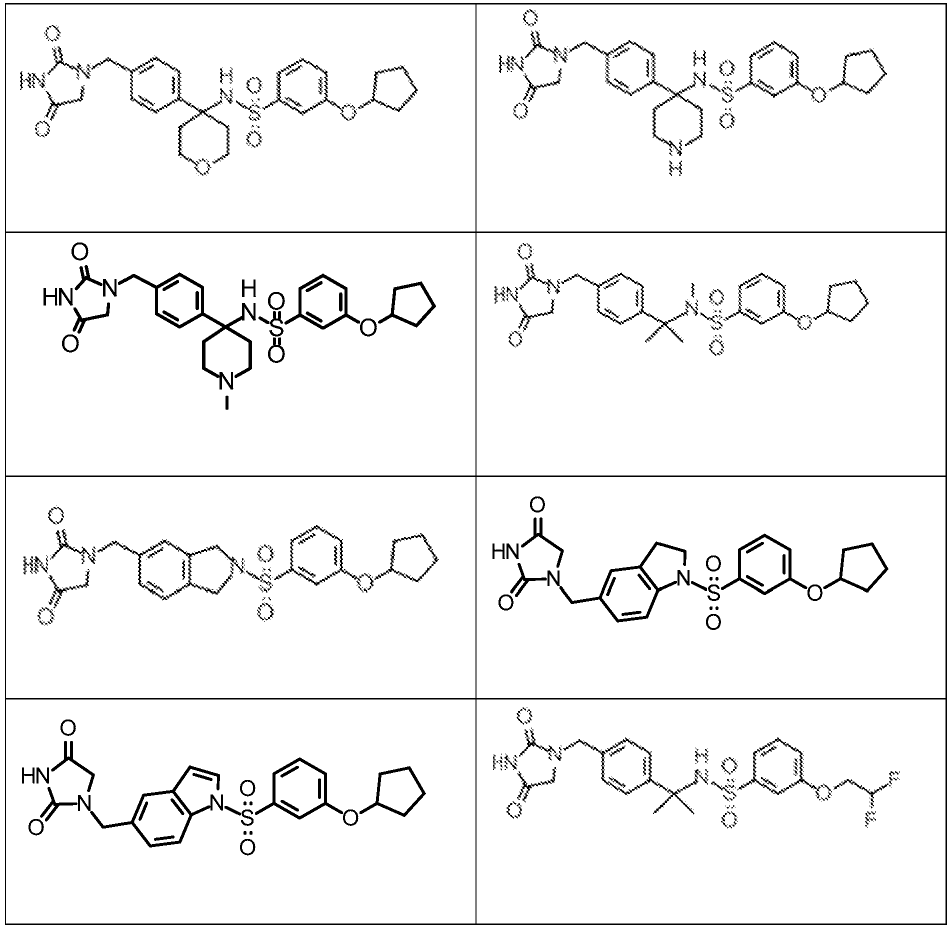

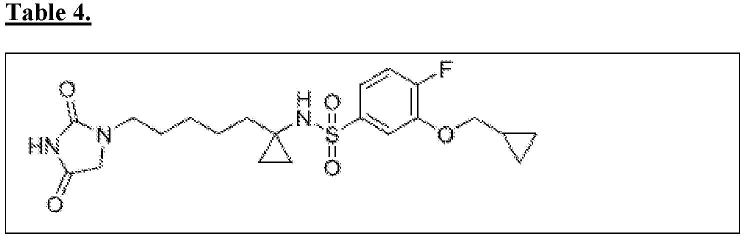

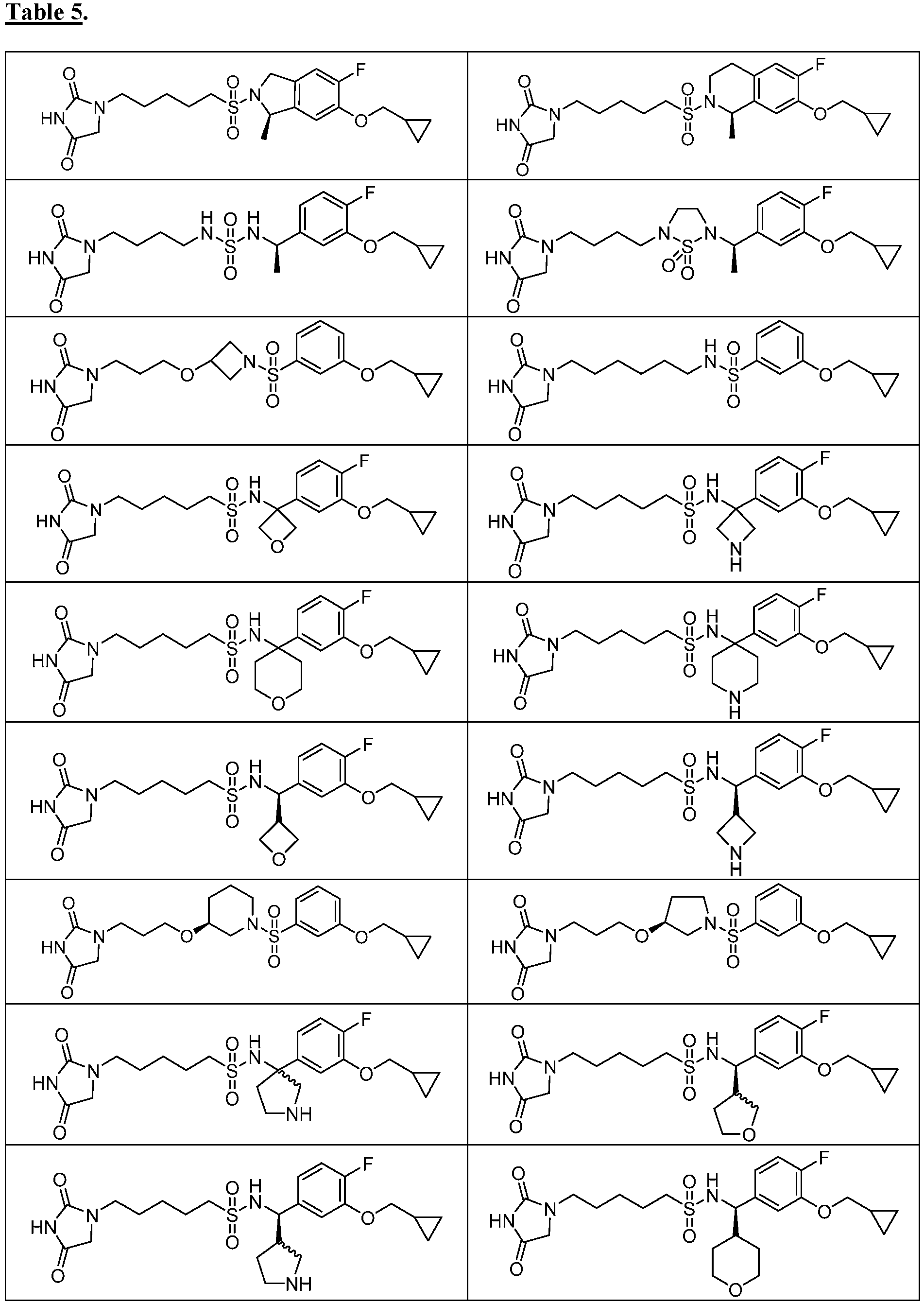

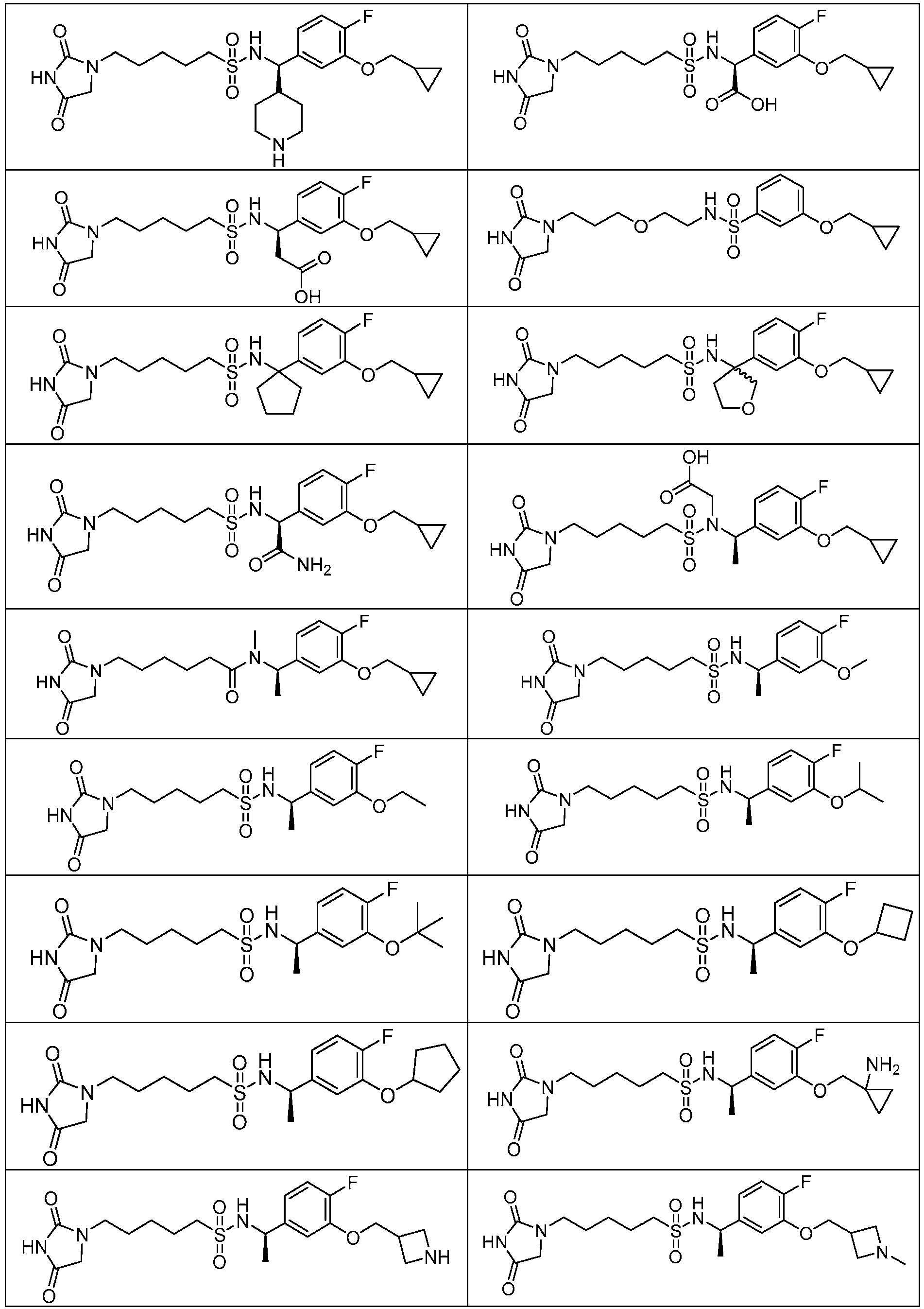

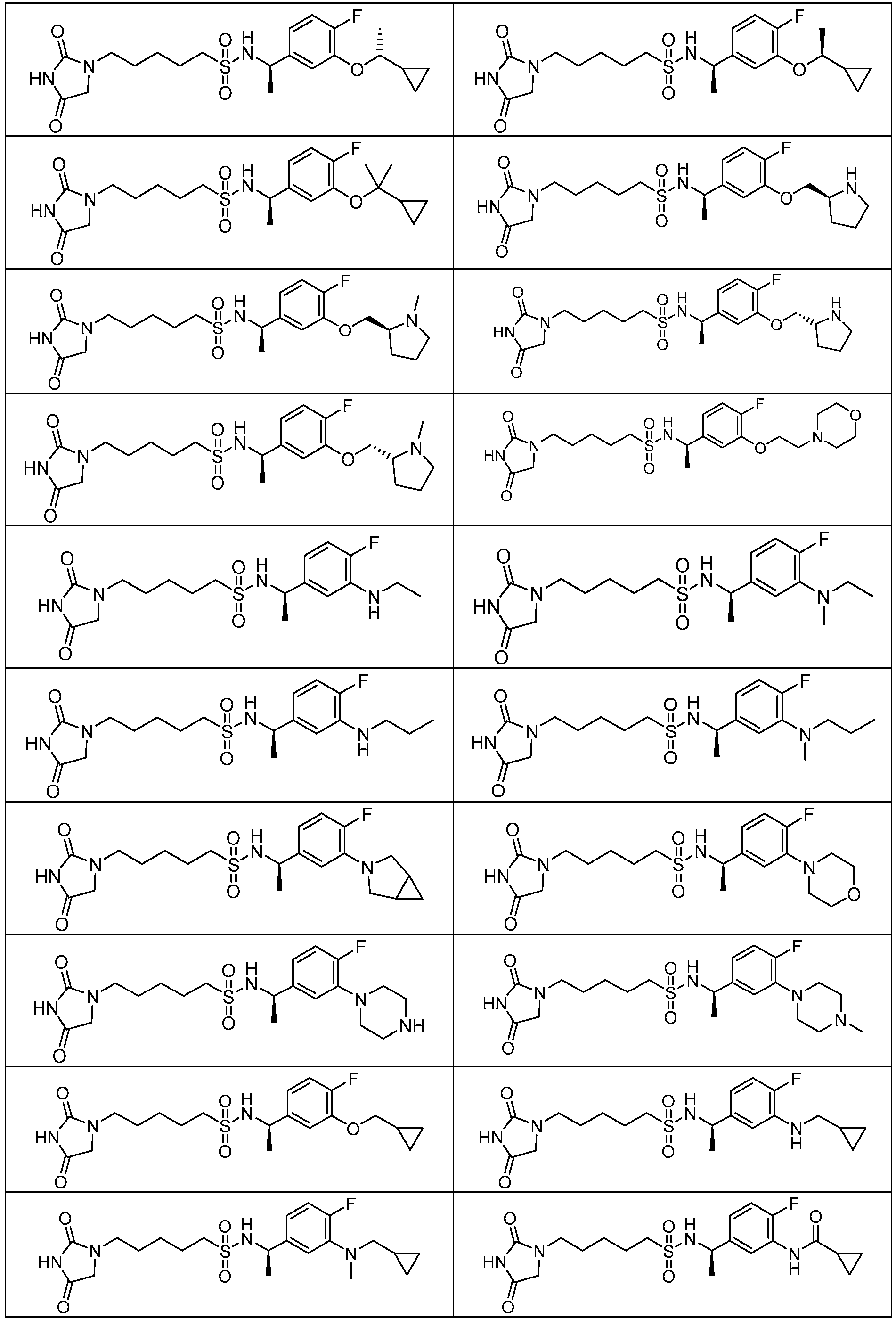

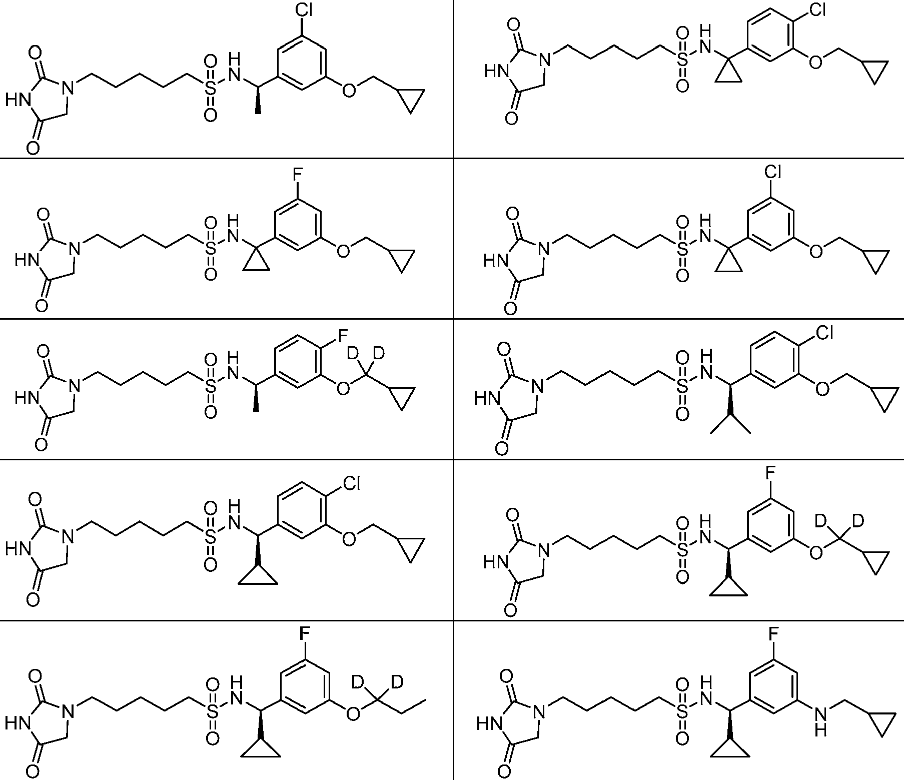

**Table 6.**

| | |
|---|---|
| 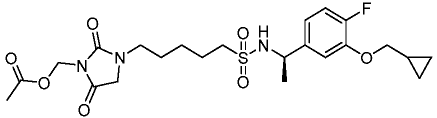 | 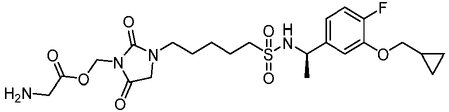 |
| 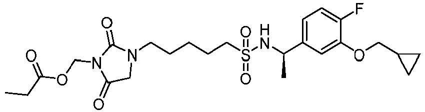 | 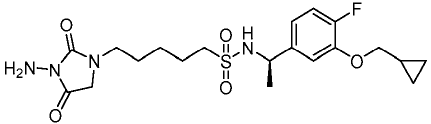 |
| 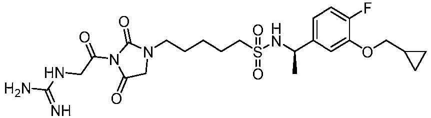 | 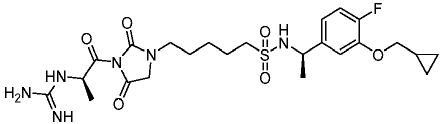 |
| 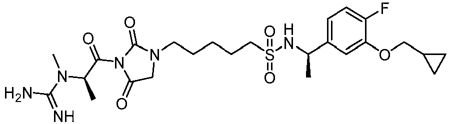 | 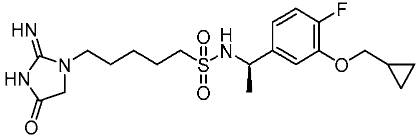 |
| 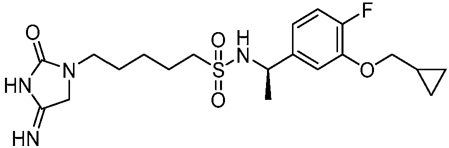 | 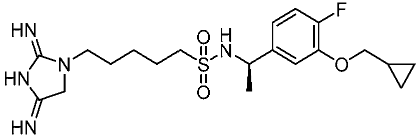 |
| 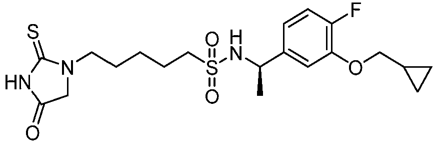 | 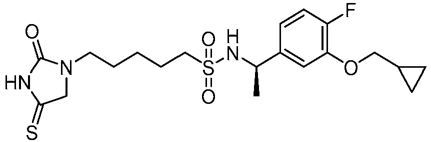 |
| 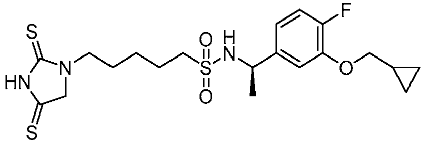 | 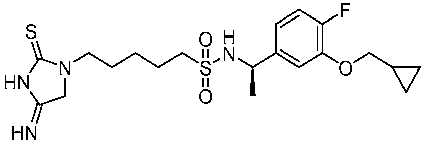 |
| 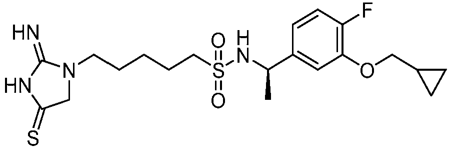 | 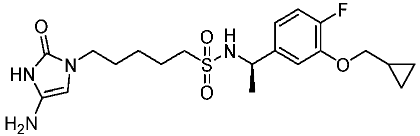 |
| 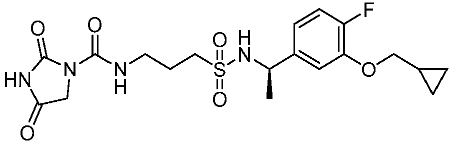 | 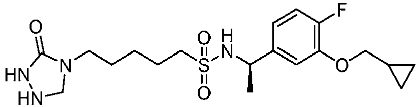 |
| 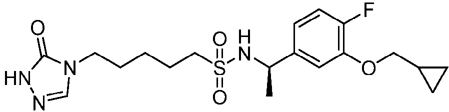 | 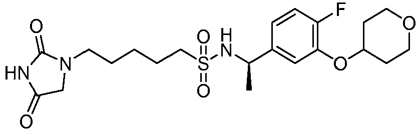 |
| 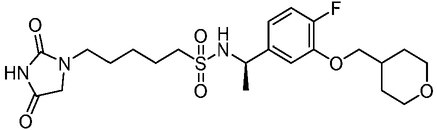 | 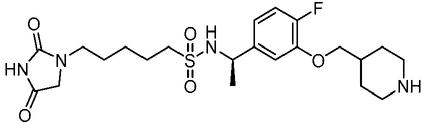 |
| 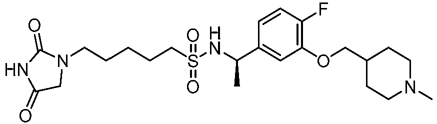 | 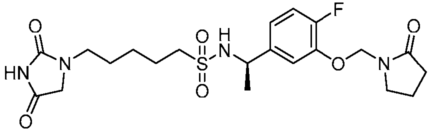 |
| 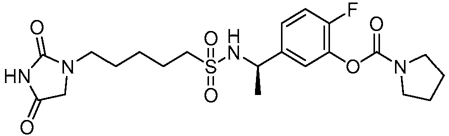 | 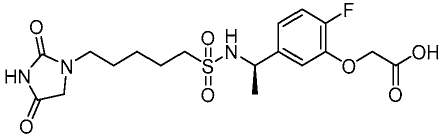 |
| 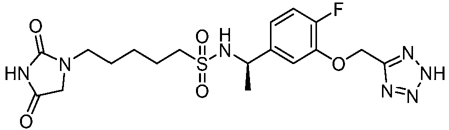 | 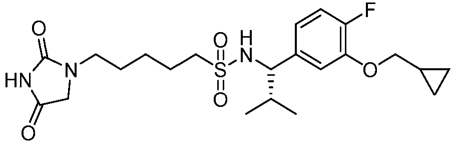 |
| 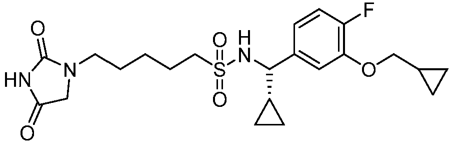 | 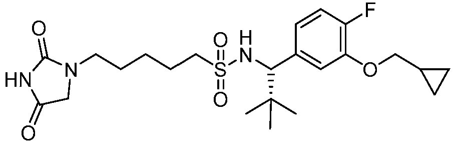 |
| 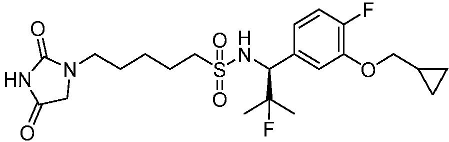 | 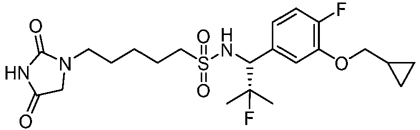 |
| 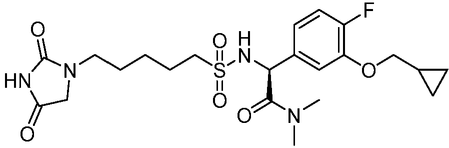 | 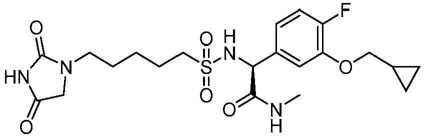 |
| 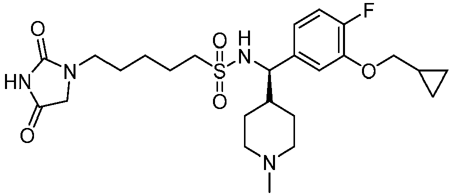 | 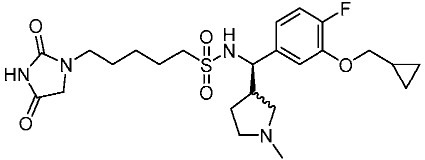 |
| 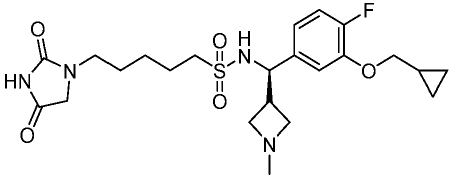 | 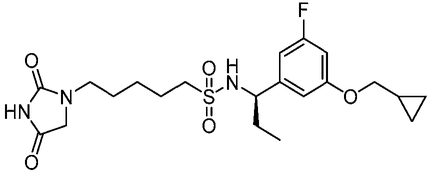 |
| 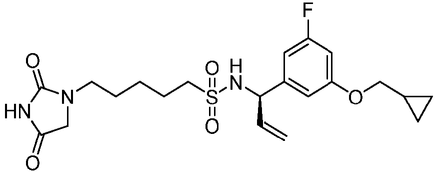 | 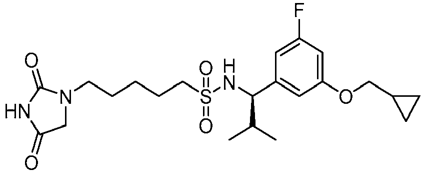 |
| 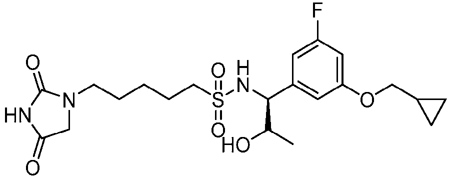 | 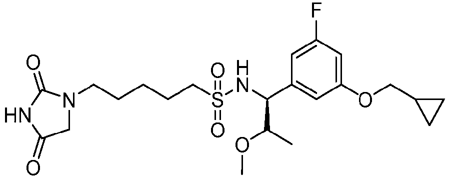 |
| 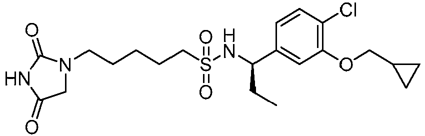 | 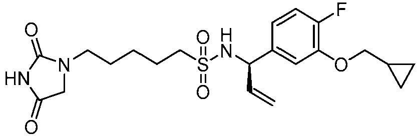 |
| 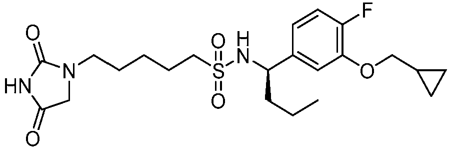 | 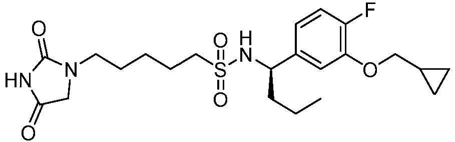 |
| 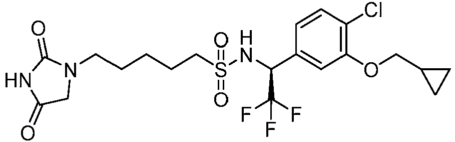 | 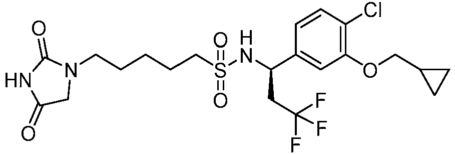 |
| 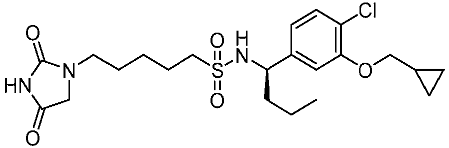 | 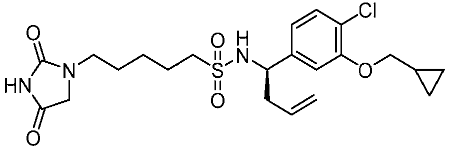 |
| 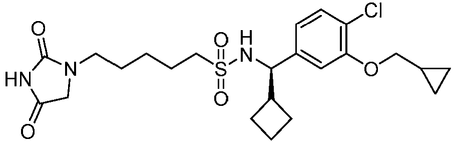 | 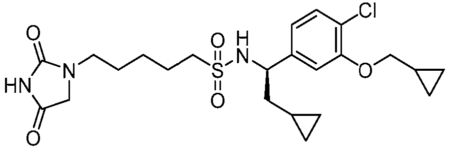 |
| 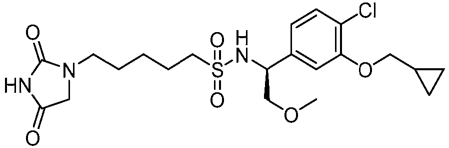 | 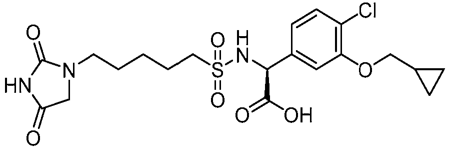 |
| 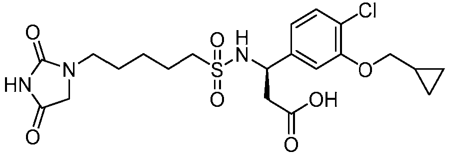 | 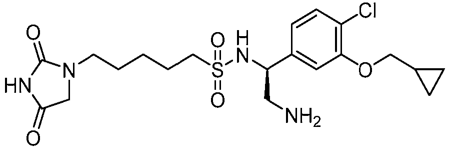 |
| 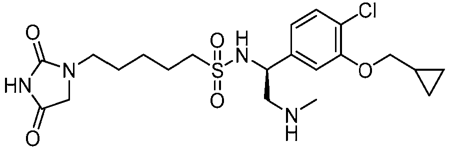 | 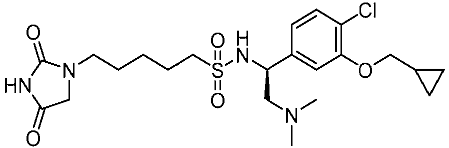 |
| 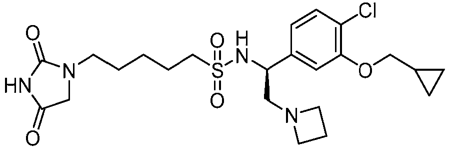 | 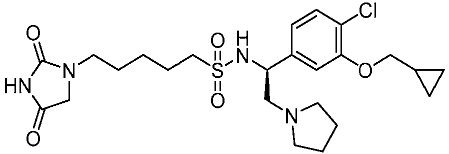 |
| 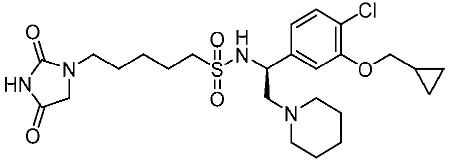 | 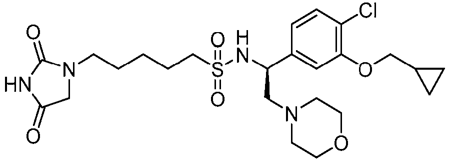 |
| 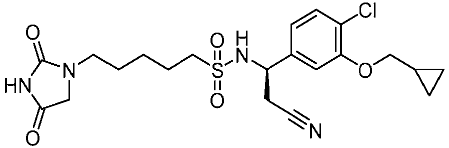 | 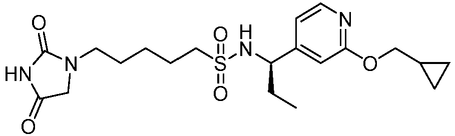 |
| 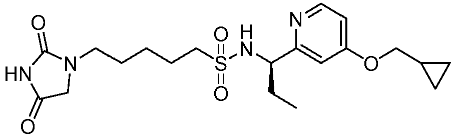 | 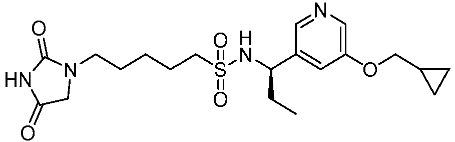 |
| 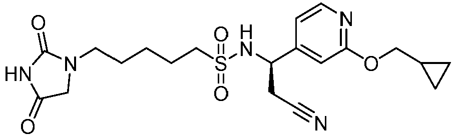 | 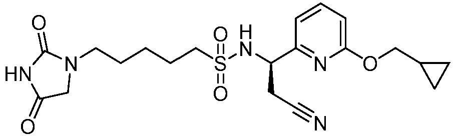 |
| 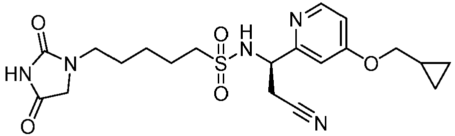 | 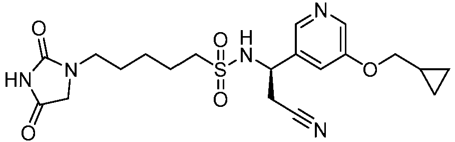 |
| 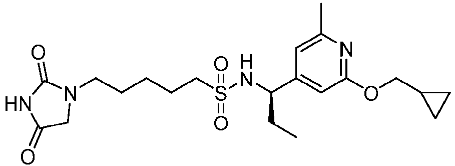 | 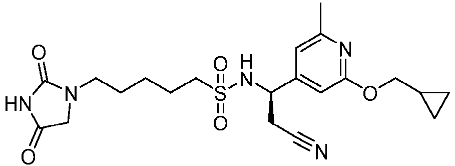 |
| 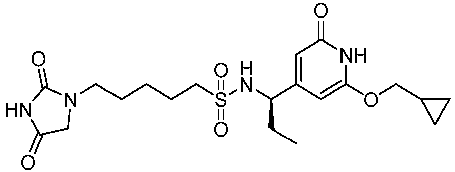 | 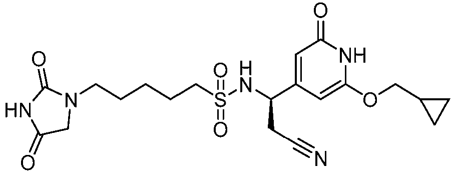 |
| 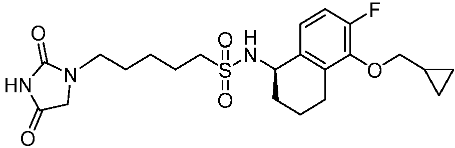 | 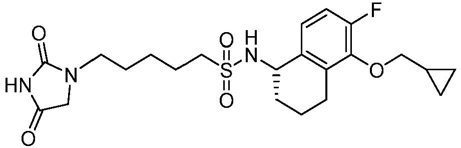 |
| 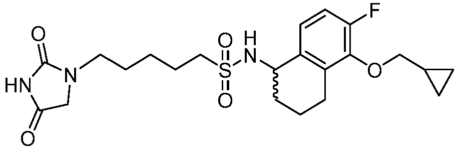 | 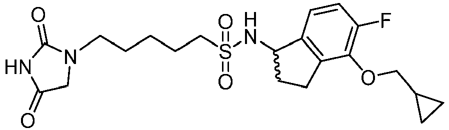 |
| 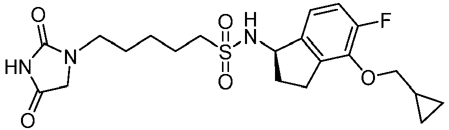 | 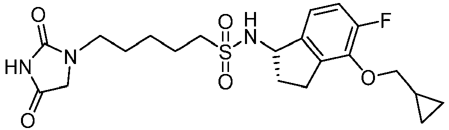 |
| 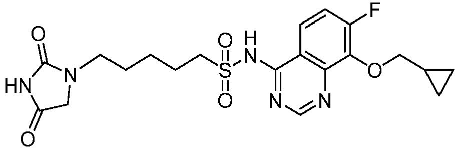 | 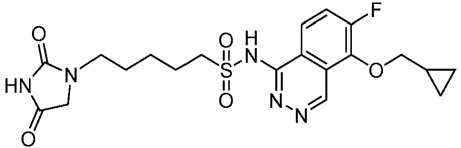 |
| 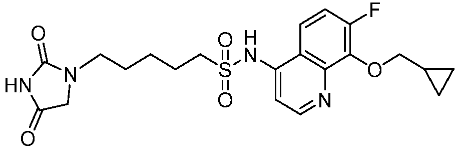 | 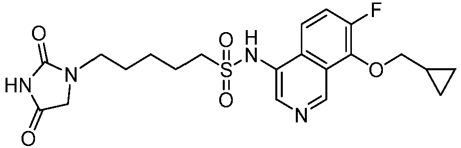 |
| 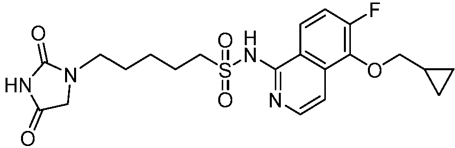 | 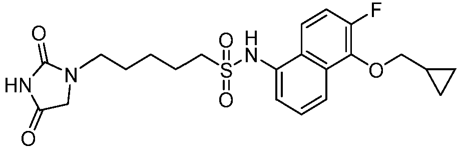 |
| 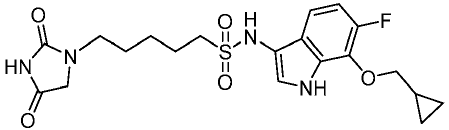 | 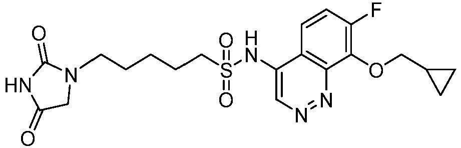 |
| 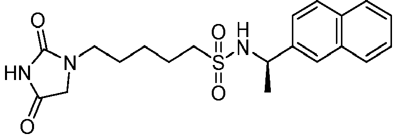 | 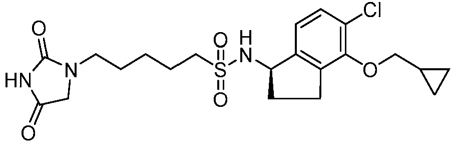 |
| 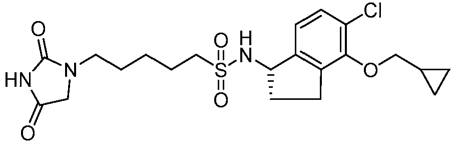 | 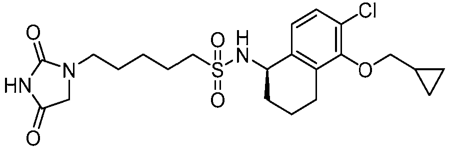 |
| 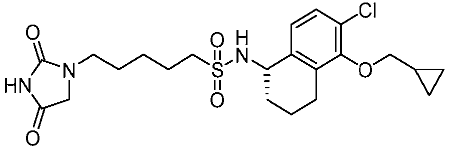 | 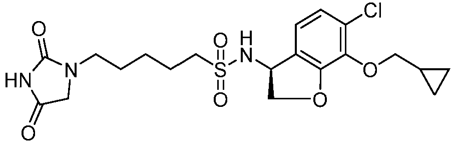 |
| 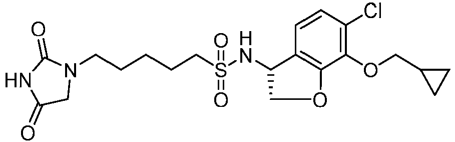 | 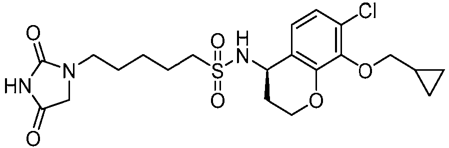 |
| 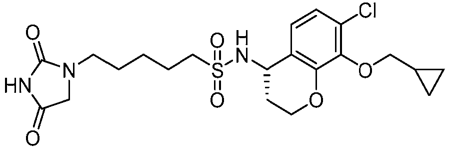 | 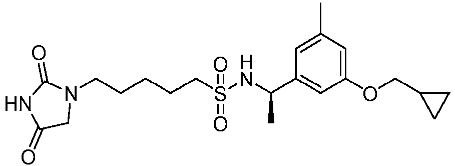 |
| 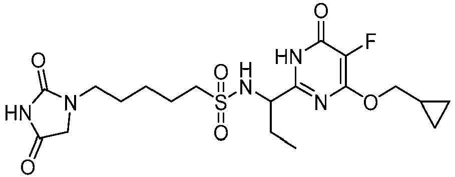 | 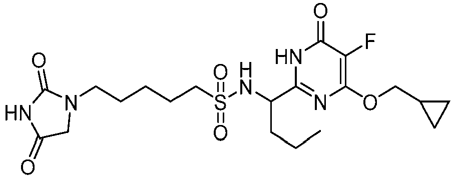 |
| 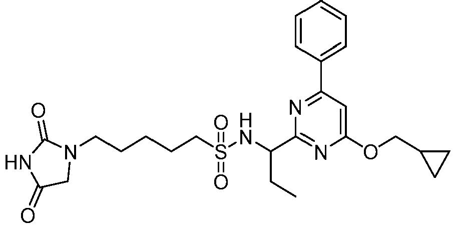 | 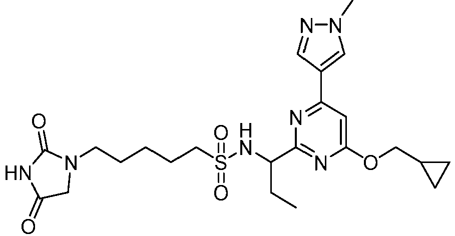 |
| 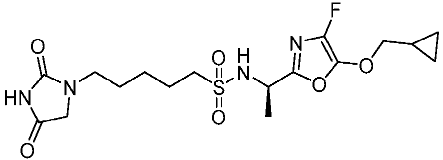 | 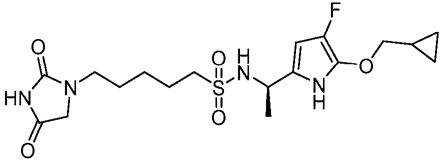 |
| 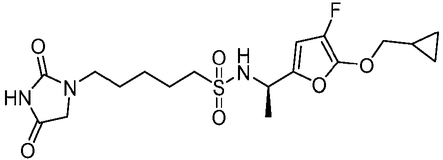 | 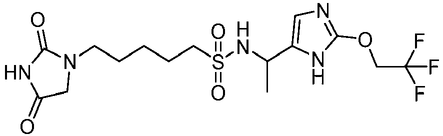 |
| 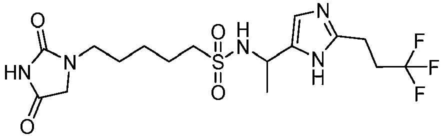 | 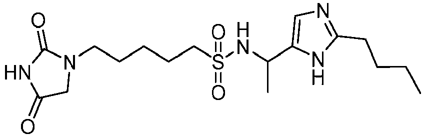 |
| 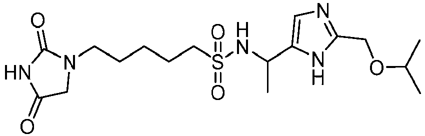 | 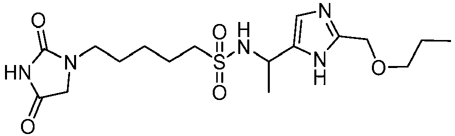 |
| 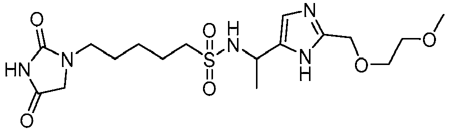 | 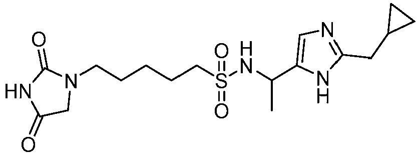 |
| 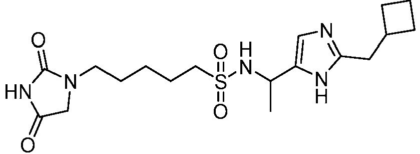 | 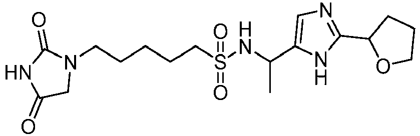 |
| 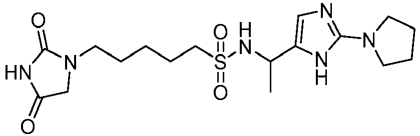 | 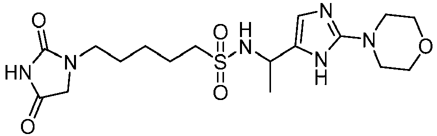 |
| 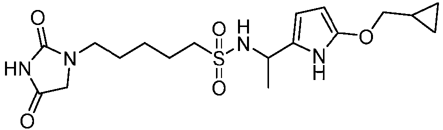 | 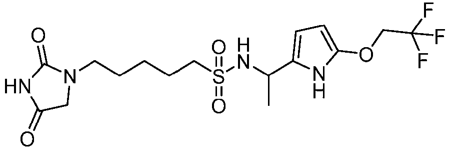 |
| 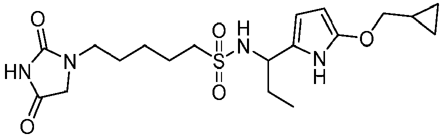 | 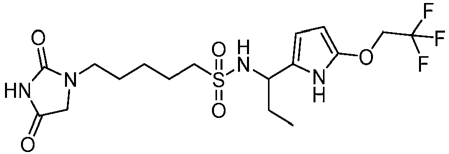 |
| 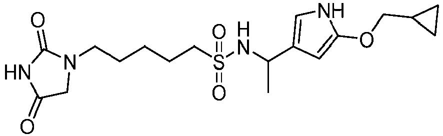 | 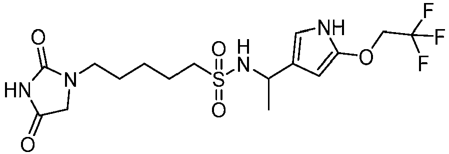 |
| 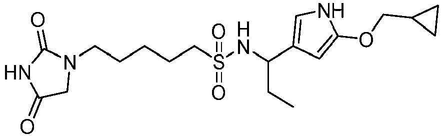 | 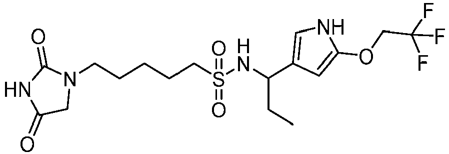 |
| 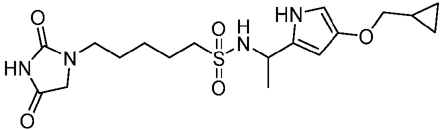 | 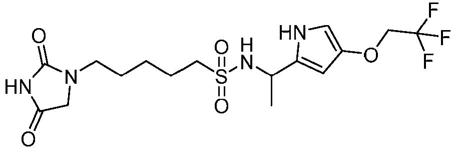 |
| 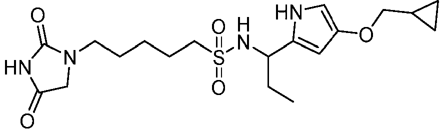 | 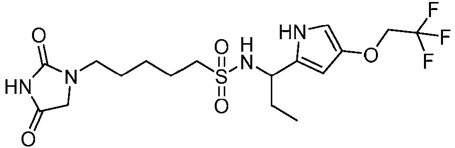 |
| 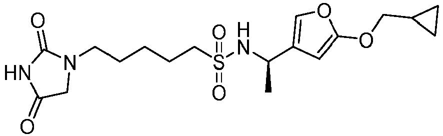 | 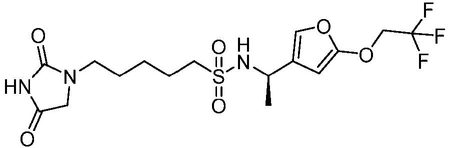 |
| 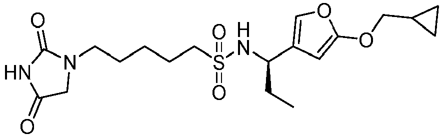 | 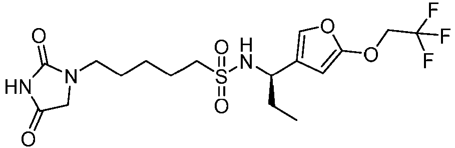 |
| 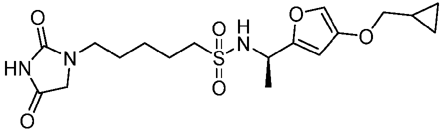 | 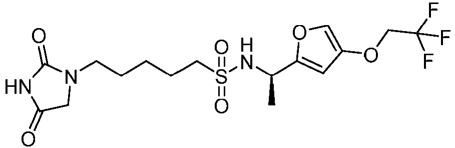 |
| 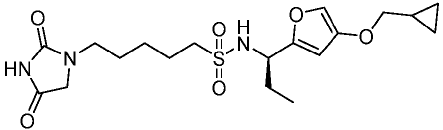 | 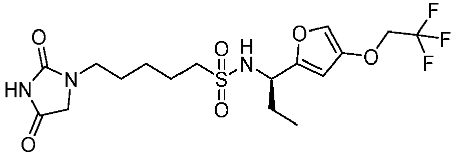 |
| 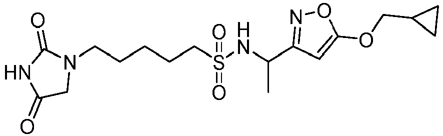 | 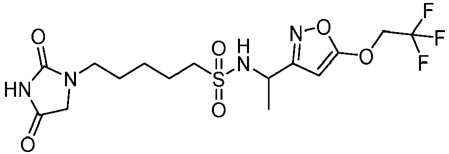 |
| 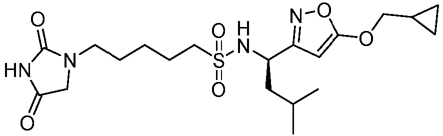 | 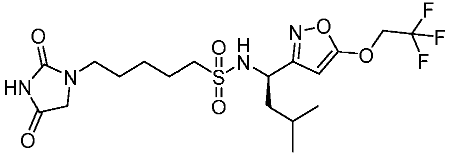 |
| 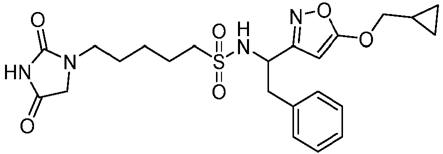 | 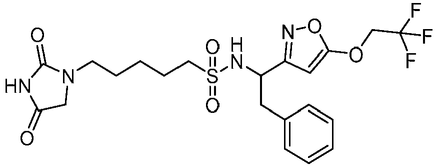 |
| 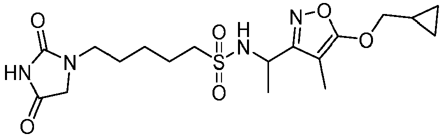 | 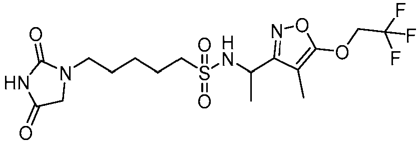 |
| 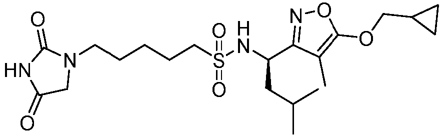 | 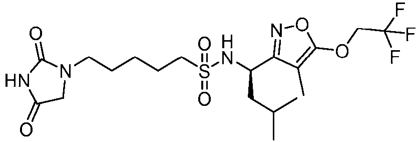 |
| 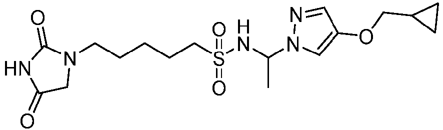 | 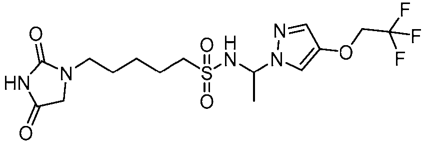 |
| 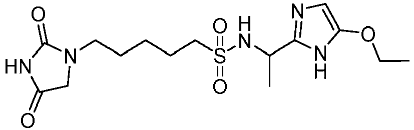 | 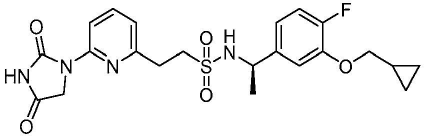 |
| 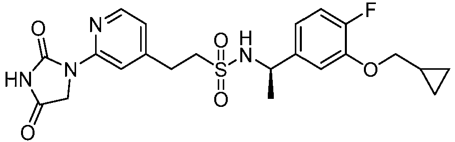 | 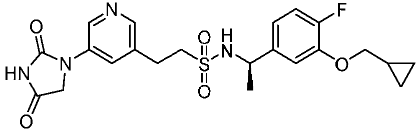 |
| 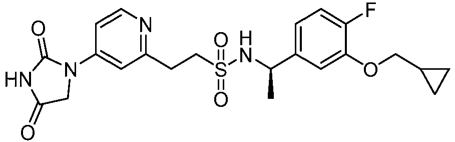 | 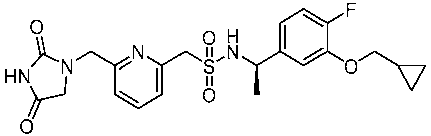 |
| 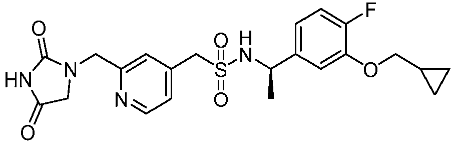 | 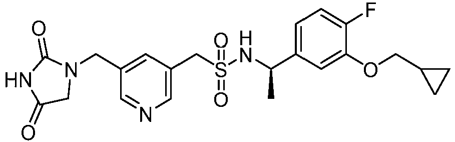 |
| 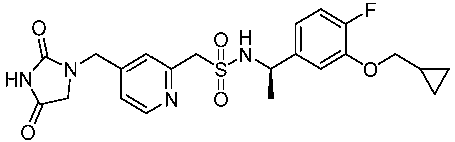 | 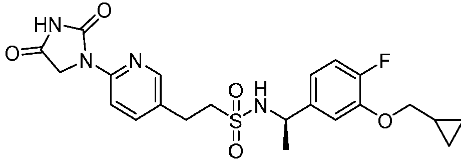 |
| 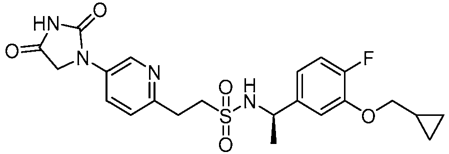 | 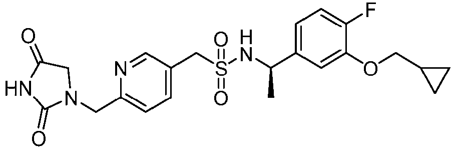 |
| 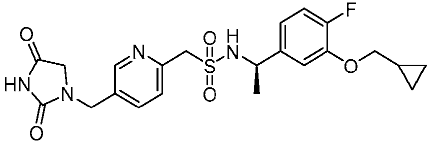 | 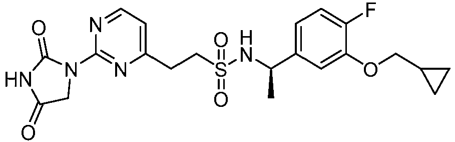 |
| 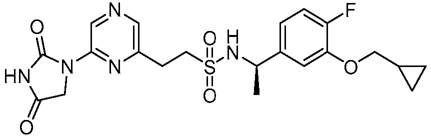 | 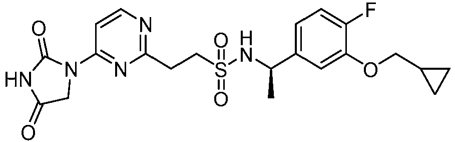 |
| 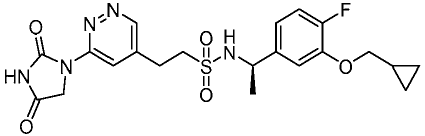 | 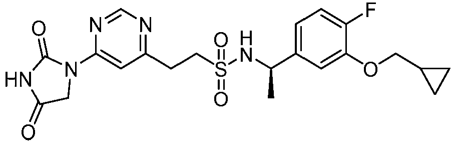 |
| 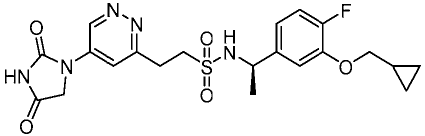 | 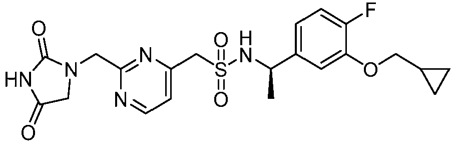 |
| 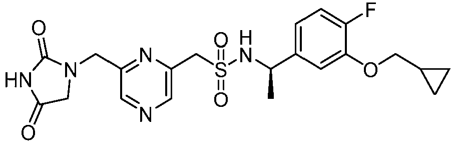 | 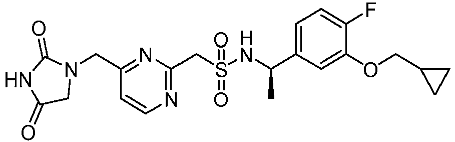 |
| 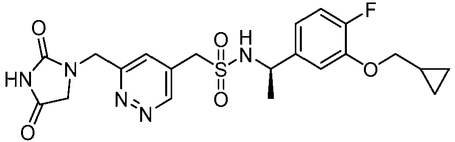 | 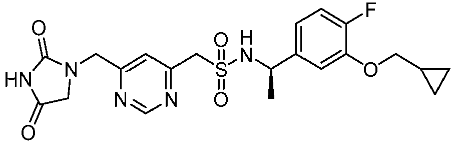 |
| 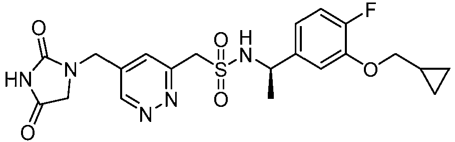 | 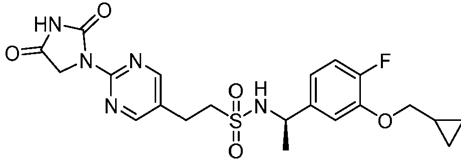 |
| 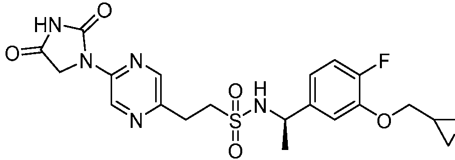 | 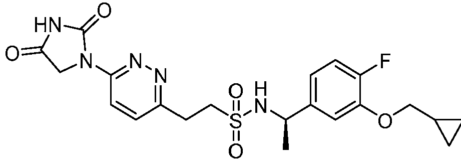 |
| 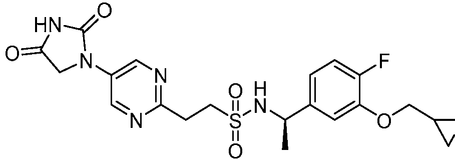 | 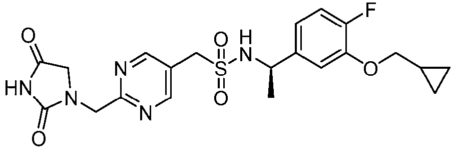 |
| 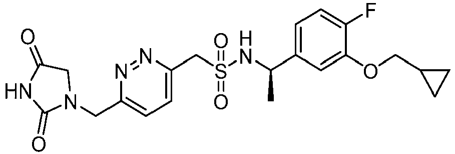 | 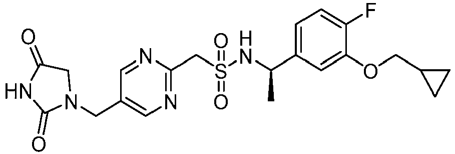 |
| 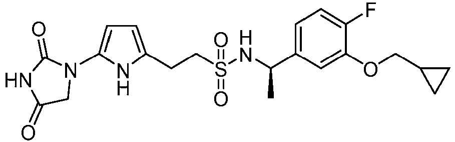 | 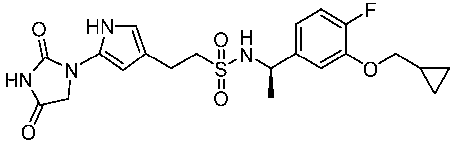 |
| 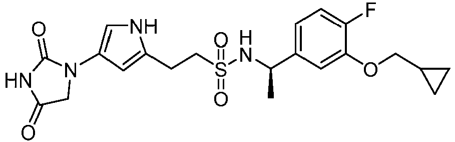 | 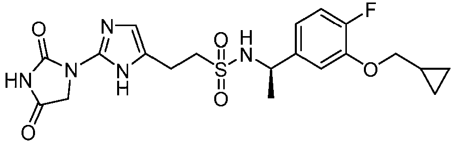 |
| 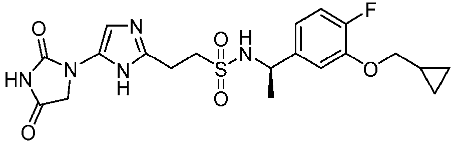 | 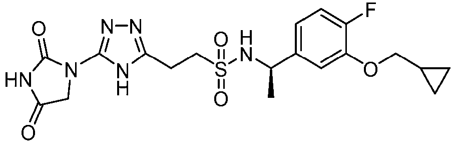 |
| 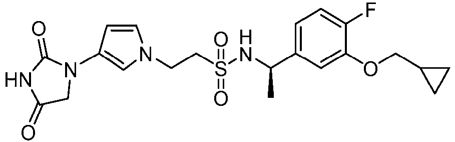 | 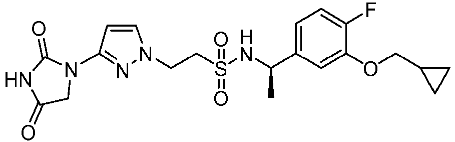 |
| 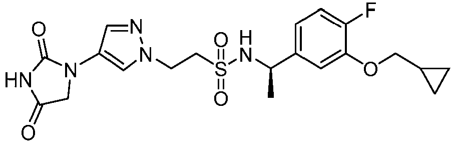 | 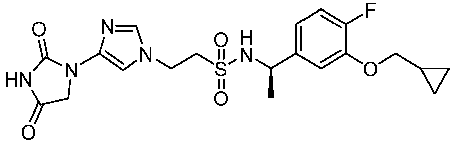 |
| 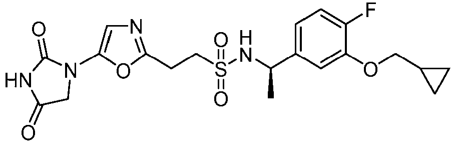 | 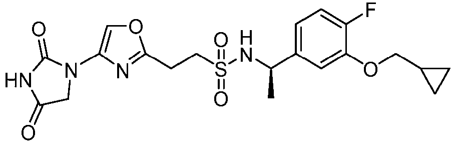 |
| 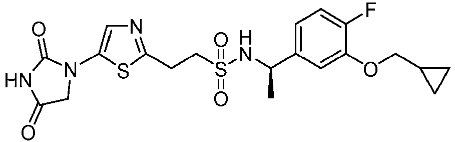 | 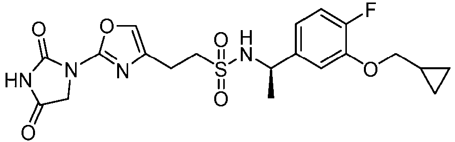 |
| 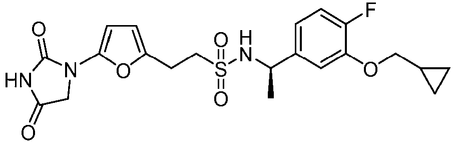 | 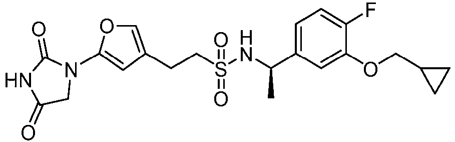 |
| 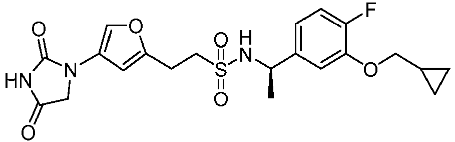 | 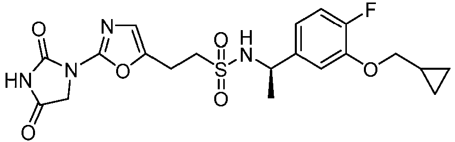 |
| 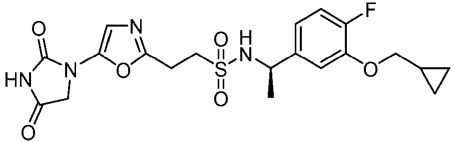 | 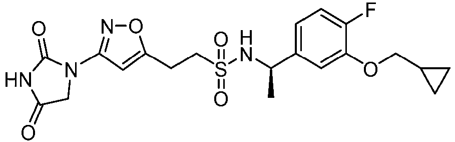 |
| 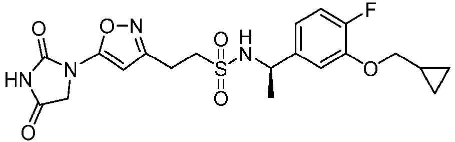 | 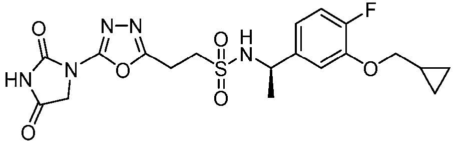 |
| 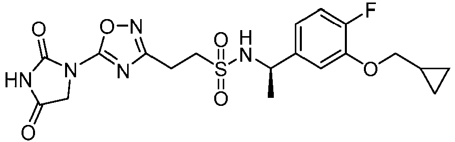 | 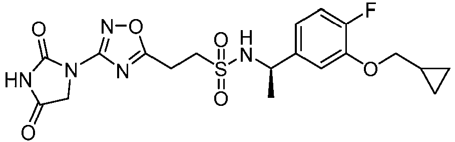 |
| 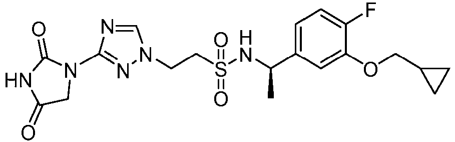 | 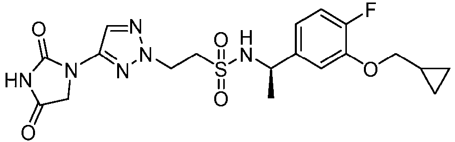 |
| 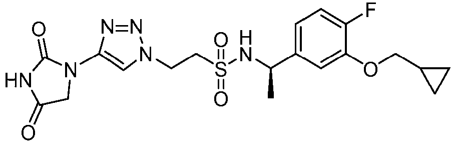 | 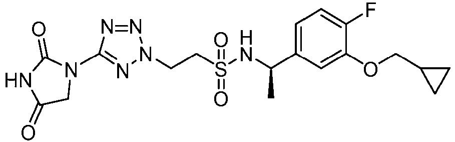 |
| 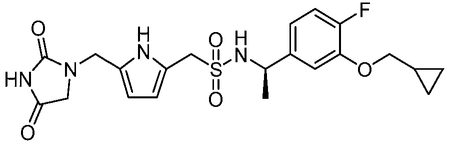 | 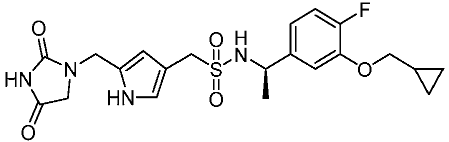 |
| 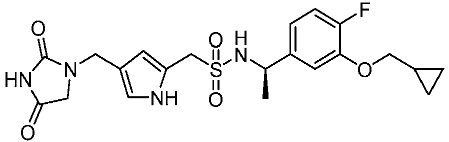 | 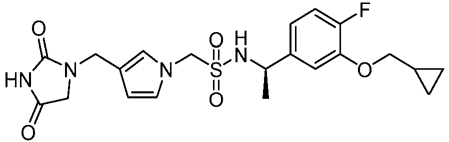 |
| 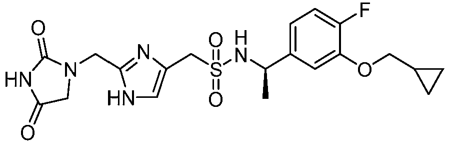 | 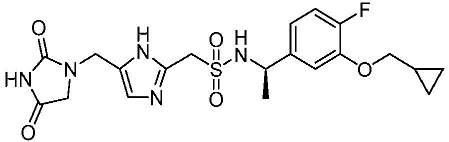 |
| 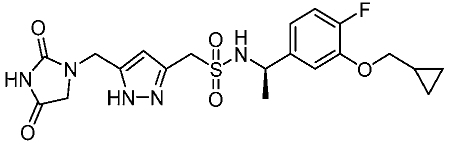 | 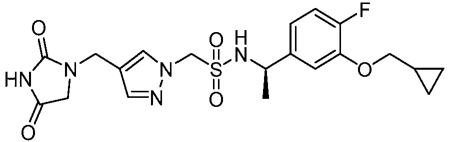 |
| 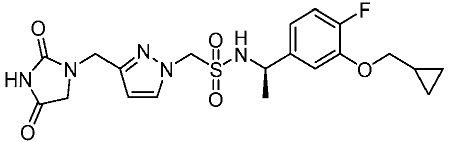 | 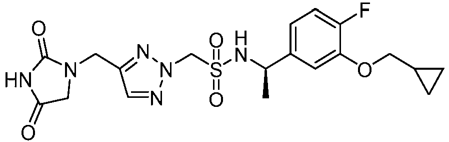 |
| 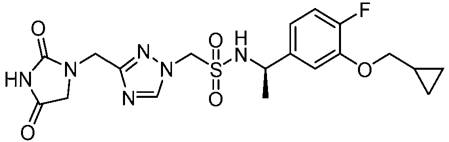 | 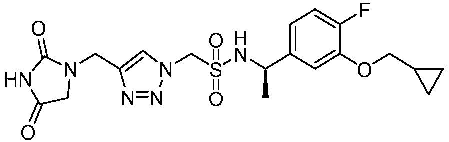 |
| 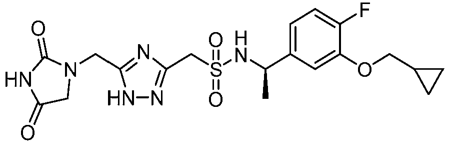 | 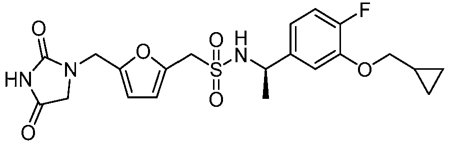 |
| 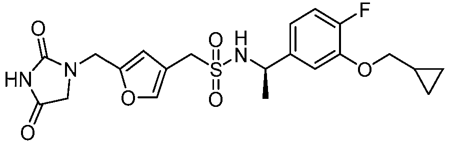 | 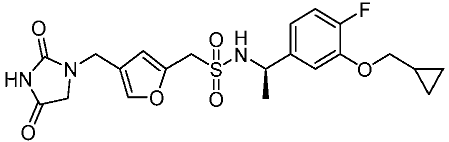 |
| 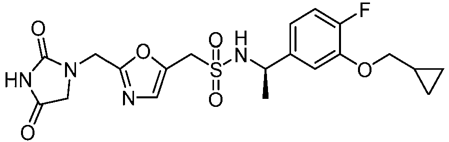 | 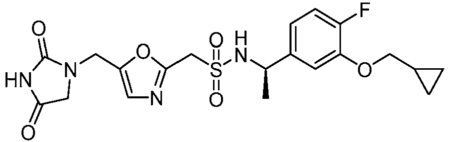 |
| 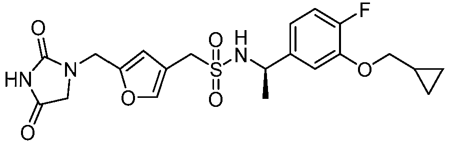 | 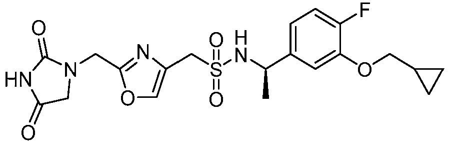 |
| 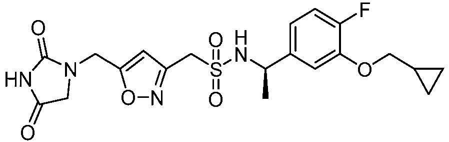 | 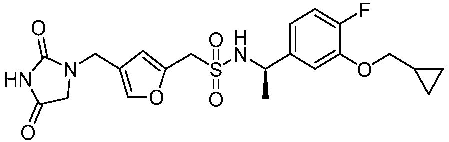 |
| 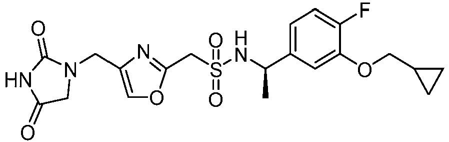 | 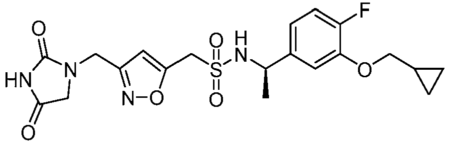 |
| 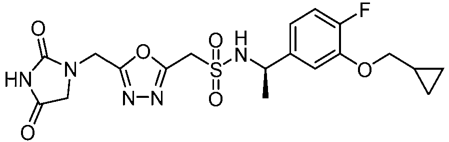 | 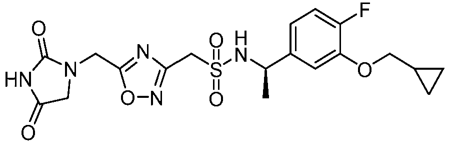 |
| 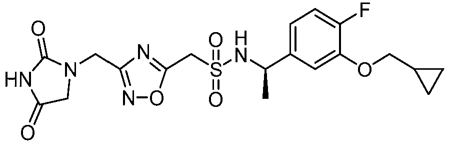 | 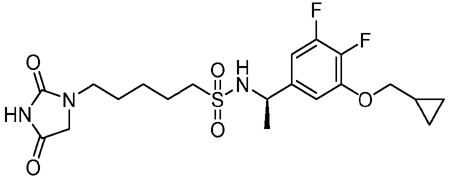 |
| 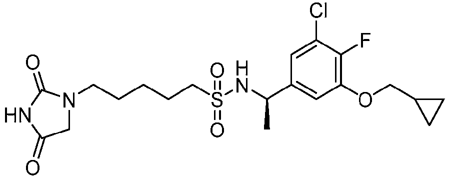 | 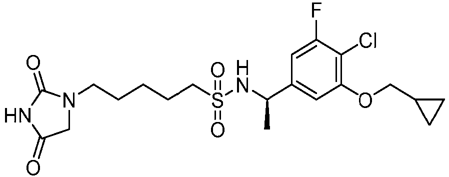 |
| 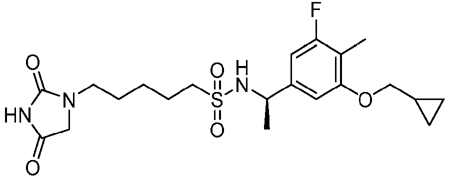 | 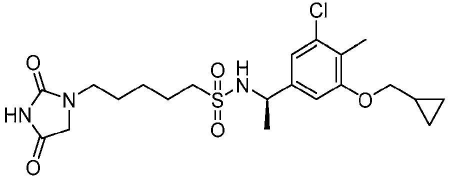 |
| 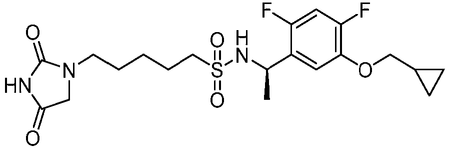 | 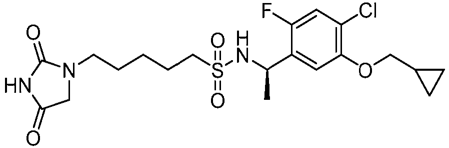 |
| 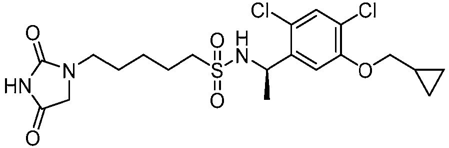 | 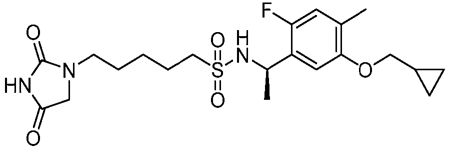 |
| 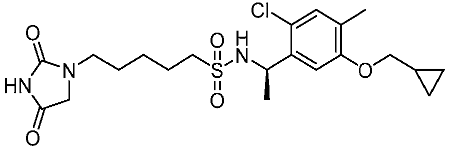 | 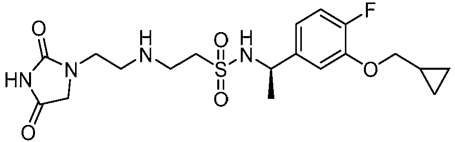 |
| 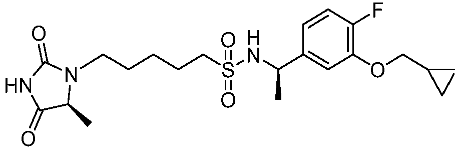 | 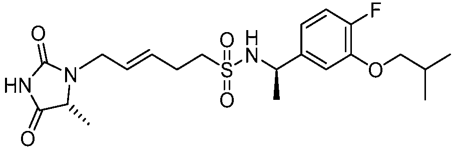 |
| 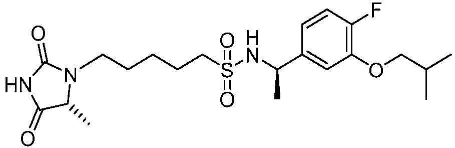 | 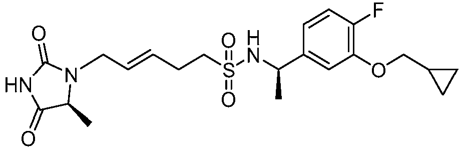 |
| 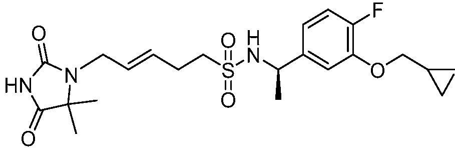 | 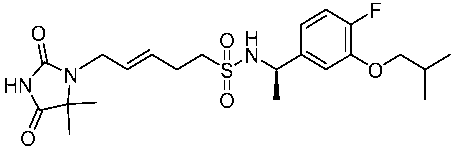 |
| 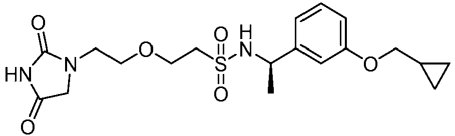 | 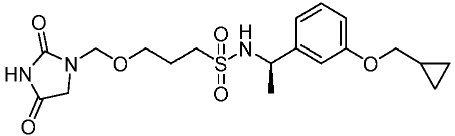 |
| 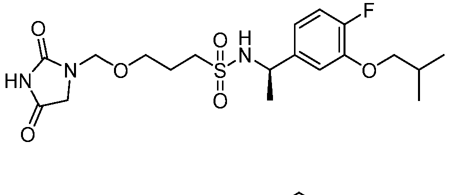 | 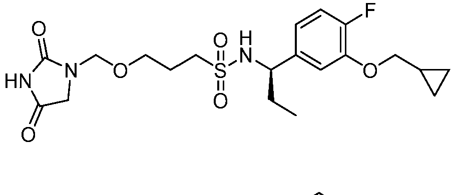 |
| 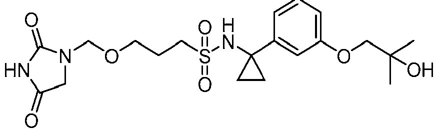 | 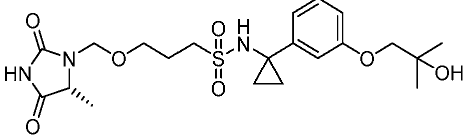 |
| 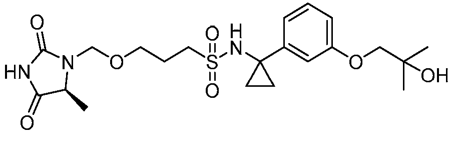 | 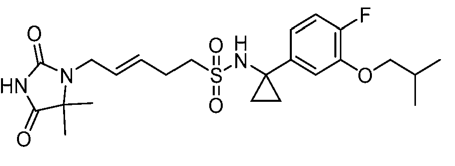 |
| 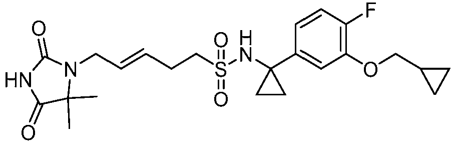 | 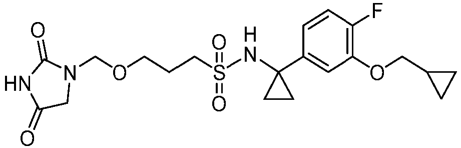 |
| 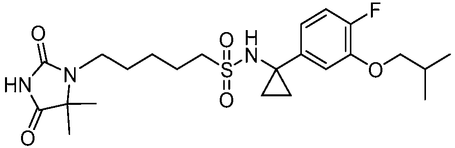 | 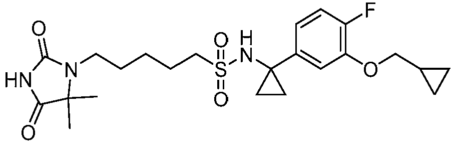 |
| 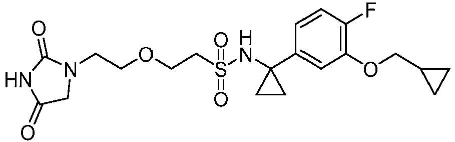 | 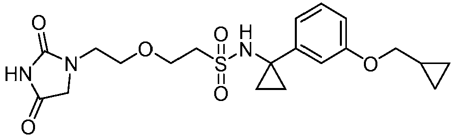 |
| 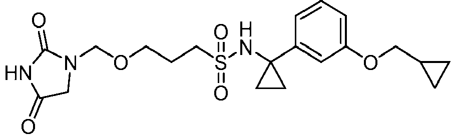 | 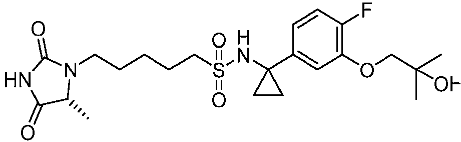 |
| 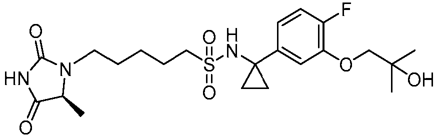 | 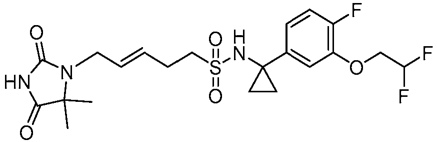 |
| 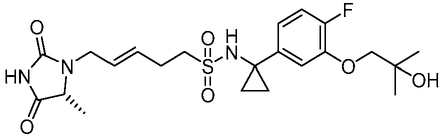 | 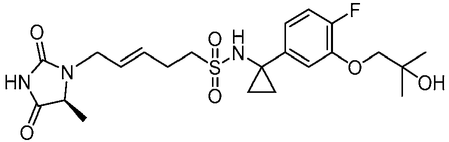 |
| 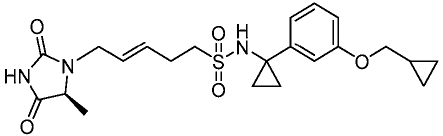 | 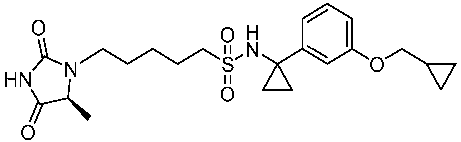 |
| 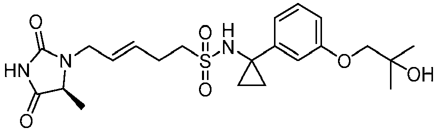 | 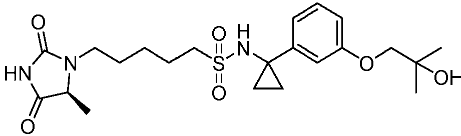 |
| 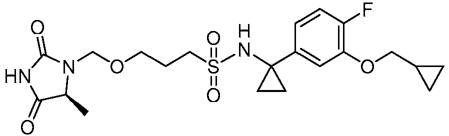 | 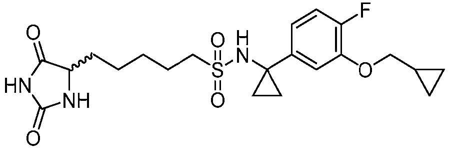 |
| 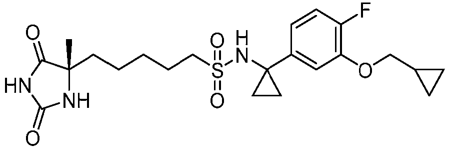 | 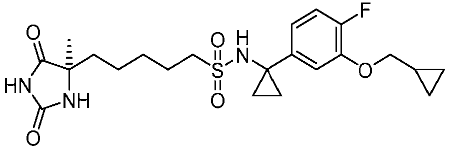 |
| 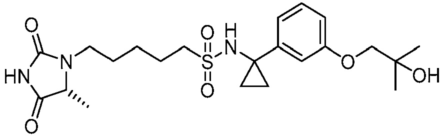 | 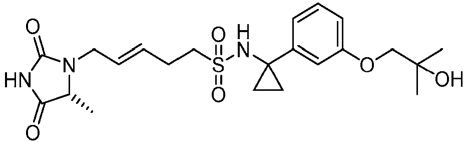 |
| 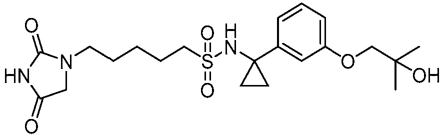 | 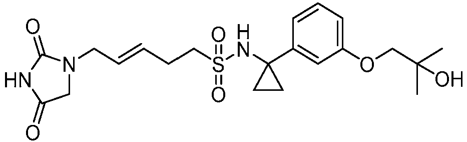 |
| 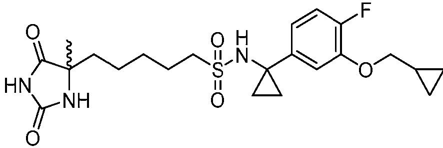 | 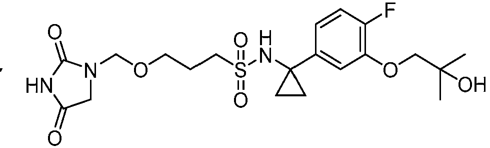 |
| 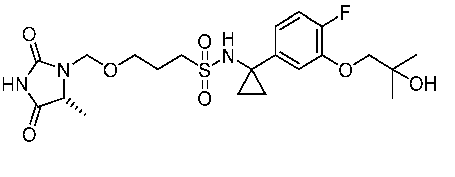 | 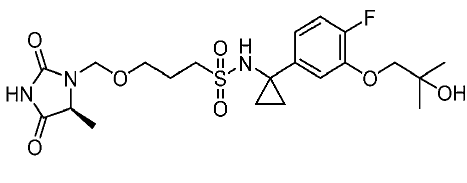 |
| 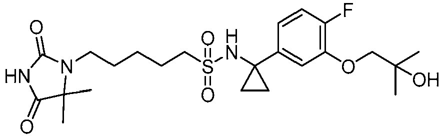 | 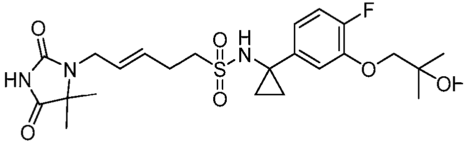 |
| 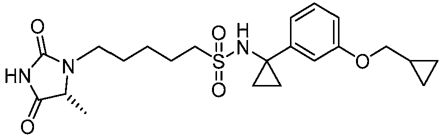 | 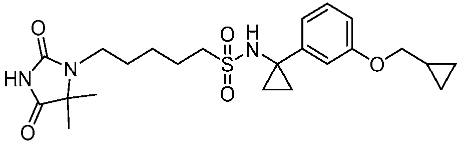 |
| 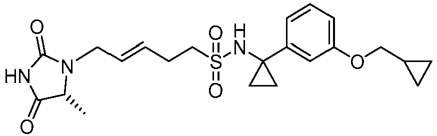 | 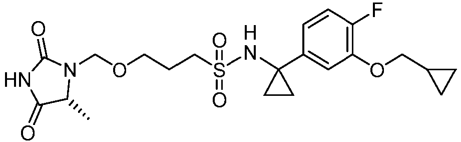 |
| 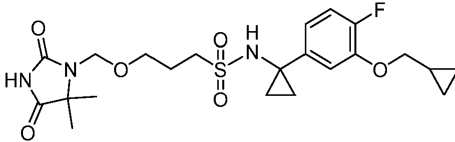 | 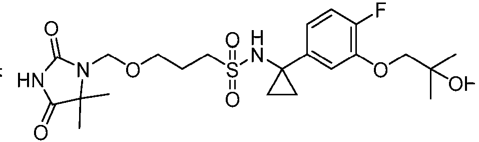 |
| 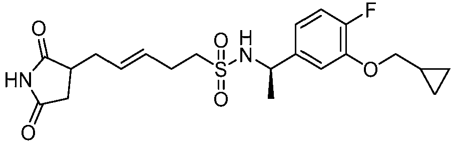 | 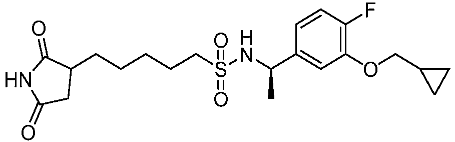 |
| 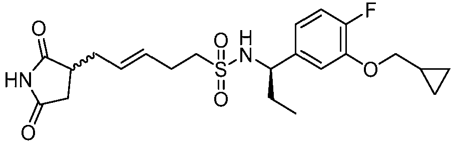 | 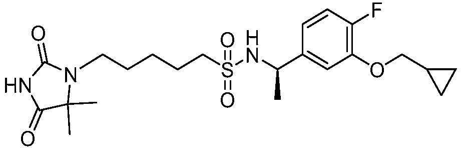 |
| 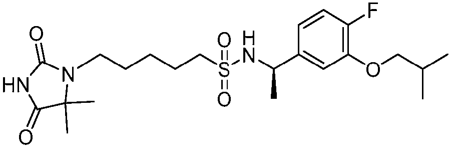 | 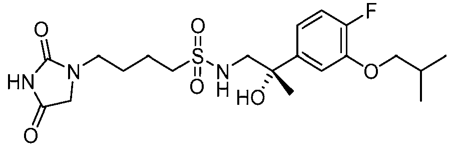 |
| 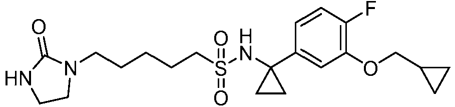 | 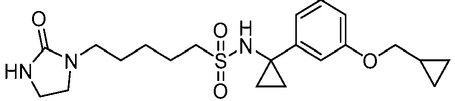 |
| 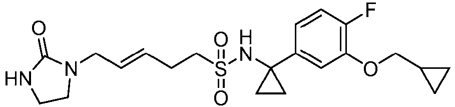 | 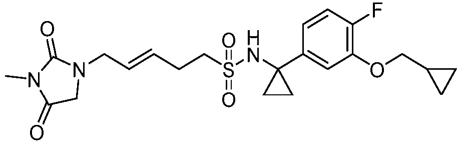 |
| 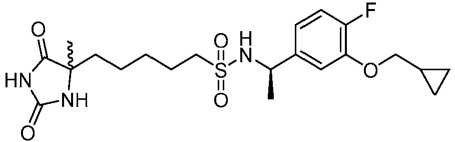 | 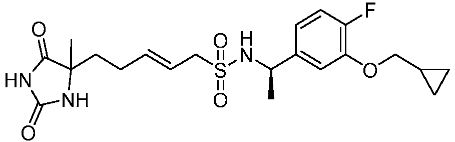 |
| 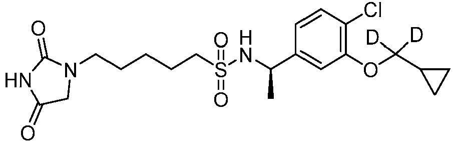 | 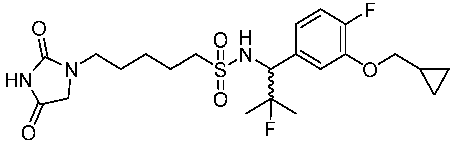 |
| 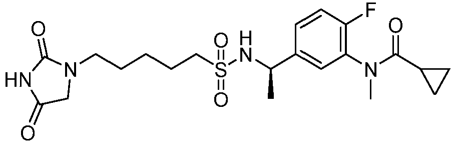 | 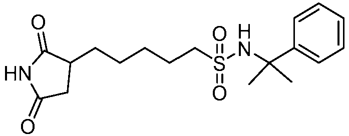 |
| 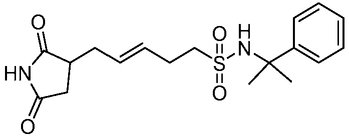 | 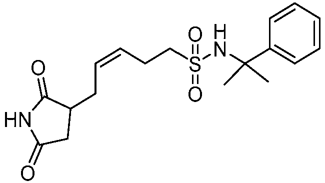 |
| 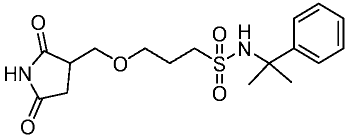 | 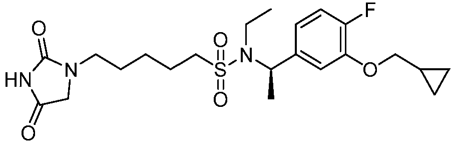 |
| 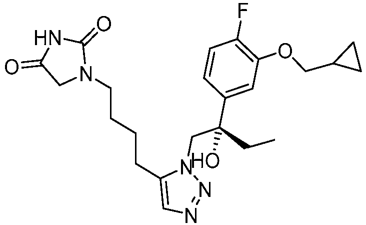 | 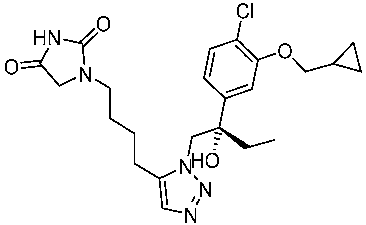 |
| 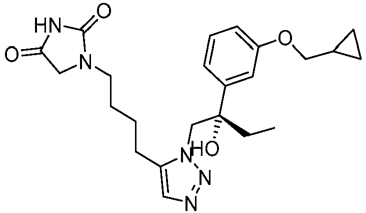 | 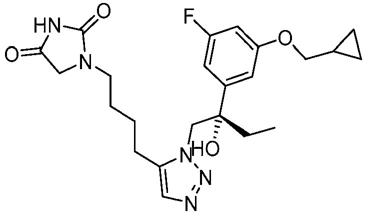 |
| 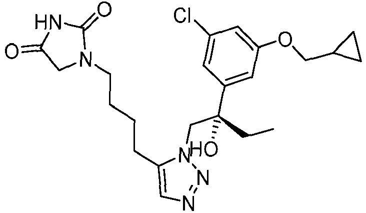 | 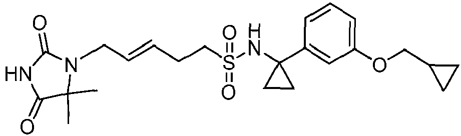 |
| 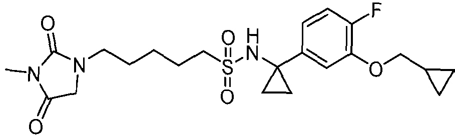 | 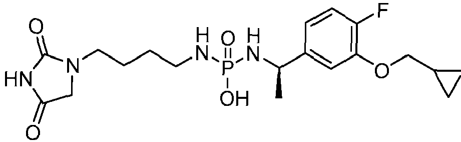 |
| 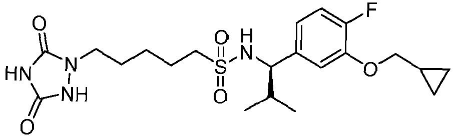 | 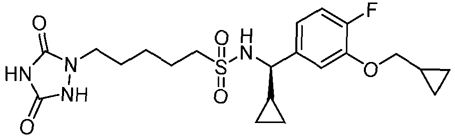 |
| 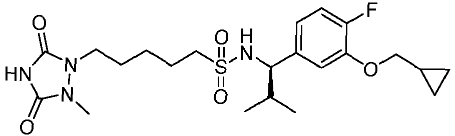 | 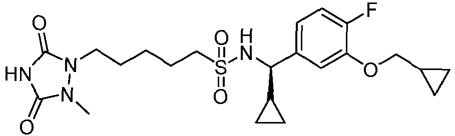 |
| 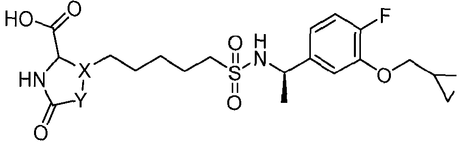 wherein X is N or CH and y is NH or CH_{2.} | 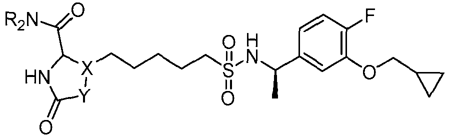 wherein X is N or CH and y is NH or CH_{2.} |
| 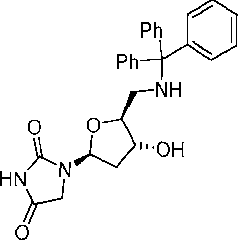 | 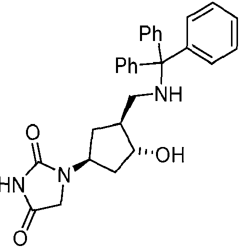 |
| 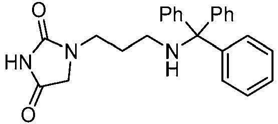 | 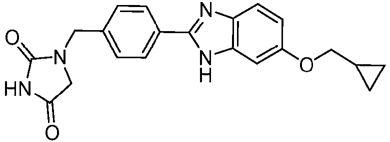 |
| 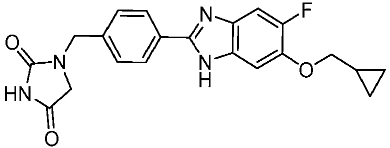 | 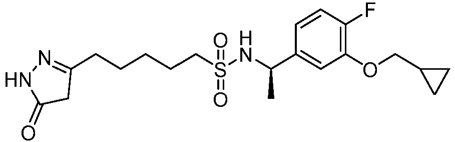 |
| 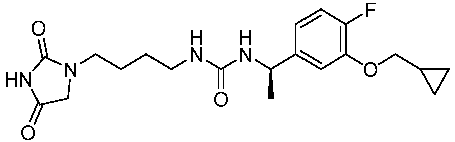 | 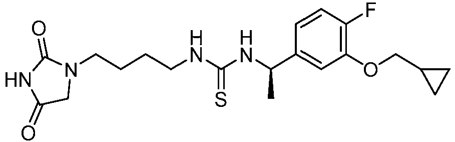 |
| 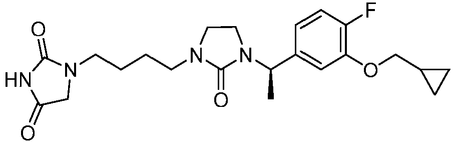 | 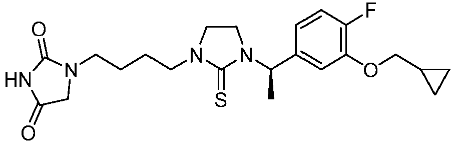 |
| 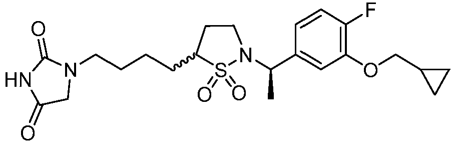 | 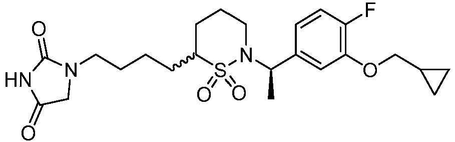 |
| 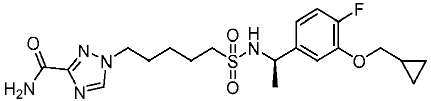 | 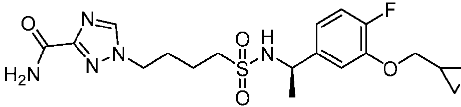 |
| 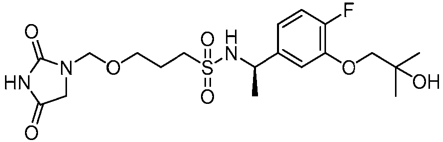 | 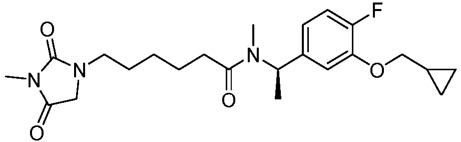 |
| 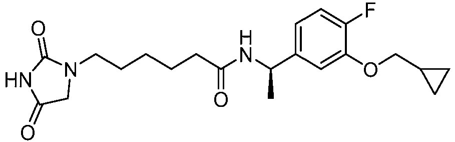 | 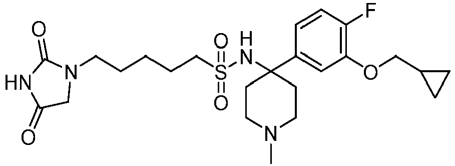 |
| 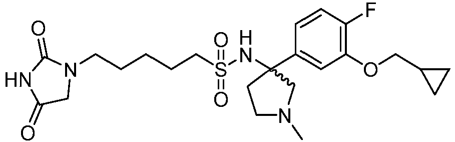 | 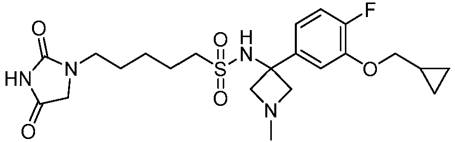 |

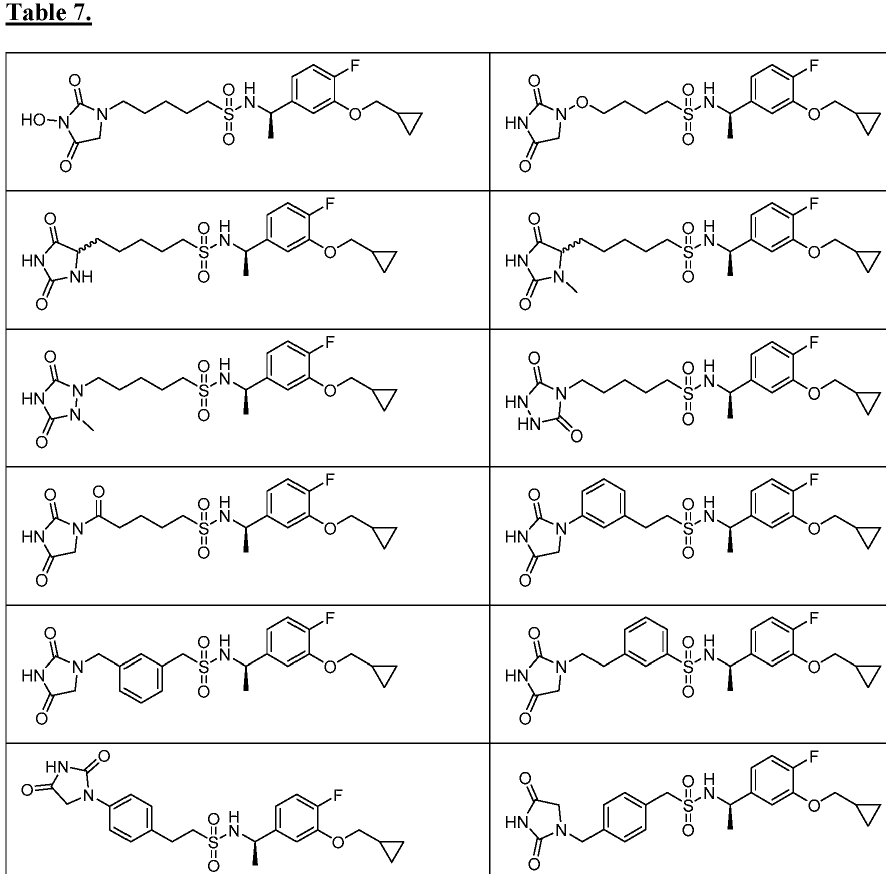

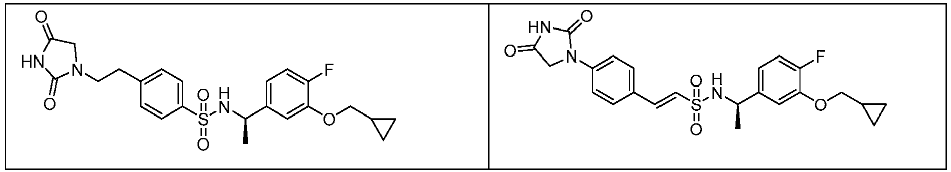

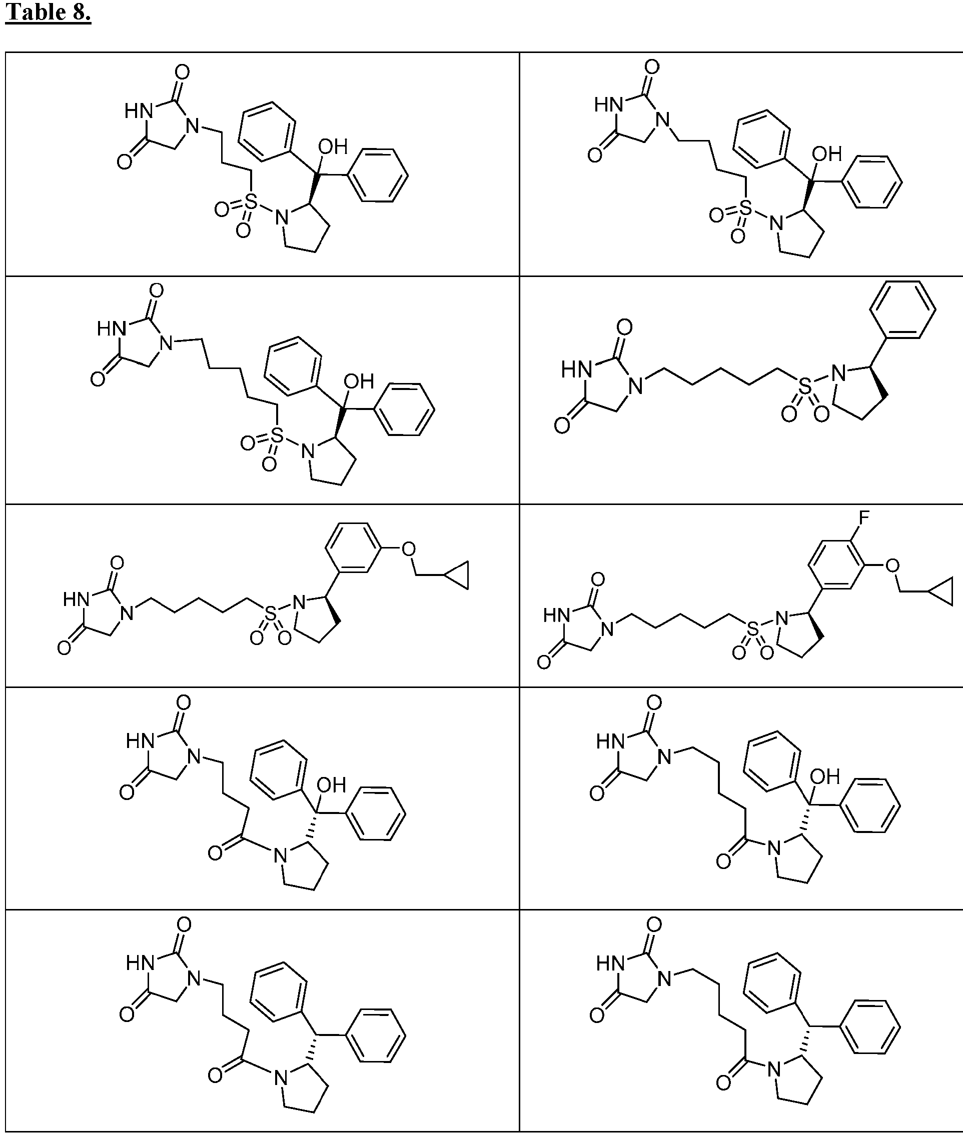

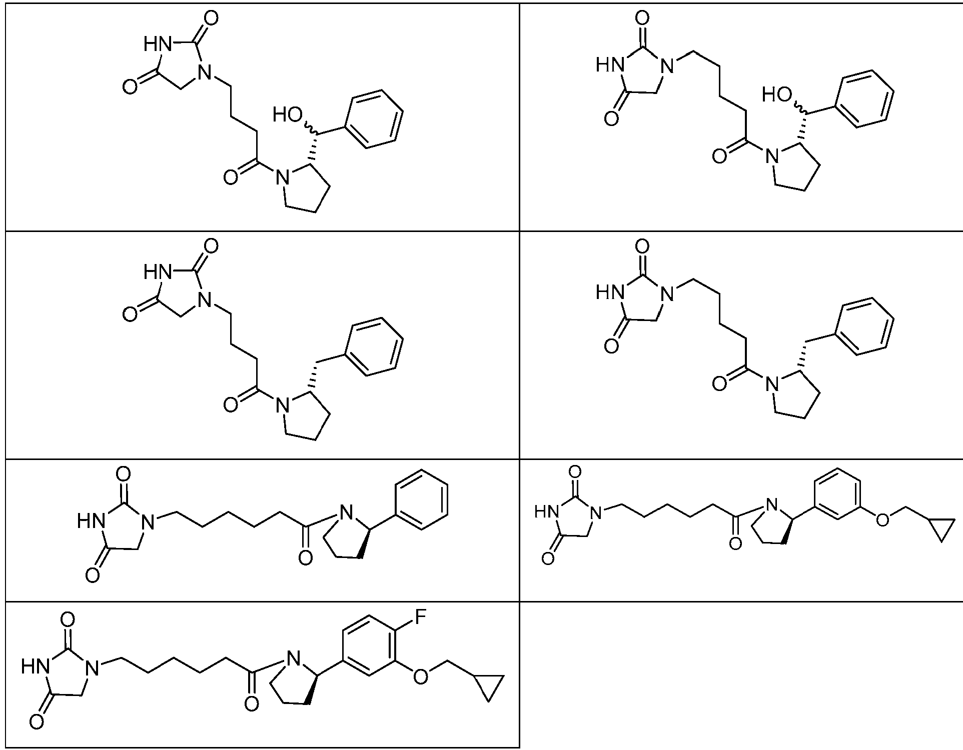

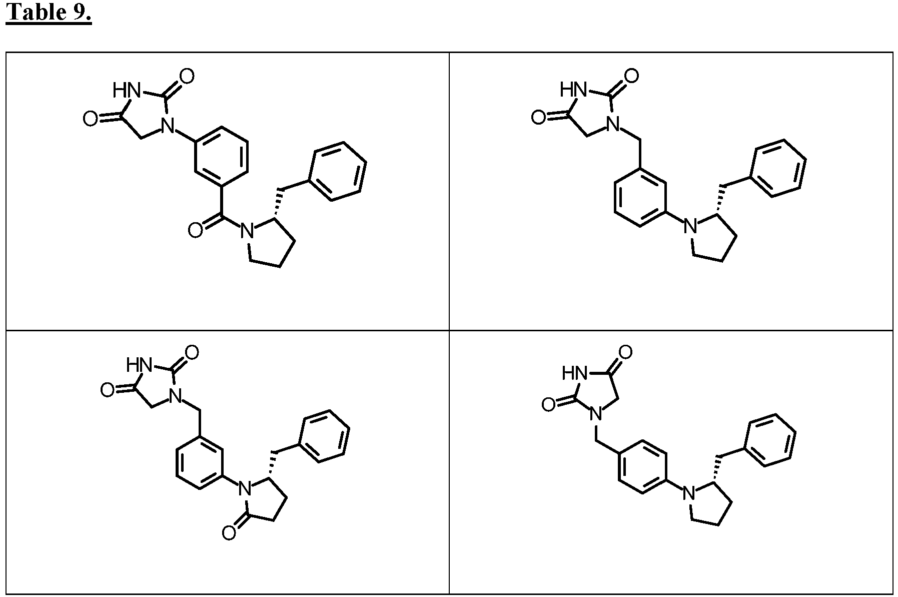

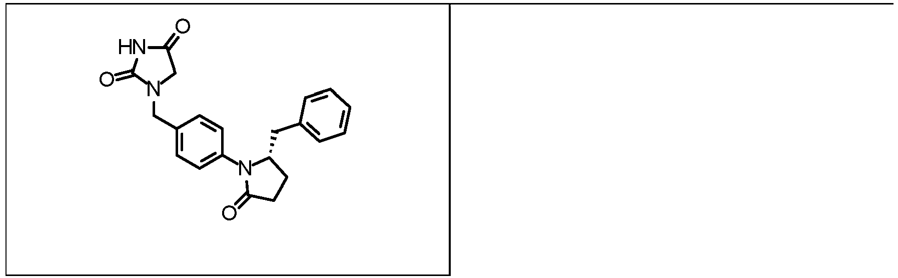

In some embodiments, the dUTPase inhibitor is a compound selected from Tables 1-9. In some embodiments, the dUTPase inhibitor is a compound selected from Tables 1 and 2.

These dUTPase inhibitors provided herein and others are synthesized following art recognized methods with the appropriate substitution of commercially available reagents as needed. For example, and without limitation, methods for synthesizing the dUTPase inhibitor disclosed herein are described in WO 2017/006282, WO 2017/006271, WO 2018/098206, WO 2018/098207, WO 2018/098208, and WO 2018/098209. Non-limiting methods for synthesizing certain other dUTPase inhibitors are described in US 2011/0082163; US 2012/0225838; WO 2014/107622; PCT/US2015/010059; Miyahara et al., J. Med. Chem. (2012) 55, 2970-2980; Miyakoshi et al., J. Med. Chem. (2012) 55, 2960-2969; Miyahara et al., J. Med. Chem. (2012) 55 (11), pp 5483-5496; and Miyakoshi et al., J. Med. Chem. (2012) 55 (14), pp 6427-6437 (each supra). Protection deprotection methods and protecting groups useful for such purposes are well known in the art, for example in Greene's Protective Groups in Organic Synthesis, 4th Edition, Wiley, 2006, or a later edition of the book.

In some embodiments, the dUTPase inhibitor is not a uracil-containing compound. In some embodiments, the dUTPase inhibitor is not a fluorouracil-containing compound. In some embodiments, the dUTPase inhibitor is not (R)-N-(1-(3-(cyclopentyloxy) phenyl)ethyl)-3-((2,4-dioxo-3,4-dihydropyrimidin-1(2H)-yl)methoxy)propane-1-sulfonamide.

### Pharmaceutical Compositions

In another aspect (described for context only), provided herein is a composition comprising, consisting essentially of, or consisting of the combination of compounds provided herein, and at least one pharmaceutically acceptable excipient.

Compositions, including pharmaceutical compositions comprising, consisting essentially of, or consisting of the combination of compounds described herein, can be manufactured by means of conventional mixing, dissolving, granulating, dragee-making levigating, emulsifying, encapsulating, entrapping, or lyophilization processes. The compositions can be formulated in conventional manner using one or more physiologically acceptable carriers, diluents, excipients, or auxiliaries which facilitate processing of the combinations of compounds provided herein into preparations which can be used pharmaceutically.

The combination of compounds of the present disclosure can be administered by parenteral (e.g., intramuscular, intraperitoneal, intravenous, ICV, intracisternal injection or infusion, subcutaneous injection, or implant), oral, by inhalation spray nasal, vaginal, rectal, sublingual, urethral *(e.g.,* urethral suppository) or topical routes of administration *(e.g.,* gel, ointment, cream, aerosol, etc.) and can be formulated in suitable dosage unit formulations containing conventional non-toxic pharmaceutically acceptable carriers, adjuvants, excipients, and vehicles appropriate for each route of administration.

In one embodiment, this technology relates to a composition comprising a combination of compounds as described herein and a carrier.

In another embodiment, this technology relates to a pharmaceutical composition comprising a combination of compounds as described herein and a pharmaceutically acceptable carrier.

In another embodiment, this technology relates to a pharmaceutical composition comprising an effective amount or a therapeutically effective amount of a combination of compounds as described herein and a pharmaceutically acceptable carrier.

The pharmaceutical compositions for the administration of the combinations of compounds can be conveniently presented in dosage unit form and can be prepared by any of the methods well known in the art of pharmacy. The pharmaceutical compositions can be, for example, prepared by uniformly and intimately bringing the compounds provided herein into association with a liquid carrier, a finely divided solid carrier or both, and then, if necessary, shaping the product into the desired formulation. In the pharmaceutical composition, each compound of the combination provided herein is included in an amount sufficient to produce the desired therapeutic effect. For example, pharmaceutical compositions of the present technology may take a form suitable for virtually any mode of administration, including, for example, topical, ocular, oral, buccal, systemic, nasal, injection, infusion, transdermal, rectal, and vaginal, or a form suitable for administration by inhalation or insufflation.

For topical administration, the combination of compounds can be formulated as solutions, gels, ointments, creams, suspensions, etc., as is well-known in the art.

Systemic formulations include those designed for administration by injection (e.g., subcutaneous, intravenous, infusion, intramuscular, intrathecal, or intraperitoneal injection) as well as those designed for transdermal, transmucosal, oral, or pulmonary administration.

Useful injectable preparations include sterile suspensions, solutions, or emulsions of the compounds provided herein in aqueous or oily vehicles. The compositions may also contain formulating agents, such as suspending, stabilizing, and/or dispersing agents. The formulations for injection can be presented in unit dosage form, *e.g.*, in ampules or in multidose containers, and may contain added preservatives.

Alternatively, the injectable formulation can be provided in powder form for reconstitution with a suitable vehicle, including but not limited to sterile pyrogen free water, buffer, and dextrose solution, before use. To this end, the combination of compounds provided herein can be dried by any art-known technique, such as lyophilization, and reconstituted prior to use.

For transmucosal administration, penetrants appropriate to the barrier to be permeated are used in the formulation. Such penetrants are known in the art.

For oral administration, the pharmaceutical compositions may take the form of, for example, lozenges, tablets, or capsules prepared by conventional means with pharmaceutically acceptable excipients such as binding agents (e.g., pregelatinised maize starch, polyvinylpyrrolidone, or hydroxypropyl methylcellulose); fillers (e.g., lactose, microcrystalline cellulose, or calcium hydrogen phosphate); lubricants (e.g., magnesium stearate, talc, or silica); disintegrants (e.g., potato starch or sodium starch glycolate); or wetting agents (e.g., sodium lauryl sulfate). The tablets can be coated by methods well known in the art with, for example, sugars, films, or enteric coatings.

Compositions intended for oral use can be prepared according to any method known to the art for the manufacture of pharmaceutical compositions, and such compositions may contain one or more agents selected from the group consisting of sweetening agents, flavoring agents, coloring agents, and preserving agents in order to provide pharmaceutically elegant and palatable preparations. Tablets contain the combination of compounds provided herein in admixture with non-toxic pharmaceutically acceptable excipients which are suitable for the manufacture of tablets. These excipients can be for example, inert diluents, such as calcium carbonate, sodium carbonate, lactose, calcium phosphate or sodium phosphate; granulating and disintegrating agents (e.g., corn starch or alginic acid); binding agents (e.g. starch, gelatin, or acacia); and lubricating agents (e.g., magnesium stearate, stearic acid, or talc). The tablets can be left uncoated or they can be coated by known techniques to delay disintegration and absorption in the gastrointestinal tract and thereby provide a sustained action over a longer period. For example, a time delay material such as glyceryl monostearate or glyceryl distearate can be employed. They may also be coated by the techniques well known to the skilled artisan. The pharmaceutical compositions of the present technology may also be in the form of oil-in-water emulsions.

Liquid preparations for oral administration may take the form of, for example, elixirs, solutions, syrups, or suspensions, or they can be presented as a dry product for constitution with water or other suitable vehicle before use. Such liquid preparations can be prepared by conventional means with pharmaceutically acceptable additives such as suspending agents (*e.g.*, sorbitol syrup, cellulose derivatives, or hydrogenated edible fats); emulsifying agents *(e.g.,* lecithin, or acacia); non-aqueous vehicles *(e.g.,* almond oil, oily esters, ethyl alcohol, cremophore^{™}, or fractionated vegetable oils); and preservatives (*e.g*., methyl or propyl-p-hydroxybenzoates or sorbic acid). The preparations may also contain buffer salts, preservatives, flavoring, coloring, and sweetening agents as appropriate.

In some embodiments, one or more compositions disclosed herein are contained in a kit. Accordingly, in some embodiments, provided herein is a kit comprising, consisting essentially of, or consisting of one or more compositions disclosed herein and instructions for their use.

### Dosages and Dosing Regimens

The deoxyuridine triphosphatase (dUTPase) inhibitor, the inhibitor of thymidylate biosynthesis, and the immunotherapy agent for use according to the invention are as defined in the claims. Other embodiments are for context only.

The appropriate amount and dosing regimen of the immunotherapy agent, the inhibitor of thymidylate biosynthesis, the inhibitor of folate-mediated one-carbon metabolism, the anthracycline or other topoisomerase II inhibitor, or the dUTPase inhibitor, when present in the combination to be administered to the subject according to any of the methods disclosed herein, may be determined by one of ordinary skill in the art.

In some embodiments, the active compounds from a combination disclosed herein, or salts or solvates thereof, may be administered to a subject suffering from abnormal cell growth, such as a human, either alone or as part of a pharmaceutically acceptable formulation, once a week, once a day, twice a day, three times a day, or four times a day, or even more frequently.

Administration of the compounds within the combinations disclosed herein may be effected by any method that enables delivery of the compounds to the site of action. These methods include oral routes, intraduodenal routes, parenteral injection (including intravenous, subcutaneous, intramuscular, intravascular or infusion), topical, and rectal administration.

Bolus doses can be used, or infusions over a period of 1, 2, 3, 4, 5, 10, 15, 20, 30, 60, 90, 120 or more minutes, or any intermediate time period can also be used, as can infusions lasting 3, 4, 5, 6, 7, 8, 9, 10. 12, 14 16, 20, 24 or more hours or lasting for 1-7 days or more. Infusions can be administered by drip, continuous infusion, infusion pump, metering pump, depot formulation, or any other suitable means.

Dosage regimens may be adjusted to provide the optimum desired response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form, as used herein, refers to physically discrete units suited as unitary dosages for the subjects to be treated; each unit containing a predetermined quantity of active compound calculated to produce the desired therapeutic effect in association with the required pharmaceutical carrier. The specification for the dosage unit forms of the disclosure are dictated by and directly dependent on (a) the unique characteristics of the chemotherapeutic agent and the particular therapeutic or prophylactic effect to be achieved, and (b) the limitations inherent in the art of compounding such an active compound for the treatment of sensitivity in individuals.

Thus, the skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a patient may also be determined, as can the temporal requirements for administering each agent to provide a detectable therapeutic benefit to the patient. Accordingly, while certain dose and administration regimens are exemplified herein, these examples in no way limit the dose and administration regimen that may be provided to a patient in practicing the present disclosure.

It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. For example, doses may be adjusted based on pharmacokinetic or pharmacodynamic parameters, which may include clinical effects such as toxic effects and/or laboratory values. Thus, the present disclosure encompasses intra-patient dose-escalation as determined by the skilled artisan. Determining appropriate dosages and regimens for administration of the chemotherapeutic agent are well-known in the relevant art and would be understood to be encompassed by the skilled artisan once provided the teachings disclosed herein.

In some embodiments, pembrolizumab is administered as a dose of 200 mg every 3 weeks.

In some embodiments, nivolumab is administered as a dose of 240 mg once every 2 weeks. In some embodiments, nivolumab is administered as a dose of 480 mg once every 4 weeks.

In some embodiments, ipilimumab is administered as a dose of 1 mg/kg, 3 mg/kg, or 10 mg/kg every 3 weeks for a total of 4 doses.

In some embodiments, avelumab is administered as a dose of 800 mg every 2 weeks

In some embodiments, durvalumab is administered as a dose of 10 mg/kg every 2 weeks

In some embodiments, atezolizumab is administered as a dose of 1200 mg intravenously over 60 minutes every 3 weeks.

In some embodiments, 5-FU is administered as a dose of 500 mg/m², i.v. bolus on day 1; and 1 hour prior to administering the 5-FU bolus, the patient is also administered leucovorin (500 mg/m², i.v.) over 2 hours. This regimen is repeated weekly on days 1, 8, 15, 22, 29, and 36 every 8 weeks for 4 to 6 cycles.

In some embodiments, 5-FU is administered in combination with radiation therapy. In further embodiments, 5-FU is administered as a dose of 500 mg/m², i.v. bolus for 5 days on days 1 and 36 beginning 22 to 70 days after surgery; and radiation therapy is administered for 6 weeks beginning on day 64 after initiation of 5-FU therapy, while 5-FU is administered at a dose of 225 mg/m²/day, i.v. continuous infusion throughout administration of radiation therapy. Then, 5-FU is administered at a dose of 450 mg/m², i.v. bolus daily for 5 days beginning 1 month after radiation (i.e., days 134 to 138) and repeated for 4 weeks.

In some embodiments, 5-FU is administered in combination with irinotecan and leucovorin, with or without bevacizumab (FOLFIRI with or without bevacizumab), wherein 5-FU is administered as a dose of 400 mg/m², i.v. bolus on day 1, followed by 5-FU 1,200 mg/m²/day on days 1 and 2 by continuous i.v. infusion (CIV) (total infusional dose, 2,400 mg/m²over 46 hours) for cycles 1 and 2. If there is no toxicity greater than grade 1, the 5-FU infusion dose may be increased to 3,000 mg/m² for all subsequent cycles.

In some embodiments, 5-FU is administered in combination with leucovorin and oxaliplatin with or without bevacizumab (FOLFOX4 with or without bevacizumab), wherein 5-FU is administered as a dose of 400 mg/m², i.v. bolus over 2 to 4 minutes, followed by 5-FU 600 mg/m² continuous i.v. infusion (CIV) over 22 hours on day 1. Prior to 5-FU bolus on day 1, oxaliplatin 85 mg/m², i.v. and leucovorin 200 mg/m², i.v. (both over 120 minutes via Y-site) are administered. If giving FOLFOX4 plus bevacizumab, bevacizumab 10 mg/kg, i.v. is administered over 30 to 90 minutes prior to chemotherapy on day 1. On day 2, a regimen of leucovorin 200 mg/m², i.v. over 2 hours followed by 5-FU 400 mg/m², i.v. bolus, followed by 5-FU 600 mg/m² CIV over 22 hours is repeated. The order of administration is bevacizumab followed by oxaliplatin and leucovorin, followed by 5-FU. This 2-day regimen is repeated every 2 weeks until disease progression or unacceptable toxicity is observed.

In some embodiments, capecitabine is administered as adjuvant following surgery (monotherapy) as a dose of 1.25 g/m² twice daily for 14 days, and subsequent courses are repeated after a 7-day interval, with recommended duration of treatment as 6 months, adjusted dose according to tolerability.

In some embodiments, capecitabine is administered as adjuvant following surgery (combination therapy) as a dose of 0.8-1 g/m² twice daily for 14 days, and subsequent courses are repeated after a 7-day interval, with recommended duration of treatment as 6 months, adjusted dose according to tolerability.

In some embodiments, capecitabine is administered as a dose of 1.25 g/m² twice daily for 14 days, and subsequent courses are repeated after a 7-day interval, adjusted dose according to tolerability.

In some embodiments, capecitabine is administered as a dose of 0.8-1 g/m² twice daily for 14 days, and subsequent courses are repeated after a 7-day interval, adjusted dose according to tolerability.

In some embodiments, capecitabine is administered in combination with a platinum based regimen, wherein capecitabine is administered as a dose of 0.8-1 g/m² twice daily for 14 days, and subsequent courses are repeated after a 7-day interval, or alternatively administered as a dose of 625 mg/m² twice daily given continuously, adjusted dose according to tolerability.

In some embodiments, methotrexate is administered orally or intramuscularly in doses of 15 to 30 mg daily for a five-day course. Such courses are usually repeated for 3 to 5 times as necessary.

In some embodiments, methotrexate is administered as a dose of 10 to 25 mg/day orally for 4 to 8 days.

In some embodiments, methotrexate is administered as a dose of 5 to 50 mg once weekly. Dose reduction or cessation is guided by patient response and hematologic monitoring.

In some embodiments, methotrexate is used in combination with other agents. In further embodiments, in addition to high-dose methotrexate with leucovorin rescue, these agents may include doxorubicin, cisplatin, and the combination of bleomycin, cyclophosphamide and dactinomycin (BCD). The starting dose for high-dose methotrexate treatment is 12 grams/m².

The following examples are included to demonstrate some embodiments of the disclosure.

### EXAMPLES

### Example 1. Efficacy of Compound A with 5-FU and an anti-PD-1 antibody in a murine MC-38 syngeneic model.

**Experimental Overview.** MC38 colon cancer cells were implanted subcutaneously into the right flank of female C57 Bl6/J mice and allowed to propagate until they reached 80-100 mm³ at which point mice were randomized into one of nine groups: Vehicle, Compound A, 5-FU, Compound A + 5-FU, anti-PD-1, anti-PD-1 + Compound A, anti-PD-1 + 5-FU, the triple combination of Compound A + 5-FU + anti-PD-1, and a triple combination of Compound A + 5-FU + IgG (as an isotype control for the antibody). Animals were treated with 200 mg/kg Compound A (3 treatments every 8 hours on day 1), 75 mg/kg 5-FU, 300 µg anti-PD-1 or IgG as appropriate. Combination regimens used combinations of the same doses used for single agent therapy and were administered concomitantly. Two seven-day cycles of treatment were administered and tumor volume and mouse bodyweight as indicators of antitumor efficacy and treatment tolerability respectively were measured until end of study. Study endpoints were selected and defined according to the Developmental Therapeutics Program of the US National Cancer Institute (dtp.nci.nih.gov) and as detailed by Holingshead in the Journal of the National Cancer Institute (Melinda G. Hollingshead. Antitumor Efficacy Testing in Rodents. J Natl Cancer Inst. 2008. 5; 100(21): 1500-1510).
**1.** Tumor volume in mm³ of each group at conclusion of study
**2.** Mouse bodyweight in each group at study conclusion

**Study Compliance.** The study was conducted in accordance with the guidelines of the Department of Health, Social Services and Public Safety (NI), (DHSSPS) and in accordance with the University Animal Welfare and Ethical Review Body (AWERB) under a Project License granted by the DHSSPS under the aforementioned Act. All procedures of the present study will be in accordance with the guidelines provided by the Animals (Scientific Procedures) Act 1986 (ASPA), and subsequent amendments. Healthy female C57 Bl6/J mice (6-8 weeks old) weighing between 17 - 20 g were procured from Envigo (Cambridgeshire, UK). Four mice were housed in each cage. Temperature and humidity was maintained at 22 ± 3 °C and 40-70 %, respectively and recorded by auto-controlled data logger system. Illumination was controlled to give a sequence of 12 hr light and 12 hr dark cycle. All the animals were provided a standard rodent diet *ad libitum.* Reverse osmosis water treated with ultraviolet light was provided *ad libitum.* Animals were granted a one week acclimitization period.

**Animal Model Selection.** Mus musculus female C57 Bl6/J of age 6-8 weeks and bodyweight 18±2.5 grams were sourced from Envigo (Cambridgeshire, UK).

**Cell Line Model Selection and Preparation.** The MC38 mouse syngeneic colon cancer cell line was obtained from the NCI. Cells were handled according to established Standard Operating Procedures. Briefly, cells were cultured and expanded in humidified incubators at 5% CO₂ until approximately 10⁸ cells were available. The day prior to allograft implantation, cell culture media was isolated from all flasks and a screen for mycoplasma was performed and confirmed negative.

**Tumor Cell Implantation.** On the day of tumor cell implantation, cells were harvested in ice-cold PBS, counted and suspended at a concentration of 1x10⁷ cells/ml in preparation for implantation. Allografts were established by the injection of 1x10⁶ cells in a volume of 100 µl ice-cold PBS containing 50% matrigel (Corning) using a 27 gauge sterile needle (Becton Dickinson).

**Drug Formulation and Preparation Procedure.** 5-FU (Sigma Aldrich, >99%) was weighed out and the appropriate mass was suspended in 0.9% NaCl at a formulation concentration of 7.5 mg/ml. The 5-FU solution required repeated vortexing and once fully solubilized was sterile-filtered through a 0.22 µm aqueous filter membrane (Millipore). The oral formulation for Compound A was prepared immediately prior to use. All reagents are supplied sterile or are sterile-filtered and pre-warmed to 37°C. The formulation consisted of the appropriate quantity of Compound A in 7.5% NMP, 10% Solutol HS-15, 30% PEG-400 and 52.5% saline to yield a formulation concentration of 13.3 mg/ml (all percentages are total w/v; please note the 60% PEG-400 is a 50% solution in saline and thus contributes 30% neat PEG-400 total to the formulation). The resulting solution was vortexed and kept at 37°C until used. Anti-PD-1 (CD279) clone RMPI-14 (BioXcell; Lot/Batch number: 717919M1; Concentration: 7.18 mg/ml; Purity: >95%) and the IgG2a isotype control clone 2A3 (BioXcell; Lot/Batch number: 71671801; Concentration: 8.56 mg/ml; Purity: >95%) were both prepared at a formulation concentration of 1.5 mg/ml in saline (0.9%w/v in NaCl) and administered at 15 mg/kg (~300 µg/mouse) at a dose volume of 10 ml/kg.

**Randomization and Dose Administration.** Seventy-two mice were weighed and randomized in to nine groups with eight mice in each group. Treatment was initiated when the mean tumor volume of all groups was within 100 mm³ ± 20 mm³. i.p. injections were performed using a Becton Dickinson 30 gauge needle and oral gavage performed using a 20 gauge stainless steel curved gavage needle (Fine Science Tools). The treatment regimen consisted of two cycles, commencing seven days apart with treatment on day 1 of each cycle. All agents were administered independently with the exception of animals receiving combinations that included 5-FU and anti-PD-1 or IgG (both i.p. routes). These agents were administered with a combined preparation of both compounds in a saline solution and the total volume injected remained the same as for the monotherapy.
1. Group 1 animals were administered with the vehicle solution of saline by i.p. injection once daily and orally with vehicle (7.5% NMP, 10% Solutol HS-15, 30% PEG-400 and 52.5% saline).
2. Group 2 animals were administered with Compound A at 200 mg/kg, p.o. at 15 ml/kg, 3 doses in a 24 hour period on day 1 of each cycle.
3. Group 3 animals were administered with 5-FU at 75 mg/kg, i.p. at 10 ml/kg on day 1 of cycle, given concomitant with first dose of Compound A.
4. Group 4 animals were co-administered with 5-FU and Compound A as described above.
5. Group 5 animals were administered with 300 µg anti-PD-1 at 10 ml/kg i.p. on day 1 of the cycle.
6. Group 6 animals were administered with the combination of 300 µg anti-PD-1 and Compound A, as described above.
7. Group 7 animals were administered with anti-PD-1 and 5-FU, as described above.
8. Group 8 animals were administered with the triple combination of Compound A, 5-FU and anti-PD-1, as described above.
9. Group 9 animals were administered with the triple combination of Compound A, 5-FU and IgG, in the place of anti-PD-1.

**Efficacy and Toxicity Analysis and Biospecimen Collections.** Tumors were measured 3 times per week during the course of the study by digital calipers by the same investigator, and tumor volume was calculated using the modified Ellipsoid equation 1/2(Length × Width²) and expressed in mm³. Animals were considered to have reached end of study and were sacrificed when tumor volume reached 1000 mm³ ± 100 mm³. Mouse bodyweight was measured 3 times per week using an Ohaus TA301 digital balance as a general indicator of toxicity and/or general physical condition. Animals were also inspected daily for signs of abnormal behavior, and/or any decline in physical condition. Both endpoints of efficacy and mouse bodyweight were analyzed statistically by a two-way ANOVA (Graphpad, Prism 6.0) and multiple comparisons testing where *p*<0.05 was considered significant. At the end of life or termination of study, tumors from each animal were excised and bisected. One half was fixed in formalin and subsequently paraffin-embedded. A small fragment from the rim of the other half was placed in RNA Later at 4°C overnight before transfer to 80°C, the remainder was snap frozen on dry ice before transfer to 80°C. Those tumors which had regressed to <50 mm³ were processed for histology only, owing to the lack of tissue.

### Results

**Antitumor Efficacy.** The combination of Compound A + 5-FU + Anti-PD-1 led to significant improvement in the antitumor efficacy when compared to all other treatment groups including all other 5-FU and anti-PD-1 combinations (Figure 1). The combination of Compound A + 5-FU + Anti-PD-1 was the only treatment group that led to complete inhibition of tumor growth from day 1 to day 19; all other treatment groups demonstrated mean tumor volume increases throughout this period (one-way ANOVA at day 19 = p<0.0001 with Tukey's Multiple Comparisons test: p<0.05 and 0.001 when Compound A + 5-FU + Anti-PD-1 is compared to 5-FU + anti-PD-1 on day 19). At end of study, 6 out of 8 animals in the Compound A + 5-FU + Anti-PD-1 remained alive (maximum permissible tumor volume not reached) compared to 4 out of 8 for the 5-FU + Anti-PD-1 treatment group. All animals in the 7 other treatment groups had reached maximum permissible tumor volume and were removed from study prior to study end (day 29). In the 5-FU + Anti-PD-1 treatment group, one animal had a complete regression with no palpable tumor remaining (Figure 2, group 7). In the Compound A + 5-FU + Anti-PD-1 treatment group, 4 animals had minimal evidence of residual tumor or inflammatory tissue with tumor volumes of <40 mm² (Figure 2, Group 8). As the tumor volume for these animals had remained exceptionally low and had not increased in size for >2 weeks since the last treatment and when removed at end of study, the biospecimen did not resemble tumor tissue, the removed specimens were sent for independent histopathological analysis. Independent histopathological analysis confirmed that these 4 animals from the Compound A + 5-FU + Anti-PD-1 treatment group had no viable tumor tissue remaining with evidence of fibrotic tissue only; these 4 animals were categorized as complete responses. These unexpected results demonstrate the surprisingly synergistic anti-tumor efficacy of a combination of a dUTPase inhibitor, an inhibitor of thymidylate biosynthesis, and an immunotherapy agent.

**Mouse Bodyweight as General Indicator of Toxicity.** All treatments were well tolerated. There were no adverse events, signs of distress or discomfort noted for the duration of the study. There was no statistically significant difference between means of each treatment group for the duration of the study (one-way ANOVA p = ns; Figure 3). All treatment groups gained bodyweight for the duration that each of the treatment groups was on study prior to reaching maximum permissible tumor volume and being removed from study (Figure 3).

Compound A is

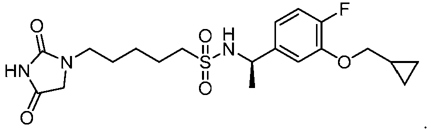

### Example 2. Biomarker study evaluating Compound A with 5-FU and an anti-PD-1 antibody in a murine MC-38 syngeneic model.

**Experimental Overview.** MC38 colon cancer cells were implanted subcutaneously into the right flank of female C57 Bl6/J mice and allowed to propagate until they reached 80-100 mm³ at which point mice were randomized into one of eight groups (n=8 per group): Vehicle, Compound A, 5-FU, Compound A + 5-FU, anti-PD-1, anti-PD-1 + Compound A, anti-PD-1 + 5-FU, the triple combination of Compound A + 5-FU + anti-PD-1. Animals were treated with 200 mg/kg Compound A (3 treatments every 8 hours on day 1), 75 mg/kg 5-FU, 300 µg anti-PD-1 or IgG as appropriate. Combination regimens used combinations of the same doses used for single agent therapy and were administered concomitantly. Two seven-day cycles of treatment were administered one week apart. Each treatment group of 8 animals was divided in to 2 groups, one group were euthanized for biospecimen collection on day 4 (48 hours post treatment in cycle 1) and the remaining 4 animals were euthanized on day 10, 48 hours post treatment in cycle 2. The primary study endpoints were the histopathological and immunohistochemical analysis of makers of immune infiltration from formalin-fixed paraffin-embedded tumor specimens removed on day 4 (48 hours post treatment in cycle 1) and day 10, (48 hours post treatment in cycle 2). Secondary endpoints included tumor volume and mouse bodyweight as general indicators of antitumor efficacy and treatment tolerability respectively were measured until end of study.

**Study Compliance.** The study was conducted in accordance with the guidelines of the Department of Health, Social Services and Public Safety (NI), (DHSSPS) and in accordance with the University Animal Welfare and Ethical Review Body (AWERB) under a Project License granted by the DHSSPS under the aforementioned Act. All procedures of the present study will be in accordance with the guidelines provided by the Animals (Scientific Procedures) Act 1986 (ASPA), and subsequent amendments. Healthy female C57 Bl6/J mice (6-8 weeks old) weighing between 17 - 20 g were procured from Envigo (Cambridgeshire, UK). Four mice were housed in each cage. Temperature and humidity was maintained at 22 ± 3 °C and 40-70 %, respectively and recorded by auto-controlled data logger system. Illumination was controlled to give a sequence of 12 hr light and 12 hr dark cycle. All the animals were provided a standard rodent diet *ad libitum.* Reverse osmosis water treated with ultraviolet light was provided *ad libitum.* Animals were granted a one week acclimitization period.

**Animal Model Selection.** Mus musculus female C57 Bl6/J of age 6-8 weeks and bodyweight 18±2.5 grams were sourced from Envigo (Cambridgeshire, UK).

**Cell Line Model Selection and Preparation.** The MC38 mouse syngeneic colon cancer cell line was obtained from the NCI. Cells were handled according to established Standard Operating Procedures. Briefly, cells were cultured and expanded in humidified incubators at 5% CO₂ until approximately 10⁸ cells were available. The day prior to allograft implantation, cell culture media was isolated from all flasks and a screen for mycoplasma was performed and confirmed negative.

**Tumor Cell Implantation.** On the day of tumor cell implantation, cells were harvested in ice-cold PBS, counted and suspended at a concentration of 1x10⁷ cells/ml in preparation for implantation. Allografts were established by the injection of 1x10⁶ cells in a volume of 100 µl ice-cold PBS containing 50% matrigel (Corning) using a 27 gauge sterile needle (Becton Dickinson).

**Drug Formulation and Preparation Procedure.** 5-FU (Sigma Aldrich, >99%) was weighed out and the appropriate mass was suspended in 0.9% NaCl at a formulation concentration of 7.5 mg/ml. The 5-FU solution required repeated vortexing and once fully solubilized was sterile-filtered through a 0.22 µm aqueous filter membrane (Millipore). The oral formulation for Compound A was prepared immediately prior to use. All reagents are supplied sterile or are sterile-filtered and pre-warmed to 37°C. The formulation consisted of the appropriate quantity of Compound A in 7.5% NMP, 10% Solutol HS-15, 30% PEG-400 and 52.5% saline to yield a formulation concentration of 13.3 mg/ml (all percentages are total w/v; please note the 60% PEG-400 is a 50% solution in saline and thus contributes 30% neat PEG-400 total to the formulation). The resulting solution was vortexed and kept at 37°C until used. Anti-PD-1 (CD279) clone RMPI-14 (BioXcell; Lot/Batch number: 717919M1; Concentration: 7.18 mg/ml; Purity: >95%) was prepared at a formulation concentration of 1.5 mg/ml in saline (0.9%w/v in NaCl) and administered at 15 mg/kg (~300 µg/mouse) at a dose volume of 10 ml/kg.

**Randomization and Dose Administration.** Sixty-four mice were weighed and randomized in to eight groups with eight mice in each group. Treatment was initiated when the mean tumor volume of all groups was within 100 mm³ ± 20 mm³. i.p. injections were performed using a Becton Dickinson 30 gauge needle and oral gavage performed using a 20 gauge stainless steel curved gavage needle (Fine Science Tools). The treatment regimen consisted of two cycles, commencing seven days apart with treatment on day 1 of each cycle. All agents were administered independently with the exception of animals receiving combinations that included 5-FU and anti-PD-1 or IgG (both i.p. routes). These agents were administered with a combined preparation of both compounds in a saline solution and the total volume injected remained the same as for the monotherapy.
1. Group 1 animals were administered with the vehicle solution of saline by i.p. injection once daily and orally with vehicle (7.5% NMP, 10% Solutol HS-15, 30% PEG-400 and 52.5% saline).
2. Group 2 animals were administered with Compound A at 200 mg/kg, p.o. at 15 ml/kg, 3 doses in a 24 hour period on day 1 of each cycle.
3. Group 3 animals were administered with 5-FU at 75 mg/kg, i.p. at 10 ml/kg on day 1 of cycle, given concomitant with first dose of Compound A.
4. Group 4 animals were co-administered with 5-FU and Compound A as described above.
5. Group 5 animals were administered with 300 µg anti-PD-1 at 10 ml/kg i.p. on day 1 of the cycle.
6. Group 6 animals were administered with the combination of 300 µg anti-PD-1 and Compound A, as described above.
7. Group 7 animals were administered with anti-PD-1 and 5-FU, as described above.
8. Group 8 animals were administered with the triple combination of Compound A, 5-FU and anti-PD-1, as described above.

**Biospecimen Collections, Efficacy and Bodyweight Analysis.** On day 4 of the study, half the mice from each treatment group were sacrificed by approved Schedule 1 method of CO₂ asphyxiation, followed by cervical dislocation. Following confirmation of cessation of heartbeat and breathing, tumors were excised, bisected and formalin fixed for subsequent paraffin-embedding. A small fragment from the rim was placed in RNA Later at 4°C overnight before transfer to 80°C. All remaining mice were sacrificed on day 10 of the study. Tumors were excised, bisected and formalin fixed for paraffin embedding. Although the study was not statistically powered to determine antitumor efficacy, tumor volume (mm³) was measured twice per week (day 4 and day 10) during the course of the study by digital calipers by the same investigator, and calculated using the modified Ellipsoid equation 1/2(Length × Width²). Mouse bodyweight was also measured 3 times per week using an Ohaus TA301 digital balance as a general indicator of toxicity and/or general physical condition. Animals were also inspected daily for signs of abnormal behavior, and/or any decline in physical condition.

### Histological and Immunohistochemical Analyses

Biospecimens were supplied to the Precision Medicine Centre (PMC) of Excellence at Queen's University Belfast for histological and immunohistochemistry analysis. All assessors were blinded to the experimental design (including time points and treatment interventions) and identity of all specimens other than the species tissue of origin (mouse) for quality control and validation purposes. Sixty-four Formalin Fixed Paraffin Embedded (FFPE) syngeneic mouse model samples were processed to FFPE blocks, sectioned at 5 µm and stained with CD3, CD4, CD8 & CD45 using the Leica Research Module, primary antibody clone LN10 (Leica) and detected using Leica Refine DAB kit with a haematoxylin counterstain. CD3, CD4, CD8 & CD45 were staining was performed according to PMC Analytical SOPs. Slides were scanned on Aperio T2 scanner to .svs image format (Leica Biosystems). QuPath v0.1.2 was used to quantify CD4, CD3, CD8 & CD45 cell populations in peripheral and intra-tumor regions. Intra-tumor and Peripheral Immune Regions of Interest (ROIs) were identified using the QuPath software for imaging, for Peripheral Immune ROIs, an average of 500 µm, either side of the invading tumor region(s) was taken. Individual area of ROIs varied according to sample. An average of values for respective ROI categories was calculated. Threshold values of 0.3 DAB Optical Density were used and regions quality controlled for immune cell identification before analysis. This threshold has been determined by a Pathologist (MST) to be most accurate in the determination of positive CD4, CD3, CD8 or CD45 cell density from previous work, submitted and under review. Results were expressed as the density of CD4, CD3, CD8 or CD45 positive cells per mm². In instances of multi ROIs, for each sample, an average value was generated. The mean data were provided in Excel spreadsheet format.

### Results

### Histopathological and Immunohistochemical Profiling of Immune Markers Demonstrates Strong Evidence that Compound A Enhances Tumor Immune Infiltration in Combination with 5-FU and an Anti-PD-1 Antibody.

To determine the impact of treatment on the immune cell content within the extracted tumor cells, histopathology and immunohistochemistry were used to assess and quantify key differences in the ratio of tumor content to fibrotic tissues and the abundance of key immune cell populations. To quantify all leukocytes present in the tumor specimens, CD45+ was examined. To quantify T-cell populations, CD3+, CD8+ and CD4+ were examined.

The combination of Compound A with 5-FU and an anti-PD-1 antibody significantly reduced the percentage of tumor tissue relative to fibrotic tissue in the tumors removed from animals on day 10 of the study when compared to vehicle control (Figure 4; *p<0.0001* for both tumor content and fibrotic tissue compared to vehicle control). When compared to 5-FU + anti-PD-1, the combination of Compound A + 5-FU + Anti-PD-1 had a significantly greater ratio of fibrotic tissue to tumor content (Figure 4; *p*<0.001 for both tumor content and fibrotic tissue by multiple comparisons test). Immunohistochemical analysis of T-cell infiltration demonstrated a highly significant increase in CD8+ cells when compared to vehicle control (Figure 5; p<0.0001) and when compared to all other treatment groups (Figure 5; p<0.0001) and the combination of Compound A + 5-FU + Anti-PD-1 led to a 838% increase in CD8+ cells per mm² when compared to 5-FU + Anti-PD-1 (Figure 5; *p<0.0001).* A similar pattern was observed for CD4+ cells where Compound A + 5-FU + anti-PD-1 had the greatest density of CD4+ cells per mm² when compared to all other treatment groups. When compared to 5-FU + Anti-PD-1, the combination of Compound A + 5-FU + Anti-PD-1 increased the infiltration of CD4+ cells by 215% (Figure 6). The combination of Compound A + 5-FU + Anti-PD-1 was the only treatment group that yielded a statistically significant increase in the intratumoral density of CD3+ cells per mm² (Figure 7, *p*<0.01) with all other treatment groups showing significant increase when compared to vehicle control (p>0.05). CD45+ cells (leukocytes) were quantified as a general indicator of immune cell infiltration. The combination of Compound A + 5-FU + Anti-PD-1 antibody exhibited the highest intratumoral density of CD45+ cells per mm² when compared to vehicle control (p<0.01) and all other treatment groups (Figure 8). When compared to 5-FU + Anti-PD-1 antibody, the combination of Compound A + 5-FU + Anti-PD-1 antibody yielded a 69% increase in intratumoral cells positive for CD45+ (Figure 8).

Taken together, these data unexpectedly demonstrate that a dUTPase inhibitor (e.g., Compound A) leads to a significant enhancement of the infiltration of both cytotoxic and regulatory T-cells (measured by CD8+ CD4+ and CD3+), leukocytes (measured by CD45+) in tumor tissue when combined with an inhibitor of thymidylate biosynthesis (e.g., 5-FU) + an immunotherapy agent (e.g., an Anti-PD-1 antibody). In addition, the decrease in remaining tumor tissue combined with the increase in fibrotic tissue strongly indicates a more robust immunological response in the tumor that lead to enhanced eradication of tumor tissue and elevated levels of fibrotic tissue as a result of wound healing post immune-destruction of tumor cells.

**Tumor Volume as a Measure of Antitumor Efficacy and Mouse Bodyweight as a General Indicator of Toxicity.** Although the study was not necessarily designed or powered to detect significant differences in antitumor efficacy, the combination of Compound A + 5-FU + Anti-PD-1 was the only group that led to a reduction in tumor volume on day 4 and day 10 and demonstrated a significant improvement in the inhibition of tumor growth when compared to all other treatment groups including all other 5-FU and anti-PD-1 combinations (Figure 9; one-way ANOVA at day 4 and day 10 = *p*<0.0001 with Tukey's Multiple Comparisons test: p<0.001 when Compound A + 5-FU + Anti-PD-1 is compared to vehicle). All treatments were well tolerated. There were no adverse events, signs of distress or discomfort noted for the duration of the study. Although the study was not designed to detect statistically significant differences in bodyweight with high statistical power, there was still no statistically significant decrease in bodyweight between vehicle group and all other treatment group at days 4 or 10 (One-way ANOVA with Dunnett's Multiple comparisons test). All treatment groups gained bodyweight for the duration that each group was on study (Figure 10).

### Example 3: Modulation of PD-L1 cellular and cell surface expression in various cancer cell line models by combination of Compound A and FUdR (described for context only)

**Experimental Overview.** Programmed death-ligand 1 (PD-L1) is a type 1 transmembrane protein that plays a major role in suppressing the innate and adaptive arms of the immune system. The PD-1/PD-L1 pathway represents an adaptive immune resistance mechanism exerted by tumor cells in response to endogenous immune anti-tumor activity and is a well-established therapeutic target in cancer therapy. Cancer cells were treated with vehicle control (DMSO), the fluoropyrimidine FUdR (also known as floxuridine) alone, Compound A alone, or the combination of Compound A and FUdR, and the expression level of the immune checkpoint protein PD-L1 was measured by both Western blotting and flow cytometry and compared to control. Cell lines assayed for total PD-L1 protein expression by Western blotting included, pancreatic cancer, PANC-1; non-small cell lung cancer, H460; colorectal cancer, HCT116; breast cancer, MCF-7; melanoma, MeWo. Two cell lines were advanced to analyze cell surface PD-L1 by flow cytometry, MCF-7 and PANC-1.

**Western Blotting.** Cells were cultured in the presence of the indicated concentrations of Compound A, 5-fluorodeoxyuridine (FUdR), or a combination of both in a P100 cell culture dishes for 12 or 24 hours. At the conclusion of specified time-points, cells were scraped in PBS containing NaF and Na₃VO₄, pelleted by centrifugation, washed with PBS and pelleted again. Cell pellets were then snap frozen in liquid nitrogen and stored at - 80°C if they are to be processed at a later time. The cell pellet was thawed and lysed with RIPA buffer containing protease and phosphatase inhibitors (HALT^{™}) on ice for 40 minutes. Cells were then physically lysed using 21-gauge needle and syringe followed by centrifugation (13,200 rpm). The concentration of the isolated supernatant was determined by BCA assay on a CLARIOstar microplate reader and the required volume of whole-cell lysate was denatured by the addition of Sample Buffer, Laemmli 2× Concentrate followed by boiling at 95 °C for 5 min. Each sample was loaded alongside PageRuler^{™} Prestained Protein Ladder onto a hand-cast 10% acrylamide gel or pre-cast 10% Mini-PROTEAN^{®} TGX Stain-Free^{™} protein gel followed by SDS-PAGE at 30 amps per gel in Tris-Glycine SDS running buffer. The proteins were electrotransferred to PVDF membranes (0.45 µm) using Trans-blot^{®} Turbo^{™} transfer system or Mini Trans-Blot^{®} Wet transfer at 100 volts for 70 minutes in Tris-Glycine transfer buffer. The PVDF was blocked in 5% milk (prepared in PBS-0.1% TWEEN^{®}-20). Immunoblots were incubated in PD-L1 antibody first, followed by β-Actin as a loading control. Following primary incubation, immunoblots were washed in PBS-0.1% TWEEN^{®}-20 for 3 x 10 minutes and incubated in the secondary HRP-conjugated antibody at room temperature for 2 hours. After incubation, immunoblots were washed as previously and 2 ml of HRP substrate was added to each immunoblot before imaging on a GBOX Chemi XX6. Images were analyzed in ImageJ to obtain PD-L1 relative density values which were normalised to relative density of loading control. Once the primary image acquisition was complete, the pre-cast gels and corresponding immunoblots were further imaged for total protein as a quality control measure following a UV-induced 1-minute reaction to produce fluorescence. Hand-cast immunoblots were stained with Ponceau S solution and imaged to evaluate total protein.

**Cell Surface Flow Cytometry.** Cells were seeded in 6-well plates and permitted to adhere overnight. Media was removed and replaced with media containing the indicated concentrations of Compound A, FUdR or a combination of both, positive control cells were treated with IFNy. Cells were then incubated for 24 hours. At the conclusion of specified timepoints, cells were washed with PBS and removed from the plates using trypsin-EDTA. Cells were counted and 300,000 cells were washed in staining buffer and incubated in PD-L1 or IgG antibody on ice for 40 minutes. Cells were then washed in staining buffer twice and finally resuspended in 300 µL of staining buffer and transferred to BD Falcon^{™} round-bottom tube. Dot plot for FSC v SSC were used to gate cell population and doublets were excluded on FL-4 histogram height versus area/width. IgG isotype control stained sample was used to identify the positively stained population. Percentage positive population and median fluorescent intensity values were exported and analyzed in Microsoft Excel and GraphPad Prism 6. Statistical analysis consisted of one-way ANOVA with Tukey's multiple comparisons testing.

### Results

A panel of heterogenous cancer cell lines were treated with vehicle control (DMSO), Compound A, FUdR and the combination of Compound A and FUdR and PD-L1 protein expression was analyzed by both Western blotting for total PD-L1 expression and subsequently by flow cytometry for cell-surface PD-L1 expression. Following treatment with vehicle control or Compound A, PD-L1 expression was unchanged in all cell lines. Treatment with 1 µM FUdR led to a significant increase in PD-L1 expression in HCT116 (colon cancer), MCF-7 (breast cancer) PANC-1 (pancreatic) and Mewo (melanoma) cell lines at 12 and 24 hours. When these cell lines were treated with 12.5 µM Compound A combined with 1 µM FUdR, PD-L1 expression was significantly reduced compared to control and was substantially less than that observed with 1 µM FUdR treatment alone (Figure 11). When used in combination with FUdR, Compound A unexpectedly exerted the ability to block the FUdR-induced increase in PD-L1 expression in HCT116, PANC-1, MeWo and MCF-7 cell lines in addition to reducing PD-L1 expression from baseline levels in the H460 cell line where PD-L1 was high at baseline and FUdR treatment did not induce it.

Based on the observed result where a significant increase in PD-L1 expression with FUdR was determined and a significant reduction in PD-L1 with the combination of Compound A and FUdR was determined, two cell lines, PANC-1 and MCF-7 were selected for further analysis. PD-L1 exerts its primary immunosuppressive effect when expressed on the cell surface. Flow cytometry was performed on intact cells to determine the cell-surface expression of PD-L1 following treatment with vehicle control (DMSO), 12.5 µM Compound A, 1 µM FUdR, and the combination of 12.5 µM Compound A and 1 µM FUdR. Interferon gamma (IFN-γ) was used as a positive control known to stimulate cell-surface PD-L1 expression in PANC-1 cells. In PANC-1 cells treated with Compound A, PD-L1 expression was the same as vehicle control cells. IFN-γ led to a 7-fold increase in cell-surface PD-L1 expression when compared to control cells. Similarly, treatment with FUdR had an approximate 10-fold increase in cell-surface PD-L1 expression. The combination of Compound A and FUdR, however, was not significantly different from control (Figure 12). Cell-surface PD-L1 expression with FUdR treatment vs Compound A + FUdR was highly statistically significant (p<0.001, one-way ANOVA with Tukey's multiple comparisons). In MCF-7 cells, IFN-y induced a small increase in cell-surface PD-L1 expression when compared to control cells that was not statistically significant. No significant difference in cell-surface PD-L1 expression was observed following Compound A treatment. Treatment with 1 µM FUdR led to a statistically significant 4.7-fold increase in cell-surface PD-L1 expression when compared to control. When cells were treated with the combination of Compound A and FUdR, cell-surface PD-L1 expression was significantly downregulated to undetectable levels below that of control cells (p<0.001, one-way ANOVA with Tukey's multiple comparisons: p<0.001 when FUdR treatment is directly compared to Compound A + FUdR treatment; Figure 12). When used in combination with an inhibitor of thymidylate biosynthesis (e.g., FUdR), a dUTPase inhibitor (e.g., Compound A) surprisingly exerted the ability to block the FUdR-induced increase in cell-surface PD-L1 expression in HCT116 and PANC-1 cancer cell lines maintaining it at similar levels to, or reducing it below that of vehicle-treated control cells.

### Example 4: Induction of passive release of HMGB1 by combination of Compound A and FUdR (described for context only)

**Experimental Overview.** High mobility group box 1 (HMGB1) is a nuclear non-histone chromatin-binding protein. The release of HMGB1 by immune cells or severely damaged cancer cells functions as an immunostimulatory chemokine and is a well-known damage-associated molecular pattern (DAMP). The release of HMGB1 during cell death is characteristic of immunogenic cell death, a sub-class of cell death that results in the potent stimulation of the innate and subsequently the stimulation of adaptive immune responses. HMGB1 functions as a pro-inflammatory cytokine-like factor and can form hetero-complexes with other immune regulators. HMGB1 binds to TLRs, RAGE and CXCR4 on immune cells stimulating chemotaxis and the secretion of pro-inflammatory factors. HCT116 colon carcinoma and JU77 mesothelioma cells were treated with vehicle control (DMSO), 12.5 µM Compound A, 1 µM FUdR, or the combination of 12.5 µM Compound A and 1 µM FUdR for 24 hours and the release of HMGB1 into the extracellular culture media was measured by ELISA.

**Cell Line Model Selection and Preparation.** The HCT116 cancer cell line was purchased from ATCC. JU77 cell line was a kind gift from Dr Dan Longley, QUB. Cells were cultured and expanded in humidified incubators at 5% CO₂ until sufficient cells were available.

**Drug Preparation and Treatments.** All drugs/compounds were prepared freshly in cell culture media from 50 mM frozen stocks prior to treatment. Cells were seeded in 6-well culture dishes and allowed to adhere overnight before treatment with Compound A, FUdR, or Compound A in combination with FUdR. Additional wells were treated with doxorubicin to act as a positive inducer of HMGB1. Twenty-four hours after treatment, the media was aspirated and centrifuged (2500 rpm, 5 min, at 4°C) to remove any cellular debris. The supernatant was collected for immediate processing or stored at -80°C for later assay.

**Detection of HMGB1 Release.** HMGB1 was quantified using the HMGB1 ELISA kit (ST51011, IBL International). Enzyme conjugate (HMGB1 conjugated to peroxidase) and standard were reconstituted in 12 ml enzyme conjugate diluent, and 1 ml diluent buffer respectively and aliquots frozen at -80°C until required. All reagents and samples were brought to room temperature immediately prior to use. A standard curve was prepared for the high sensitive detection range (0.313 - 10 ng/ml). 50 µl diluent buffer was pipetted into each well of the microtiter plate. 50 µl of standard, sample, positive control or negative control (diluent buffer) was pipetted, in duplicate, into respective wells as determined by the plate set up. The plate was covered with adhesive foil and incubated at 37°C for 20 to 24 hours on an orbital shaker (450 rpm). Following the incubation period the incubation solution was discarded and the plate washed (5 x 300 µl per well). Excess wash buffer was removed by inversion on to a paper towel. 100 µl enzyme conjugate was added to each well, the plate covered with adhesive foil and incubated at 25°C for 2 hours on an orbital shaker (450 rpm). Following the incubation the solution was discarded and the plate washed as before. Fresh color solution was prepared immediately prior to use and 100 µl added to each well using a multichannel pipette. The plate was then incubated for 30 minutes at room temperature. The color reaction was stopped by pipetting 100 µl of stop reagent to each well using a multichannel pipette. The plate was shaken gently to mix and the backs of the wells were cleaned using a lint-free tissue. The concentration of HMGB1 was determined on a CLARIOstar microplate reader by measuring the absorbance at 450 nm using a reference reading at 640 nm. Data was exported to Microsoft Excel for processing and statistical analysis consisting of one-way ANOVA with Tukey's Multiple Comparisons Test was performed in GraphPad Prism 6.

### Results

When HCT116 cells were treated with either Compound A or FUdR single agents, there was no significant difference in the amount of extracellular HMGB1 detected in the cell culture media. When cells were treated with the combination of 12.5 µM Compound A and 1 µM FUdR, the concentration of HMGB1 in the cell culture media increased by 245% when compared to control (p<0.01) (Figure 13). The increase in HMGB1 observed over control with the combination of Compound A and FUdR was similar to that of doxorubicin which increased extracellular HMGB1 over control by 213% (p<0.01). Compound A unexpectedly stimulated the extracellular release of HMGB 1 in combination with FUdR in the HCT116 colon cancer cell line. When JU77 cells were treated with either Compound A or FUdR single agents, there was no significant difference in the amount of extracellular HMGB1 detected in the cell culture media. When cells were treated with the combination of 12.5 µM Compound A and 1 µM FUdR, the concentration of HMGB1 in the cell culture media increased by 493% when compared to control (p<0.01) (Figure 14). In the JU77 cells, doxorubicin failed to elicit any significant increase in HMGB1. Compound A unexpectedly stimulated the extracellular release of HMGB1 in combination with FUdR in the JU77 mesothelioma cancer cell line. Accordingly, it was demonstrated that a combination of a dUTPase inhibitor and an inhibitor of thymidylate biosynthesis surprisingly induces the release of DAMP from a cancer cell.

### Example 5: Induction of cell surface expression of calreticulin by combination of Compound A and FUdR (described for context only)

**Experimental Overview.** Tumor cells undergoing immunogenic cell death (ICD) emit damage associated molecular patterns (DAMPs). ICD is a sub-class of cell death that results in the potent stimulation of the innate immune system and subsequently the stimulation of adaptive immune responses. The cell surface expression of calreticulin during cell death is one of several established DAMPs characteristic of immunogenic cell death. Calreticulin translocates from the endoplasmic reticulin to the cell membrane where it functions as a potent "eat me" signal for macrophages and dictates tumor antigen transfer and therefore the immunogenicity of cancer cell death. PANC1 pancreatic carcinoma cells were treated with vehicle control (DMSO), 6.25 µM Compound A, 1 µM FUdR, or the combination of 6.25 µM Compound A and 1 µM FUdR for 4 hours and the concentration of cell surface calreticulin was deteremined by flow cytometry.

**Cell Line Model Selection and Preparation.** The PANC1 pancreatic cancer cell line was purchased from ATCC. Cells were cultured and expanded in humidified incubators at 5% CO₂ until sufficient cells were available.

**Drug Preparation and Treatments.** All drugs/compounds were prepared freshly in cell culture media from 50 mM frozen stocks prior to treatment. Cells were seeded in 6-well plates and permitted to adhere overnight. Media was removed and replaced with media containing 6.25 µM Compound A, 1 µM FUdR or a combination of 6.25 µM Compound A and 1 µM FUdR. Positive control cells were treated with 500 nmol/L doxorubicin. Cells were then incubated in drug-containing media for 4 hours. At the conclusion of specified drug incubation timepoints, cells were processed immediately for flow cytometry detection of cell surface calreticulin.

**Detection of Cell Surface Calreticulin.** Cell surface calreticulin was quantified by flow cytometry. At the conclusion of specified drug incubation timepoints, cells were washed with PBS and removed from the plates using trypsin-EDTA. Cells were counted and 300,000 cells were washed in staining buffer and incubated in Calreticulin (D3E6) XP Alexa Fluor 488 Conjugate antibody (Cell Signaling) or IgG antibody (negative control antibody) on ice for 40 minutes. Cells were then washed in staining buffer twice and finally resuspended in 300 µL of staining buffer and transferred to BD Falcon^{™} round-bottom tube. Dot plot for FSC v SSC were used to gate cell population and doublets were excluded on FL-4 histogram height versus area/width. The IgG isotype control stained sample was used to correctly identify the positively stained population in which median fluorescence intensity was determined. Median fluorescence intensity values were exported and analyzed in Microsoft Excel and GraphPad Prism 6. Statistical analysis consisted of one-way ANOVA with Tukey's multiple comparisons testing.

### Results

When PANC1 cells were treated with either Compound A or FUdR single agents, there was no significant difference in the amount of calreticulin detected on the cell surface. When cells were treated with the combination of 6.25 µM Compound A and 1 µM FUdR, the cell surface expression of calreticulin increased by 700% when compared to control (p<0.01) (Figure 15). The increase in cell surface calreticulin observed over control with the combination of Compound A and FUdR was greater than that of doxorubicin which increased cell surface calreticulin over control by 450% (p<0.05, Figure 15). Compound A unexpectedly stimulated the cell surface expression of calreticulin in combination with FUdR in the PANC1 pancreatic cancer cell line. Accordingly, it was demonstrated that a combination of a dUTPase inhibitor and an inhibitor of thymidylate biosynthesis surprisingly induces the release of DAMP from a cancer cell.

### Example 6: Induction of the release of nuclear self-DNA into the cytoplasm by the combination of Compound A and FUdR. (described for context only)

**Experimental Overview.** Aberrant host DNA metabolism as a result of failed cell division or genotoxic stress leads to the release of nuclear DNA into the cytoplasm and is a well-known damage-associated molecular pattern. The subsequent detection of cytoplasmic double-stranded DNA (dsDNA) by pattern recognition receptors triggers a variety of innate immune signaling and biological responses. HCT116 colon and PANC-1 pancreatic carcinoma cells were seeded on coverslips and treated with vehicle control (DMSO), 12.5 µM Compound A, 1 µM FUdR, or the combination of 12.5 µM Compound A and 1 µM FUdR for 24 hours. At 24 hours post-treatment cells were fixed and stained with an anti-dsDNA (double-stranded DNA) antibody and the nuclear stain DAPI. Immunofluorescence was detected and images acquired by fluorescence microscopy to quantify levels of dsDNA in the cytoplasm.

**Cell lines, Drug Treatments and Fluorescence Microscopy.** HCT116 colon and PANC-1 pancreatic carcinoma cells (5x10⁴) were seeded onto glass coverslips within 24-well plates in relevant media. The following day, seeding media was removed and replaced with drug-containing media and incubated for 24 hours. At 24 and 48 hours post-treatment cells were fixed and processed. Briefly, cell membranes were permeabilised, incubated with FITC-conjugated dsDNA marker antibody (HYB331-01) from Santa Cruz Biotechnology, sc-58749 (1:500 dilution) and mounted onto slides with ProLong Gold antifade with DAPI (Invitrogen P36931). Images were acquired on a Nikon Fluorescent Microscope. Exposure time was selected for FITC using negative and positive controls and kept constant throughout image acquisition. Images were analyzed using ImageJ software. Cell nuclei were identified using DAPI staining. Intensity of cytoplasmic dsDNA staining was established by quantifying the intensity of the staining in the immediate area surrounding the nucleus. Software settings: Gaps to ring = 3 and Ring size = 25 (Figure 16).

### Results

The HCT116 colon cancer cell line and the PANC-1 pancreatic cancer cell line were treated with vehicle control (DMSO), 12.5 µM Compound A, 1 µM FUdR and the combination of 12.5 µM Compound A and 1 µM FUdR for 24 hours and the presence of double-stranded nuclear self-DNA in the cytoplasm was measured by immunofluorescent microscopy. Following treatment with Compound A, there was no statistically significant difference in the mean relative cytoplasmic DNA in HCT116 colon cancer cells treated with either 12.5 µM Compound A or 1 µM FUdR when compared to vehicle control-treated cells. In contrast, the combination of 12.5 µM Compound A and 1 µM FUdR led to a statistically significant 67% increase in mean cytoplasmic DNA when compared to vehicle-treated control (p<0.001, one-way ANOVA with Tukey's multiple comparisons test; Figure 17). The combination of 12.5 µM Compound A and 1 µM FUdR was also highly statistically significant compared to either 12.5 µM single agent Compound A or single agent 1 µM FUdR, (p<0.001, one-way ANOVA with Tukey's multiple comparisons test for both treatments; Figure 17). Representative images depicting 3 individual cells for each treatment group (vehicle control, 12.5 µM Compound A, 1 µM FUdR and the combination of 12.5 µM Compound A and 1 µM FUdR) is provided in Figure 18 and illustrates the increase in cytoplasmic fluorescence intensity depicted by bright haze and white specks in the cytoplasm outside the central nucleus. The white specks in particular represent DNA micronuclei which act as potent sources of immunostimulatory extra-nuclear DNA (Figure 18).

In PANC-1 pancreatic cancer cells, treatment with Compound A, led to a statistically significant increase in the mean relative cytoplasmic DNA of 74% when compared to vehicle-treated control (p<0.001, one-way ANOVA with Tukey's multiple comparisons test). Similarly, 1 µM FUdR led to a 56% increase in mean relative cytoplasmic DNA when compared to vehicle control-treated cells (p<0.001, one-way ANOVA with Tukey's multiple comparisons test). However, the combination of 12.5 µM Compound A and 1 µM FUdR led to a highly statistically significant increase of 266% in mean cytoplasmic DNA when compared to vehicle-treated control (p<0.001, one-way ANOVA with Tukey's multiple comparisons test; Figure 19). The combination of 12.5 µM Compound A and 1 µM FUdR was also highly statistically significant compared to either 12.5 µM single agent Compound A or 1 µM FUdR, p<0.001, one-way ANOVA with Tukey's multiple comparisons (p<0.001, one-way ANOVA with Tukey's multiple comparisons test for both treatments; Figure 19). Representative images depicting 3 individual cells for each treatment group (vehicle control (DMSO), 12.5 µM Compound A, 1 µM FUdR and the combination of 12.5 µM Compound A and 1 µM FUdR) is provided in Figure 20 and illustrates the increase in cytoplasmic fluorescence intensity depicted by bright haze and white specks in the cytoplasm outside the central nucleus. The white specks in particular represent DNA micronuclei which act as particularly strong sources of immunostimulatory extra-nuclear DNA (Figure 20). Accordingly, a dUTPase inhibitor (e.g., Compound A) unexpectedly and significantly increased the release of immunostimulatory nuclear dsDNA into the cytoplasm when used in combination with an inhibitor of thymidylate biosynthesis (e.g., FUdR) in both cell lines and as a single agent in the PANC-1 cell line.

It should be understood that although the present invention has been specifically disclosed by certain aspects, embodiments, and optional features, modification, improvement and variation of such aspects, embodiments, and optional features can be resorted to by those skilled in the art. The invention is as defined in the claims.

Where features or aspects of the disclosure are described in terms of Markush groups, those skilled in the art will recognize that the disclosure is also thereby described in terms of any individual member or subgroup of members of the Markush group.

## Claims

1. A deoxyuridine triphosphatase (dUTPase) inhibitor for use in a method of:
(i) enhancing a therapeutic efficacy of an immunotherapy agent in a subject in need thereof; or
(ii) treating cancer in a subject in need thereof;
wherein the method comprises administering to the subject an effective amount of the deoxyuridine triphosphatase (dUTPase) inhibitor, an effective amount of an inhibitor of thymidylate biosynthesis, and an immunotherapy agent, wherein the dUTPase inhibitor is 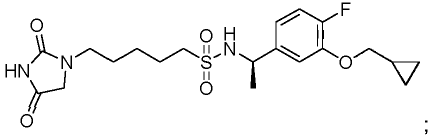 the inhibitor of thymidylate biosynthesis is 5-fluorouracil (5-FU); and the immunotherapy agent is an anti-PD-1 (CD279) antibody.

2. An inhibitor of thymidylate biosynthesis for use in a method of:
(i) enhancing a therapeutic efficacy of an immunotherapy agent in a subject in need thereof; or
(ii) treating cancer in a subject in need thereof;
wherein the method comprises administering to the subject an effective amount of a deoxyuridine triphosphatase (dUTPase) inhibitor, an effective amount of the inhibitor of thymidylate biosynthesis, and an immunotherapy agent, wherein the dUTPase inhibitor is 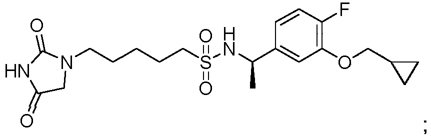 the inhibitor of thymidylate biosynthesis is 5-fluorouracil (5-FU); and the immunotherapy agent is an anti-PD-1 (CD279) antibody.

3. An immunotherapy agent for use in:
(i) a method of enhancing a therapeutic efficacy of the immunotherapy agent in a subject in need thereof; or
(ii) a method of treating cancer in a subject in need thereof;
wherein the method comprises administering to the subject an effective amount of a deoxyuridine triphosphatase (dUTPase) inhibitor, an effective amount of an inhibitor of thymidylate biosynthesis, and the immunotherapy agent, wherein the dUTPase inhibitor is 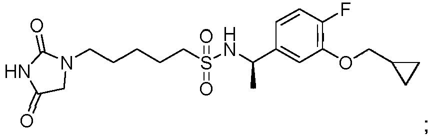 the inhibitor of thymidylate biosynthesis is 5-fluorouracil (5-FU); and the immunotherapy agent is an anti-PD-1 (CD279) antibody.

4. A deoxyuridine triphosphatase (dUTPase) inhibitor, an inhibitor of thymidylate biosynthesis and an immunotherapy agent, for use in a method of:
(i) enhancing a therapeutic efficacy of the immunotherapy agent in a subject in need thereof; or
(ii) treating cancer in a subject in need thereof;
wherein the method comprises administering to the subject an effective amount of the deoxyuridine triphosphatase (dUTPase) inhibitor, an effective amount of the inhibitor of thymidylate biosynthesis, and the immunotherapy agent, wherein the dUTPase inhibitor is 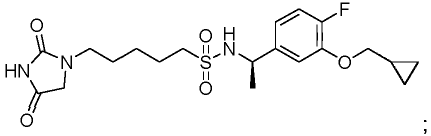 the inhibitor of thymidylate biosynthesis is 5-fluorouracil (5-FU); and the immunotherapy agent is an anti-PD-1 (CD279) antibody.

5. The dUTPase inhibitor for use according to claim 1, the inhibitor of thymidylate biosynthesis for use according to claim 2, the immunotherapy agent for use according to claim 3, or the dUTPase inhibitor, the inhibitor of thymidylate biosynthesis, and the immunotherapy agent for use according to claim 4, wherein the use is for treating cancer, and wherein the subject after treatment experiences one or more endpoints selected from tumor response, reduction in tumor size, reduction in tumor burden, increase in overall survival, increase in progression free survival, inhibiting metastasis, improvement of quality of life, minimization of toxicity, and avoidance of side-effects.

6. The dUTPase inhibitor for use according to claim 1 or 5, the inhibitor of thymidylate biosynthesis for use according to claim 2 or 5, the immunotherapy agent for use according to claim 3 or 5, or the dUTPase inhibitor, the inhibitor of thymidylate biosynthesis, and the immunotherapy agent for use according to claim 4 and 5, wherein the use is for treating cancer, and wherein
(i) the cancer is selected from cancers of the: circulatory system; respiratory tract; gastrointestinal system; genitourinary tract; liver; bone; nervous system; reproductive system; hematologic system; oral cavity; skin; and other tissues comprising connective and soft tissue, retroperitoneum and peritoneum, eye, intraocular melanoma, and adnexa, breast, head or/and neck, anal region, thyroid, parathyroid, adrenal gland and other endocrine glands, and lymph nodes, optionally wherein the cancer is a solid tumor or alternatively wherein the cancer is a liquid cancer, and further optionally wherein the cancer is a primary cancer or a metastasis; or
(ii) the cancer comprises a carcinoma, a sarcoma, a myeloma, a leukemia, or a lymphoma.

## Patentansprüche

1. Desoxyuridin-Triphosphatase-(dUTPase)Hemmer zur Verwendung bei einem Verfahren zum:
(i) Verstärken einer therapeutischen Wirksamkeit eines Immuntherapeutikums bei einem Individuum, bei dem diesbezüglich Bedarf besteht, oder
(ii) Behandeln von Krebs bei einem Individuum, bei dem diesbezüglich Bedarf besteht;
wobei das Verfahren Verabreichen einer wirksamen Menge des Desoxyuridin-Triphosphatase(dUTPase)-Hemmers, einer wirksamen Menge eines Hemmers der Thymidylat-Biosynthese und eines Immuntherapeutikums an das Individuum umfasst, wobei es sich bei dem dUTPase-Hemmer um 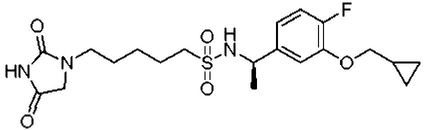 handelt; es sich bei dem Hemmer der Thymidylat-Biosynthese um 5- Fluoruracil (5-FU) handelt und es sich bei dem Immuntherapeutikum um einen Anti-PD-1 (CD279)-Antikörper handelt.

2. Hemmer der Thymidylat-Biosynthese zur Verwendung bei einem Verfahren zum:
(i) Verstärken einer therapeutischen Wirksamkeit eines Immuntherapeutikums bei einem Individuum, bei dem diesbezüglich Bedarf besteht, oder
(ii) Behandeln von Krebs bei einem Individuum, bei dem diesbezüglich Bedarf besteht;
wobei das Verfahren Verabreichen einer wirksamen Menge eines Desoxyuridin-Triphosphatase(dUTPase)-Hemmers, einer wirksamen Menge des Hemmers der Thymidylat-Biosynthese und eines Immuntherapeutikums an das Individuum umfasst, wobei es sich bei dem dUTPase-Hemmer um 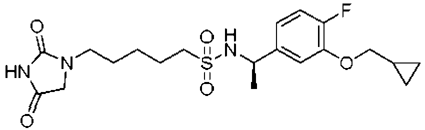 handelt; es sich bei dem Hemmer der Thymidylat-Biosynthese um 5-Fluoruracil (5-FU) handelt und es sich bei dem Immuntherapeutikum um einen Anti-PD-1 (CD279)-Antikörper handelt.

3. Immuntherapeutikum zur Verwendung bei:
(i) einem Verfahren zum Verstärken einer therapeutischen Wirksamkeit des Immuntherapeutikums bei einem Individuum, bei dem diesbezüglich Bedarf besteht, oder
(ii) einem Verfahren zum Behandeln von Krebs bei einem Individuum, bei dem diesbezüglich Bedarf besteht;
wobei das Verfahren Verabreichen einer wirksamen Menge eines Desoxyuridin-Triphosphatase(dUTPase)-Hemmers, einer wirksamen Menge eines Hemmers der Thymidylat-Biosynthese und des Immuntherapeutikums an das Individuum umfasst, wobei es sich bei dem dUTPase-Hemmer um 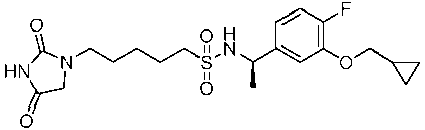 handelt; es sich bei dem Hemmer der Thymidylat-Biosynthese um 5-Fluoruracil (5-FU) handelt und es sich bei dem Immuntherapeutikum um einen Anti-PD-1 (CD279)-Antikörper handelt.

4. Desoxyuridin-Triphosphatase(dUTPase)-Hemmer, Hemmer der Thymidylat-Biosynthese und Immuntherapeutikum zur Verwendung bei einem Verfahren zum:
(i) Verstärken einer therapeutischen Wirksamkeit des Immuntherapeutikums bei einem Individuum, bei dem diesbezüglich Bedarf besteht; oder
(ii) Behandeln von Krebs bei einem Individuum, bei dem diesbezüglich Bedarf besteht;
wobei das Verfahren Verabreichen einer wirksamen Menge des Desoxyuridin-Triphosphatase(dUTPase)-Hemmers, einer wirksamen Menge des Hemmers der Thymidylat-Biosynthese und des Immuntherapeutikums an das Individuum umfasst, wobei es sich bei dem dUTPase-Hemmer um 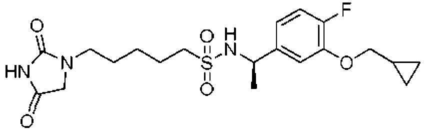 handelt; es sich bei dem Hemmer der Thymidylat-Biosynthese um 5-Fluoruracil (5-FU) handelt und es sich bei dem Immuntherapeutikum um einen Anti-PD-1 (CD279)-Antikörper handelt.

5. dUTPase-Hemmer zur Verwendung nach Anspruch 1, Hemmer der Thymidylat-Biosynthese zur Verwendung nach Anspruch 2, Immuntherapeutikum zur Verwendung nach Anspruch 3 oder dUTPase-Hemmer, Hemmer der Thymidylat-Biosynthese und Immuntherapeutikum zur Verwendung nach Anspruch 4, wobei die Verwendung der Behandlung von Krebs dient, und wobei das Individuum nach der Behandlung einen oder mehrere Endpunkte erreicht, die aus Tumorreaktion, Verringerung der Tumorgröße, Verringerung der Tumorlast, Erhöhung des Gesamtüberlebens, Erhöhung des progressionsfreien Überlebens, Hemmen von Metastase, Verbesserung der Lebensqualität, Minimierung der Toxizität und Vermeidung von Nebenwirkungen ausgewählt sind.

6. dUTPase-Hemmer zur Verwendung nach Anspruch 1 oder 5, Hemmer der Thymidylat-Biosynthese zur Verwendung nach Anspruch 2 oder 5, Immuntherapeutikum zur Verwendung nach Anspruch 3 oder 5 oder dUTPase-Hemmer, Hemmer der Thymidylat-Biosynthese und Immuntherapeutikum zur Verwendung nach Anspruch 4 und 5, wobei die Verwendung der Behandlung von Krebs dient und wobei
(i) der Krebs aus Krebserkrankungen des Kreislaufsystems, der Atemwege, des Magen-Darmtrakts, der Urogenitaltrakte, der Leber, Knochen, des Nervensystems, Fortpflanzungssystems, hämatologischen Systems, der Mundhöhle, Haut und anderer Gewebe, die Binde- und Weichgewebe, Retroperitoneum und Peritoneum, Auge, intraokulares Melanom und Adnexe, Brust, Kopf oder/und Hals, Analregion, Schilddrüse, Nebenschilddrüse, Nebenniere und andere endokrine Drüsen und Lymphknoten umfassen, ausgewählt ist,
wobei es sich gegebenenfalls bei dem Krebs um einen soliden Tumor handelt oder wobei es sich alternativ bei dem Krebs um einen flüssigen Krebs handelt und wobei es sich ferner gegebenenfalls bei dem Krebs um einen primären Krebs oder eine Metastase handelt; oder
(ii) der Krebs ein Karzinom, ein Sarkom, ein Myelom, eine Leukämie oder ein Lymphom umfasst.

## Revendications

1. Inhibiteur de désoxyuridine triphosphatase (dUTPase) destiné à être utilisé dans un procédé de :
(i) amélioration de l'efficacité thérapeutique d'un agent immunothérapeutique chez un sujet qui en a besoin ; ou
(ii) traitement d'un cancer chez un sujet qui en a besoin ;
dans lequel le procédé comprend l'administration au sujet d'une quantité efficace de l'inhibiteur de désoxyuridine triphosphatase (dUTPase), d'une quantité efficace d'un inhibiteur de la biosynthèse de thymidylate, et d'un agent immunothérapeutique, l'inhibiteur de la dUTPase étant 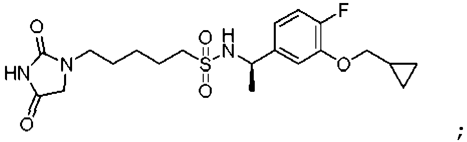 l'inhibiteur de la biosynthèse de thymidylate étant le 5-fluorouracile (5-FU) ; et l'agent immunothérapeutique étant un anticorps anti-PD-1 (CD279).

2. Inhibiteur de biosynthèse de thymidylate destiné à être utilisé dans un procédé de :
(i) amélioration de l'efficacité thérapeutique d'un agent immunothérapeutique chez un sujet qui en a besoin ; ou
(ii) traitement d'un cancer chez un sujet qui en a besoin ;
le procédé comprend l'administration au sujet d'une quantité efficace d'un inhibiteur de désoxyuridine triphosphatase (dUTPase), d'une quantité efficace de l'inhibiteur de la biosynthèse de thymidylate, et d'un agent immunothérapeutique, l'inhibiteur de la dUTPase étant 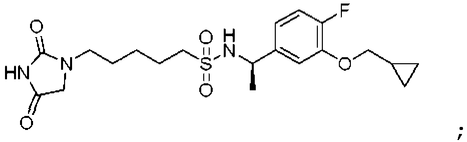 l'inhibiteur de la biosynthèse de thymidylate étant le 5-fluorouracile (5-FU) ; et l'agent immunothérapeutique étant un anticorps anti-PD-1 (CD279).

3. Agent immunothérapeutique destiné à être utilisé dans :
(i) un procédé d'amélioration de l'efficacité thérapeutique de l'agent immunothérapeutique chez un sujet qui en a besoin ; ou
(ii) un procédé de traitement d'un cancer chez un sujet qui en a besoin ;
dans lequel le procédé comprend l'administration au sujet d'une quantité efficace d'un inhibiteur de désoxyuridine triphosphatase (dUTPase), d'une quantité efficace d'un inhibiteur de la biosynthèse de thymidylate, et de l'agent immunothérapeutique, l'inhibiteur de la dUTPase étant 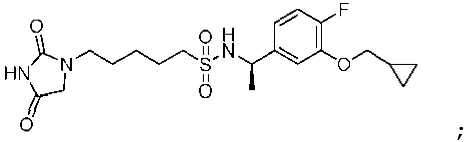 l'inhibiteur de la biosynthèse de thymidylate étant le 5-fluorouracile (5-FU) ; et l'agent immunothérapeutique étant un anticorps anti-PD-1 (CD279).

4. Inhibiteur de désoxyuridine triphosphatase (dUTPase), inhibiteur de la biosynthèse de thymidylate et agent immunothérapeutique, pour une utilisation dans un procédé de :
(i) amélioration de l'efficacité thérapeutique de l'agent immunothérapeutique chez un sujet qui en a besoin ; ou
(ii) traitement d'un cancer chez un sujet qui en a besoin ;
dans lequel le procédé comprend l'administration au sujet d'une quantité efficace de l'inhibiteur de désoxyuridine triphosphatase (dUTPase), d'une quantité efficace de l'inhibiteur de la biosynthèse de thymidylate, et de l'agent immunothérapeutique, l'inhibiteur de la dUTPase étant 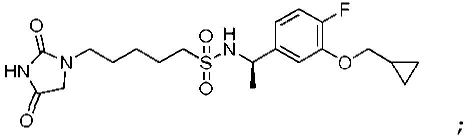 l'inhibiteur de la biosynthèse de thymidylate étant le 5-fluorouracile (5-FU) ; et l'agent immunothérapeutique étant un anticorps anti-PD-1 (CD279).

5. Inhibiteur de dUTPase pour utilisation selon la revendication 1, inhibiteur de biosynthèse de thymidylate pour utilisation selon la revendication 2, agent immunothérapeutique pour utilisation selon la revendication 3, ou inhibiteur de la dUTPase, inhibiteur de la biosynthèse de thymidylate, et agent immunothérapeutique pour utilisation selon la revendication 4, dans lequel l'utilisation est pour le traitement d'un cancer, et dans lequel le sujet, après le traitement, présente un ou plusieurs points finaux sélectionnés parmi la réponse tumorale, la réduction de la taille de la tumeur, la réduction de la charge tumorale, l'augmentation de la survie globale, l'augmentation de la survie sans progression, l'inhibition des métastases, l'amélioration de la qualité de vie, la minimisation de la toxicité et l'évitement d'effets secondaires.

6. Inhibiteur de dUTPase pour utilisation selon la revendication 1 ou 5, inhibiteur de biosynthèse de thymidylate pour utilisation selon la revendication 2 ou 5, agent immunothérapeutique pour utilisation selon la revendication 3 ou 5, ou inhibiteur de dUTPase, inhibiteur de biosynthèse de thymidylate, et agent immunothérapeutique pour utilisation selon les revendications 4 et 5, dans lesquels l'utilisation est pour le traitement d'un cancer, et dans lequel
(i) le cancer est choisi parmi les cancers du : système circulatoire ; voies respiratoires ; système gastrointestinal ; appareil génito-urinaire ; foie ; os ; système nerveux ; système reproducteur ; système hématologique ; cavité buccale ; peau ; et autres tissus comprenant les tissus conjonctifs et mous, rétropéritoine et péritoine, œil, mélanome intraoculaire, et annexes, sein, tête et/ou cou, région anale, thyroïde, péritoine, glandes surrénales et autres glandes endocrines, et ganglions lymphatiques,
éventuellement, dans lequel le cancer est une tumeur solide ou, éventuellement, dans lequel le cancer est un cancer liquide, et éventuellement, dans lequel le cancer est un cancer primaire ou une métastase ; ou
(ii) le cancer comprend un carcinome, un sarcome, un myélome, une leucémie ou un lymphome.
